(19) Europäisches Patentamt
European Patent Office
Office européen des brevets

(11) Publication number: **0 002 590 B1**

# (12) EUROPEAN PATENT SPECIFICATION

(45) Date of publication of patent specification: 30.09.81

(51) Int. Cl.$^3$: C 07 C 177/00, A 61 K 31/557

(21) Application number: 78300772.7

(22) Date of filing: 08.12.78

(54) **Novel prostaglandin compounds, processes for the preparation thereof and pharmaceutical compositions containing them.**

(30) Priority: 08.12.77 US 858487
08.12.77 US 858588
08.12.77 US 858589
08.12.77 US 858504
08.12.77 US 858580
08.12.77 US 858579

(43) Date of publication of application:
27.06.79 Bulletin 79/13

(45) Publication of the grant of the European patent:
30.09.81 Bulletin 81/39

(84) Designated Contracting States:
BE CH DE FR GB IT NL SE

(56) References cited:
RECUEIL DES TRAVAUX CHIMIQUES DES PAYS BAS, vol. 85 (1966) Pages 1251—1253

(73) Proprietor: AMERICAN CYANAMID COMPANY
Berdan Avenue
Wayne, New Jersey 06904 (US)

(72) Inventor: Wissner, Allan
31 Wood Avenue
Ardsley, New York (US)

(74) Representative: Allam, Peter Clerk, et al,
LLOYD WISE, TREGEAR & CO.
Norman House, 105—109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 590 B1

0 002 590

# Novel prostaglandin compounds, processes for the preparation thereof and pharmaceutical compositions containing them

The prostaglandins are currently of great interest because of the broad physiological responses which they elicit in animals, including man. The present invention provides novel prostaglandin compounds exhibiting potentially useful therapeutic activity, and also processes for the preparation thereof.

This invention relates to compounds of the formula:

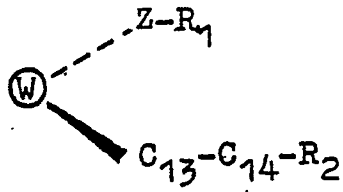

wherein Z is $-(CH_2)_f-$ or $-CH_2-CH=CH-(CH_2)-_g$, wherein f is an integer from 5 to 7 inclusive, and g is an integer from 2 to 4, inclusive: $C_{13}-C_{14}$ is ethylene of *trans*-vinylene; W is selected from the group consisting of:

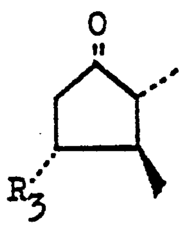
,
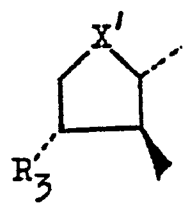
or
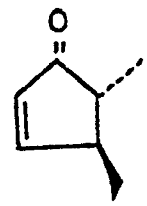

wherein X' is:

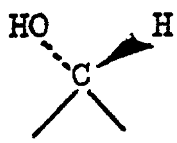
or
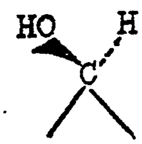

and $R_3$ is hydrogen or hydroxyl; $R_1$ is selected from the group consisting of:

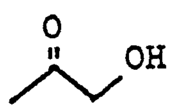

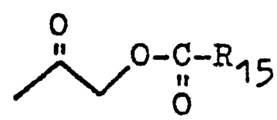

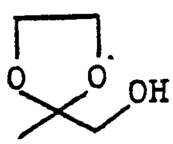
or
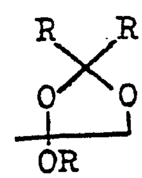

wherein R is $C_1-C_4$ alkyl, and $R_{15}$ is selected from the group consisting of $C_1-C_4$ alkyl, di-($C_1-C_4$)-alkylamino, and phenyl or phenyl substituted with one or more substituents selected from the group consisting of $C_1-C_4$ alkyl, —OR, —SR, F or Cl wherein R is as previously defined, with the proviso that when W is

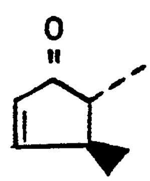
then $R_1$ cannot be
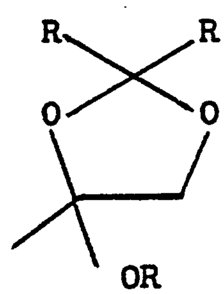

and $R_2$ is selected from the group consisting of:

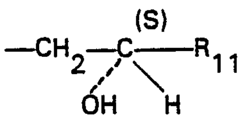

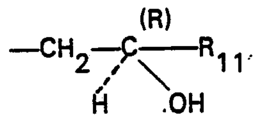

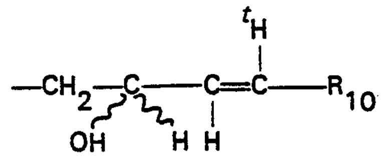

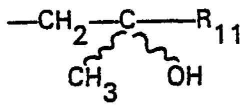

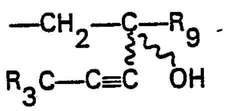

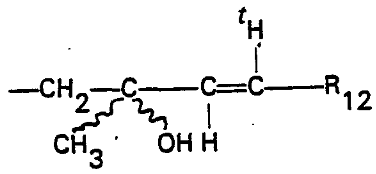

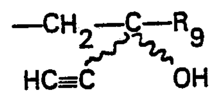

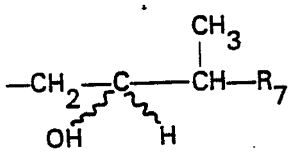

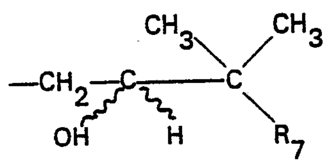

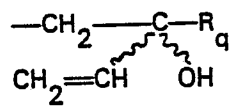

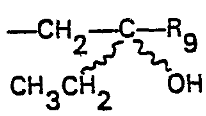

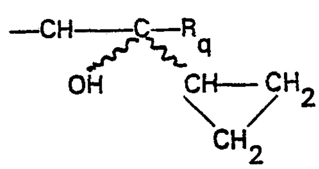

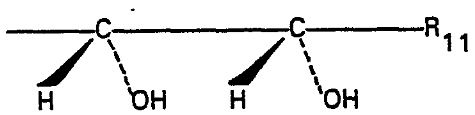

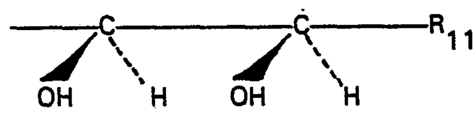

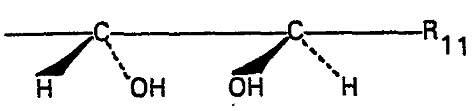

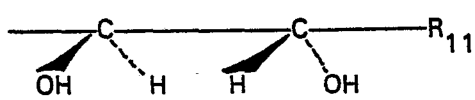

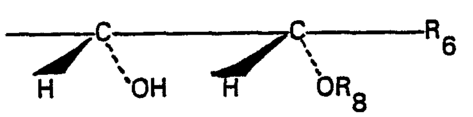

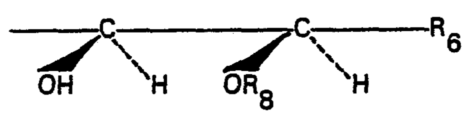

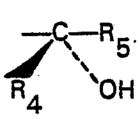

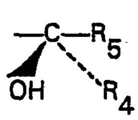

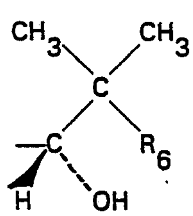

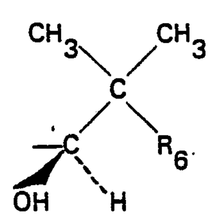

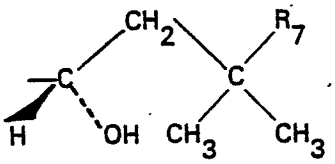
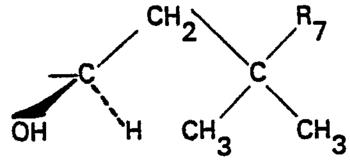
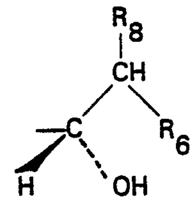
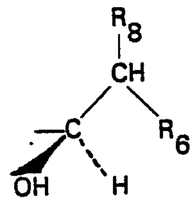
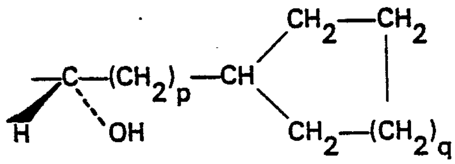
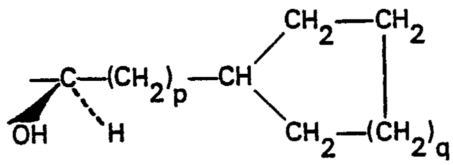
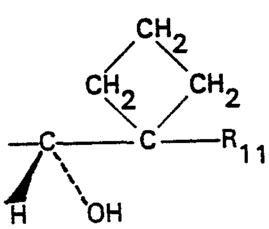
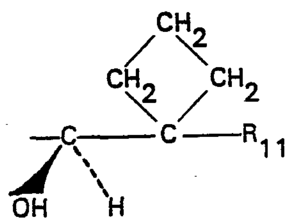
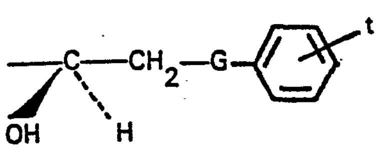
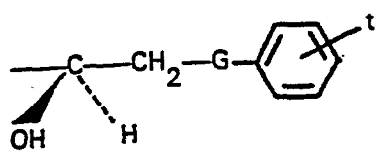

and 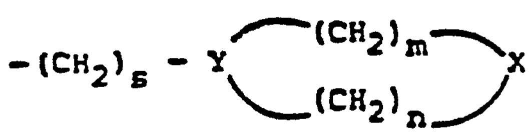

wherein $R_4$ is hydrogen or $C_1$—$C_3$ alkyl; $R_5$ is selected from the group consisting of straight chain $C_4$—$C_7$ alkyl; $R_6$ is selected from the group consisting of straight chain $C_3$—$C_6$ alkyl; $R_7$ is selected from the group consisting of straight chain $C_2$—$C_4$ alkyl; $R_8$ is selected from the group consisting of $C_1$—$C_2$ alkyl; $R_9$ is selected from the group consisting of straight chain $C_3$—$C_8$ alkyl; $R_{10}$ is selected from the group consisting of straight chain $C_1$—$C_4$ alkyl; $R_{11}$ is selected from the group consisting of straight chain $C_3$—$C_7$ alkyl; $R_{12}$ is selected from the group consisting of straight chain $C_1$—$C_4$ alkyl; p is an integer from 0 to 3; q is 1 or 2; X is a divalent radical selected from the group consisting of:

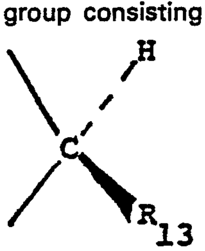

and

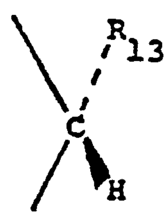

wherein $R_{13}$ is selected from the group consisting of $C_1$—$C_7$ alkyl, hydrogen, and a phenoxy group optionally substituted with a substituent selected from the group consisting of halogen, trifluoromethyl and $C_1$—$C_4$ alkyloxy; Y is a divalent radical selected from the group consisting of:

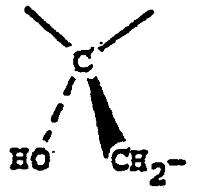

and

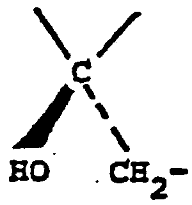

G is a divalent radical selected from the group consisting of —O— and —$CH_2$—; m is zero or an integer from 1 to 4 inclusive; n is zero or an integer from 1 to 4, inclusive; with the proviso that the sum of m and n has the value of 1 to 4; s is zero or the integer 1; and t is selected from the group consisting of hydrogen, chloro, fluoro, dichloro, trifluoromethyl and methoxy.

The compounds of the present invention include the optically active isomers of Formula (I) above as well as racemic mixtures thereof; and mirror images thereof.

This invention also relates to the method of preparing the above-described compounds.

DETAILED DESCRIPTION OF THE INVENTION

The compounds of this invention may, be administered in various ways for various purposes, e.g., intravenously, intramuscularly, subcutaneously, orally, intravaginally, rectally, bucally, sublingually, topically and in the form of sterile implants for prolonged action.

For intravenous injection or infusion, sterile aqueous isotonic suspensions are preferred. For subcutaneous or intramuscular injection, sterile suspensions of the compounds in aqueous or nonaqueous media are used. Tablets, capsules, and liquid preparations such as syrups, elixirs, and simple solutions, with the usual pharmaceutical carriers are used for oral or sublingual administration. For rectal or vaginal administration, suppositories prepared as known in the art are used. For tissue implants, a sterile tablet or silicone rubber capsule or other object containing or impregnated with the substance is used. On certain occasions it may be advantageous to administer the compounds of this invention as clathrate compounds with substances such as $\alpha$-cyclodextrin.

The prostaglandins are a family of closely related compounds which have been obtained from various animal tissues and which stimulate smooth muscle, lower arterial blood pressure, antagonize epinephrine-induced mobilization of free fatty acids, and have other pharmacological and autopharmacological effects in mammals. See Bergstom, *et al.*, J. Biol. Chem., *238*,, 3555 (1963) and Horton, Experientia, *21*, 113 (1965) and references cited therein. All of the so called natural prostaglandins are derivatives of prostanic acid:

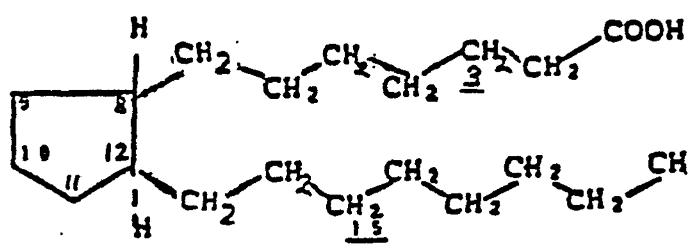

The hydrogen atoms attached to C—8 and C—12 are in *trans*-configuration. The natural prostaglandins represent only one of the possible optical isomers. The compounds of this invention include all possible optical isomers and mixtures (including racemates) thereof.

The configuration of substituents on the prostaglandin molecule are designed to be in the $\alpha$-configuration if they lie beneath the plane of the molecule as drawn above and are designated with a ---- bond. Those substituents which lie above the plane of the molecule as drawn above are designated $\beta$ and are represented by a ▶bond.

The compounds of this invention which have the structure are shown in formula (A) wherein $R_1$, Z, $R_3$, Y, m, n, s and X are as herein above defined are said to be in the same configuration as the natural prostaglandins with respect to the configurations at $C_8$, $C_{11}$ and $C_{12}$ and are designated by the prefix *nat*. The enantiomer, represented by formula (B) is said to be in the mirror image or *ent* configuration. A substituent at $C_{11}$ drawn with a dotted line ($C_{11}$—$R_3$) is said to have an $\alpha$ configuration; a solid line $C_{11}$—$R_3$) indicates a $\beta$ configuration. The configuration at Y and X will be expressed in terms of R and S as is understood in the art. For example, the compound represented by formula (C) is named *nat*-15S,16S—11$\alpha$, 15 - dihydroxy - 1 - (hydroxymethyl) - 1,9 - dioxo - 15,16 - trimethylene - 13 - *trans*-prostene; its enantiomer (formula D) is named *ent* - 15R,16R - 11$\alpha$,15 - dihydroxy - 1 - (hydroxymethyl) - 1,9 - dioxo - 15,16 - trimethylene - 13 - *trans* - prostene. The racemate (1:1 mixture of (C) and (D)) is named *nat* - 15S,16S - (and *ent* - 15R,16R) 11$\alpha$, 15 - dihydroxy - 1 -

(hydroxymethyl) - 1,9 - dioxo - 15,16 - trimethylene - 13 - *trans* - prostene. In a similar manner, the compounds represented by formulae (E) to (J) have the configurations shown below.

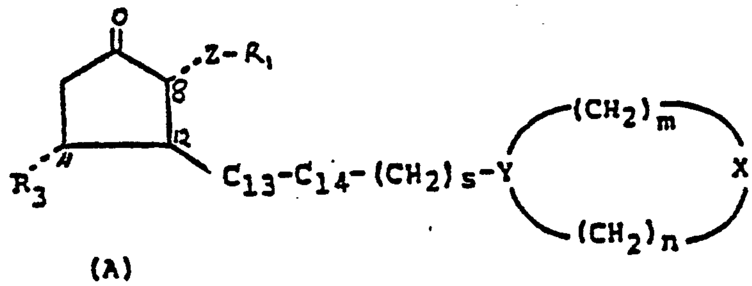

(A)

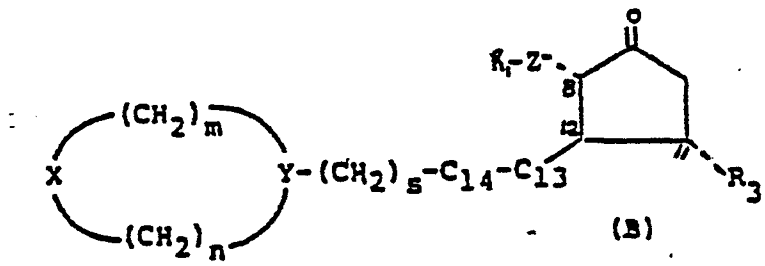

(B)

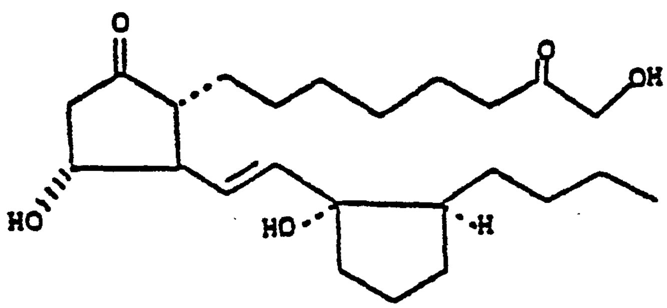

nat-15S,16S

(C)

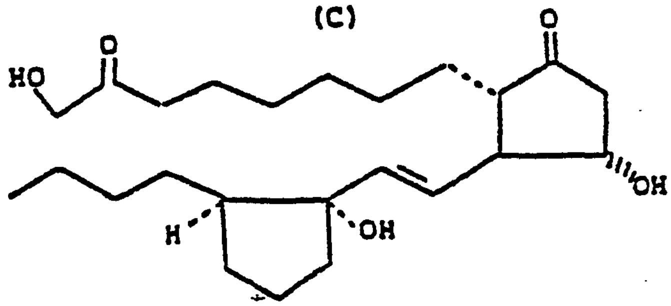

ent-15R,16R

(D)

racemate nat-15S,16S-(and ent-15R,16R)

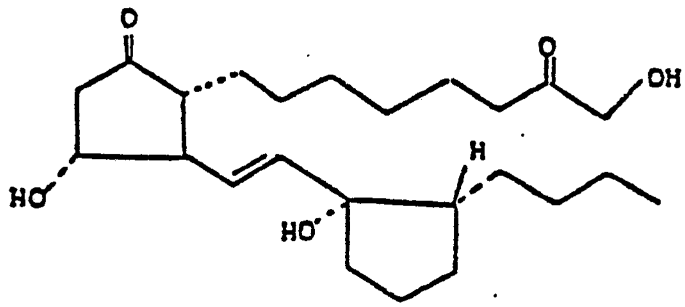

nat-15S,16R

(E)

racemate
nat-15S,16R
(and
ent-15R,16S)

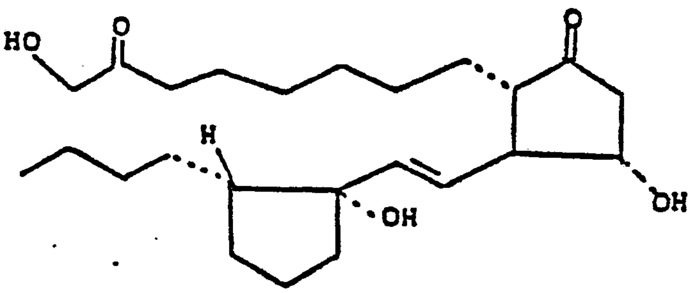

ent-15R,16S

(F)

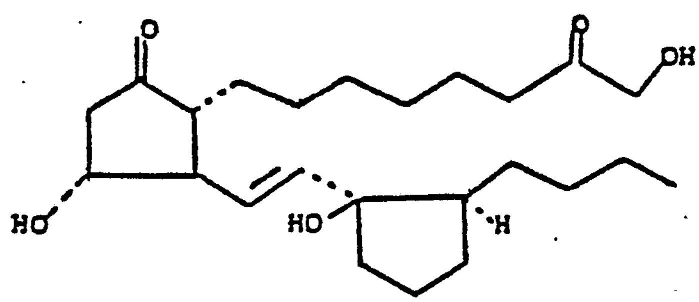

nat-15R,16S

(G)

racemate
nat-15R,16S
(and
ent-15S,16R)

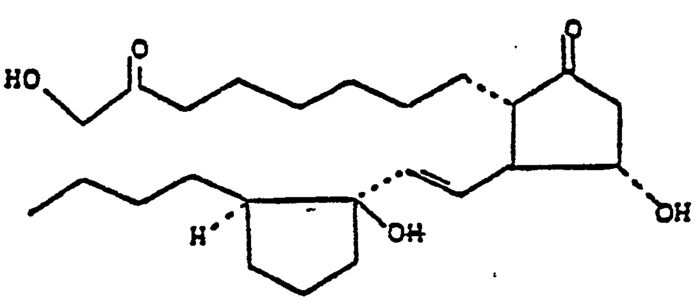

ent-15S,16R

(H)

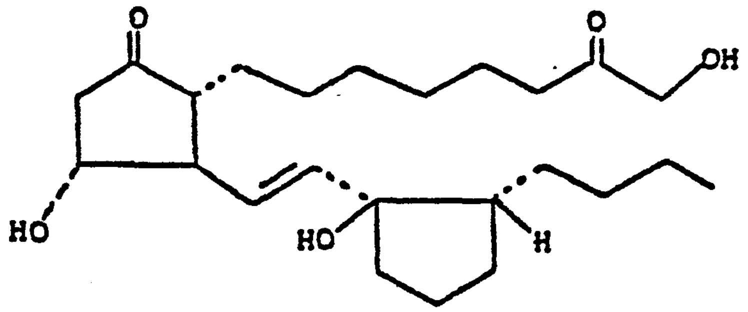

nat-15R,16R

(I)

racemate nat-15R,16R (and ent-15S,16S)

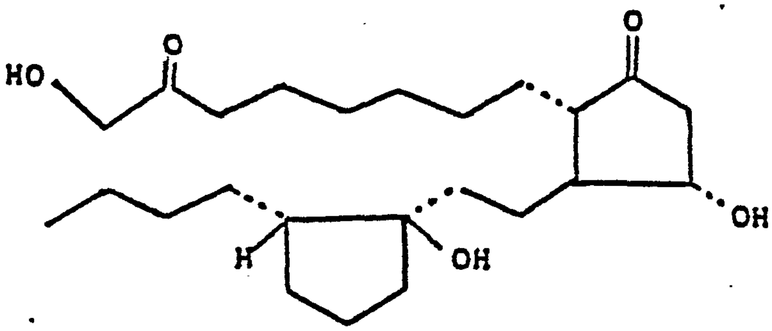

ent-15S,16S

(J)

In each of the above formulae (C to J) the hydroxy group at $C_{11}$ is named "-11$\alpha$-hydroxy".

The novel compounds of this invention can be prepared by a novel 1,4-conjugate-addition procedure involving treatment of the ether blocked cyclopentenone such as (129) or (111) with a lithiocuprate reagent such as (117), (118), or (119) prepared as illustrated in Flowsheets A through N.

The 1,4 - conjugate - addition procedure is described hereinbelow in Flowsheet N. The preparation of the various requisite 1 - iodo - *trans* - 1 - alkenyl or 1 - tributylstannyl - *trans* - 1 alkenyl derivative is illustrated in Flowsheets A—H and the novel and important methods of preparation of the 4-hydroxycyclopentenones embracing the 1 - (hydroxymethyl) - 1 - ox $\alpha$ chain is described with Flowsheets I—M.

FLOWSHEET A

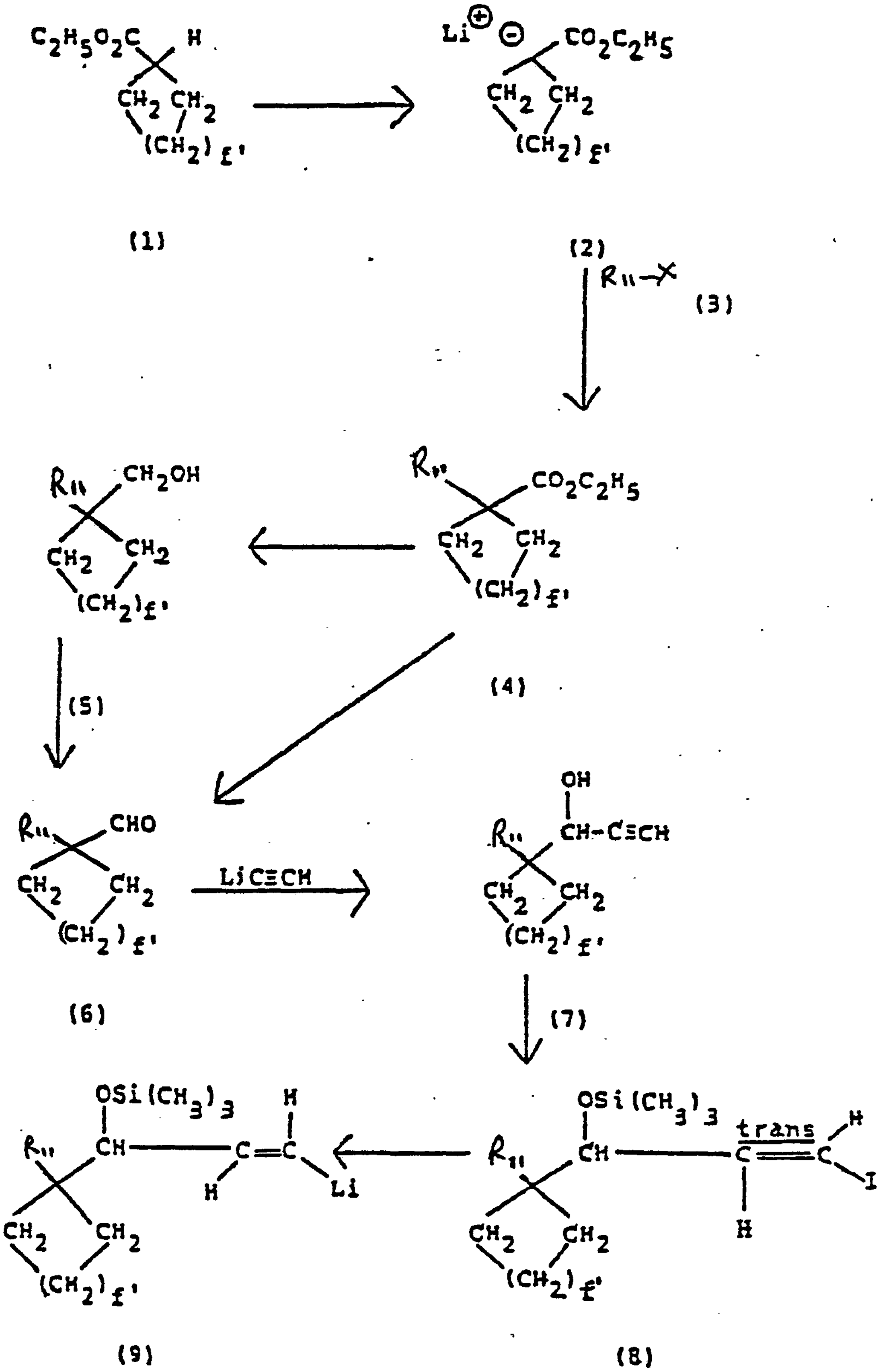


wherein f' is one or two inclusive.

In accordance with the scheme as outlined hereinabove in Flowsheet A, carbethoxycyclobutane or carbethoxycyclopentane is converted to its enolate anion (2) by treatment with a strong base such as lithium cyclohexylisopropylamide, prepared from the corresponding amine and *n*-butyl lithium (hexane solution) in a solvent, such as anhydrous tetrahydrofuran, at very low temperatures, such as —78°C. The resulting enolate anion (2) is then alkylated with $R_{11}$—X (3) to provide (4), the ester group of which is reduced to alcohol (5) by reaction with 2 equivalents of diisobutyl aluminum hydride, lithium aluminum hydride or the like. Oxidation of alcohol (5) with dipyridine chromium oxide complex. ("Reagents for Organic Synthesis", L.F. Fieser and M. Fieser, John Wiley and Sons, Inc., New York, *4*, 215 (1974)), prepared *in situ* in methylene chloride solution, provides the corresponding aldehyde (6), which can also be obtained directly from ester (4) by partial reduction with one equivalent of diisobutyl aluminum hydride at —78°C, but the former two-step procedure is preferable. Reaction of aldehyde (6), with lithium acetylide ethylene diamine complex provides the 3 - hydroxy - 1 - alkyne (7), which is converted to its trimethylsilyl ether in the usual manner. The silylated derivative is then treated with diisoamylborane (prepared *in situ* in tetrahydrofuran solution at ice bath temperatures from 2 - methyl - 2 - butene, sodium borohydride and boron trifluoride ethereate) and then anhydrous trimethylamine oxide. The resulting solution and an iodine solution in tetrahydrofuran are then added simultaneously to an aqueous solution of sodium hydroxide to give the 1 - iodo - 3 - trimethylsilyloxy-4,4 - methylene - 1 - alkene (8).

FLOWSHEET B

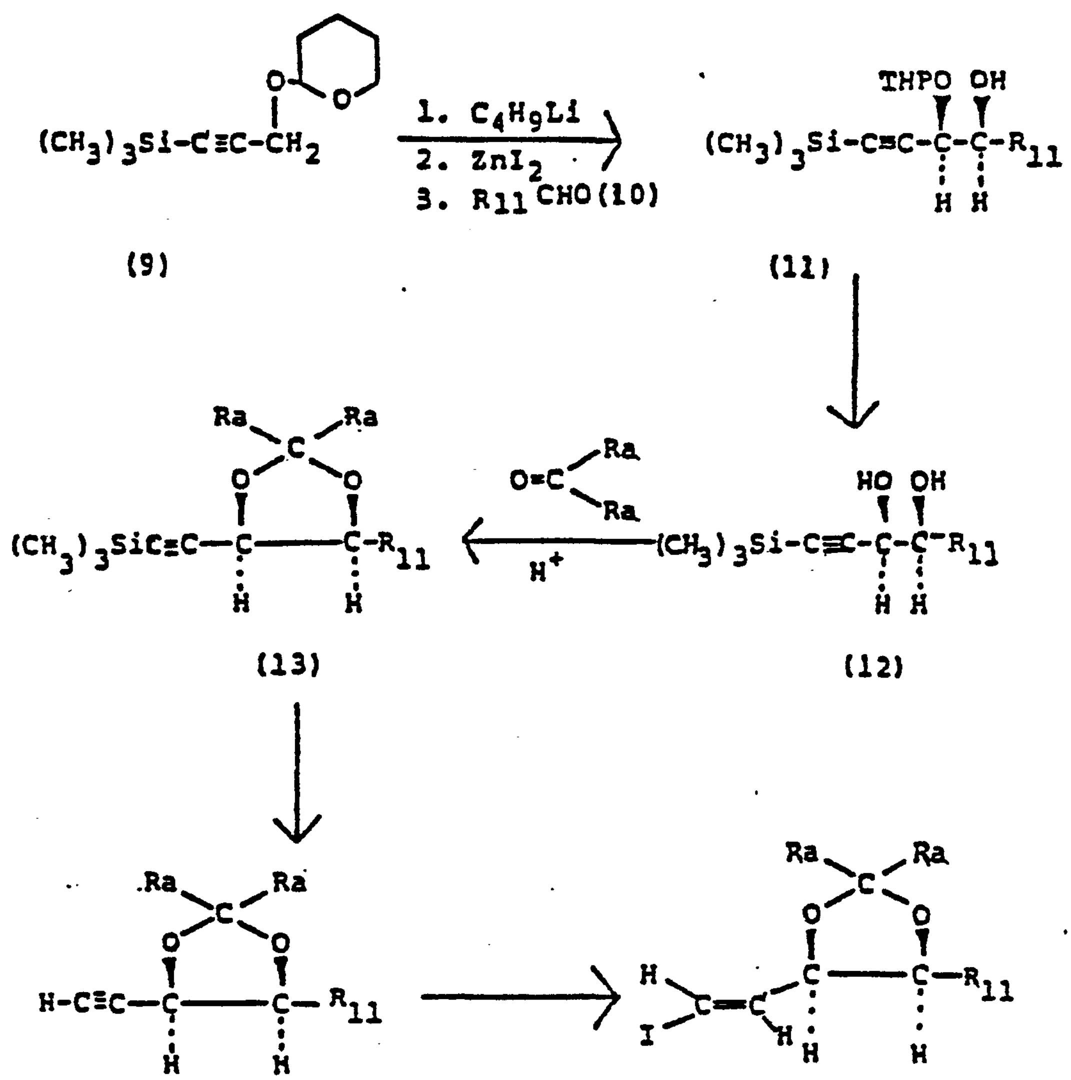


(9) (11) (12) (13) (14) (15)

Flowsheet B Continued

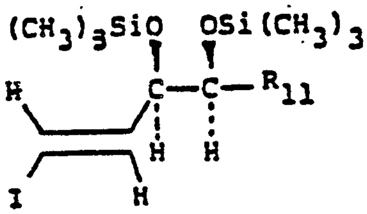

(16)

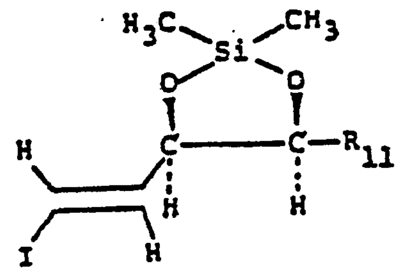

(17)

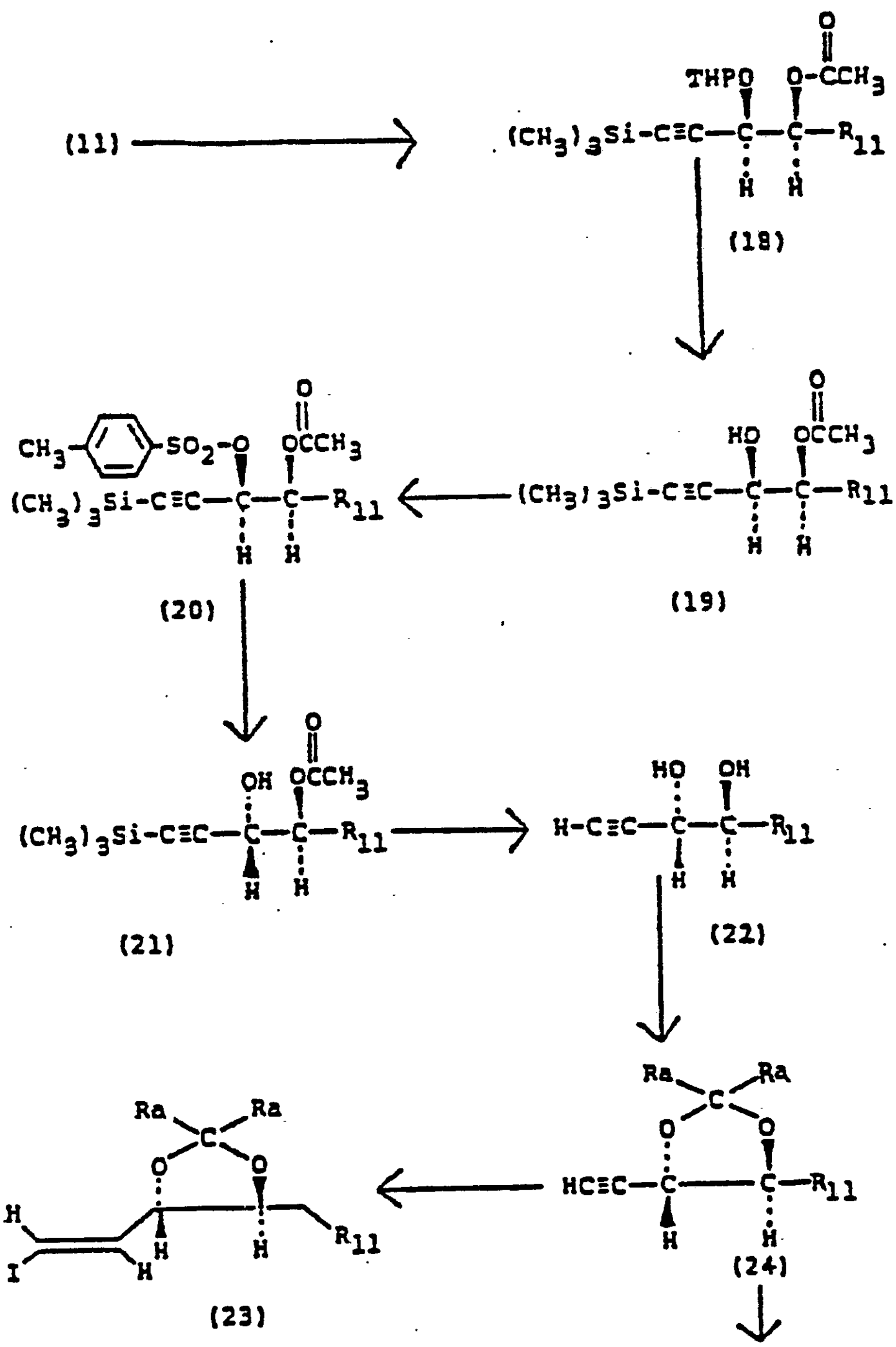

(11)

(18)

(20)

(19)

(21)

(22)

(24)

(23)

(25)

Flowsheet B Continued

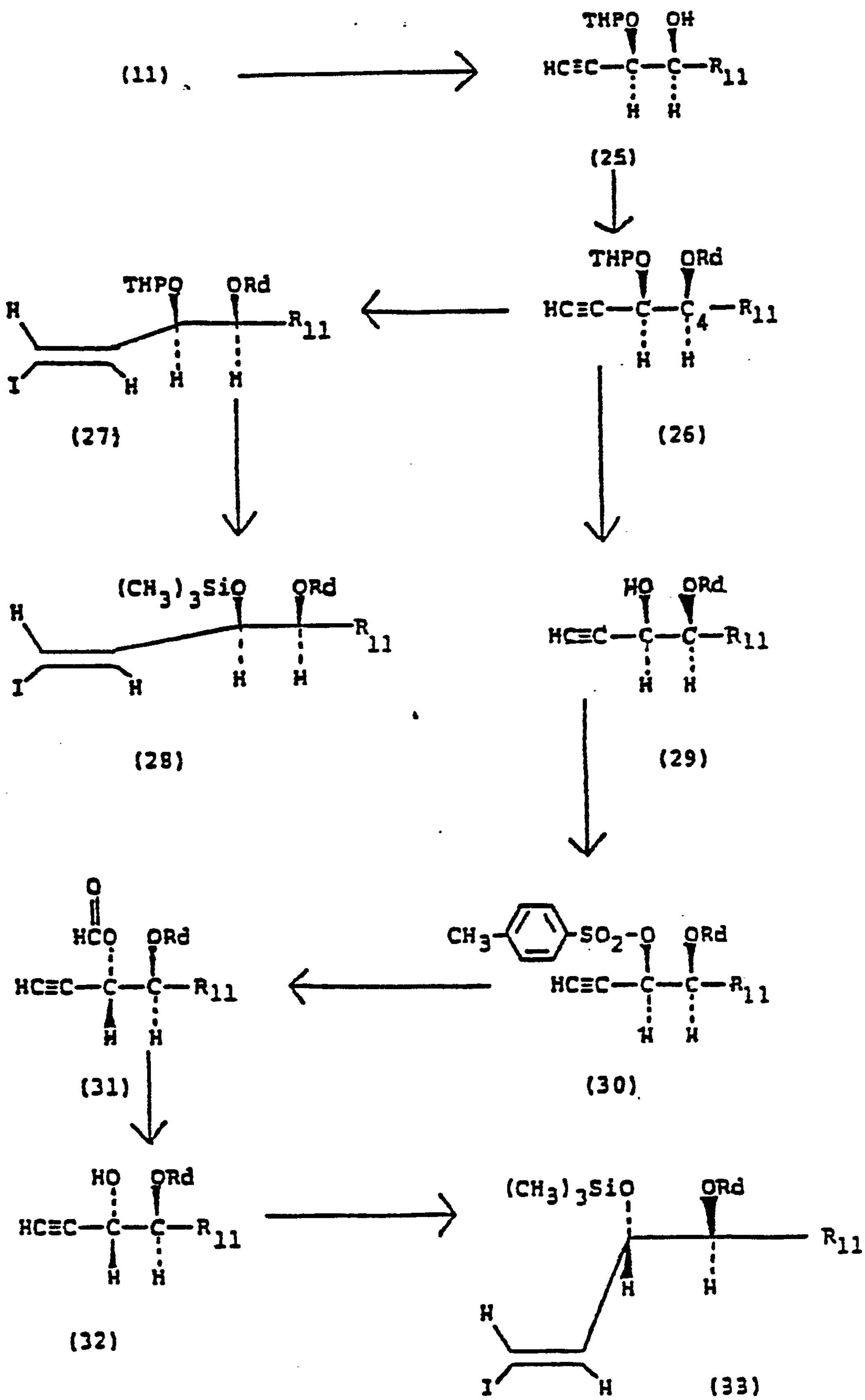

(11)

(25)

(27)

(26)

(28)

(29)

(31)

(30)

(32)

(33)

In accordance with the scheme as outlined hereinabove in Flowsheet B, 1 - trimethylsilyl - 3 - tetrahydropyranyloxy - 1 - propyne (9) is treated with *n*-butyllithium at −78°C and then with a freshly prepared solution of zinc iodide in anhydrous tetrahydrofuran, also at−78°C. Reaction of aldehyde (10) with the resulting reagent then provides the 4 - hydroxy - 3 - tetrahydropyranyloxy - 1 - alkyne (11). This reaction proceeds with great stereoselectivity and the product (11) is in the *erythro* configuration. (For additional information concerning this reaction see the examples which follow and F. Mercier, R. Epstein and S. Holland, Bull. Soc. Chim. France, 690(1972).

The tetrahydropyranyl group in (11) is removed on weak acid treatment and the resulting erythro diol (12) can be reblocked by treating with an appropriate aldehyde or ketone

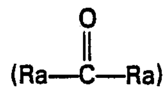

in the presence of strong acid catalyst in the usual way to give the ketal or acetal (13). Acetone is a useful ketone for this purpose and the product (13) is then a 3,4 - isopropylidenedioxy - 1 - alkyne. It is also possible to utilized silyl blocking groups (introduced after removal of the 1-trimethylsilyl group) to ultimately give the vinyl iodides (16) or (17). Weak base treatment of (13), for example heating for about one hour in refluxing methanol with potassium carbonate, results in desilylation to give (14). The 1-alkene (14) is converted to the corresponding 1 - iodo - *trans* - 1 - alkene (15) by treatment with disiamylborane (prepared *in situ* in tetrahydrofuran solution at ice bath temperatures form 2 - methyl - 2 - butene, sodium borohydride and boron trifluoride ethereate) and then anhydrous trimethylamine oxide. The resulting solution and an iodine solution in tetrahydrofuran are then added simultaneously to an aqueous solution of sodium hydroxide to give (15).

For the preparation of the *threo* derivatives, the 4 - hydroxy - 3 - tetrahydropyranyloxy - 1 - alkyne (11) is acetylated to provide the corresponding 4-acetoxy derivative (18). The tetrahydropyranyl group is preferentially hydrolized with weak acid to (19), which is then tosylated in the usual manner to afford the *erythro* - 3 - tosyloxy - 4 - acetoxy -1 - alkyne (20). Solvolysis of (20) under essentially neutral conditions by heating in aqueous tetrahydrofuran in the presence of an insoluble acid-acceptor, such as calcium carbonate, results in inversion of $C_3$, furnishing the *threo* - 3 - hydroxy - 4 - acetoxy - 1 - alkyne (21) which is then deblocked with aqueous base to give the *threo* - 3,4 - diol (22). Diol (22) is converted to an acetal or ketal (23) (or silyl derivatives as in (16) or (17)) and thence to the 1 - iodo - *trans* - 1 - alkene (23) as described hereinabove wherein Ra is lower alkyl ($C_1$ to $C_3$).

For the preparation of the 16-alkoxyprostanoic acids of this invention, the *erythro* - 4 - hydroxy - 3 - tetrahydropyranyl - oxy - 1 - alkyne (11) is desilylated by methanol-potassium carbonate treatment and the resulting (25) is alkylated to give the 4 - alkoxy - 3 - tetrahydropyranyloxy - 1 - alkyne (26). A useful procedure for this last step involves treatment of (25) with a molar equivalent of sodium hydride to give the 4-alkoxide which is then alkylated with the appropriate alkylating agent, for example methyl iodide. The 4 - alkoxy - 1 - alkyne (26) is then converted to the corresponding 1 - iodo - *trans* - 1 - alkene (27) as described hereinabove for the preparation of (15). If desired the tetrahydropyranyl blocking group in (27) can be hydrolyzed (weak acid) and the resulting free 3-ol corresponding to (27) converted to the 3-trimethylsilyloxy derivative (28), all in the usual manner wherein Rd is methyl or ethyl.

For the *threo* series, the tetrahydropyranyl group in *erythro* - 4 - alkoxy - 1 - alkyne (26) is cleaved and the resulting 3 - hydroxy - 4 - alkoxy - 1 - alkyne (29) is tosylated to give the *erythro* - 3 - tosyloxy - 4 - alkoxy - 1 - alkyne (30). $Sn_2$ displacement reaction with (30) with reagents such as tetrahydroammonium formate results in inversion to the *threo* derivative (31) saponification of which provides *threo* - 3 - hydroxy - 4 - alkoxy - 1 - alkyne (32). Trimethylsilylation followed by the vinyl iodide conversion procedure described hereinabove furnishes the *threo* - 1 - iodo - 1 - alkene (33) wherein Rd is hydrogen, methyl or ethyl.

The 15-alkyl and/or 16-alkyl derivatives of this invention can be prepared by substituting

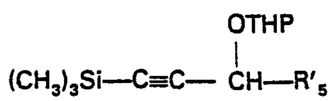 for (9) and/or 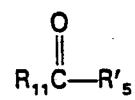

for (10) ($R'_5$ = lower alkyl of 1 to 3 carbons) in Flowsheet B.

In accordance with the procedure as outlined in Flowsheet C, an aldehyde (34) is treated with propargylic magnesium halide to form the homopropargylic alcohol (35), which is converted to its trimethylsilyl ether in the usual manner. The silylated derivative is then treated with disiamylborane (prepared *in situ* in tetrahydrofuran solution at ice bath temperatured from 2 - methyl - 2 - butene, sodium borohydride and boron trifluoride ethereate) and then anhydrous trimethylamine oxide. The resulting solution and an iodine solution in tetrahydrofuran are added simultaneously to a sodium hydroxide to give the 1 - iodo - 4 - trimethylsilyloxy - *trans* - 1 - alkene (36), precursors for 16 - hydroxy - prostaglandin.

The trimethylsilyl protecting group is removed with mild acid and the resulting vinyl iodide alcohol is oxidized with pyridinium chlorochromate to provide the 1 - iodo - 4 - oxo - *trans* - 1 - alkene (37), which upon treatment with a Grignard reagent ($R'_{13}MgX$) provides the 1 - iodo - 4 - hydroxy - *trans* - 1 - alkene, which is silylated in the usual manner to provide the silyl ether (38) wherein $R'_{11}$ is lower alkyl ($C_3$ to $C_6$) or lower alkenyl group ($C_3$ to $C_5$) and $R'_{13}$ is vinyl, cyclopropyl or ethyl.

FLOWSHEET C

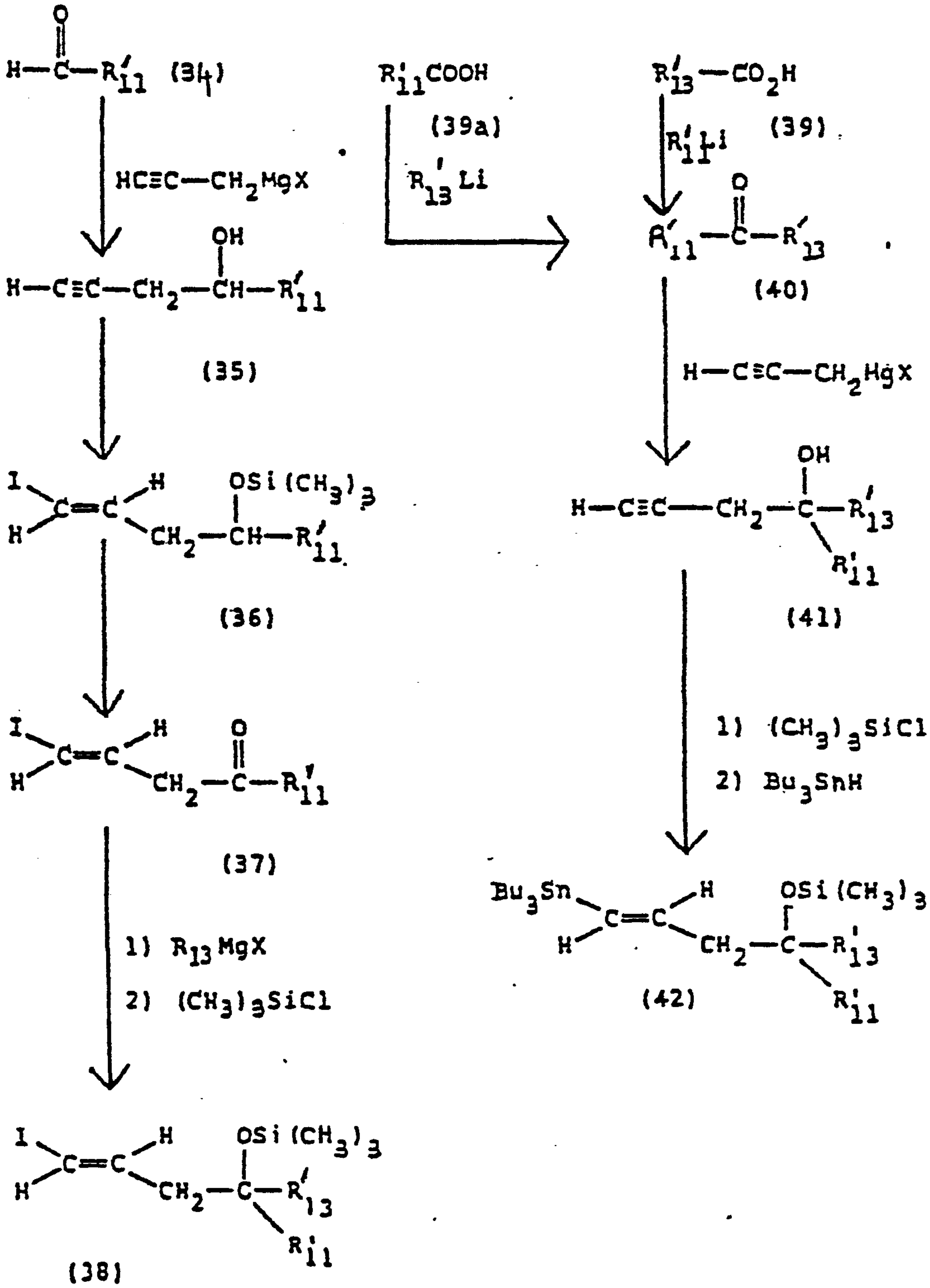


(34) (39a) (39) (40) (35) (41) (36) (37) (42) (38)

A more preferred method for the preparation of vinyllithium precursor is also described in Flowsheet C. Treatment of the requisite carboxylic acid (39a or 39) with the appropriate organolithium reagent ($R'_{13}Li$ or $R'_{11}Li$ respectively), wherein $R'_{11}$ and $R'_{13}$ are hereinabove defined, give the corresponding ketone (40) which upon treatment with propargylic magnesium halide provides the homopropargylic alcohol (41) which is converted to the *trans* vinylstannyl derivative by sequential treatment with chlorotrimethylsilane and tri - *n* - butyltin hydride. Treatment of the vinylstannyl reagent (42) with *n*-butyllithium at a temperature of −10°C to −78°C generates the corresponding vinyllithium reagent.

FLOWSHEET D

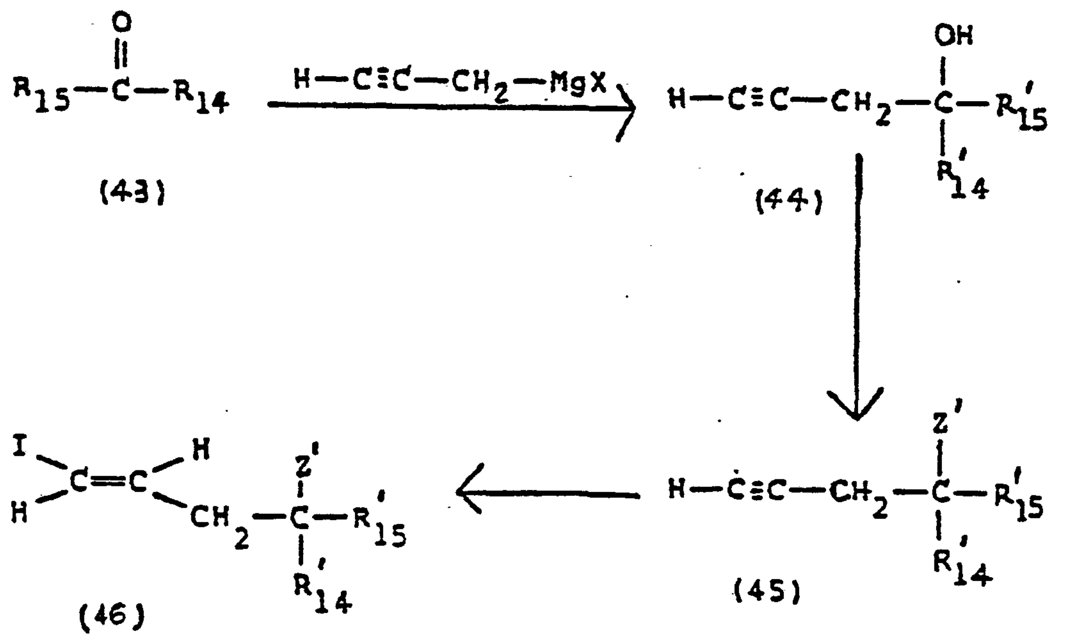

(43) (44) (45) (46)

In accordance with Flowsheet D hereinabove, the precursors for other 16-hydroxy prostallandins are prepared by treating an appropriate aldehyde or ketone (43) with a propargylic magnesium halide to yield the requisite homopropaargylic alcohol (44). The alcohol is protected as a tritylether (45) (for secondary alcohols) or as a trimethylsilyl ether (45) (for tertiary alcohols). These ethers are then converted to the appropriate *trans*-vinyliodide (46) by treatment with disiamylborane generated *in situ* from 2 - methyl - 2 - butene, sodium borohydride, and boron trifluoride, followed by treatment with trimethylamine oxide and then iodine and sodium hydroxide, wherein $R'_{15}$ is hydrogen or methyl: Z' is $O—C(C_6H_5)_3$ when $R'_{15}$ is hydrogen and Z' is $O—Si(CH_3)_3$ when $R'_{15}$ is methyl: $R'_{14}$ is selected from the group comprising lower alkyl ($C_3$ to $C_7$), lower 1-alkenyl ($C_3$ to $C_6$),

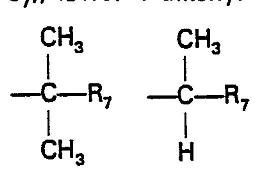 :

wherein $R_7$ is as defined above with the proviso that when $R'_{14}$ is

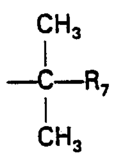 or 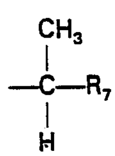

then $R'_{15}$ must be hydrogen.

FLOWSHEET E

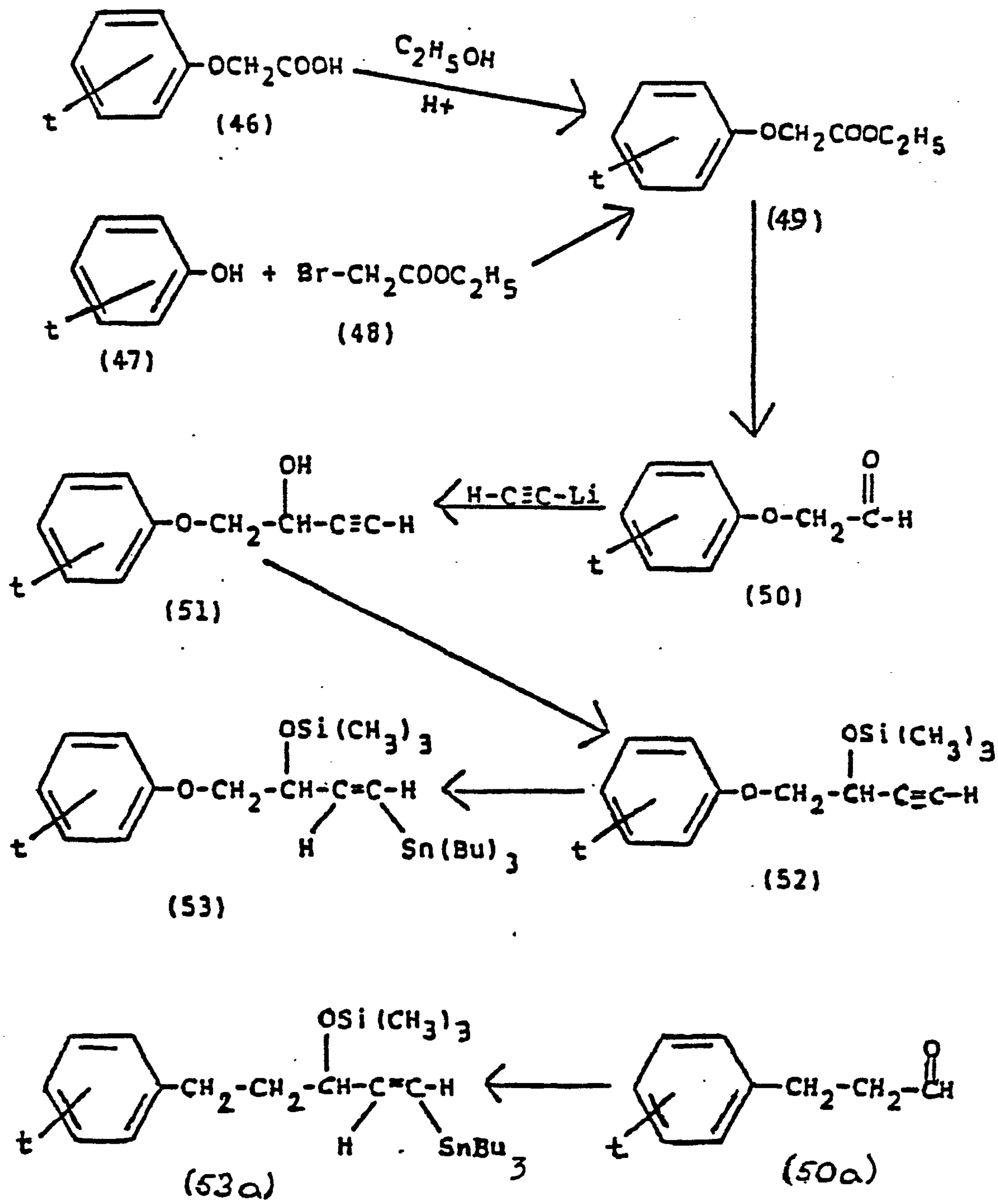


(46) (47) (48) (49) (50) (51) (52) (53) (53a) (50a)

The preparation of the precursors for the synthesis of 16-aryloxy congeners is described in accordance with Flowsheet E hereinabove. The aryl esters (49) are prepared by esterifying the commercially available acids or by treatment of ethyl bromoacetate with the appropriate phenol. The ester (49) is carefully reduced to the aldehyde (50) which upon treatment with lithium acetylide provides the propargylic alcohol (51). Treatment of the alcohol (51) with the chlorotrimethylsilane followed by tri - *n* - butyltin hydride furnishes the requisite vinylstannyl derivative (53). Similar treatment starting with substituted hydrocinnamaldehyde (50a) provides the respective vinylstannyl derivative (53a).

The preparation of the precursors for the synthesis of secondary 15-hydroxy congeners are

described in the literature. The preparation of the precursor for 15 - methyl - 15 - hydroxy is described in Flowsheet F hereinbelow. In accordance with Flowsheet F, an acid chloride, wherein $R_5$ is hereinabove defined, is treated with acetylene and aluminum trichloride to provide the vinylchloride (55) which upon treatment with sodium iodide furnishes the vinyliodide (56). Treatment of (56) with methylmagnesium halide followed by chlorotrimethylsilane gives the requisite protected vinyliodide (57).

FLOWSHEET F

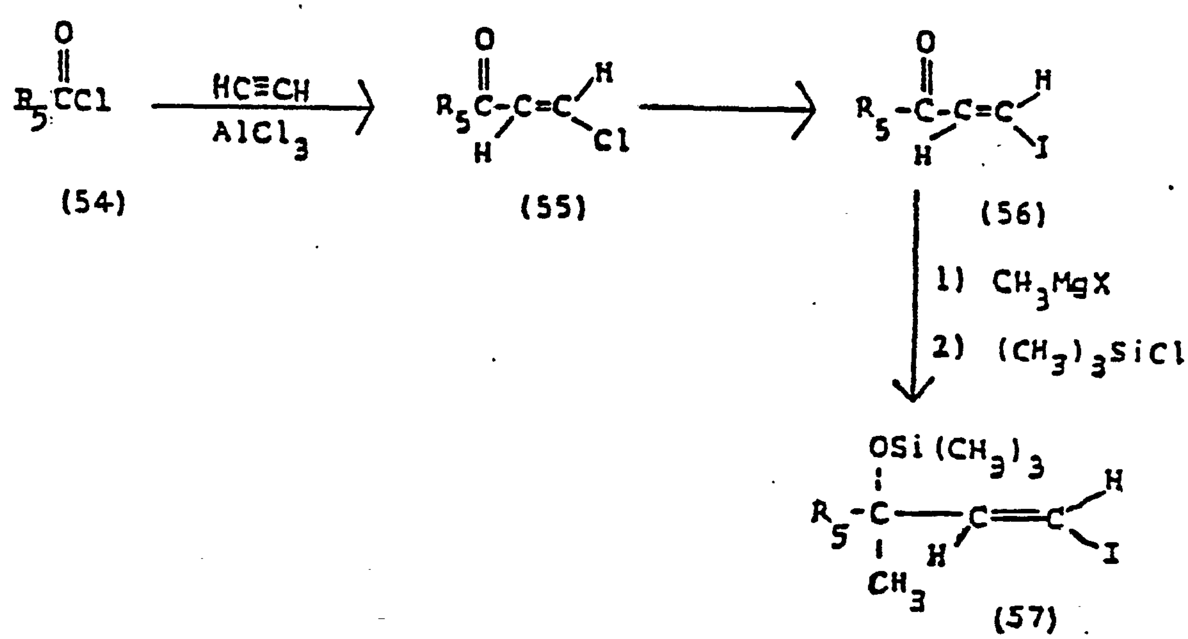

(54) (55) (56) (57)

The precursors for the novel compounds of this invention have a $\beta$ chain represented by Formula K

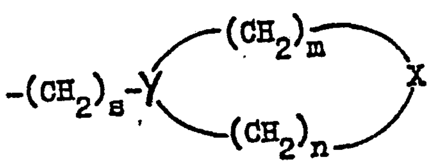
(K)

wherein s, Y, m, n, and X are hereinabove defined is shown in Flowsheet G and Flowsheet H.

Flowsheet G

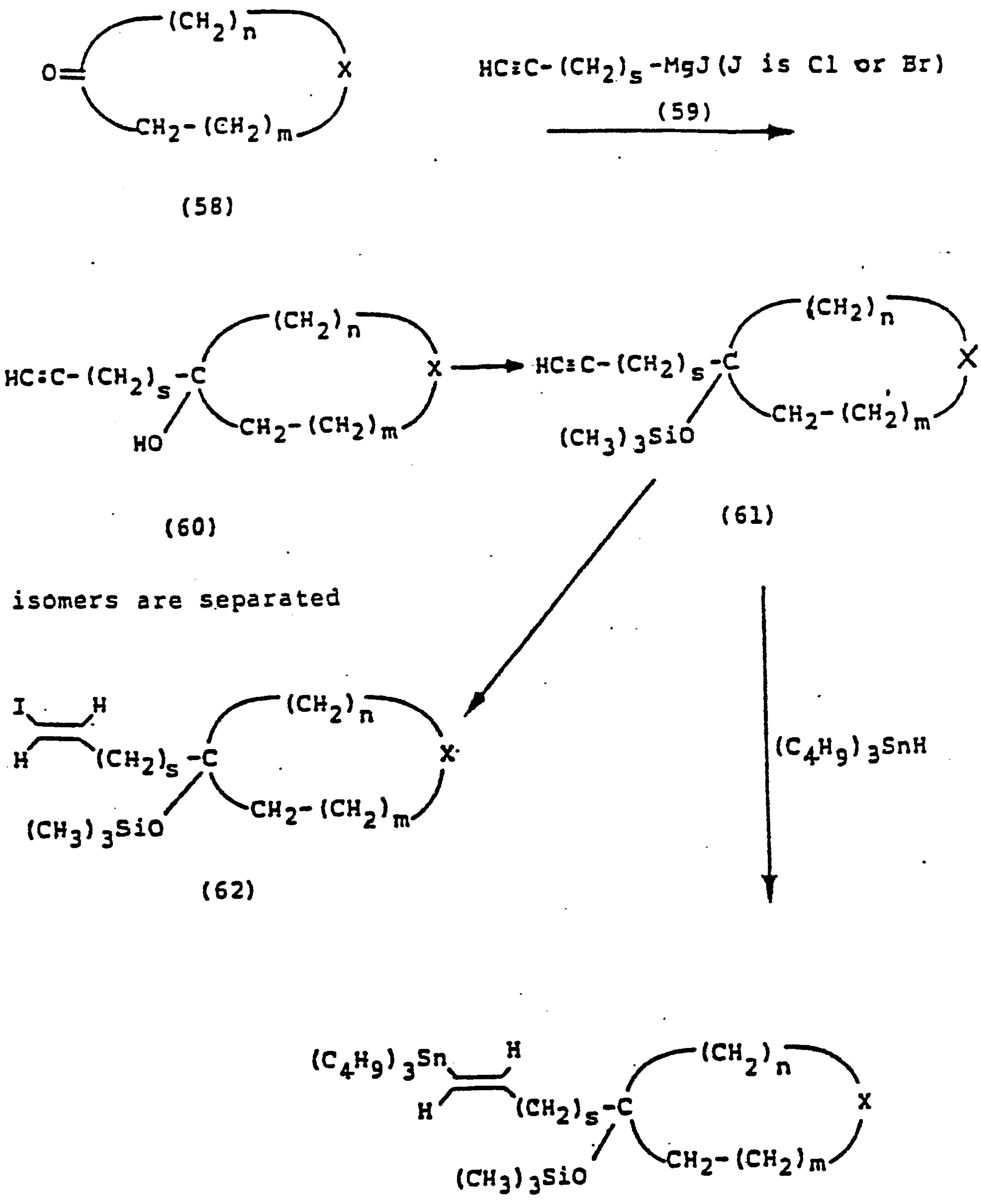


(58)

(60)

(61)

(62)

(63)

In accordance with the scheme as outlined hereinabove in Flowsheet G a ketone (58) is reacted with Grignard reagent (59) such as acetylene magnesium chloride (59, s = 0) or propargyl magnesium bromide (59, s = 1) to give the acetylenic alcohols (60). In those cases where X is not $—CH_2$ two isomeric acetylenic alcohols are formed. These isomers can be separated by procedures well known to the art including fractional cystallization, fractional distillation and various chromatographic procedures. The individual isomers can then be carried through the remaining reactions outlined in Flowsheet G.

The acetylenic alcohol (60) is converted to its trimethylsilyl ether in the usual manner. The silylated derivative (61) is then treated with diisomaylborane (prepared *in situ* in tetrahydrofuran solution at ice bath temperatures from 2 - methyl - 2 - butene, sodium borohydride and boron trifluoride ethereate) and then anhydrous trimethylamine oxide. The resulting solution and an iodine solution in tetrahydrofuran are then added simultaneously to an aqueous solution of sodium hydroxide to give the 1 - iodo - 3 - trimethylsilyloxy - *trans* - 1 - alkene (62).

(63) in turn is readily prepared by the addition of tri - *n* - butyltin hydride to the acetylene (61) in the presence of bisazoisobutyronitrile followed by vacuum distillation at a high enough temperature (about 170°C) to isomerize any of the *cis*-vinyl tin compound to the *trans*-vinyl tin compound.

Certain of the ketones (67) of this invention are prepared as indicated in Flowsheet H below:

## Flowsheet H

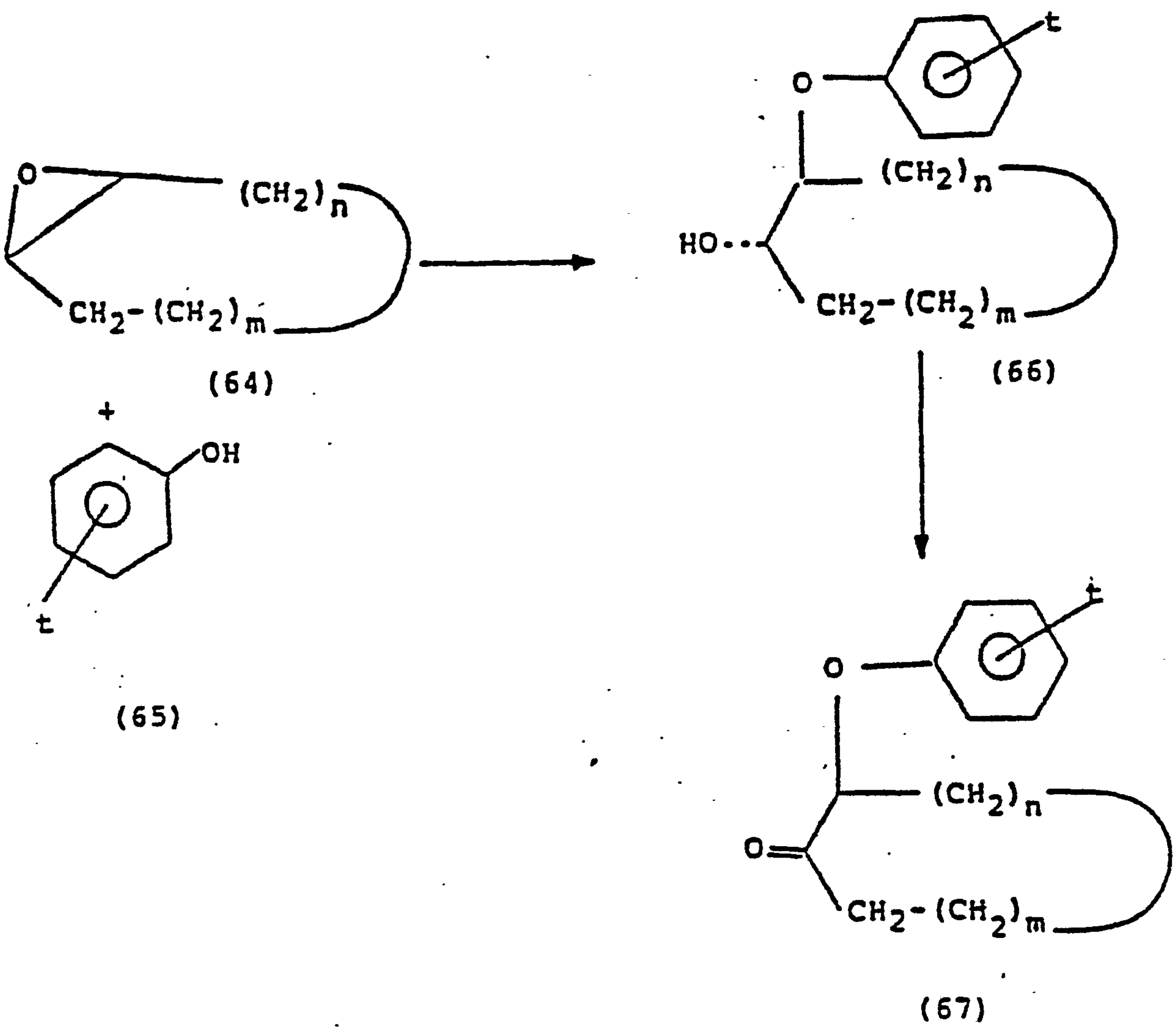

(64) (65) (66) (67)

wherein n and m are are hereinabove defined and moiety 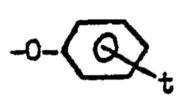 represents a phenoxy group which is optionally substituted with one or more halogen, trifluoromethyl, and lower alkoxy ($C_1$ to $C_4$) groups.

As indicated in Flowsheet H the reaction of an epoxide (64) with a substituted or unsubstituted phenol (65) in the presence of a catalytic amount of aqueous sodium hydroxide and a phase transfer catalyst such as methyl tricapryly ammonium chloride and the like at 70°—80°C gives the phenoxy substituted alcohol (66) which in turn is oxidized with an oxidizing reagent such as pyriidinium chlorochromate in methylene chloride to give the phenoxy substituted ketone (67). This ketone (67) is then carried through the reactions shown in Flowsheet G above.

The other ketones (58) used in this invention are known in the literature or can be made by procedures well known to the art (G. Lardelli, U. Lamberti, W. T. Walles and A. P. de Jonge, *Rec. Trav. Chem. Pays-Bas, 86* 481 (1967); Ng. Ph. Buu-Hoi, T. B. Loc and Ng. Dat Xuong., *Bull. Soc. Chem. France,* 174 (1958); and G. H. Posner, *Organic Reactions, 19,* 1 (1972)).

The preparation of the cyclopentenones containing the hydroxyketone feature (68) wherein Z is hereinabove defined and $R_3$ is hydrogen or a hydroxy group can be accomplished in several ways one of which involves the conversion of the corresponding cyclopentenone containing a carboxylate function (69) to the respective hydroxyketone analog (68).

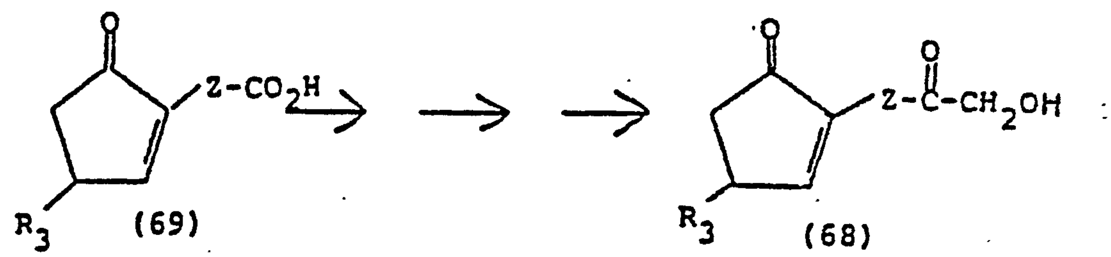

(69) (68)

Most of the cyclopentenone carboxylic acids (69) required for the purposes of this invention have been described in the literature or can be prepared by procedures quite analogous to those already described. Appropriate references are provided in the examples which follow. The synthesis of certain non-reference requisite cyclopentenone carboxylic acids (69) is also described herein.

The conversion of the cyclopentenone carboxylic acid (69) to the respective hydroxyketone analogs (68) and the protection of these compounds for a conjugate addition reaction is described hereinbelow in Flowsheets J and K.

For the preparation of cyclopentenones of the type (79) wherein Z is hereinabove defined, the carboxylic acid (76) is converted to the acid chloride (77) by first forming the sodium salt with sodium hydride in tetrahydrofuran (THF) and then reacting the resulting suspension with oxalyl chloride in the presence of a catalytic amount of dimethylformamide (DMF). The resulting acid chloride (77), dissolved in ether, is then added dropwise to an ether solution containing two to three equivalents of diazomethane to produce the diazoketone (78). The diazoketone can be hydrolized to the hydroxy ketone (79) by refluxing an etheral solution in the presence of a dilute aqueous solution of sulfuric acid.

Alternatively, the acid chloride (77) can be heated with two equivalents of 1,1,2 - tris - trimethylsilyloxyethylene at 90—100°C for 2 to 4 hours to produce compound (81). Compound (81) can be readily hydrolized and decarboxylated to give the hydroxyketone (79) by treatment with dilute hydrochloric acid in tetrahydrofuran (THF).

Protection of the hydroxy ketone function of 79, suitable for a conjugate addition reaction, can be accomplished in two ways. Ketalization of 79 with ethylene glycol is accomplished by refluxing a benzene or toluene solution of 79 and ethylene glycol into a Dean-Stark trap. The resulting ketal (82) is then treated with trimethylsilylchloride (TMSCl) and imidazole in dimethylformamide (DMF) to give 83 which is suitably protected for a conjugate addition reaction.

Alternatively 79 can be protected by the reaction with a mixture of 2 - methoxy - 1 - propene (84) and 2,2-dimethoxypropane (85) in benzene in the presence of an acid catalyst such as p-toluenesulfonic acid to give the ketal 86 which is suitably protected for a conjugate addition reaction.

Flowsheet J

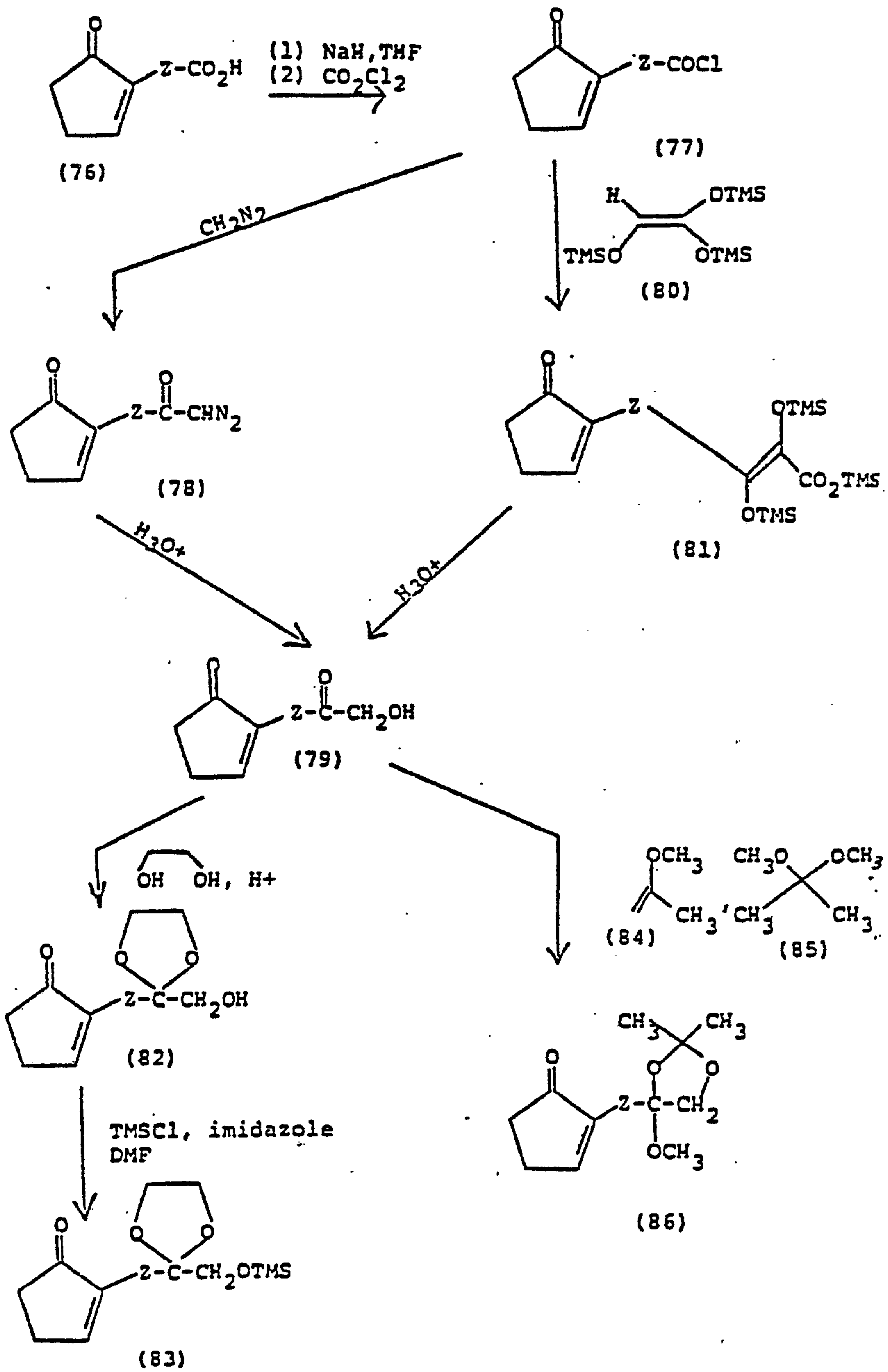


(76) (77) (78) (80) (81) (79) (82) (83) (84) (85) (86)

The preparation of the 4-hydroxycyclopentenones of this invention (92) wherein Z is hereinabove defined is outlined in Flowsheet K below. The reaction of the hydroxy acid (87) with at least two equivalents of dimethyl - t - butylsilylchloride in the presence of imidazole in dimethylformamide at 30—40°C gives the *bis* - dimethyl - t - butylsilated compound 88. The carboxylate dimethyl - t - butylsilyl group can be selectively removed by treatment with acetic acid, tetrahydrofuran and water (4:2:1) to give the carboxylic acid (89). The acid chloride (90) is prepared by first treating the acid (89) with sodium hydride in tetrahydrofuran to give the soidum salt. The resulting suspension of the sodium salt is then treated with oxalyl chloride in the presence of a catalytic amount of dimethylformamide. Alterantively the acid chloride (90) can be prepared directly by the reaction of the acid (89) or the dimethyl - t - butylsilyl ester (88) with oxalyl chloride in tetrahydrofuran in the presence of a catalytic amount of dimethylformamide at 0°C. The slow addition of an etheral solution of the acid chloride (90) to an etheral solution of two to three equivalents of diazomethane gives the diazoketone (91) which on acid hydrolysis gives the 4-hydroxycyclopentenone (92) containing the hydroxyketone function.

Alternatively the acid chloride (90) can be heated with at least two equivalents of 1,1,2 - *tris* - trimethylsilyloxyethylene at 90—120°C in the absence of a solvent to give compound (93) which is readily hydrolized and decarboxylated to give the 4-hydroxycyclopentenone (92) containing the hydroxyketone feature. Protecting of 92 can be accomplished by treatment with an excess of a mixture of 2 - methoxy - 1 - propene (84) and 2,2-dimethoxypropane (85) in benzene with an acid catalyst such as p-toluenesulfonic acid to give the *bis*-ketal (94) which is suitably protected for a conjugate addition reaction.

Alternatively, the two hydroxyl moieties may be protected using 2 equivalents of 2-methoxypropene per equivalent of *92* in the presence of a catalyst such as chloroacetic acid to provide compounds such as *94A*. Other useful protecting dihydro - 2H - pyran, ethylvinylether, and the like.

Other acid sensitive protecting groups for the two hydroxyl groups are the triloweralkylsilyls (from silylchlorides), triphenylmethane (from tritylchloride or bromide), mono - *p* - methoxytriphenylmethane (from mono - *p* - methoxytriphenylmethylchloride or bromide), methoxymethyl (from chloromethylmethylether) and the like.

Flowsheet K

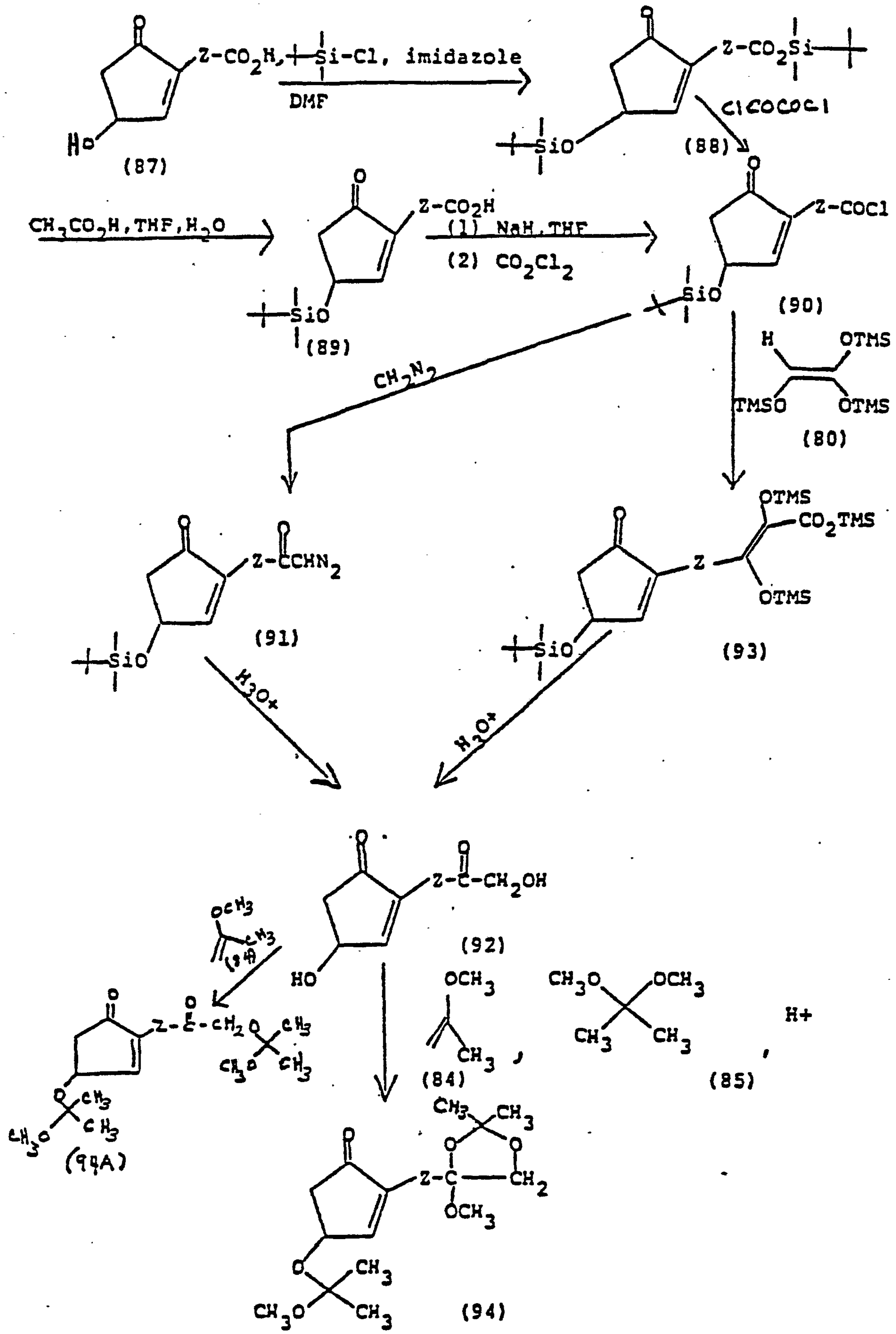


(87) (88) (89) (90) (80) (91) (93) (92) (84), (85), (94A) (94)

Another preparation of the 4-hydroxycyclopentenones which contain a *cis* double bond in the potential $\alpha$ chain (109) is shown hereinbelow in Flowsheet L wherein g is as hereinabove defined. As illustrated in Flowsheet L there are three methods available to prepare the important intermediate 98. The reaction of the $\omega$-bromo carboxylic acid (95) with oxalyl chloride in an inert solvent such as benzene gives the acid chloride (96). Addition of the acid chloride (96) in ether to an etheral solution of diazomethane (2 to 3 equivalent) yields the diazoketone (97) which can be hydrolized in a two phase system consisting of ether and dilute sulfuric acid to the hydroxyketone (98). Alternatively the acid chloride (96) can be treated with an excess of 1,1,2 - *tris* - trimethylsilyloxyethylene in the presence of a catalytic amount of stannic chloride in the absence of solvent to give compound (99) which can readily by hydrolized and decarboxylated to the desired hydroxyketone (98) using dilute hydrochloric acid in tetrahydrofuran. An alternate method to prepare (98) involves the reaction of the bromoolefin (100) with aqueous N-bromosuccinimide (NBS) in the presence of a catalytic amount of acetic acid to give a mixture of bromohydrins (101 and 102). Oxidation of the mixture of bromohydrins with an oxidizing agent such as pyridinium chlorochromate in methylene chloride gives a mixture of bromoketone (102a) and bromoaldehyde (102b). Refluxing this mixture with sodium formate in methanol then gives the desired intermediate (98). Protection of the ketone function of 98 is accomplished using ethylene glycol in refluxing toluene using a catalytic amount of p-tolouenesulfonic acid. The ketal (103) is then reacted with dimethyl - t - butylsilylchloride and imidazole in dimethylformamide to give the fully protected compound 104. The phosphonium salt (105) is obtained by refluxing a solution of 104 and triphenylphosphine in acetonitrile. Treatment of the phosphonium salt (105) with sodium methylsulfinylmethide in dimethylsulfoxide generates a phosphonium yield which on reaction with aldehyde 106 gives 107. Refluxing a water-dioxane solution of 107 in the presence of a phosphate buffer (PH 5 to 6) gives the cyclopentenone (108). Treatment of 108 with chloral and triethylamine in ether gives (109) which on hydrolysis in a mixture of tetrahydrofuran and dilute hydrochloric acid at 50—70°C then gives the desired 4-hydroxycyclopentenone (110) which can be protected as described hereinabove in Flowsheet K.

Treatment of 109 with trimethylsilylchloride and imidazole in DMF gives 111 which is also suitably protected for a conjugate addition reaction.

Flowsheet L

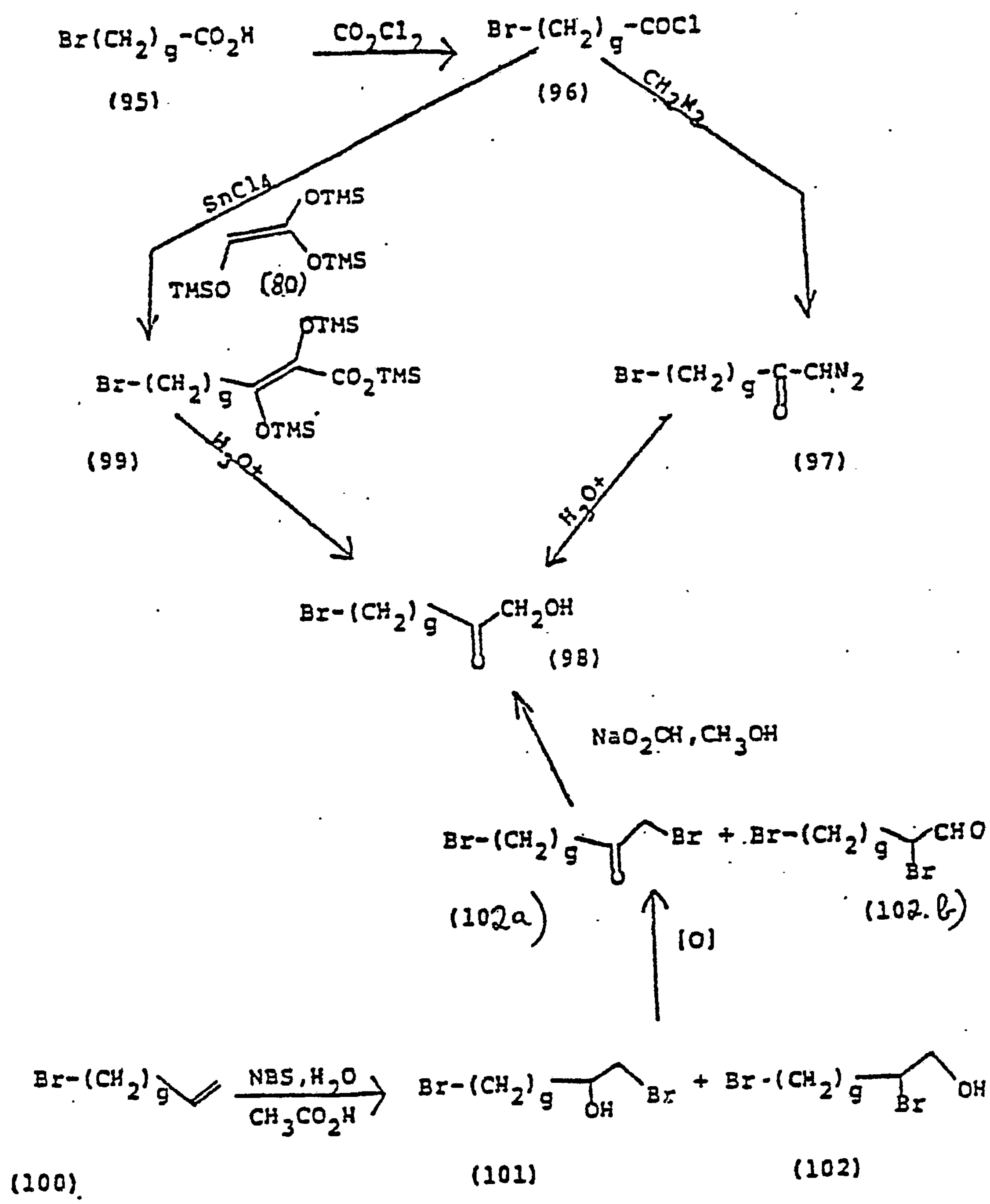

(95)
$CO_2Cl_2$
(96)
$CH_2N_2$
$SnCl_4$
(80)
(99)
(97)
$H_3O^+$
$H_3O^+$
(98)
$NaO_2CH, CH_3OH$
(102a)
(102 b)
[O]
$NBS, H_2O$
$CH_3CO_2H$
(100)
(101)
(102)

Flowsheet L (continued)

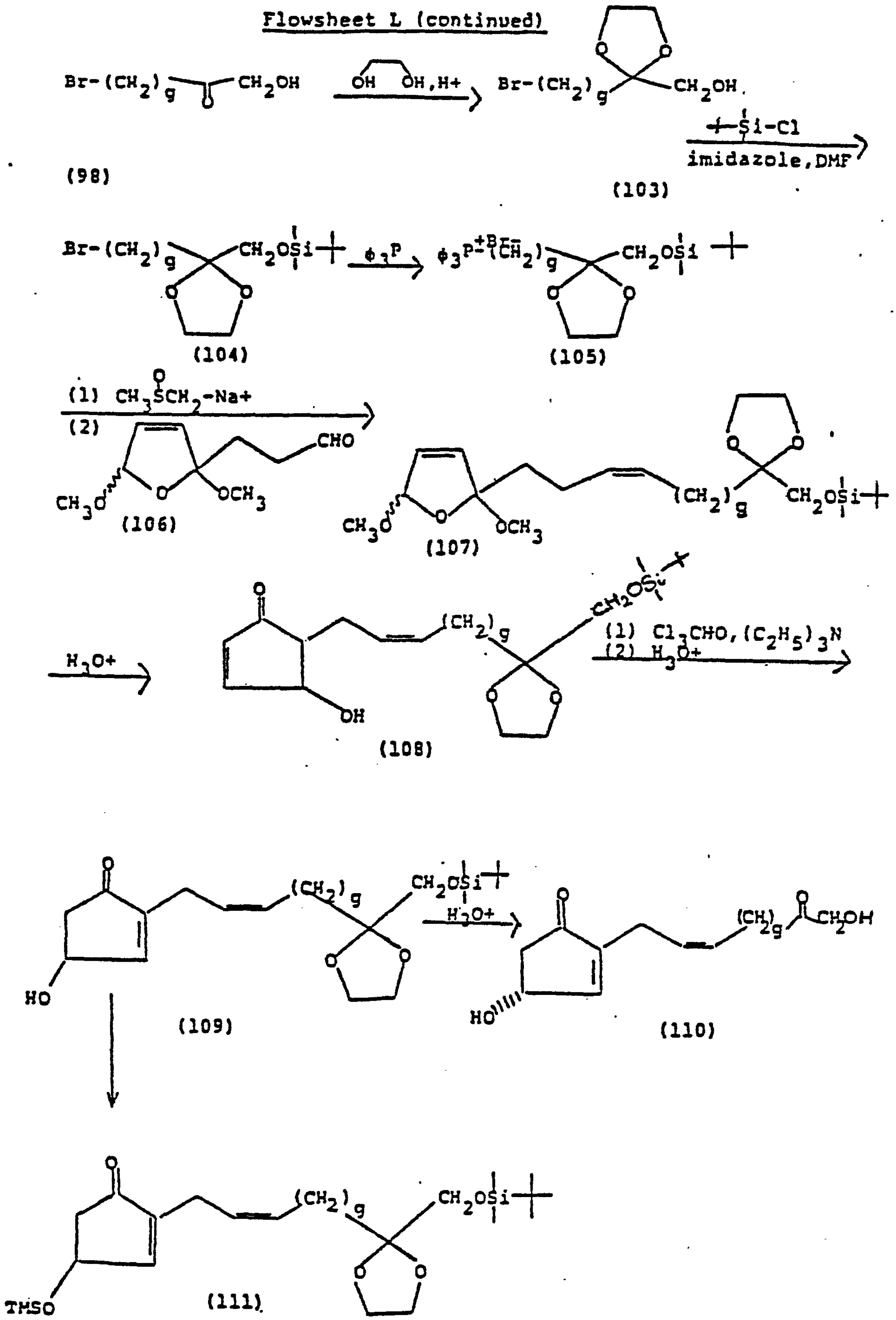


(98) (103) (104) (105) (106) (107) (108) (109) (110) (111)

The preparation of the reagent 1,1,2 - *tris* - trimethylsilyloxyethylene (80) is described hereinbelow in Flowsheet M. The reaction of glycolic acid with 1,1,1,3,3,3-hexamethyldisilazane and trimethylsilylchloride in pyridine gives *bis*-trimethylsilated glycolic acid (113). Addition of (113) to a tetrahydrofuran solution of one equivalent of lithium 1,1,1,3,3,3-hexamethyldisilazane amide at −78°C generates lithium enolate which is trapped with trimethylsilylchloride to produce the desired reagent (80).

Flowsheet M

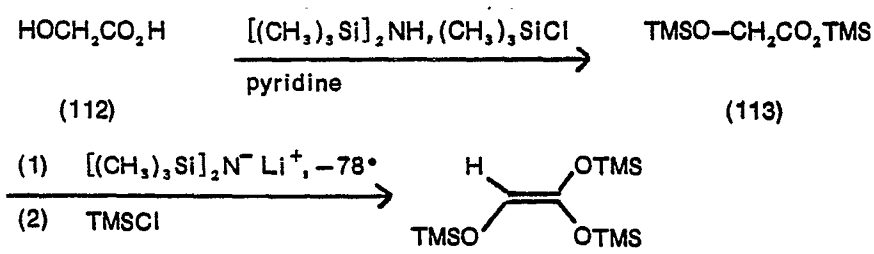


(112) (113)

(80)

The preparation of the prostaglandin congeners of this invention are described hereinbelow in Flowsheet N wherein Z is as hereinabove defined; $R''_3$ is hydrogen, a protected hydroxy group such as 1 - methoxy - 1 - methylethoxy(—$OC(CH_3)_2OCH_3$) or trimethylsilyloxy; $R_3$ is hydrogen or hydroxy; T′ is the radical

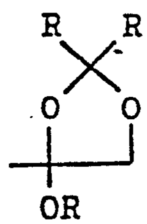 , 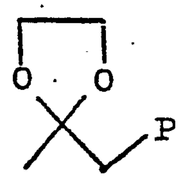 or 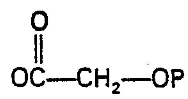

wherein R is as hereinabove defined, P is a protected hydroxy group such as tri($C_1$—$C_4$)alkylsiloxy or 1 - methoxy - 1 - methylethoxy, $R'_2$ is selected from the group consisting of:

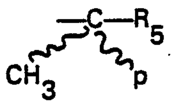 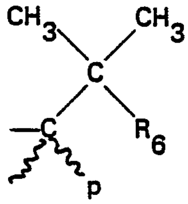 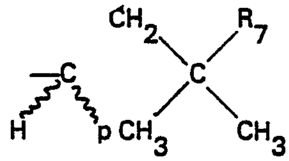

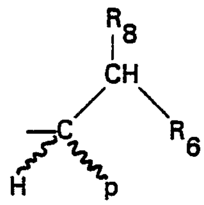 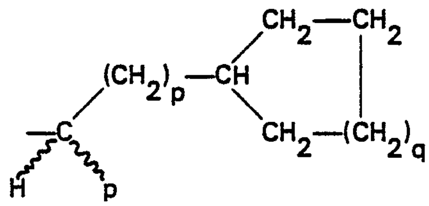

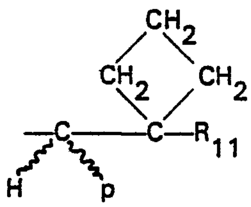 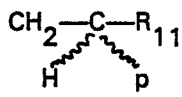 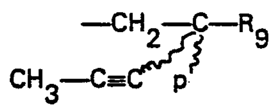

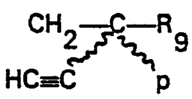 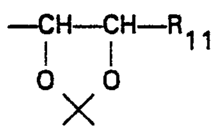 (erythro or threo),

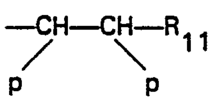 (erythro or threo) 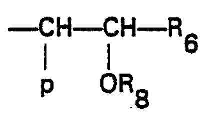

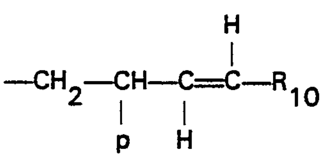 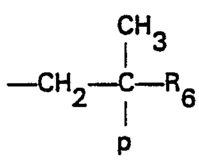 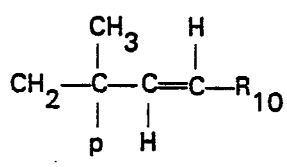

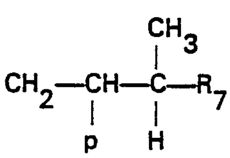 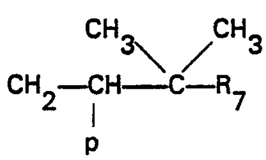 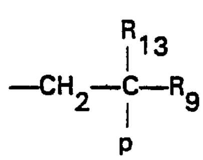

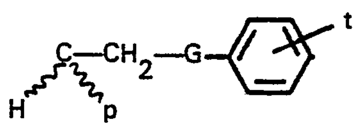

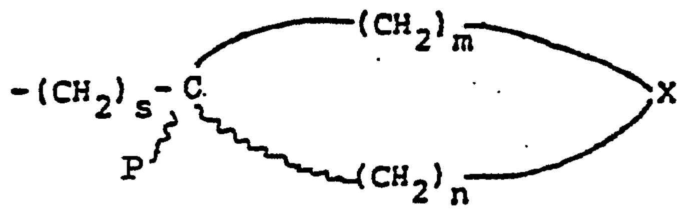

wherein $R_5$, $R_6$, $R_7$, $R_8$, $R_9$, $R_{10}$, $R_{11}$, $R'_{13}$, G, p, q, t, s, m, n and X are as hereinabove defined.

In accordance with Flowsheet N the vinyliodide (114) is treated with either one equivalent of *n*-butyllithium or 2 equivalents of *t*-butyllithium at low temperature, preferably —30° to —70°C in an inert solvent, e.g. hexane, ether or toluene to provide the *trans* alkenyl-lithium reagent (116).

Alternatively, the vinyllithium reagent (116) can be prepared by treatment of a vinylstannyl derivative such as (115) with *n*-butyllithium at —10° to —78°C in ether or THF.

For the preparation of the asymmetrical lithio cuprate (117) or the like, a solution of one molar equivalent of copper (I)-1-alkyne, preferably copper (I)-1-pentyne in anhydrous hexamethylphosphorous triamide preferably one to five molar equivalents, and anhydrous ether is added to one molar equivalent of the aforementioned vinyllithium solution cooled to about — 78°C. After about one hour at this temperature, a molar equivalent of the requisite cyclopentenone (120) is added. After several hours at —78°C to —20°C the reaction mixture is quenched with aqueous ammonium chloride solution and the blocked product (121) is isolated in the usual manner.

It is also possible to effect conjugate 1,4-addition with the asymmetrical lithio cuprate (119) derived from vinyllithium (116) and cuprous thiophenoxide. A solution of vinyllithium (116) in ether at —78°C is reacted with an equimolar amount of a reagent prepared by admixture, in ether at a temperature of 0°C to —78°C, of equimolar amounts of cuprous thiophenoxide and copper (I) iodide tributylphosphonium complex. After about 30 minutes at this temperature, the lithio cuprate (119) is treated with the requisite cyclopentenone (120) as described hereinabove for the conjugate addition with 1-alkynyl lithio cuprate (117).

For the preparation of the symmetrical lithio cuprate (118) one molar equivalent of copper (I) iodide tributylphosphine complex, dissolved in anhydrous ether, is added at about —78°C to two molar equivalents of the aforementioned vinyl lithium (116) solution in hexanes, cooled to —78°C. After about one hour at this temperature, the lithio cuprate (118) is treated with the requisite cyclopentenone (120) as described hereinabove for the conjugate addition with the 1-alkynyl-lithio cuprate (117).

The procedures for conjugate addition involving organocopper reagents are well known in the art, see for example, C. J. Sih, *et. al.*, J. A. C. S., *97*, 865 (1975).

All available evidence leads us to believe that the —CH=CH—$R'_2$ function introduced by the cuprate process occupies a position *trans* to the 11-oxy function. Similarly, we are led to the conclusion that in the product (121) the two sides-chains attached to $C_8$ and $C_{12}$ are *trans* to each other. However, we are not certain of this configurational relationship in the product as it is obtained directly from the cuprate process. These products may have the side-chains in a *trans* or *cis*-relationship or they may be a mixture containing both the *trans*- and *cis*-isomers. This is indicated in the nomenclature of the compounds involved by the designation S. In order to ensure a *trans*-relationship in (121) these products can be submitted to conditions known in the literature to equilibrate and *cis*-8-iso-$PGE_1$ to a mixture containing about 90% of the *trans* product. These conditions involve treatment with potassium acetate in aqueous methanol for 96 hours at room temperature.

FLOWSHEET N

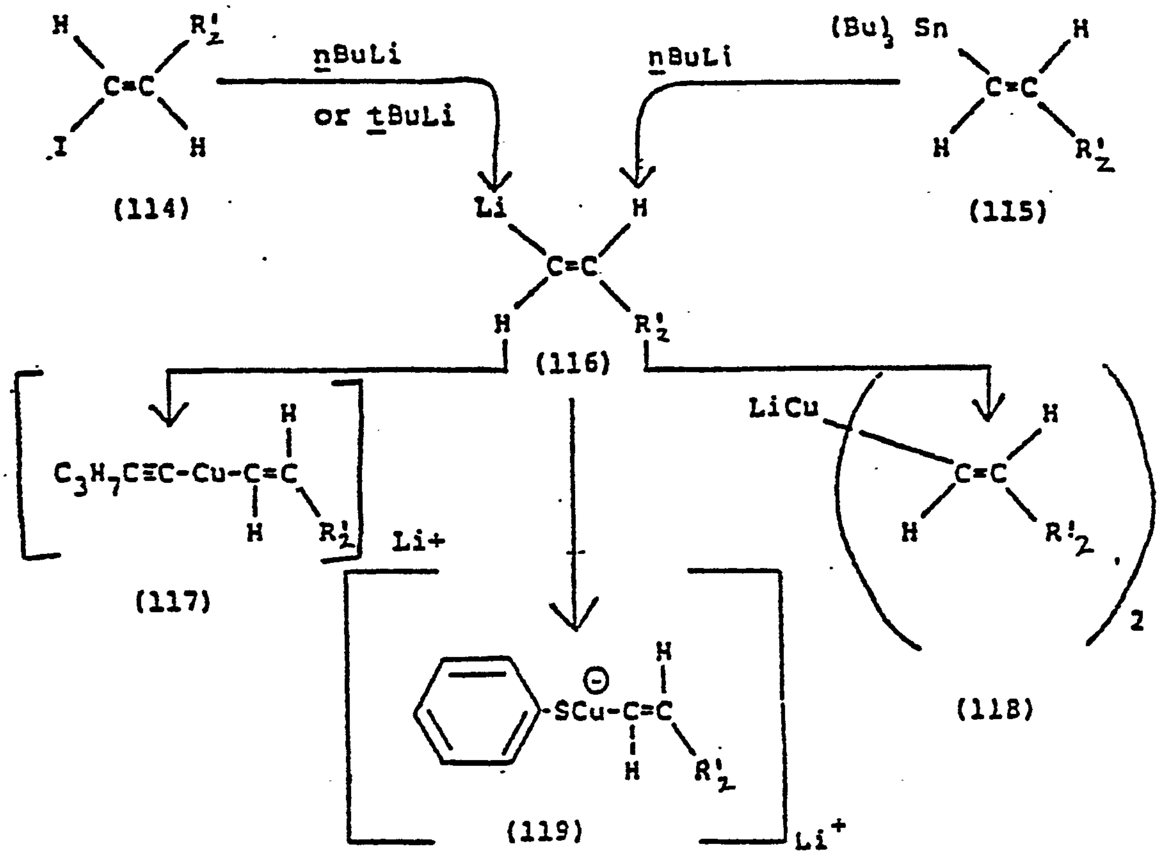


(114) (115) (116) (117) (118) (119)

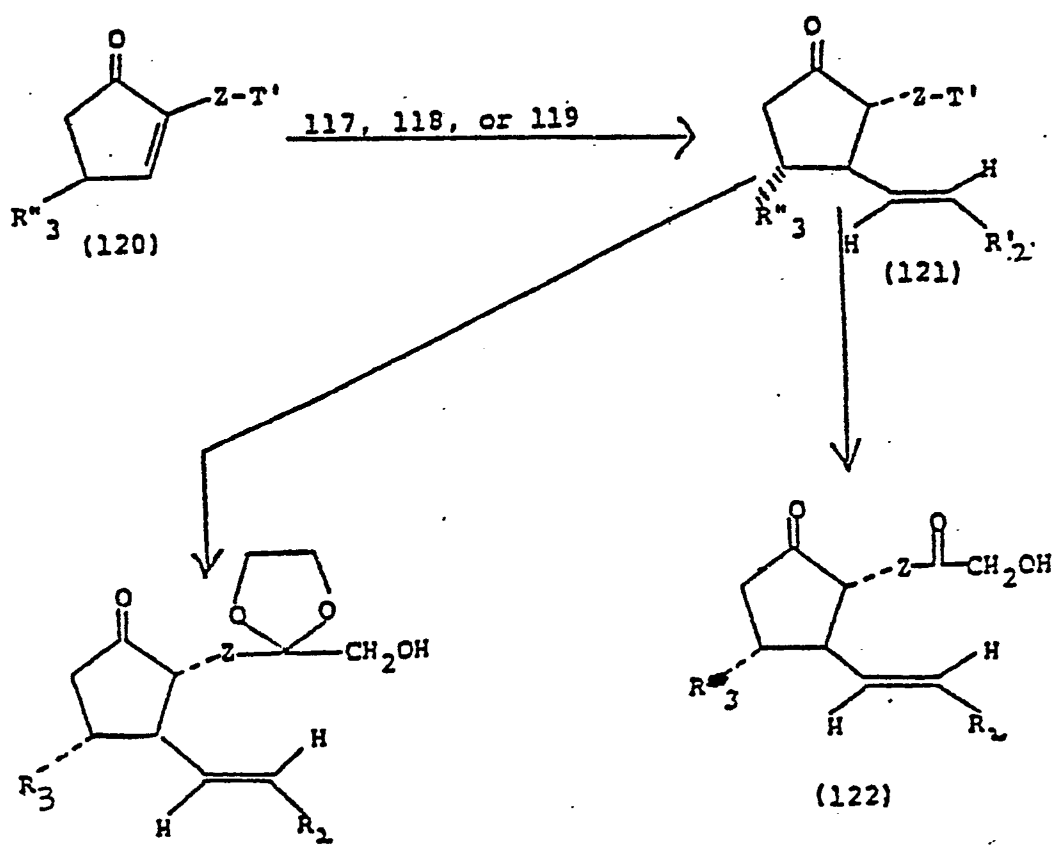


(120) (121) (122) (123)

When T′ is

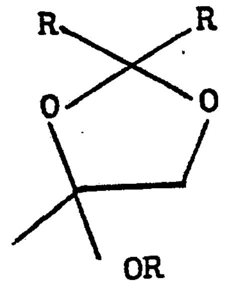

removal of the blocking groups from (121) to give the prostaglandin congener (122) is accomplished by treatment of (121) with a mixture of acetic acid, tetrahydrofuran and water (4:2:1) at 25° to 55°C.

When T′ is

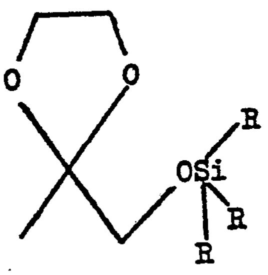

(121) can be partially deblocked to give ketal (123) by treatment of (121) with 0.6N hydrochloric acid in tetrahydrofuran at room temperature for 4 to 7 hours.

In certain cases it is possible to convert the carboxylic acid function of a prostaglandin congener into a terminal hydroxymethyl ketone function as shown in Flowsheet O hereinbelow wherein Z, $C_{13}$—$C_{14}$, $R_2'$ and $R_2$ is as hereinabove defined. Treatment of a prostaglandin congener (124) in which the 11-hydroxy group and the hydroxy groups of the β-chain are protected with a suitable group such as acetate or a dimethyl-t-butylsilyl ether, with oxalyl chloride in benzene for 2 to 5 hours furnishes the acid chloride (125), wherein $R_3''$ is hydrogen or a protected hydroxy group. The protected 11-hydroxyl-acid chloride compounds may be prepared in the manner described above in Flowsheet K.

The prostaglandin congeners (124) may be prepared by the 1,4 conjugate addition of the suitably protected cyclopentenones (76) or (87) such as cyclopentenone (88) and the lithiocuprate (117), (118) or (119) by the procedures disclosed herein by the examples and Flowsheet N. Addition of the acid chloride (125), dissolved in ether, to an ether solution of at least three equivalents of diazomethane gives the diazoketone (126). Hydrolysis of the diazoketone using aqueous sulfuric acid and tetrahydrofuran at about 0—55°C gives the hydroxymethyl ketone analog (127). The acetate protecting group can be removed by refluxing with acidified methanol. The dimethyl-*t*-butylsilyl ether protecting group can be removed by treatment with aqueous hydrochloric acid in tetrahydrofuran at 25 to 60°C.

## Flowsheet O

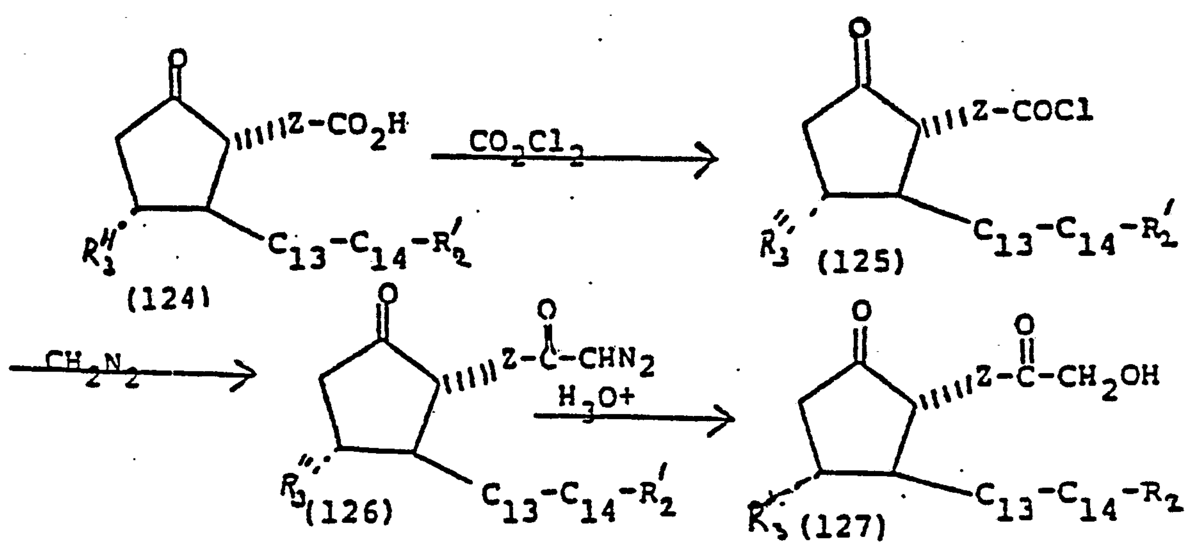


(124) (125) (126) (127)

When the compounds of this invention are prepared from racemic starting compounds two racemates are obtained. In appropriate instances these racemates can be separated from each other by careful application of the usual chromatogrpahic procedures. In the more difficult instances it may be necessary to apply high pressure liquid chromatography including recycling techniques. (See G. Fallick, *American Laboratory,* 19—27 (August, 1973) as well as references cited therein. Additional information concerning high speed liquid chromatography and the instruments necessary for its application is available from Waters Associate, Inc., Maple Street, Milford, Mass.)

It is also possible to prepare the compounds of this invention in their optically active forms by the conversion of the optically active 4-hydroxycyclopent-2-en-1-one carboxylic acids (128) to the optically active protected hydroxy ketone analog (129) using the methods outlined hereabove in Flowsheet K.

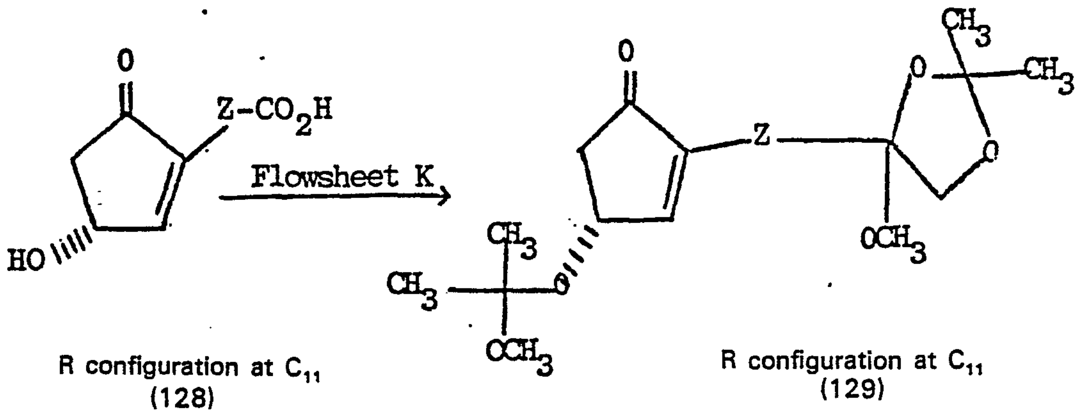


R configuration at $C_{11}$
(128)

R configuration at $C_{11}$
(129)

Conjugate addition of the vinyl cuprates to (129) followed by deblocking as described hereinabove in Flowsheet N then gives the compounds of this invention in their optically active forms. Although in some cases two diastereoisomers will be formed, each optically active, they can be separated by chromatographic procedures as described hereinabove.

In accordance with the following reaction scheme prostanoids are prepared as described by Stork in J.A.C.S. *97,* 4 (1975) and J.A.C.S. *97* 6260 (1975).

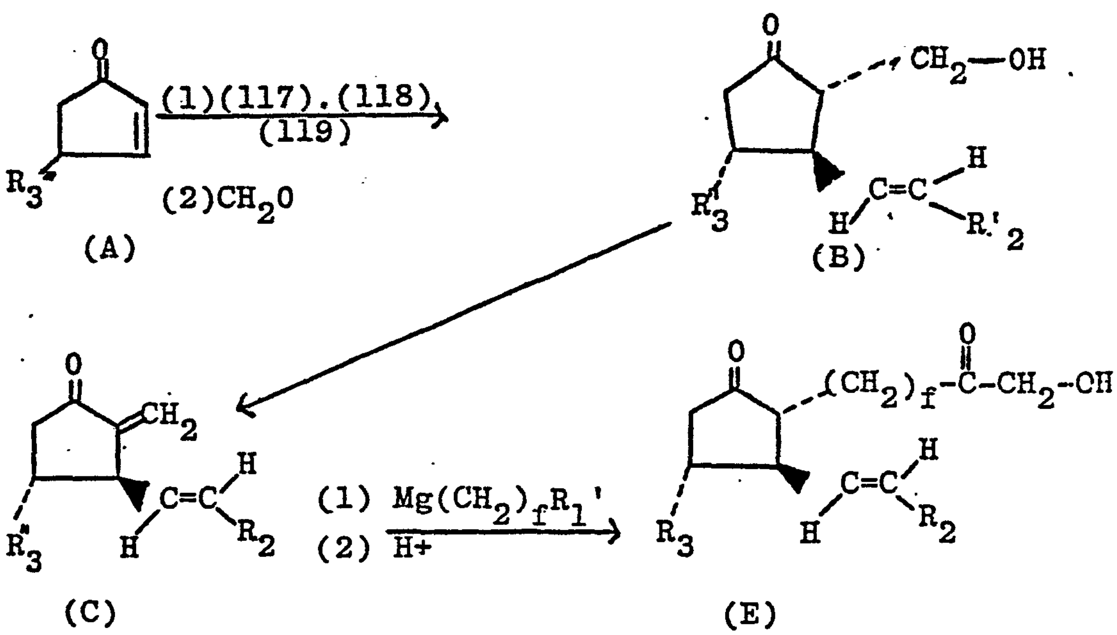


Treatment of the cyclopentenone (A) with a cuprate such as (117), (118) or (119) as hereinabove defined, followed by quenching with formaldehyde to provide the hydroxymethyl analog (B) which is dehydrated with reagents such as excess methansulfonyl chloride in pyridine followed by treatment of the crude mesylate with diissopropylethylamine in ether overnight to provide all the methylene cyclopentenone C, wherein $R_3''$, $R_3'''$ and $R_2'$ are as previously defined. Addition of the methylenecyclopentanone (C) to a solution of the Grignard $Mg(CH_2)_fR_1'$ wherein f is as previously defined and $R_1'$ is a substituent selected from the group $R_1$ wherein the hydroxyl group of the substituent is protected by a suitable blocking group such as 2-methoxypropyl-2-oxy, and a catalytic amount of $Bu_3P.CuI$ ($Bu_3$ is

tertiary butyl) followed by mild hydrolysis of the adduct provides the product prostanoid (E) wherein f, $R_3$ and $R_2$ are as previously defined.

The preparation of optically active 4-hydroxycyclopent-2-en-1-ones such as (128) is described hereinbelow.

The 4-hydroxycyclopentenone racemates may be resolved into their component enantiomers (130) and (131) by derivatizing the ketone function with a reagent having an optically active centre. The resulting diastereomeric mixture can then be separated by fractional crystallization, or by chromatography, or by high speed liquid chromatography involving, if necessary, recycling techniques. Among the useful optically active ketone derivatizing reagents are *1*-*α*-aminoxy-*γ*-methylpentanoic acid hydrochloride (to give (132)), (R)-2-aminoxy-3,3-dimethyl-butyric acid hydrochloride, and 4-*α*-methylbenzyl semicarbazide. After separation of the diastereomeric derivatives, reconstitution of the keto function provides the individual 4-hydroxycyclopentenone enantiomers (130) and (131). A useful procedure for the resolution of a 4-hydroxy-cyclopentenone racemate via an oxime such as (132) is described in the art (R. Pappo, P. Collins and C. Jung, *Tetrahedron Letters,* 943 (1973)).

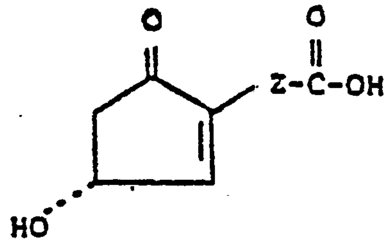

(130)

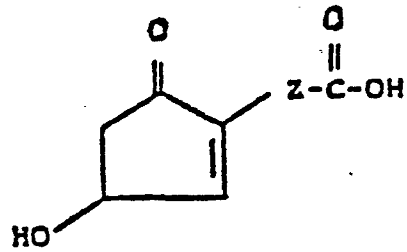

(131)

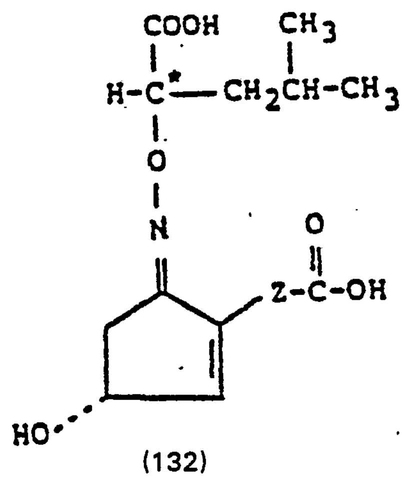

(132)

An alternate procedure for the preparation of the 4(R)-hydroxycyclopentenone enantiomers such as (130) involves as a key step the selective microbiological or chemical reduction of trione (133) to the 4(R)-hydroxycyclopentanedione (134). A wide variety of micro-organisms are capable of accomplishing this asymmetric reduction, one of the most useful being *Dipodascus unincleatus.* This step also can be achieved chemically by catalytic hydrogenation in the usual manner (for example, under about one atmosphere of hydrogen in methanol) using a soluble rhodium catalyst with chiral phosphine ligands, such as (1,5-cyclooctadiene)-*bis*-(*o*-anisylcyclohexylmethylphosphine)rhodium (I) tetrafluoroborate in the presence of one equivalent of organic base, such as triethylamine.

Conversion of hydroxycyclopentanedione (134) to an enol ether or enol ester, (135, E. = alkyl, preferably *iso*-propyl; aroyl such as benzoyl; or arylsulfonyl such as 2-mesitylenesulfonyl), is accomplished by treatment, for example, with isopropyl iodide and a base such as potassium carbonate in refluxing acetone for from 15 to 20 hours, or with a base such as triethylamine and 0.95 equivalents of benzoyl chloride or a slight excess of 2-mesitylenesulfonyl chloride, in a non-prototropic solvent at a temperature of about −10° to −15°C. Reduction of (135) with excess sodium bis (2-methoxyethoxy)aluminum hydride in a solvent such as tetrahydrofuran or toluene at low temperatures, such as −60° to −78°C, followed by mild acid hydrolysis (representative conditions: aqueous dilute hydrochloric acid, pH 2.5; or oxalic acid, sodium oxalate in chloroform) at ambient temperatures from 1 to 3 hours provides the 4(R)-hydroxycyclopentenone ester (136). The ester (136), can then by hydrolized to acid (130).

For a description of these procedures in the art see: C. J. Sih, *et al, J. A. C. S., 95,* 1676 (1973): J. B. Heather, *et al., Tetrahedron Letters,* 2213 (1973); R. Pappo and P. W. Collins, *Tetrahedron Letters,* 2627 (1972); and R. Pappo, P. Collins, and C. Jung, *Ann. N. Y. Acad. Sci., 180,* 64 (1971).

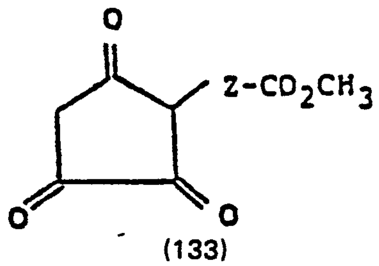

(133)

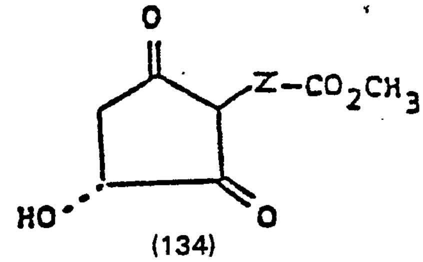

(134)

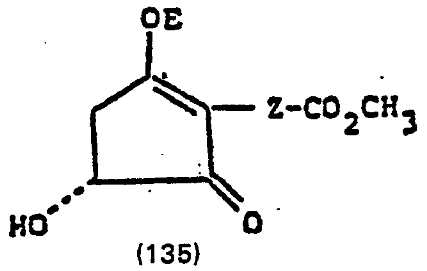

(135)

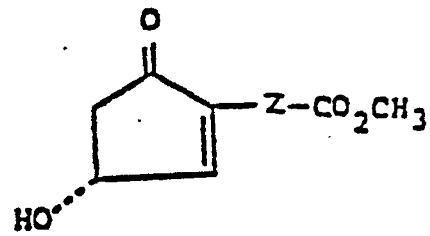

(136)

Procedures for the preparation of the requisite cyclopentanetriones (133) are well-established in the art and generally involve the treatment of an ester (137) with methyl or ethyl oxalate and a base such as sodium methoxide in methanol, followed by treatment with dilute hydrochloric acid in aqueous methanol to effect the dealkoxalylation of the intermediate (138). See J. Kutsube and M. Matsui, *Agr. Biol. Chem., 33* 1078 (1969): P. Collins, C. J. Jung and R. Pappo, *Israel Journal of Chemistry, 6,* 839 (1968); R. Pappo, P. Colins and C. Jung, *Ann. N. Y. Acad. Sci., 180,* 64 (1971); C. J. Sih, *et al., J. A. C. S., 95,* 1676 (1973) (See reference 7); and J. B. Heather, *et al., Tetrahedron Letters,* 2313 (1973) for pertinent background literature.

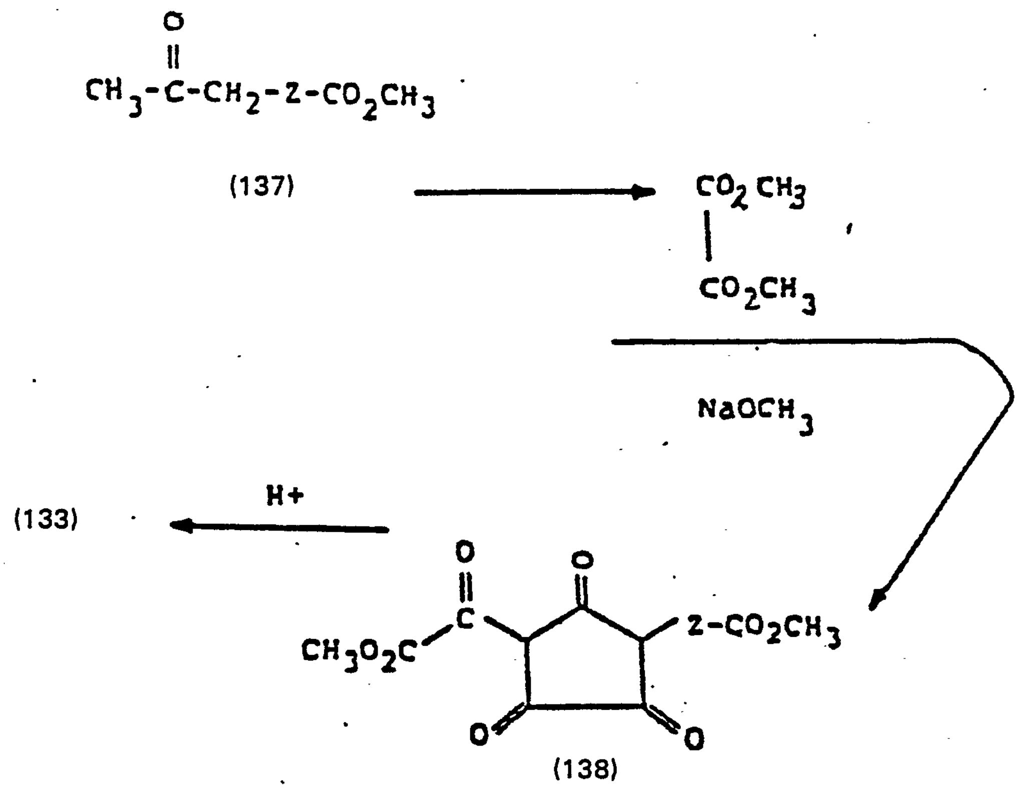


(137)

(133)

(138)

The intermediate keto esters (137) may be prepared by a variety of methods known to the art. One useful procedure is outlined below and involves alkylation of ethyl acetoacetate sodium salt (139) in the usual manner with the appropriate side-chain precursor (140, X = Cl, Br, I, preferably Br or I) followed by decarbethoxylation and reesterification, all in the usual manner.

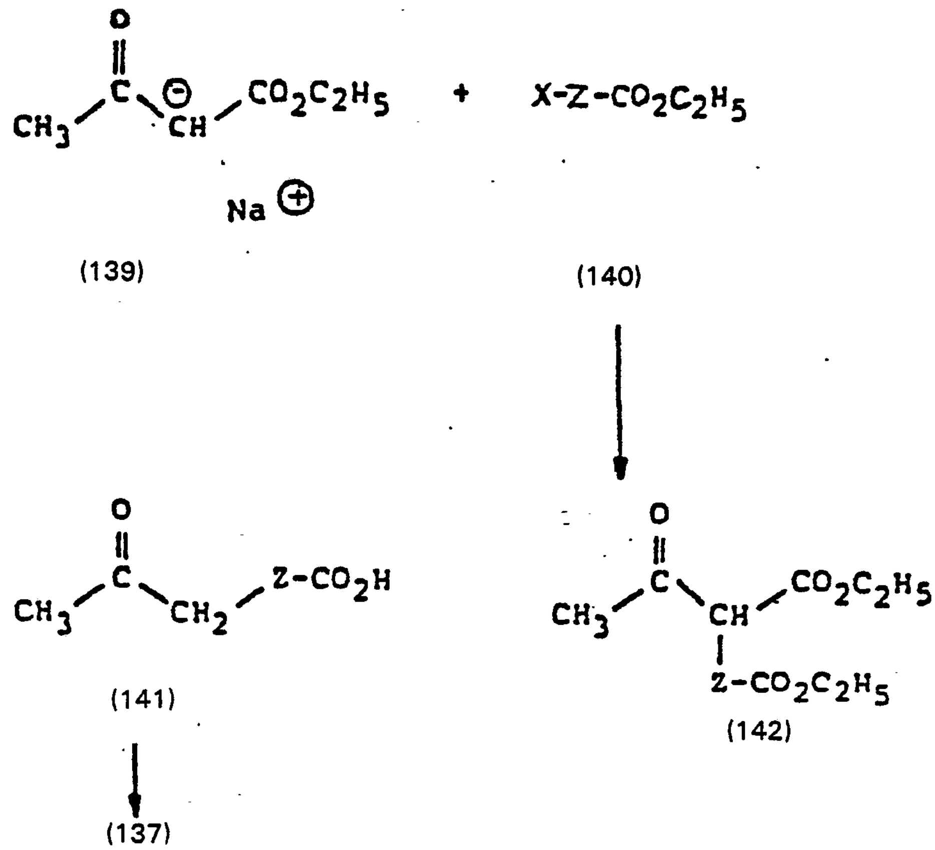

(139) (140)

(141) (142)

(137)

The side-chain precursors (140) are commercially available where Z is $-(CH_2)_4-$, and can be prepared as described in Belgian Patent 786,215 (granted and opened to inspection January 15, 1973).

It is also possible to resolve the 4-hydroxycyclopentenone racemate (146) by microbiological means. Thus, treatment of the 4-*O*-alkanoyl or aroyl derivatives (147, $R_{18}$ = aryl or alkyl) of racemate (146) (preferably the 4-*O*-acetyl and 4-*O*-propionyl derivatives) with an appropriate micro-organism, preferably a Saccharomyces species *e.g.,* 1375—143, affords preferential de-*O*-acylation of the 4(R)-enantiomer to give (148), which is then separated from the unreacted 4(S)-*O*-acyl enantiomer (149) by chromatographic procedures. After separation, mild hydrolysis of the 4(S) derivative (149) provides the 4(S)-hydroxycyclopentenone (150). (See N. J. Marsheck and M. Miyano, *Biochima et Biphysica Acta, 316,* 363 (1973) for related examples.)

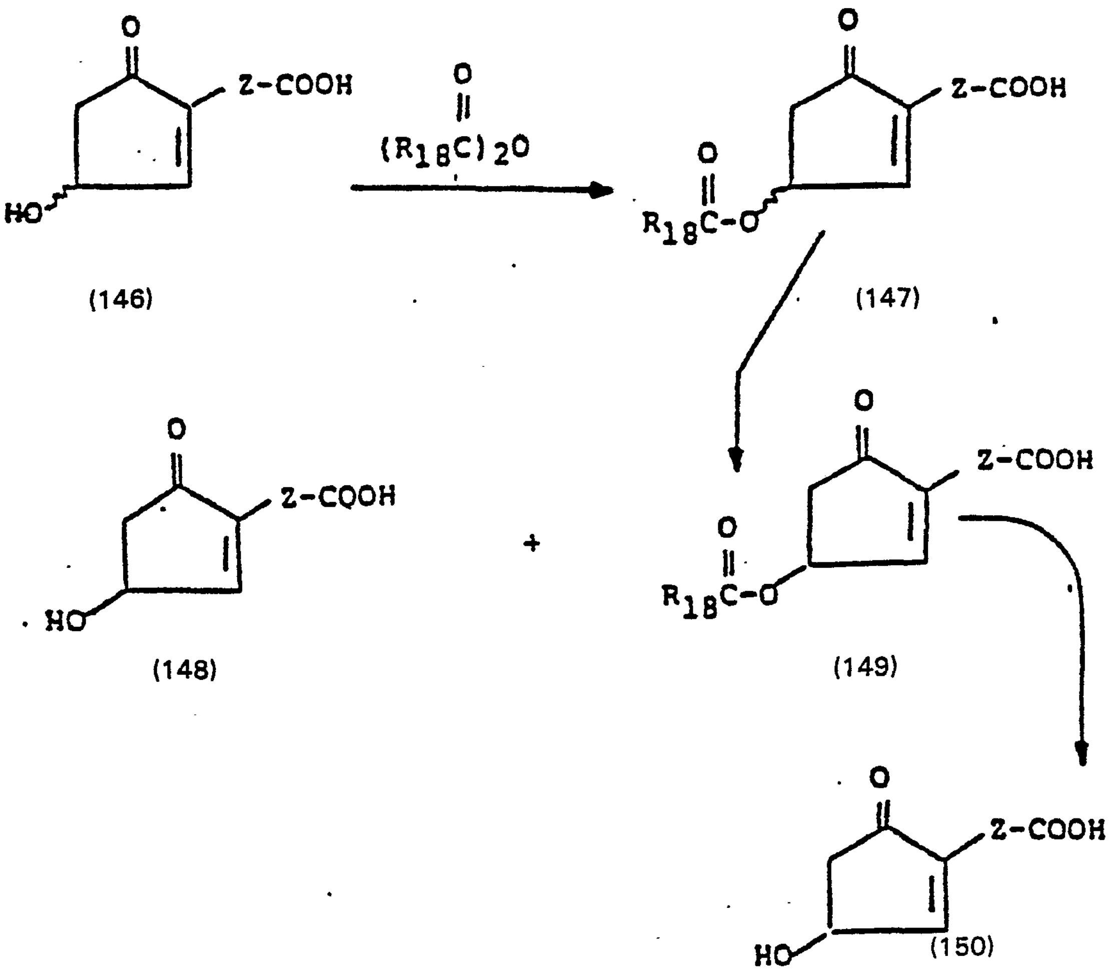


(146) (147)

(148) + (149)

(150)

It is also possible to prepare the individual 4-hydroxycyclopentenones (148) and (150) directly by selective microbial hydroxylations of the corresponding 4-unsubstituted cyclopentenone (151). For example, with *Aspergillus niger* ATCC 9142: a selective 4(R)-hydroxylation of (151, $Z = (CH_2)_6$) has been reported; See S. Kurozum, T. Tora and S. Ishimoto, *Tetrahedron Letters,* 4959 (1973). Other micro-organisms can also accomplish this hydroxylation.

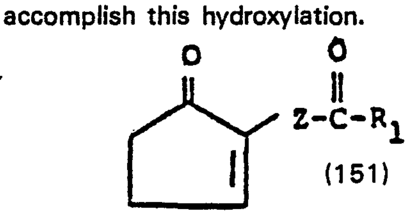

(151)

The ring system of the novel compounds of this invention allow them to be characterized as follows:

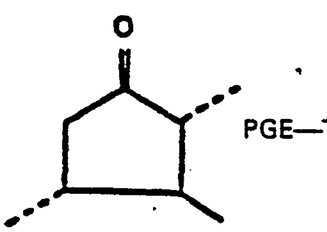

PGE—Type

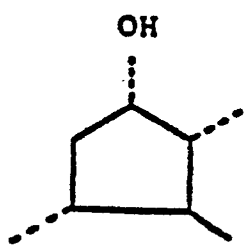

PGFα—Type

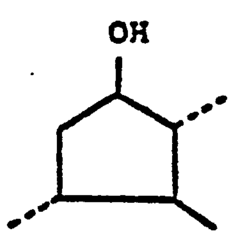

PGFβ—Type

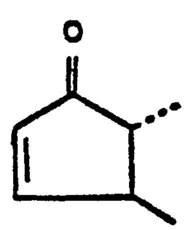

PGA—Type

The 9α-hydroxy PGF compounds of this invention (154) are prepared by a conjugate addition reaction as described hereinabove in Flowsheet N. The initial conjugate addition product (121) (wherein Z, T', $R_3''$ and R' are as hereinabove defined) is not deblocked but dissolved in tetrahydrofuran. An excess of lithium perhydro-9b-borophenalyhydride (PBPH) in tetrahydrofuran is added at −78°C. After warming to 0°C., the reaction mixture is quenched with saturated ammonium chloride solution. The product (153) is isolated and deblocked with acetic acid-tetrahydrofuran-water 4:2:1 at 40°C. in the cases where T' is

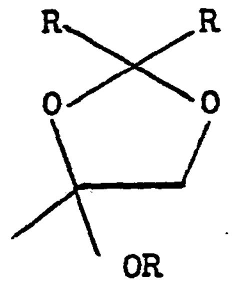

and with dilute hydrochloric acid in the cases where T' is

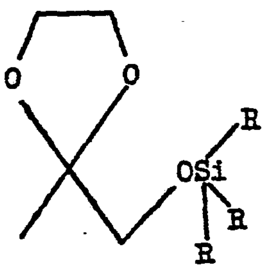

to give the 9α-hydroxy compounds of this invention (154). See Flowsheet P hereinbelow wherein Z, T', $R_3''$, $R_2$, R' and $R_3'''$ are as hereinabove defined.

FLOWSHEET P

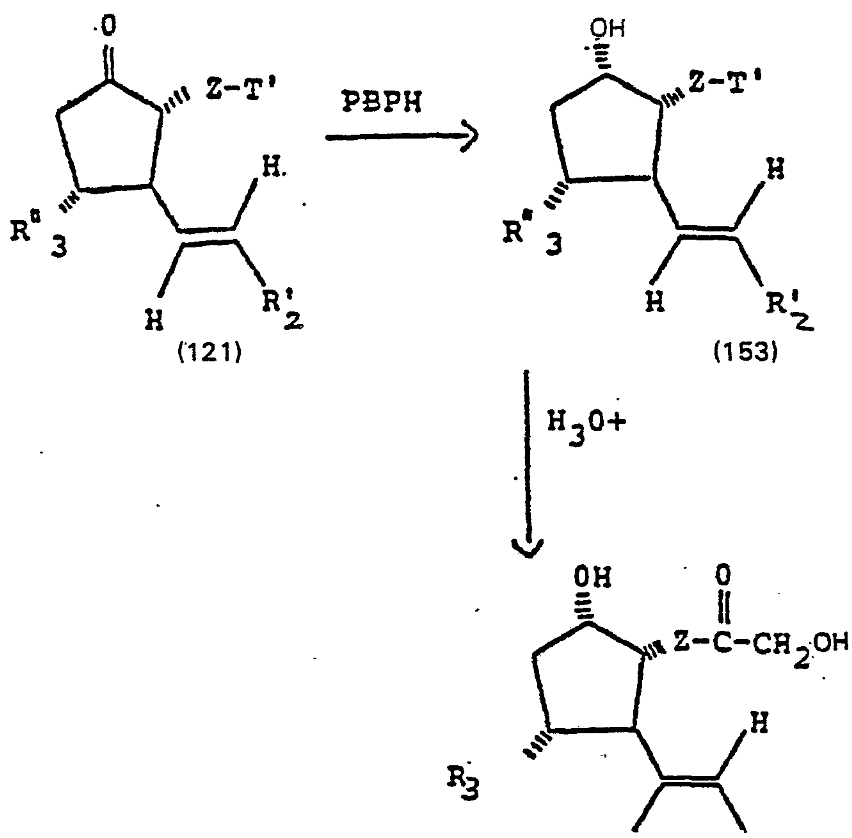


(121) (153) (154)

The 9β-hydroxy PGF compounds of this invention (156) are prepared by performing a conjugate addition as described hereinabove in Flowsheet N. The initial conjugate addition product (121) (wherein Z, T′, $R_3''$, and $R_2'$ are as hereinabove defined) is not deblocked but dissolved in ethanol and an excess of sodium borohydride is added. The mixture is stirred for 8 hours, poured into water and the reduced products (153) and (155) are obtained. These are deblocked with acetic acid-tetrahydrofuran-water 4:2:1 at 40°C. in the cases where T′ is

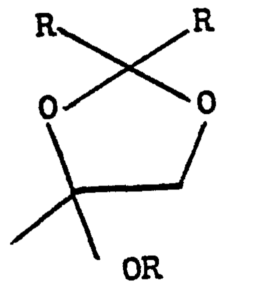

and with dilute hydrochloric acid in tetrahydrofuran in the cases where T′ is

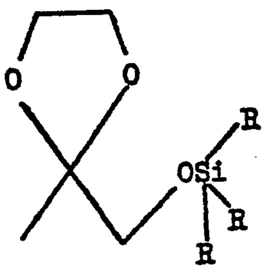

to give the 9α-hydroxy (154) and 9β-hydroxy (156) compounds of this invention which can be separated by silica gel chromatography. See Flowsheet Q hereinbelow wherein Z, T′, R, $R_2'$, $R_3''$, $R_2$ and $R_3$ are as defined hereinabove.

FLOWSHEET Q

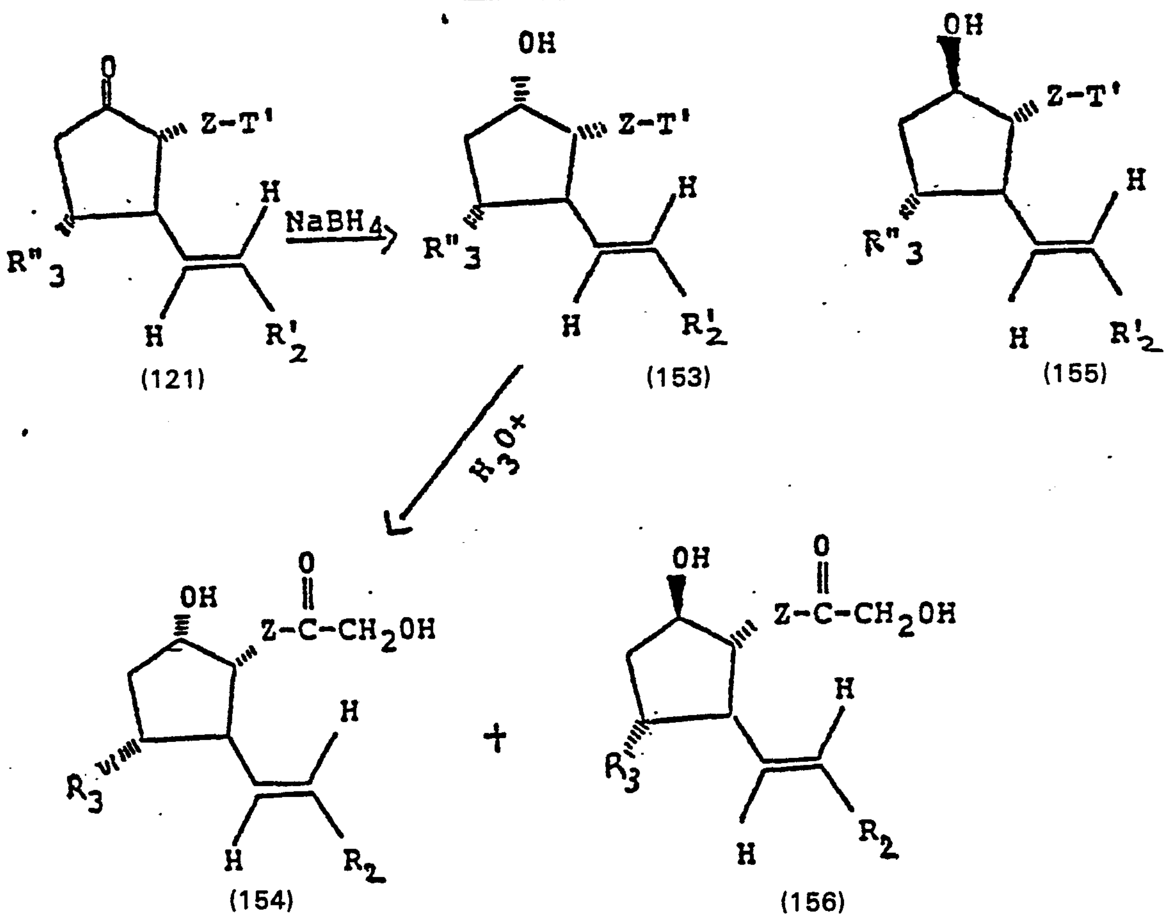


(121) (153) (155)

(154) + (156)

The 1-hydroxymethyl group of the PGF compounds of this invention can be selectively esterified by dissolving the compound (154) or 156) in pyridine and adding one equivalent of an anhydride $(R_{15}—CO)_2O$ or the acid chloride $R_{15}$

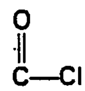

and allowing the mixture to stand overnight to give the desired esters (157 and 158).

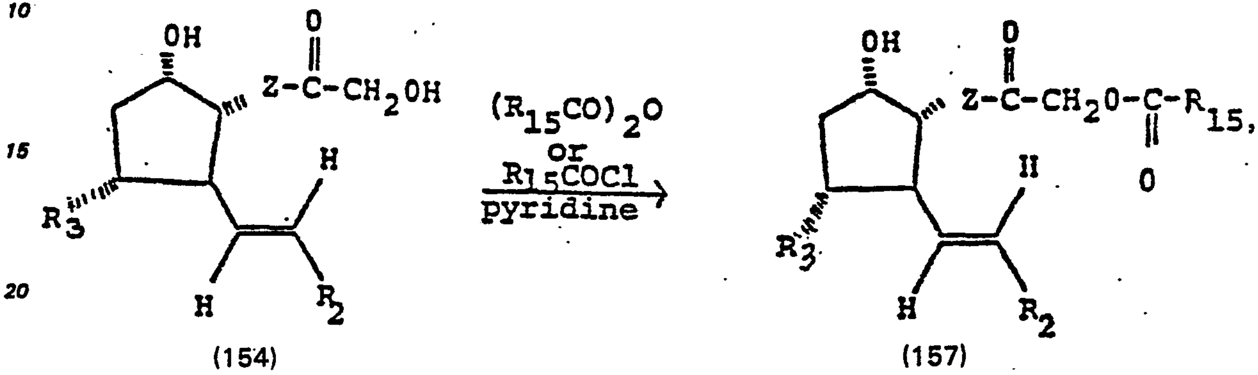


(154) (157)

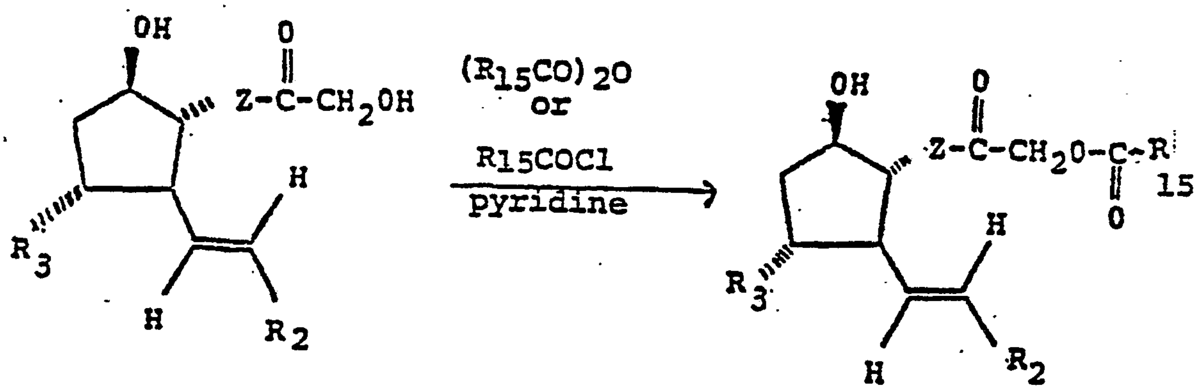


(156) (158)

$R_{15}$ is phenyl or phenyl substituted with one or more groups such as alkyl ($C_1$—$C_4$), OR, SR, F, Cl: dialkylamino; or $C_1$—$C_4$ alkyl, wherein R is $C_1$—$C_4$ alkyl. The E—Series compound (122) may also be esterified by this procedure.

FLOWSHEET R

PGA

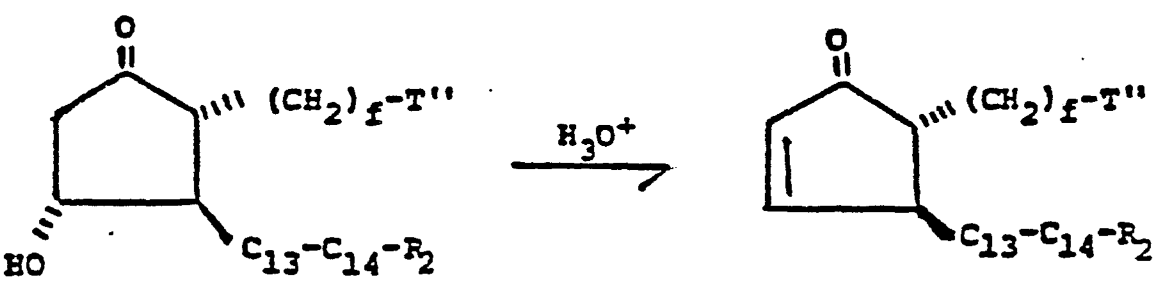


(159) (160)

The PGA, compounds of this invention (160) are prepared from the corresponding 11-hydroxy analogs (159) by treatment of (159) with dilute hydrochloric acid in tetrahydrofuran for 2 to 4 days at

room temperature as shown hereinabove in Flowsheet R, wherein $C_{13}$—$C_{14}$, $R_2$ and f are as hereinabove defined and T″ is a moiety selected from a group consisting of:

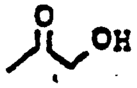

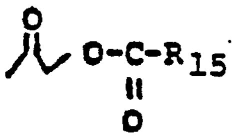

and

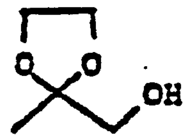

wherein $R_{15}$ is hereinabove defined.

As shown in Flowsheet S, hereinbelow, the compounds of this invention wherein T″ is

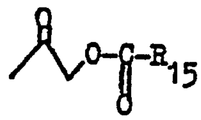

(162 can also be prepared by selective esterification of the terminal hydroxyl group of compound (161), which is also part of this invention.

This esterification can be accomplished by treatment of a pyridine solution of (161) with one equivalent of an anhydride (163a) or acid chloride (163b) at room temperature for 24 hours.

FLOWSHEET S

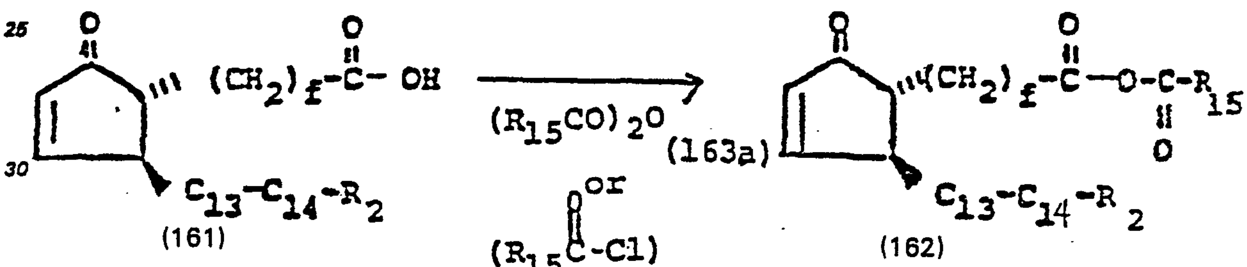


(161) (163b) (162)

FLOWSHEET T
$PGA_2$

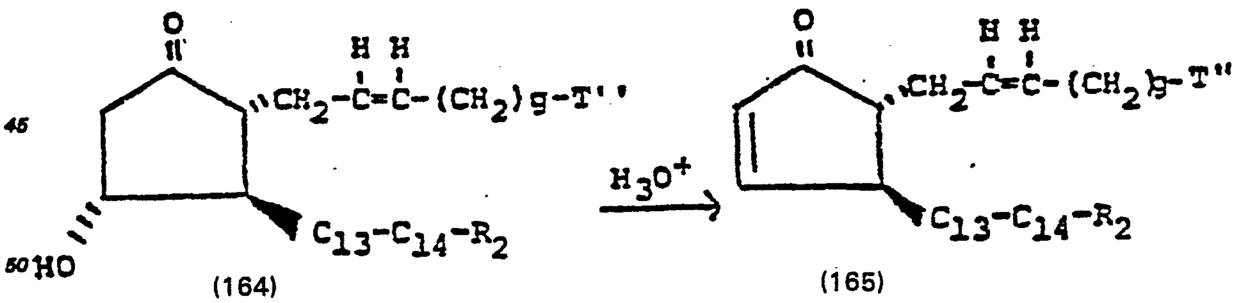


(164) (165)

The $PGA_2$ compounds of this invention (165) are prepared from the corresponding 11-hydroxy analog (164) by treatment of (164) with dilute hydrochloric acid in tetrahydrofuran for 2 to 4 days at room temperature as shown hereinabove in Flowsheet T wherein $C_{13}$—$C_{14}$, $R_2$ and g are as hereinabove defined and T″ is a moiety selected from a group consisting of:

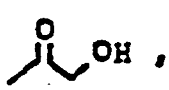

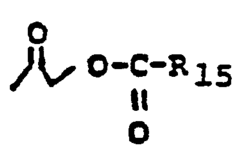

and

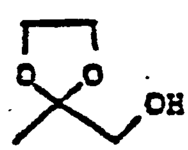

wherein $R_{15}$ is hereinabove defined.

As shown in Flowsheet U hereinbelow, the compounds of this invention wherein T″ is

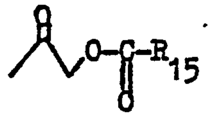

(167) can also be prepared by selective esterification of the terminal hydroxyl group of compound (166) which is also part of this invention.

This esterification can be accomplished by treatment of a pyridine solution of (166) with one equivalent of an anhydride (168a) or acid chloride (168b) at room temperature for 24 hours.

FLOWSHEET U

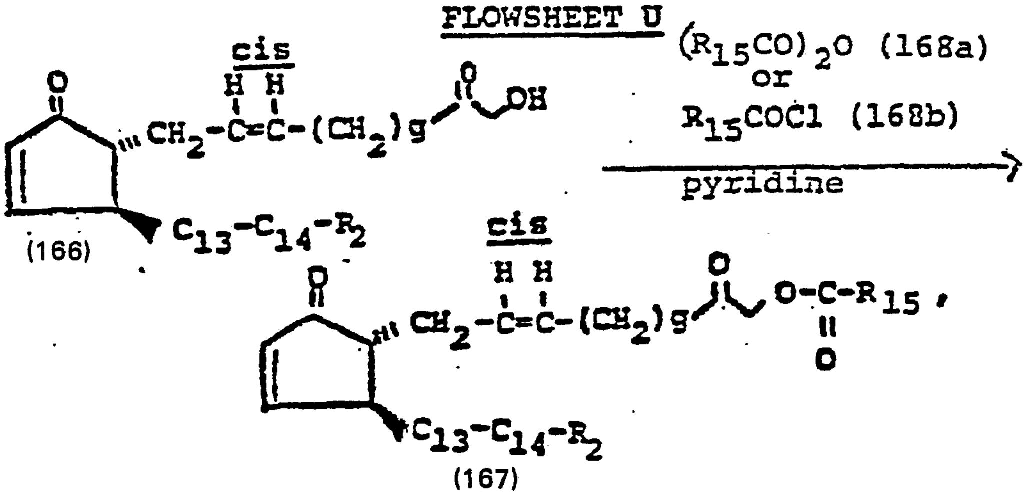


(166)

(167)

In accordance with the process of Bundy et al. J.A.C.S. *94,* 2123 (1972) or E. J. Corey J.O.C. *38,* 3187 (1973), the $PGA_1$, $PGA_0$ or $PGA_2$ series compounds of this invention may be converted to the corresponding $PGE_0$, $PGE_1$ or $PGE_2$ compound.

This conversion is accomplished by treating either the protected or unprotected PGA compounds (i.e. 160 or 165) with alkaline hydrogen peroxide to provide a mixture of isomeric 10,11-epoxides which, without separation is reduced with chromous acetate in acidic acid or by aluminum amalgam to provide after hydrolysis (if necessary) and silica gel chromatography, the 11$\alpha$-hydroxy-PGE compounds and a lesser amount of the corresponding 11$\beta$-epimer.

The novel compounds of the present invention have potential utility as hypotensive agents, antiulcer agents for the treatment of gastric hypersecretion and gastric erosion, agents to provide protection against the ulcerogenic and other gastric difficulties associated with the use of various non-steroidal anti-inflammatory agents (e.g., indomethacin, aspirin, and phenylbutazone), bronchodilators, anti-inflammatory agents, abortifacients, agents for the induction of labour, agents for the induction of menses, fertility-controlling agents, oestrus regulators for use in animal husbandry with cattle and other domestic animals and central nervous system regulatory agents. Certain of the novel compounds of this invention possess utility as intermediates for the preparation of the other novel compounds of this invention.

The novel compounds of this invention possess the pharmacological activity described below as associated with the appropriate above-described prostaglandin types.

The known PGE, PGF$\alpha$, PGF$\beta$ and PGA compounds are all potent in causing multiple biological responses even at low doses. For example, $PGE_1$, $PGE_2$, $PGA_1$ and $PGA_2$ are extremely potent in causing vasodepression and smooth muscle stimulation, and also are potent as antilipolytic agents. Moreover, for many applications, these known prostaglandins have an inconveniently short duration of biological activity. In striking contrast, preferred prostaglandin analogs of this invention are substantially more specific with regard to potency in causing prostaglandin-like biological responses, and/or having a susbtantially longer duration of biological activity. For example, the 11-deoxy-PGE compounds of this invention are selective in that they are at most relatively weak stimulants of smooth muscle. A further advantage of these novel compounds lies in their increased stabilities and lengthened shelf-lives.

Another advantage of novel compounds of this invention, compared with the known prostaglandins, is that the compounds may be administered effectively orally, sublingually, intravaginally, buccally, or rectally, in addition to the usual intravenous, intramuscular, or subcutaneous injection or infusion methods indicated above for the uses of the known prostaglandins. These qualities are advantageous because they facilitate maintaining uniform levels of these compounds in the body with fewer, shorter or smaller doses, and make possible self-administration by the patient.

$PGE_1$, $PGE_2$, $PGE_3$, dihydro-$PGE_1$, PGF$\alpha$, PGF$\beta$ and PGA compounds, their esters and pharmacologically acceptable salts, are extremely potent in causing various biological responses. For that reason, these compounds are useful for pharmacological purposes. See, for example, Bergstrome, *et al.,*

*Pharmacol. Rev. 20,* 1 (1968), and references cited herein. A few of those biological responses are systemic arterial blood pressure lowering in the case of the PGA and PGE compounds as measured, for example in anaesthetized (phenobarbital sodium) pentolinium-treated rats with indwelling aortic and right heart cannulas; pressor activity, similarly measured, for the PGF compounds; stimulation of smooth muscle as shown, for example, by tests on strips of guinea pig ileum, rabbit duodenum, of gerbil colon; potentiation of other smooth muscle stimulants; antilipolytic activity as shown by antagonism of epinephrine-induced mobilization of free fatty acids or inhibition of the spontaneous release of glycerol from isolated rat fat pads; inhibition of gastric secretion in the case of PGE compounds, as shown in dogs with secretion stimulated by food or histamine infusion; activity on the central nervous system; decrease of blood platelet adhesiveness in the case of PGE, as shown by platelet-to-glass adhesiveness, and inhibition of blood platelet aggregation and thrombus formation induced by various physical stimuli, e.g., arterial injury, and various biochemical stimuli, e.g. ADP, ATP, serotonin, thrombin, and collagen, and in the case of the PGE and PGA compounds, stimulation of epidermal proliferation and keratinization, as shown when they are applied in culture to embryonic chick and rat skin segments.

Because of these biological responses, these known prostaglandins are useful to study, prevent, control or alleviate a wide variety of disease and undesirable physiological conditions in birds and mammals including humans, useful domestic animals, pets, and zoological specimens, and in laboratory animals, e.g. mice, rats, rabbits and monkeys.

For example, these compounds are useful in mammals, including man, as nasal decongestants. For this purpose, the compounds are used in a dose range of about 10 $\mu$g to about 10 mg per ml of a pharmacologically suitable liquid vehicle or as an aerosol spray, both for topical application.

PGA, PGF$\alpha$ and PGE compounds are useful as hypotensive agents to reduce blood pressure in mammals including man. For this purpose, the PGF compounds are administered by intravenous infusion at the rate of about 0.01 mg to about 40 mg per Kg of body weight per minute, or in a single dosage or multiple doses of about 25 mg to 2500 mg per Kg of body weight total per day. The PGE and PGA compounds are administered by intravenous infusion at the rate of about 0.01 to about 50 mg per Kg of body weight per minute, or in a single dose of multiple doses of about 25 to 2500 mg per Kg of body weight total per day.

The PGE, PGF$\alpha$ and PGF$\beta$ compounds are useful in place of oxytocin to induce labour in pregnant female animals, including humans, cows, sheep and pigs, at or near term or in pregnant animals with intrauterine death of the foetus from about 20 weeks to term. For this purpose, the PGF compound is infused itnravenously at a dose of 0.01 mg to 50 mg per Kg of body weight per minute until or near the termination of the second stage of labour, i.e. expulsion of the foetus. Similarly, the PGE compound is infused intravenously at a dose of 0.01 to 50 mg per Kg of body weight per minute until or near the expulsion of the foetus. These compounds are especially useful when the female is one or more weeks postmature and natural labour has not started, or 12 to 60 hours after the membranes have ruptured and natural labour has not yet started.

The PGE, PGF$\alpha$ and PFG$\beta$ compounds are useful for controlling the reproductive cycle in ovulating female mammals, including humans and other animals. For that purpose, PGF$_2\alpha$, for example, is administered systemically at a dose level in the range of 0.01 mg to about 20 mg per Kg of body weight, advantageously during a span of time starting approximately at the time of ovulation and ending approximately at the time of menses or just prior to menses. Likewise, a PGE compound is administered in the same fashion at a dose level of 0.01 mg to about 50 mg per Kg of body weight. Additionally, expulsion of an embryo or foetus is accomplished by similar administration of the compound during the first third or the second third of the normal mammalian gestation period. Accordingly, such compounds are useful as abortifacients. They are also useful for induction of menses during approximately the first two weeks of a missed menstrual period and thus, are useful as contraceptive anti-fertility agents.

PGE compounds are extremely potent in causing stimulation of smooth muscle, and are also highly active in potentiating other known smooth muscle stimulators, for example, oxytocic agents, e.g., oxytocin, and the various ergot alkaloids including derivatives and analogs thereof. Therefore PGE$_2$, for example, is useful in place of or in combination with less than usual amounts of these known smooth muscle stimulators for example, to relieve the symptoms of parlytic ileus, to control or prevent uterine bleeding after abortion or delivery, to aid in expulsion of the placenta, and during the puerperium. For the latter purpose, the PGE compound is administered by intravenous infusion immediately after abortion or delivery at a dose in the range about 0.01 to about 50 mg per Kg of body weight per minute until the desired effect is obtained. Subsequent doses are given by intravenous, subcutaneous, or intramuscular injection or infusion during puerperium in the range of 0.01 to 2 mg per Kg of body weight per day, the exact dose depending on the age, weight, and condition of the patient or animal.

The novel PGA, PGE and PGF$\beta$ of this invention are also useful as bronchodilators for the treatment of asthma and chronic bronchitis. As such they may be conveniently administered by inhalation of aerosol sprays prepared in a dose range of about 10 $\mu$g to about 10 mg/ml of a pharmacologically suitable liquid vehicle. Relative to the natural prostaglandins, the PGA and PGE compounds in particular have the significant advantage of inducing prolonged effects.

The PGE and PGA compounds are also useful in mammals, including man and certain useful animals, e.g., dogs and pigs, to reduce and control excessive gastric secretion, thereby reducing or avoiding gastric erosion or gastrointestinal ulcer formation, and accelerating the healing of such ulcers already present in the gastrointestinal tract. For this purpose, the compounds are injected or infused intravenously, subcutaneously, or intramuscularly in an infusion dose range of about 0.1 mg. to about 500 mg per Kg of body weight per minute, or in a total daily dose by injection or infusion in the range of about 0.1 to about 20 mg per Kg of body weight per day, the exact dose depending on the age, weight, and condition of the patient or animal, and on the frequency and route of administration. These compounds may also be useful in conjunction with various non-steroidal anti-inflammatory agents, such as asprin, phenylbutazone, indomethacin and the like, to minimize the well-known ulcerogenic effects of the latter.

The PGE and PGA compounds also stimulate epidermal proliferation and keratinization, and in such a capacity are useful to promote and accelerate healing of skin which has been damaged, for example, by burns, wounds, abrasions or surgery. The nasopressor action of the PGA compounds makes them particularly useful in speeding the adherence and growth of skin autografts, especially small, deep (Davies) grafts which are intended to cover skinless areas by subsequent outward growth rather than initially, and in retarding rejection of homografts.

For these purposes, these compounds are preferably administered topically at or near the site where cell growth and keratin formation is desired, advantageously as an aerosol liquid or micronized powder spray, as an isotonic aqueous solution in the case of wet dressings, or as a lotion cream, or ointment in combination with the usual pharmaceutically acceptable diluents. In some instances, when there is substantial fluid loss as in the case of extensive burns or skin loss due to other causes, systemic administration of PGE is advantageous, for example, by intravenous injection or infusion, separate or in combination with the usual infusions of blood, plasma, or substitutes thereof. Alternative routes of administration are subcutaneous or intramuscular near the site, oral, sublinqual, buccal, rectal, or vaginal. The exact dose depends on such factors as the route of administration, and the age, weight, and condition of the subject. Illustrative of a wet dressing for topical application to second and/or third degree burns of skin area 5 to 25 square centimeters is the use of an isotonic aqueous solution containing one to 500 mg/ml of the PGA compound or several times that concentration of the PGE compound. Especially for topical use, these prostaglandins are useful in combination with antibiotics such as gentamycin, neomycin, polymyxin $\beta$, bacitracin, spectinomycin, and oxytetracycline; with other antibacterials such as mafenide hydrochloride, sulfadiazine, furazolium chloride, and nitrofurazone; and with corticoid steroids, such as hydrocortisone, prednisolone, methylprednisolone, and fluoroprednisolone; each of those being used in the combination at the usual concentration suitable for its use alone.

The PGA compounds and derivatives and salts thereof increase the flow of blood in the mammalian kidney, thereby increasing the volume and electrolyte content of the urine. For that reason, PGA compounds are useful in managing cases of renal disfunction, especially in cases of severely impaired renal blood flow, for example, the hepatorena syndrome and early kidney transplant rejection. In case of excessive or inappropriate antidiuretic hormone ADH vasopressin secretion, the diuretic effect of these compounds is even greater. In anephretic states, the vasopressin action of these compounds is especially useful.

The PGE compounds of this invention are also useful as topical vasodilators.

The $PGE_1$ compounds of this invention are useful whenever it is desired to inhibit platelet aggregation, to reduce the adhesive character of platelets, and to remove or prevent the formation of thrombi in mammals including man, rabbits, and rats. For example, these compounds are useful to treat and prevent myocardial infarcts and post-operative thrombosis. For these purposes, these compounds are administered systemically, e.g., intravenously, subcutaneously, intramuscularly, and in the form of sterile implants for prolonged action. For rapid response, especially in emergency situations, the intravenous route of administration is preferred. Doses in the range of about 0.005 to about 20 mg per Kg of body weight per day are used, the exact dose depending on the age, weight, and condition of the patient or animal, and on the frequency and route of administration.

It is well known that platelet aggregation inhibitors may be useful as anti-thrombotic drugs. Inhibition of platelet aggregation can be conveniently measured *in vitro* by monitoring changes in optical density and/or light transmission in platelet rich plasma upon addition of suitable aggregating agents such as adenosine diphosphate, epinephrine, thrombin or collagen. Alternatively, platelet aggregation can be measured *in vitro* using platelet rich plasma obtained at various time intervals from animals given inhibitors by an oral or parenteral route.

The PGE compounds of the present invention exhibit the ability to inhibit platelet aggregation *in vitro* when tested by the following procedure.

Human protein rich plasma is incubated with modified Tyrode's solution in a proportion of 40—50% human protein rich plasma. The test compounds are added at varying concentrations and after 5 minutes incubation, an aggregating agent such as adenosine diphosphate or collagen is added. The change in optical density (light transmission) is monitored by eye and inhibition is recorded as a (—) or lack of inhibition is recorded as a (+). Test compounds are considered active if they inhibit adenosine

diphosphate or collagen induced aggregation at a concentration of 0.025 mg/ml or less within 5—10 minutes. For example, a compound of this invention, 1,9-dioxo-15-hydroxy-1-hydroxy-methyl-13-*trans*-prostene, shows inhibition of platelet aggregation induced by both adenosine diphosphate and collagen at a concentration of 0.025 mg/ml.

The PGE compounds of this invention also have bronchodilator activity as determined in a test using dogs anaesthetized, artificially ventilated and submitted to a continuous respiratory spasm induced by pilocarpine.

Mongrel dogs of either sex weighing between 5 and 10 kg are used. They are premedicated with morphine HCl by subcutaneous injection at 1.5 mg/Kg. An intravenous perfusion of 5%(W/V) chloralose is started 1/2 hour after the morphine injection in such a way that 60 mg/Kg are administered within 15 minutes. After completion perfusion of 10 mg/Kg/hour is maintained throughout the experiment. The dogs are artificially ventilated by means of a Starling pump at a rate of 20 breaths/minute. The volume is adjusted according to the weight of the animal. (Kleinman and Radford, *J. Appl. Physiol., 19,* 360 (1964)). All the measurements are made with the dogs positioned supine in a heated, V-shaped table. Curarization is obtained by succinylcholine chloride using a starting injection of 3 mg/Kg lasting 3 minutes, followed by a continuous perfusion of 0.1 mg/Kg/minute.

The respiratory spasm is induced by a starting injection of 400 mcg/Kg of pilocarpine HCl lasting 5 minutes. An increase or decrease in the dose of pilocarpine HCl may occur as a function of the observed effect on the airway's resistance. A 15 minute delay is observed before the start of a continuous perfusion of pilocarpine HCl at a dose of 4 mcg/Kg/minute to maintain a constant spasm during the test.

A metallic cannula is inserted and fixed, after tracheotomy, into the upper part of the trachea. The two cephalic veins and the two femoral veins are catheterized to inject the various agents. The femoral artery is catheterized to measure the systemic blood pressure. An esophageal balloon (11 cm × 2.5 cm) is inserted into the lower third of the oesophagus to measure the endothoracic pressure. The measurement of air flow is made with a Fleish pneumotachograph connected to the tracheal tube.

The transpulmonary pressure is measured as follows: the tracheal cannula is equipped with a stainless steel axial tube (1.5 mm) which is closed at its distal end and projected 2.5 cm beyond the end of the cannula. Three holes with a diameter of one mm are pierced on this latter segment. This tube, which is used to measure the tracheal pressure, is connected to one of the two chambers of a Sanborn 267 B/C differential transducer. The other chamber is connected to the esophageal balloon by means of a polyethylene catheter of the same length and characteristics as the balloon's.

The airflow is measured from the Fleish pneumotachograph by means of a Sanborn 270 differential transducer.

The tidal volume is obtained by electronic integration of the flow signal using an R.C. integrator.

The systemic and pulmonary blood pressures are gauged by means of a Sanborn 267 B/C or 1280B pressure transducer.

An electrocardiogram is taken in lead 2. Its use is to monitor a cardiac rate-meter.

All these parameters are recorded on a Sanborn polygraph. The transpulmonary pressure and the tidal volume are also displayed as rectangular co-ordinates on an oscilloscope.

The airway's resistance, expressed in cm of water/litre/second, is measured by subtracting from the electrical equivalent of the transpulmonary pressure, a voltage proportional to the flow so as to synchronize the pressure and volume signals on the oscilloscope (Mead and Whittenberger, *J. Appl. Physiol., 5,* 779 (1953)).

The value of the pulmonary elastance, expressed in cm of water/litre, is obtained by means of the same principal, i.e., an electrical signal proportioned to the volume is subtracted from the transpulmonary pressure signal, in order to optimize the pressure-flow loop on the oscilloscope.

The details of this method are described by Lulling, *et al.* (*Med. Pharmacol. Exp., 16,* 481 (1967)).

The computing operations are carried out with an analogical computer which allows the direct reading, cycle to cycle, of the values of resistance and elastance.

The test compounds are administered by an Aerosol route. The micronebulizer of a Bird Mark 7 respirator is fitted on the metallic cannula just after the pneumotachograph. The "puff" of the test compound, in Aerosol is driven by a 2 Kg/cm$^2$ pressure, allowed into the micronebulizer just during one inspiration cycle. The micronebulizer is fitted on the respiratory tube only during the "puff". It is weighed just before and just after administration to determine the amount of test compound administered.

The % inhibition of spasms indiced by pilocarpine for some of the compounds of this invention is shown below in Table A. Approximately 50 mg of the solution is administered to each dog.

The bronchodilator activity of some of the PGE compounds of this invention is determined in guinea pigs against bronchospasms elicited by intravenous injections of 5-hydroxy-tryptamine, histamine or acetylcholine by Konzett procedure. (See J. Lulling, P. Lievens, F. El Sayed and J. Prignot, *Arzneimittel-Forschung, 18,* 995 (1968)).

In the Table B which follows, bronchodilator activity for representative compounds of this invention against one or more of the three spasmogenic agents is expressed as an $ED_{50}$ determined from the results obtained with three logarithmic cumulative intravenous doses.

TABLE A

Bronchodilator Activity (Pilocarpine Assay) % Inhibition of Spasm

| | Dose mg/ml | % Inhibition of pilocarpine induced spasm as a function of the after drug administration | | |
|---|---|---|---|---|
| | | 5 min. | 30 min. | 60 min. |
| 1,9-dioxo-11α,16-dihydroxy-16-methyl-1-hydroxymethyl-5-*cis*-13-*trans* prostadiene | 3.2 | 90 | 90 | 70 |
| 1,9-dioxo-11α,16-dihydroxy-16-vinyl-1-hydroxymethyl-5-*cis*-13-*trans* prostadiene | 3.2 | 80 | 80 | 66 |
| 1,9-dioxo-11α,15-dihydroxy-16,16-trimethylene-1-hydroxymethyl-5-*cis*-13-*trans* prostadiene | 3.2 | 80 | 74 | 68 |
| 1,9-dioxo-15-hydroxy-15-methyl-1-hydroxymethyl-13-*trans* prostene | 3.2 | 37 | 30 | 30 |
| 1,9-dioxo-15-hydroxy-1-hydroxy-methyl prostane | 3.2 | 70 | 45 | 30 |
| 1,9-dioxo-11α,16-dihydroxy-16-vinyl-1-acetoxymethyl-5-*cis*-13-*trans* prostadiene | 3.2 | 92 | 75 | 55 |
| 1,9-dioxo-11α,16-dihydroxy-16-ethyl-1-hydroxymethyl-5-*cis*-13-*trans*-prostadiene | 3.2 | 70 | 50 | 35 |
| 1,9-dioxo-11α,16-dihydroxy-16-methyl-1-hydroxymethyl-13-*trans*-prostene | 3.2 | 90 | 70 | 45 |
| 1,9-dioxo-11α,16-dihydroxy-16-vinyl-1-hydroxymethyl-13-*trans*-prostene | 3.2 | 90 | 80 | 70 |
| 1,9-dioxo-11α,15α-dihydroxy-16,16-trimethylene-1-hydroxymethyl-13-*trans*-prostene | 3.2 | 90 | 75 | 60 |
| 1,9-dioxo-11α,15α-dihydroxy-1-hydroxymethyl-13-*trans*-prostene | 3.2 | 80 | 40 | 15 |
| 1,9-dioxo-11α,16-dihydroxy-16,20-dimethyl-1-hydroxymethyl-13-*trans*-prostene | 3.2 | 55 | 70 | 55 |

TABLE B
Bronchodilator Activity (Konzett Assay) ED 50 mg/kg

| | Spasmogenic Agent | | |
|---|---|---|---|
| | 5-Hydroxytryptamine | Histamine | Acetylcholine |
| 1,9-dioxo-11α,16-dihydroxy-16-methyl-1-hydroxymethyl-5-*cis*-13-*trans* prostadiene | $3.18 \times 10^{-3}$ | $3.0 \times 10^{-3}$ | $6.4 \times 10^{-3}$ |
| 1,9-dioxo-11α,16-dihydroxy-16-vinyl-1-hydroxymethyl-5-*cis*-13-*trans* prostadiene | $402 \times 10^{-6}$ | $254 \times 10^{-6}$ | $2.66 \times 10^{-3}$ |
| 1,9-dioxo-15-hydroxy-15-methyl-1-hydroxymethyl-13-*trans* prostene | $288 \times 10^{-6}$ | $373 \times 10^{-6}$ | $293 \times 10^{-6}$ |
| 1,9-dioxo-15-hydroxy-1-hydroxymethyl prostane | $890 \times 10^{-6}$ | $471 \times 10^{-6}$ | |
| 1,9-dioxo-15-hydroxy-1-hydroxymethyl-13-*trans* prostene | $19.4 \times 10^{-3}$ | $4.8 \times 10^{-3}$ | >320 |
| 1,9-dioxo-11α,16-dihydroxy-16-vinyl-1-acetoxymethyl-5-*cis*-13-*trans* prostadiene | $88.1 \times 10^{-6}$ | $780 \times 10^{-6}$ | $795 \times 10^{-6}$ |
| 1,9-dioxo-11α,16-dihydroxy-16-methyl-1-hydroxymethyl-13-*trans*-prostene | $3.0 \times 10^{-3}$ | $943 \times 10^{-6}$ | $4.7 \times 10^{-3}$ |
| 1,9-dioxo-11α,16-dihydroxy-16-vinyl-1-hydroxymethyl-13-*trans*-prostene | $147 \times 10^{-6}$ | $250 \times 10^{-6}$ | $1.7 \times 10^{-3}$ |
| 1,9-dioxo-11α,15α-dihydroxy-16,16-trimethylene-1-hydroxymethyl-13-*trans*-prostene | $1.4 \times 10^{-3}$ | $734 \times 10^{-6}$ | $1.3 \times 10^{-3}$ |
| 1,9-dioxo-11α,15α-dihydroxy-1-hydroxymethyl-13-*trans*-prostene | $936 \times 10^{-6}$ | $547 \times 10^{-6}$ | $3.9 \times 10^{-3}$ |
| 1,9-dioxo-11α,15α-dihydroxy 16,16-trimethylene-5-*cis*, 13-*trans*-prostadiene | $1.49 \times 10^{-3}$ | $781 \times 10^{-6}$ | $995 \times 10^{-6}$ |
| 1,9-dioxo-11α,16-dihydroxy-16,20-dimethyl-1-hydroxymethyl-5-*cis*,13-*trans*-prostadiene | $47 \times 10^{-3}$ | $8.8 \times 10^{-3}$ | $19.0 \times 10^{-3}$ |

PROTOCOL FOR EVALUATION OF GASTRIC ANTI-SECRETORY AGENTS

A. MODEL:

Unanesthetized mongrel dogs weighing 10—15 Kg are used.

The animals have a surgically prepared denervated (Heidenhain) pouch which drains by gravity through a titanium cannula. These animals are trained to stand quietly in a Pavlov support. Gastric secretion is stimulated at the lowest rate giving stable secretion (25—40% of maximal) with histamine acid phosphate, 30—50 μg/Kg/hr. Under such stimulation, a stable gastric secretory output can generally be maintained for a period of at least three hours.

Gastric juice is collected continuously during secretory studies and pooled into 15-minute collections. Determination of collection volume, pH and titratable acidity is performed. Acid is determined by titrating an aliguot of gastric sample with 0.1 N NaOH to pH 7.0 using an automatic titrator.

Drugs are administered on a background of submaximal gastric secretory stimulation and the results compared with control secretory studies without the use of drug. Depending upon the duration of action of a particular drug, it may be possible for a single drug-secretory study to serve as its own control. The route of drug administration is oral administration into the main stomach. This route is easy to perform and does not interfere with the smooth collection of pouch gastric juice.

For Example, one of the PGE compounds of this invention, 1,9-dioxo-11$\alpha$,16-dihydroxy-16-methyl-1-hydroxymethyl-5-*cis*,13-*trans* prostadiene on administration into the stomach of one of these dogs at a dose of 100 mg/Kg gives the results shown hereinbelow in Table D. The data in Table D is the average of four tests in one dog.

TABLE D

Volume and titratable activity of gastric juice after administration of 1,9 - dioxo - 11$\alpha$,16 - dihydroxy - 16 - methyl - 1 - hydroxymethyl - 5 - *cis*,13 - *trans* prostadiene (100 mg/Kg).

| Time after administration, min. | 0 | 15 | 30 | 45 | 60 | 75 | 90 | 105 | 120 |
|---|---|---|---|---|---|---|---|---|---|
| Volume (ml) | 3.4 | 2.6 | 1.5 | 1.3 | 0.7 | 0.9 | 1.5 | 1.9 | 2.7 |
| Ac.1 (meg/15 min.) | 0.43 | 0.35 | 0.22 | 0.20 | 0.09 | 0.13 | 0.21 | 0.27 | 0.40 |

*Gastric Acid Secretion in the Dog Fistula*

Three mongrel dogs (20—32 kg) were surgically prepared with stainless steel cannulae. These were inserted into the most dependent portion of the ventral stomach and exteriorized through the abdomen for the collection of gastric secretions. The dogs were trained to stand quietly in a Pavlov support and were conscious during subsequent secretory studies.

TABLE E

Effect of 1,9 - dioxo - 11$\alpha$,16 - dihydroxy - 16 - methyl - 1 - hydroxymethyl - 13 - *trans* - prostene, and 1,9 - dioxo - 11$\alpha$,16 - dihydroxy - 16 - methyl - 1 - hydroxymethyl - 5 - *cis* - 13 - *trans* prostadiene on the Gastric Acid Secretion[a] in the Dog Fistula Preparation

| treatment | dose (μg/kg I.G.) | n[b] | cumulative two-hour gastric secretion[c] | |
|---|---|---|---|---|
| | | | volume (ml) | acid (mEq $H^+$) |
| Control | — | 20 | 130 ± 11 | 17.2 ± 1.6 |
| Compound A | 10 | 6 | 59 ± 19 (p<0.01) | 6.9 ± 2.8 (p<0.005) |
| Compound B | 10 | 6 | 76 ± 16 (p<0.05) | 7.5 ± 2.1 (p<0.005) |

Footnotes to Table E

ᵃAfter a 26-hour fast, gastric acid secretion was submaximally stimulated, beginning 45 minutes after treatment, using a constant intravenous infusion of histamine acid phosphate (40 g/kg/hr.) ᵇEach of three dogs was treated six to seven times with vehicle (control) and one to three times with each compound; n is the number of experiments conducted with each treatment. ᶜTotal secretion from 45—120 minutes following intragastric (I.G.) administration of drugs or vehicle. Mean effects of treatment were compared to control means by Student's t test. Compound A is 1,9 - dioxo - 11$\alpha$,16 - dihydroxy - 16 - methyl - 1 - hydroxymethyl - 13 - *trans* - prostene, and Compound B is 1,9 dioxo - 11$\alpha$,16 - dihydroxy - 16 - methyl - 1 - hydroxymethyl - 5 - *cis* - 13 - trans - prostadiene.

*Restraint — Immersion Stress Ulcers in the Rat (Wilson)*

A. *Induction of Ulcers*

Male "Sprague — Dawley Derived" rats (Locke — Erickson Laboratories, Maywood, Illinois) weighing 150 to 200 g, are fasted, and provided drinking water for 16 to 19 hours prior to the start of the study. At 0 time the rats are dosed orally with compound or vehicle, (approximately 0.5 ml) and then secured in a Bollman-type restraining cage. Five minutes after dosing, the animals are immersed (tail downward) to "shoulder level" into a 22°C water bath for 100 minutes. At the end of this period, the animals are decapitated and their stomachs promptly excised with about 5 mm each of esophagus and duodenum.

With a blunt tipped scissor the stomach is opened along the greater curvature, starting at the tip of the rumen through the glandular mucosa into the pyrolus and opening the bit of duodenum along its amesenteric border. Debris is removed from stomach by rinsing in saline at room temperature and gently blotting it with gauze or filter paper. The stomach is then spread out, mucosal surface upward on a 3 x 5 file card or similar material.

B. *Evaluation of Lesions*

With an illuminated magnifier the lesions are counted (for total lesion count) and scored (for weighted score) after the method described by G. Osterloh, *et al.,* Arzneimittel-Forschung, 16 (8a):901910, August 1966. The score for each lesion is given in the following table, modified from the paper.

| LESION TYPE | SCORE |
|---|---|
| No lesions | 0 |
| Erythema | 1 |
| Petechial Hemorrhages | 2 |
| Erosion | 3 |
| Pinpoint Ulcer | 4 |
| Small Ulcer (0.5—1mm) | 5 |
| Medium Ulcer (1—3mm) | 6 |
| Large Ulcer (3mm) | 7 |
| Perforation | 8 |

Very large (area) non-perforating lesions are frequently seen. These are measured as so many 3 mm diameter ulcers and scored as multiples of #7. For example, a 3 $mm^2$ diameter ulcer is taken to have an area of 7.1 $mm^2$, so a lesion measuring 9 x 4 mm will have an area of 36 $mm^2$ and hence is equivalent to 5 lesions with a score #7.

TABLE F
Effect of Prostaglandins on Stress-Induced (Immersion) Ulcers in Rats

| Compound | Dose (mg/Kg P.O.) | Ulcer Score (Mean ± SEM (n)) | % Reduction |
|---|---|---|---|
| Control | — | 40 ± 5 (27) | |
| A* | 500 | 2 ± 1 (10) | 95 ($p < .001$) |
| | 200 | 6 ± 3 ( 5) | 85 ($p < .01$) |
| B* | 500 | 7 ± 2 ( 6) | 82 ($p < .01$) |
| | 200 | 13 ± 7 (13) | 67 ($p < .005$) |
| | 80 | 7 ± 6 ( 7) | 82 ($p < .005$) |
| | 16 | 6 ± 4 ( 6) | 85 ($p < .005$) |
| | 3 | 15 ± 11( 6) | 62 ($p < .05$) |

A* is: 1,9 - dioxo - 11α,16 - dihydroxy - 16 - methyl - 1 - hydroxymethyl - 5 - *cis*, 13 - *trans* - prostadiene.
B* is: 1,9 - dioxo - 11α,16 - dihydroxy - 16 - methyl - 1 - hydroxymethyl - 13 - *trans* - prostene.

Rats were dosed by gavage with compound or vehicle and submerged into a 22°C water bath for 100 minutes. At the end of this period, animals were sacrificed and their stomachs removed and scored for ulcers.

*Indomethacin — Induced Ulcers in the Rat*

Male Wistar rats (Royal Hart, New Hampton, N.Y.), weighing 190—210 g are distributed among control and treatment groups (5 rats/group) and housed one rat per cage. During the 52 hour test period, the rats are permitted free access to food and drinking water for the first 33 hours but are fasted overnight prior to sacrifice. Indomethacin is suspended (4 mg/ml) in a 1.5% starchphosphate buffer solution (SPBS). 1,9 - dioxo - 11α,16 - dihydroxy - 16 - methyl - 1 - hydroxymethyl - 13 - *trans* - prostene is dissolved at 10 mg/ml in ethanol and then diluted in SPBS to 0.1 mg/ml or to a lower concentration as required. The indomethacin suspension is injected subcutaneously (10 mg/kg) and the prostaglandin suspension is given by gavage (0.5 mg/kg or less) b.i.d. on day 0 and 1. Rats receive only one dose of prostaglandin and indomethacin on day 2 and 6 hours later are killed with chloroform. The stomachs are dissected, opened along the greater curvature, and rinsed briefly in tap water. They are then spread, mucosal surface facing upward and pinned onto uniform size corks (2.5 inch diameter) individually numbered on the back. The identification number for each stomach is unknown to the investigator and the stomachs are randomly graded according to the following scheme (10).

0 — Normal
1 — Petechial hemorrhage, or pin-point ulcers
2 — One or two small ulcers or hemorrhagic erosions
3 — Many areas of hemorrhage erosion or ulcers, a few large
4 — Massive areas of hemorrhagic erosion or many ulcers, mainly large

Intestines are also removed and examined for the presence of ulcers which are graded according to the scheme outlined below.

0 — Normal
1 — Mucosa thin, petechial hemorrhage
2 — "Blow-outs", when intestine inflated with air
3 — Few ulcers. Gut more fragile than normal, tears along line of mesentery attachments when removed
4 — Many large perforating lesions. Adhesions. Gut hemorrhagic and very fragile. Tears readily and cannot be removed intact.
*Graded in situ.*

TABLE G

Effect of 1,9 - dioxo - 11α,16 - dihydroxy - 16 - methyl - 1 - hydroxymethyl - 13 - *trans* - prostene (Compound B) on Indomethacin-Induced Ulcers in Rats

| Treatment[a] Indomethacin (mg/kg subcut.) | Compound B (mg/kg P.O.) | N | Gastric Ulcer Score (Mean ± S.E.M.) | Intestinal Ulcer Score (Mean ± S.E.M.) | Hematocrit (Mean ± S.E.M.) |
|---|---|---|---|---|---|
| 10 | — | 10 | 2.9 ± 0.2 | 3.7 ± 0.2 | 33.1 ± 2.1 |
| 10 | .5 | 10 | 2.1 ± 0.4 | 2.0 ± 0.4[b] | 40.0 ± 1.4[b] |
| 10 | .1 | 10 | 1.7 ± 0.3[b] | 2.7 ± 0.2[b] | 41.1 ± 2.4[b] |
| 10 | .02 | 8 | 1.9 ± 0.4[b] | 3.5 ± 0.2 | 35.6 ± 1.9 |
| — | — | 10 | 0.3 ± 0.2[b] | 0[b] | 48.0 ± 0.6[b] |

[a] Treatment is B.I.D. on days 0 and 1, and once on day 2, six hours prior to sacrifice for scoring
[b] Significantly different from indomethacin-only treatment at $p<.05$

Certain of the novel compounds of this invention are useful for the preparation of other novel compounds of this invention.

The invention is illustrated by the following specific examples.

Example 1

Preparation of ethyl 2.2-trimethylenehexanoate

To a stirred solution of 27.6 g of freshly distilled N-isopropylcyclohexylamine in 200 ml of dry tetrahydrofuran cooled to −78°C is added at a fast rate 96 ml of 2.04M *n*-butyllithium in hexane. To the resulting solution is added dropwise 25 g of ethyl cyclobutanecarboxylate. After 30 minutes the resulting solution is allowed to warm to ambient temperature, is transferred to a dropping funnel under nitrogen and is added dropwise over a period of 1½ hours to a solution of 54 g of *n*-butyl iodide in 100 ml of dry dimethylsulfoxide maintaining the temperature at 16°—20°C. Stirring is continued for an additional 30 minutes. The separated salts are removed by filtration, the mother liquor is taken to a small volume and the resulting oil is diluted with hexanes. This solution is washed with 2% hydrochloric acid, saturated sodium chloride solution, and dried with anhydrous magnesium sulfate. The solvent is removed and the residual oil is distilled to give 14.6 g (41%) of product, bp 84°—87°C (10 mm).

Example 2

Preparation of ethyl 2,2-tetramethylenehexanoate

In the manner described in Example 1, treatment of the lithium salt of ethyl cyclopentanecarboxylate with *n*-butyl iodide furnishes the subject product.

Example 3

Preparation of 2,2-trimethylenehexan-1-ol

To a stirred solution of 20 g of ethyl 2,2-trimethylenehexanoate (Example 1) in 100 ml of dry toluene, in an argon atmosphere and cooled in an ice bath, is added dropwise 250 ml (2 molar equivalents) of 0.89M diisobutylaluminum hydride in toluene. The resulting solution is stirred at ambient temperature for 2 hours and then poured into excess iced 5% hydrochloric acid. The organic phase is separated and washed with 5% hydrochloric acid, saturated sodium chloride solution, dried with anhydrous magnesium sulfate and taken to dryness to give 14.8 g (96%) of oil; bp 92°—93°C (10 mm).

Example 4

Preparation of 2,2-tetramethylenehexan-1-ol

In the manner described in Example 3, treatment of ethyl 2,2-tetramethylenehexanoate (Example 2) with 0.89 molar diisobutylaluminum hydride furnishes the subject product.

Example 5

Preparation of 2,2-trimethylenehexaldehyde

Chromium trioxide (61.5 g), dried in a vacuum desiccator over phosphoros pentoxide, is added to an ice cold solution of 97 g of dry pyridine in one litre of dry methylene chloride. The deep red suspension is stirred for 15 minutes at 0°C and then for 45 minutes at ambient temperature. A solution

of 14.5 g of 2,2-trimethylenehexanol-1 (Example 3) in 55 ml of methylene chloride is added all at once to the suspension. A black tarry deposit is formed immediately. After stirring at ambient temperature for 15 minutes the solution is decanted from the tarry deposit which is then triturated four times with small portions of methylene chloride. The combined extracts are washed twice with ice cold 5% sodium hydroxide, ice cold 5% hydrochloric acid and finally with saturated sodium chloride solution, dried with magnesium sulfate and taken to dryness. Distillation gives 12.9 g of product; bp 69°C (11 mm).

Example 6

Preparation of 2,2-tetramethylenehexaldehyde

Oxidation of 2,2-tetramethylenehexan-1-ol (Example 4) with chromium trioxide-pyridine complex in the manner described in Example 5 furnishes the subject product.

Example 7

Preparation of 4,4-trimethylene-1-octyn-3-ol

To a solution of lithium acetylide-ethylenediamine complex (9.4 g) in 90 ml of dry dimethylsulfoxide, cooled in an ice bath, is added 12.94 g of 2,2-trimethylenehexaldehyde (Example 5) in 10 ml of dimethylsulfoxide dropwise, at such a rate that the temperature is maintained at 20°—25°C. The solution is stirred at ambient temperature 12 hours and then poured into a mixture of ice cold 2% hydrochloric acid and ether. The ether layer is separated and the aqueous phase is extracted with ether. The combined ether extracts are washed with saturated sodium chloride solution, dried with anhydrous magnesium sulfate and taken to dryness. Distillation provides 13.53 g of product; bp 108°—109°C (13 mm).

Example 8

Preparation of 4,4-tetramethylene-1-octyn-3-ol

Treatment of 2,2-tetramethylenehexaldehyde (Example 6) with lithium acetylide-ethylenediamine complex in dimethylsulfoxide in the manner described in Example 4 is productive of the subject compound.

Example 9

Preparation of 4,4-trimethylene-3-trimethylsilyloxy-1-octyne

To a stirred solution of 5.3 g of 4,4-trimethylene-1-octyn-3-ol (Example 7) and 5.42 g of imidazole in 32 ml of dry dimethylformamide, cooled in an ice bath under argon atmosphere is added 4.35 g of chlorotrimethylsilane. After stirring at 0°C for 15 minutes, the solution is stirred at ambient temperature for 18 hours and then poured into 200 ml of hexanes. The solution is washed twice with ice cold water, saturated sodium chloride solution, dried with anhydrous magnesium sulfate and taken to dryness. Distillation furnishes 6.02 g (80%) of colourless oil; bp 110°—112°C (14 mm).

Example 10

Preparation of 4,4-tetramethylene-3-trimethylsilyloxy-1-octyne

Treatment of 4,4-tetramethylene-1-octyn-3-ol (Example 8) with chlorotrimethylsilane in dimethylformamide containing imidazole as described in Example 5 furnishes the subject product.

Example 11

Preparation of 1-iodo-4,4-trimethylene-3-trimethylsilyloxy-1-octyne

To a solution of 25 g of 4,4-trimethylene-3-trimethylsilyloxy-1-octyne (Example 9), stirred under argon atmosphere at —78°C, is added dropwise 93 ml of 2.3M *n*-butyllithium in hexane at a rate to maintain the temperature below —40°C. After stirring for 40 minutes, a solution of iodine is allowed to warm to ambient temperature and 10% aqueous sodium thiosulfate solution is added until the purple colour is removed. The organic phase is washed with dilute aqueous sodium thiosulfate solution, saturated sodium chloride solution, dried with anhydrous sodium sulfate and taken to dryness to afford the subject product as an oil.

Example 12

Preparation of 1-iodo-4,4-trimethylene-3-trimethylsilyloxy-1-*cis*-octene

To a solution of 30 g of 1 - iodo - 4,4 - trimethylene - 3 - trimethylsilyloxy - 1 - octyne (Example 10) in 100 ml of methanol, under argon atmosphere is added 54 g of potassium azodicarboxylate (J. Thiele, Annalen der Chemie, *271*, 127 (1892)) to this solution is added dropwise 45 ml of acetic acid over a period of about 2 hours. The solids are removed by filtration and the mother liquor is reduced to a small volume, diluted with water and extracted with ether. The ether is evaporated and the residual oil is stirred with 250 ml of 1M sodium bicarbonate solution. The solution is extracted several times with ether and the combined extracts are washed with saturated sodium chloride solution, dried with anhydrous sodium sulfate and taken to dryness to furnish the subject product as an oil.

Example 13

Preparation of 1-iodo-4,4-trimethylene-3-trimethylsilyloxy-1-*trans*-octene

To a mixture of 4.76 g of sodium borohydride and 23.6 g of 2 - methyl - 2 - butene in 220 ml of dry tetrahydrofuran at −5°C is added dropwise 23.8 g of freshly distilled borontrifluoride etherate. The resulting mixture is stirred at −5°C to −0°C for 2 hours and to it is added dropwise a solution of 20 g of 4,4 - trimethylene - 3 - trimethylsilyloxy - 1 - octyne (Example 9) in 20 ml of dry tetrahydrofuran. The resulting mixture is stirred at ambient temperature for 2½ hours. The mixture is then cooled to −5°C and there is added 44 g of trimethylene oxide portionwise over a period of 20 minutes, maintaining the temperature at 15°—20°C. The mixture is stirred at ambient temperature for 2 hours and then poured simultaneously, with a solution of 119 g of iodine in 290 ml of tetrahydrofuran, into 1490 ml of 15% aqueous sodium hydroxide solution. After stirring for 30 minutes the organic phase is separated and the aqueous phase is extracted with ether. The combined organic phase is washed with 5% aqueous sodium thiosulfate solution, saturated sodium chloride solution, dried with anhydrous magnesium sulfate and taken to dryness to give 27 g of oily material. Chromatography on 135 g of "Florisil" (registered Trade Mark) and eluting with 500 ml of hexanes furnishes 24 g of oily product which is shown to be contaminated with starting material and iodoform by infrared and thin layer chromatography. The material is purified by removing the trimethylsilyl group in the following manner. The crude product is dissolved in 350 ml of acetic acid-tetrahydrofuran-water (4:2:1) by stirring at ambient temperature for 5 minutes. The solvent is removed under reduced pressure and the residual oil containing mainly 1 - iodo - 3 - hydroxy - 4,4 - trimethylene - 1 - *trans* - octene is applied to a 2" (flat) dry column containing 1200 g of Woelm silica gel. The column is developed with benzene, cut into one-inch segments and each segment is eluted with chloroform. Combination of the appropriate fractions affords 300 mg of iodomethane, 2.8 g of 4,4 - trimethylene - 1 - octyne - 3 ol, and 11.6 g of 1 - iodo - 3 - hydroxy - 4,4 - trimethylene - 1 - *trans* - octene. Silylation of this material in the manner described above followed by distillation of the residual oil furnishes 13 g of pure product; bp 83°—84°C (0.2 mm).

Example 14

Preparation of 1-iodo-4,4-tetramethylene-3-trimethylsilyloxy-1-*trans*-octene

Treatment of 4,4-tetramethylene-3-trimethylsilyl-oxy-1-octene (Example 10) in the manner described in Example 13 furnishes the subject product.

Examples 15—20

Treatment of the lithium salt of ethyl cyclobutane-carboxylate with the alkyl halides listed in Table I below by the procedure described in Example 1 furnishes the 2,2-trimethylene esters of the table.

TABLE I

| Example | Alkyl Halides | Product 2,2-Trimethylene esters |
|---|---|---|
| 15 | propyl iodide | ethyl 2,2-trimethylenepentanoate |
| 16 | amyl iodide | ethyl 2,2-trimethyleneheptanoate |
| 17 | hexyl iodide | ethyl 2,2-trimethyleneoctanoate |
| 18 | benzyl iodide | ethyl 2,2-trimethylene-3-phenylpropionate |
| 19 | 2-cyclopentyl-1-ethyl bromide | ethyl 2,2-trimethylene-4-cyclopentylbutyrate |
| 20 | 1-chloro-2-butyne | ethyl 2,2-trimethylene-4-hexynoate |

Examples 21—27

Reduction of the various esters listed in Table II below with diisobutylaluminum hydride all in the manner described in Example 3 above is productive of the alcohols of the table.

TABLE II

| Example | Starting Esters of Example | Product Alcohols |
|---|---|---|
| 21 | 15 | 2,2-trimethylenepentan-1-ol |
| 22 | 16 | 2,2-trimethyleneheptan-1-ol |
| 23 | 17 | 2,2-trimethyleneoctan-1-ol |
| 24 | 18 | 2,2-trimethylene-3-phenylpropan-1-ol |
| 25 | 19 | 2,2-trimethylene-4-cyclopentylbutan-1-ol |
| 26 | 20 | 2,2-trimethylene-4-hexyn-1-ol |
| 27 | 55 | 2,2-trimethylene-4-*cis*-hexen-1-ol |

Examples 28—34

Oxidation of the alcohols listed in Table III below with chromium trioxide-pyridine complex by the procedure described in Example 5 above furnishes the corresponding aldehydes of the table.

TABLE III

| Example | Starting Alcohols of Example | Product of 2,2-Trimethylenealdehydes |
|---|---|---|
| 28 | 21 | 2,2-trimethylenevaleraldehyde |
| 29 | 22 | 2,2-trimethyleneheptaldehyde |
| 30 | 23 | 2,2-trimethyleneoctaldehyde |
| 31 | 24 | 2,2-trimethylene-3-phenylpropionylaldehyde |
| 32 | 25 | 2,2-trimethylene-4-cyclopentylbutyraldehyde |
| 33 | 26 | 2,2-trimethylenehex-4-yn-1-al |
| 34 | 27 | 2,2-trimethylene-4-*cis*-hexene-1-al |

Examples 35—41

Treatment of the various aldehydes listed below in Table IV with lithium acetylide-ethylenediamine complex in the manner described in Example 7 furnishes the hydroxyacetylenes of the table.

TABLE IV

| Example | Starting Aldehydes of Example | Product Hydroxyacetylenes |
|---|---|---|
| 35 | 28 | 4,4-trimethylene-1-heptyn-3-ol |
| 36 | 29 | 4,4-trimethylene-1-nonyn-3-ol |
| 37 | 30 | 4,4-trimethylene-1-decyn-3-ol |
| 38 | 31 | 4,4-trimethylene-5-phenyl-1-pentyn-3-ol |
| 39 | 32 | 4,4-trimethylene-6-cyclopentyl-1-hexyn-3-ol |
| 40 | 33 | 4,4-trimethylene-1,6-octadiyn-3-ol |
| 41 | 34 | 4,4-trimethylene-4-*cis*-hexene-3-ol |

Examples 42—48

Treatment of the various alcohols listed below in Table V with chlorotrimethylsilane in the manner described in Example 9 furnishes the corresponding trimethylsilyloxy acetylenes of the table.

TABLE V

| Example | Starting Alcohols of Example | Product Trimethylsilyloxyacetylenes |
|---|---|---|
| 42 | 35 | 4,4-trimethylene-3-trimethylsilyloxy-1-heptyne |
| 43 | 36 | 4,4-trimethylene-3-trimethylsilyloxy-1-nonyne |
| 44 | 37 | 4,4-trimethylene-3-trimethylsilyloxy-1-decyne |
| 45 | 38 | 4,4-trimethylene-3-trimethylsilyloxy-5-phenyl-1-pentyne |
| 46 | 39 | 4,4-trimethylene-3-trimethylsilyloxy-6-cyclopentyl-1-hexyne |
| 47 | 40 | 4,4-trimethylene-3-trimethylsilyloxy-1,6-octadiyne |
| 48 | 41 | 4,4-trimethylene-3-trimethylsilyloxy-4-*cis*-octene-1-yne |

Examples 49—54

In the manner described in Example 13 treatment of the various acetylenes of Table VI below with disiamylborane, made *in situ* from sodium borohydride and 2 - methyl -2 - butene, followed by oxidation of the so formed organoborane with trimethylamine oxide followed by treatment of this product with iodine and sodium hydroxide furnishes the trimethylsilyliodovinylcarbinols of the table.

TABLE VI

| Example | Starting Acetylenes of Example | Product Trimethylsilylvinylcarbinols |
|---|---|---|
| 49 | 42 | 1-iodo-4,4-trimethylene-3-trimethylsilyloxy-1-*trans*-heptene |
| 50 | 43 | 1-iodo-4,4-trimethylene-3-trimethylsilyloxy-1-*trans*-nonene |
| 51 | 44 | 1-iodo-4,4-trimethylene-3-trimethylsilyloxy-1-*trans*-decene |
| 52 | 45 | 1-iodo-4,4-trimethylene-3-trimethylsilyloxy-5-phenyl-1-*trans*-pentene |
| 53 | 46 | 1-iodo-4,4-trimethylene-3-trimethylsilyloxy-6-cyclopentyl-1-*trans*-hexene |
| 54 | 47 | 1-iodo-4,4-trimethylene-3-trimethylsilyloxy-1-*trans*-octen-6-yne |

There is no Example 55

Example 56

Preparation of 3-tetrahydropyranyloxy-1-propyne

To a stirred solution of 112 g (2.0 mol) of 3 - hydroxy - 1 - propyne and 260 g (3.0 mol.) of dihydropyran in 1.20 litres of methylene chloride cooled to 0°C in an ice bath, is added a solution of 20 mg of para-toluenesulfonic acid in 100 ml of methylene chloride, dropwise. The reaction mixture is stirred at 0°C for one-half hour, and at ambient temperature for one hour. It is then poured into 200 ml of a 5% solution of sodium bicarbonate, the organic phase is separated, the aqueousphase extracted with 100 ml of methylene chloride, the combined organic phases washed with 100 ml of a solution of brine, dried over sodium sulfate, and evaporated under vacuum (12 mm) at 45°C to give 300 g of crude product, which is purified by fractional distillation, bp 71°—73°C (14 mm) to yield 250 g (89%) of a liquid.

Example 57

Preparation of 3-tetrahydropyranyloxy-1-trimethylsilyl-1-propyne

To a stirred —20°C solution of 125 g (0.89 mol.) of 3 - tetrahydropyranyloxy - 1 - propyne (Example 56) in 450 ml of ether, under a nitrogen atmosphere, is added dropwise, over one hour, a solution of 45 ml (0.89 mol.) of 2.0 N *n*-butyllithium in hexane. After 150 ml of dry ether is added and the mixture stirred at —20°C for 30 minutes, a solution of 98 g (0.89 mol.) of trimethylchlorosilane in 73 ml of ether is added dropwise. Stirring is continued for 30 minutes at —20°C and at ambient temperature for 18 hours. The reaction mixture is again cooled to —20°C, and a solution of 90 ml of acetic acid in 300 ml of ether is added dropwise, followed by 90 ml of water. It is then diluted with 500 ml of water, and extracted 3 times with 300 ml of 5% sodium bicarbonate solution. The organic phase is separated, washed with 500 ml of a saturated brine solution, dried over sodium sulfate, and evaporated at 40°C under vacuum (12 mm.). The crude product is fractionally distilled, bp 120°—125°C (18 mm.), to yield 120 g of an oil.

Example 58

Preparation of d,1-*erythro*-3-tetrahydropyranyloxy-4-hydroxy-1-trimethylsilyl-1-octyne

To a stirred —78°C solution of 62 ml (124 mmol.) of a 2.0 M solution of *n*-butyllithium in hexane and 50 ml of dry tetrahydrofuran, under a nitrogen atmosphere is added dropwise, a solution of 24 g (113 mmol.) of 3 - tetrahydropyranyl - oxy - 1 - trimethylsilyl - 1 - propyne (Example 57) in 35 ml of tetrahydrofuran. This red solution is stirred one hour at —78°C, then a freshly prepared solution of zinc iodide (135 mmol.) in 125 ml of tetrahydrofuran (F. Mercier, R. Epstein, and S. Holand, Bull. Soc. Chim. France, *2*, 690 (1972)) is added dropwise at —78°C, until the mixture turns yellow. After stirring an additional hour at —78°C, a solution of 21 g (250 mmol.) of *n*-valeraldehyde in 35 ml of tetrahydrofuran is added dropwise and the reaction mixture stirred for one hour at —78°C and 18 hours at ambient temperature. It is then cooled to 0°C and a solution of 12 ml of acetic acid in 65 ml of ether is added dropwise, followed by 75 ml of ice-water. The phases are separated and the aqueous phase is extracted twice with ether. The combined organic phases are washed 3 times with saturated sodium

bicarbonate solution, until the last wash is basic, then with a saturated brine solution, dried over sodium sulfate, and evaporated to give 40 g of yellow oil. The crude product may be purified on a 4" x 40" dry column of alumina, and eluted with chloroform. I.R.: neat; 3550 (OH), 2200 (C=C), 840, 750 ($(CH_3)_3Si$), $cm^{-1}$.

Example 59

Preparation of d,1-*erythro*-3,4-dihydroxy-1-trimethylsilyl-1-octyne

A solution of 19.6 g (.066 mol.) of d, 1-*erythro*-3-tetrahydropyranyloxy-4-hydroxy-1-trimethylsilyl-1-octyne (Example 58) in 55.5 ml of ethanol, 22.2 ml of acetic acid, and 22.2 ml of water is heated at reflux for 3 hours. The cooled mixture is taken to dryness and evaporated twice with benzene. The residue is taken up in hexane, washed 3 times with saturated potassium bicarbonate solution, dried with magnesium sulfate, and evaporated to give 17.0 g of crude product IR: neat, 3500—3400, broad (two OH).

Example 60

Preparation of d,1-*erythro*-3,4-isopropylidenedioxy-1-trimethylsilyl-1-octyne

To a stirred solution of 17.0 g (79.5 mmol.) of crude d,1 - *erythro* - 3,4 - dihydroxy - 1 - trimethylsilyl - 1 - octyne (Example 59) is 33.6 ml of 2,2-dimethoxy propane at 0°C, is added 0.05 ml of 60% perchloric acid. After 30 minutes at ambient temperature, the mixture is shaken with 50 ml of hexane and 25 ml of saturated sodium bicarbonate solution. The hexane phase is separated, dried with magnesium sulfate, and evaporated to give 19.0 g of crude product.

Example 61

Preparation of d,1-*erythro*-3,4-isopropylidenedioxy-1-octyne

A mixture of 19.0 g (75.0 mmol.) of crude d,1 - *erythro* - 3,4 - isopropylidenedioxy - 1 - trimethylsilyl - 1 - octyne (Example 60) with 95 ml of methanol and 3.0 g of potassium carbonate is refluxed for one hour. The mixture is cooled and evaporated at 50°C (13 mm), taken up in 250 ml of benzene, and washed with 100 ml of water. The water is saturated with salt, the organic phase separated, dried with magnesium sulfate, and evaporated to give 12 g of crude product. Fractional distillation yields 7.0 g of the subject compound as a colorless oil, bp 103°—106°C (13 mm).

IR: neat; 3300 sharp (H—C≡C), 2100, (C≡C), 780 (erythro configuration) $cm^{-1}$

nmr: $\delta CDCl_3$

TMS; 4.75 (dd., 1, C≡C—*CH*—CH, J=2Hz, J=5Hz), 4.10 (m, 1, C≡C—CH—*CH*—$CH_2$, 2.5 (d, 1, *H*—C C—CH), 1.9—1.2 (m, 14, alkyl), .90 (m, 3H, $CH_2CH_3$).

Example 62

Preparation of d,1-*erythro*-1-iodo-3,4-isopropylidenedioxy-*trans*-1-octene

To a stirred 0°C slurry of 0.852 g (.023 mol.) of sodium borohydride and 4.21 g (.060 mol) of 2 - methyl - 2 - butene in 40 ml of dry tetrahydrofuran, under an argon atmosphere, is added dropwise 4.26 g (.030 mol.) of boron trifluoride etherate complex. A solution of 2.73 g (.015 mol.) of d,1 - *erythro* - 3,4 - isopropylidenedioxy - 1 - octyne (Example 61) in 5 ml of tetrahydrofuran is added dropwise, the ice bath removed, and the mixture allowed to stir at ambient temperature for two hours. It is then cooled again to 0°C, and 2.88 g (.105 mol.) of dry trimethylamine oxide is added in portions over 30 minutes. After stirring 3 hours at room temperature, this mixture is poured simultaneously with a 0°C solution of 2.13 g of iodine in 53 ml of tetrahydrofuran into 766 ml of a 0°C 15% solution of sodium hydroxide in water and the whole stirred vigorously at 0°C for 45 minutes. The organic phase is separated, the aqueous phase is extracted twice with ether, the combined organic phases are washed with a 5% solution of sodium thiosulfate, dried with magnesium sulfate, and evaporated. The crude product is chromatographed on a 2" by 40" dry column of silica gel, by eluting with chloroform, to yield 1.2 g (25%) of a yellow oil.

H

IR: neat; 1599 sharp, 945 (C=C), $cm^{-1}$l

H

Example 63

Preparation of d,1 - *erythro* - 3 - tetrahydropyranyloxy - 4 - acetyloxy - 1 - trimethylsilyl - 1 - octyne

A solution of 3.0 g (13.2 mmol.) of d,1 - *erythro* - 3 - tetrahydropyranyloxy - 4 - hydroxy - 1 - trimethylsilyl - 1 - octyne is heated at 100°C for 15 hours with 3 ml of acetic anhydride and 10 ml of pyridien. The mixture is evaporated to dryness, dissolved in ether, washed with sodium bicarbonate solution and water. The organic phase is dried over magnesium sulfate and evaporated to give 2.5 g of the subject compound as an oil.

IR: neat; 2200 (C C), 1730 (C=O), 830, 760 ($(CH_3)_3(Si)$, $cm^{-1}$.

Example 64

Preparation of d,1-*erythro*-3-hydroxy-4-acetyloxy-1-trimethylsilyl-1-octyne

In the manner of Example 59, 2.5 g (7.4 mmol.) of d,1 - *erythro* - 3 - tetrahydropyranyloxy - 4 - acetyloxy - 1 - trimethylsilyl - 1 - octyne (Example 63) in a solution of ethanol, acetic acid and water is heated at 100°C for 3 hours. After workup, the crude product is chromatographed on a 7/8" x 22" dry column of silica gel, and eluted with chloroform to give 1.0 g of a yellow oil.
IR: neat; 3500 (OH), 1730 (C=O), $cm^{-1}$.

Example 65

Preparation of d,1 - *erythro* - 3 - paratoluenesulfonyloxy - 4 - acetyloxy - 1 - trimethylsilyl - 1 - octyne

To a solution of 7.5 g (41.0 mmol.) of d,1 - *erythro* - 3 - hydroxy - 4 - acetyloxy - 1 - trimethylsilyl - 1 - octyne (Example 64) in 41 ml of dry pyridine is added 11.0 g (58 mmol.) of *para*-toluenesulfonyl chloride and the resulting solution is stirred at 25°C for 15 hours. The mixture is then warmed at 40°C for one hour, and after cooling, partitioned between 500 ml of diethyl ether and 100 ml of 1.0 N hydrochloric acid. The organic phase is washed three times with 100 ml of 1.0 N hydrochloric acid, once with dilute sodium bicarbonate solution, dried over magnesium sulfate, and evaporated under reduced pressure to give an oil. The crude product is purified on a 2" x 24" dry column of silica gel, and eluted with chloroform to yield a yellow oil.
IR: neat; 1730 (C=O), 1595 (aromatic) $cm^{-1}$.

Example 66

Preparation of d,1-*threo*-3-hydroxy-4-acetyloxy-1-trimethylsilyl-1-octyne

A mixture of 15.5 g (39.0 mmol.) of d,1 - *erythro* - 3 - *para* - toluenesulfonyloxy - 4 - acetyloxy - 1 - trimethylsilyl - 1 - octyne (Example 65), 5.0 g of calcium carbonate, 25 ml of water and 250 ml of tetrahydrofuran is refluxed with stirring for 4 days. The mixture is cooled, 100 ml of water added and the organic phase separated. The aqueous phase is extracted with ether, the combined organic phases dried with magnesium sulfate, and evaporated. The crude product is chromatographed on a 3" x 30" dry column of silica gel, and eluted with chloroform to give 7.0 g of an oil.
IR: neat; 3500, (OH), $cm^{-1}$.

Example 67

Preparation of d,1-*threo*-3,4-dihydroxy-1-octyne

A solution of 7.0 g (28 mmol.) of d,1 - *threo* - 3 - hydroxy - 4 - acetyloxy - 1 - trimethylsilyl - 1 - octyne (Example 66) in 50 ml of methanol is stirred at room temperature for 24 hours with a solution of 6.3 g (112 mmol.) of potassium hydroxide in 50 ml of water. The mixture is extracted twice with hexane, washed with 0.5M hydrochloric acid, brine, and dried with magnesium sulfate. After evaporation, the subject compound is obtained as a yellow oil.
IR: neat, 2500 broad (2—OH), $cm^{-1}$.

Example 68

Preparation of d,1-*threo*-3,4-isopropylidenedioxy-1-octyne

In the manner of Example 60, treatment of a solution of d,1 - *threo* - 3,4 - dihydroxy - 1 - octyne (Example 67) in dimethoxypropane with 60% perchloric acid, and fractional distillation (12 mm) is productive of the subject compound as a colourless oil, containing 15% of d,1 - *erythro* - 3,4 - isopropylidenedioxy - 1 - octyne (Example 60), as an impurity.
IR: neat; 810 (*threo* configuration).
nmr: $\delta$ $CDCl_3$
TMS: 4.2 (dd, I, —C≡C—CH—, J's — $2H_z$, $6H_z$), 4.1—3.9 (m, I, —C≡C—CH—*CH*—$CH_2$—), 2.5 (d, I, H—C≡C—, J = $2H_z$), 1.9—1.2 (m,14, alkyl), .90 (m, 3H, $CH_2$—$CH_3$).

Example 69

Preparation of d,1-*threo*-1-iodo-3,4-isopropylidenedioxy-*trans*-1-octene

In the manner of Example 62, d,1-*threo*-3,4-isopropylidenedioxy-*trans*-1-octyne (Example 68) is treated successively with disiamylborane, trimethylamine oxide, iodine, and sodium hydroxide to give the subject compound.

Example 70

Preparation of d,1-*erythro*-3-tetrahydropyranyloxy-4-hydroxy-1-octyne

Alkaline hydrolysis of d,1 - *erythro* - 3 - tetrahydropyranyloxy - 4 - hydroxy - 1 - trimethylsilyl - 1 - octyne (Example 58) by the procedure of Example 61 is productive of the subject compound.

### Example 71

Preparation of d,1-*erythro*-3-tetrahydropyranyloxy-4-methoxy-1-octyne

To a stirred slurry of 6.0 g (150 mmol.) of a 60% oil dispersion of sodium hydride and 96 g of iodomethane, under an argon atmosphere, is added 700 ml of dry tetrahydrofuran. The stirred mixture is cooled to −20°C and a solution of 30 g (133 mmol.) of d,1 - *erythro* - 3 - tetrahydropyranyloxy - 4 - hydroxy - 1 - octyne (Example 70), is added dropwise, followed by 0.1 ml of methanol. The mixture is stirred at ambient temperature for 24 hours, 10 ml of methanol is added, and evaporated. The residue is taken up in ether, washed 3 times with water, dried over magnesium sulfate, and evaporated. The crude product is purified by fractional distillation to yield 16.3 g of a colourless oil, bp 137°—140°C (12 mm).

### Example 72

Preparation of d,1-*erythro*-3-tetrahydropyranyloxy-4-methoxy-1-iodo-*trans*-1-octene

In the manner of Example 62, 1.20 g (5.0 mmol.) of d,1 - *erythro* - 3 - tetrahydropyranyloxy - 4 - methoxy - 1 - octyne (Example 71) is treated successively with disiamylborane, trimethylamine oxide, iodine, and sodium hydroxide. Chromatography on a 2″ x 36″ dry column of silica gel and elution with chloroform is productive of 0.80 g (40%) of the subject compound as an oil.

nmr: $\delta$ $CDCl_3$

TMS: 7.9—6.1 (m,2, HC=CH), 4.9—4.6 (2m, 2, c=C—CH, O—CH—O), 4.3—4.0 (m, l, c=c—CH—*CH*—$CH_2$), 3.9—3.0 (m, 6, $C\mathit{H}_2$—O—*CH*, $OCH_3$), 1.8—1.2 (m, 12H, alkyl), 0.9 (m, 3 —$CH_3$).

### Example 73

Preparation of d,1-*erythro*-3-hydroxy-4-methoxy-1-iodo-*trans*-1-octene

A solution of 3.10 g (8.24 mmol.) of d,1 - *erythro* - 3 - tetrahydropyranyloxy - 4 - methoxy - 1 - iodo - trans - 1 - octene (Example 72) in 60 ml of acetic acid, 30 ml of tetrahydrofuran, and 15 ml of water is stirred at ambient temperature for 18 hours. It is then evaporated at 70°C under high vacuum 1.0 mm), and three times with 40 ml of toluene to give the crude product as an oil.

### Example 74

Preparation of d,1-*erythro*-3-trimethylsilyloxy-4-methoxy-1-iodo-*trans*-1-octene

To a stirred solution of 3.0 g (10.2 mmol.) of d,1 - *erythro* - 3 - hydroxy - 4 - methoxy - 1 - iodo - *trans* - 1 - octene (Example 73) in 11.0 ml of dry dimethylformamide and 1.90 g (28.0 mmol.) of imidazole cooled to 0°C is added, dropwise, 1.35 g (12.5 mmol.) of trimethylsilyl chloride. The reaction mixture is stirred a further 4 hours at room temperature. It is then poured into a mixture of 100 ml of hexane and 25 ml of water, the organic phase is separated, washed twice with water once with a solution of saturated sodium chloride, dried over magnesium sulfate, and evaporated. The crude product is purified by fractional distillation to yield 2.0 g of a colourless oil, bp 82°—83°C (0.3 mm).

H
IR: neat; 1602 sharp (C=C), 840, 750 broad ($(CH_3)_3Si$-), $cm^{-1}$
H

### Example 75

Preparation of d,1-*erythro*-1-iodo-3,4-dihydroxy-*trans*-1-octene

A solution of 1.40 g (4.50 mmol.) of d,1 - *erythro* - 1 - iodo - 3,4 - isopropylidenedioxy - *trans* - 1 - octene (Example 62) in 30 ml of acetic acid, 10 ml of tetrahydrofuran and 10 ml of water is stirred and heated at 50°C for five hours. It is then evaporated at 40°C under high vacuum (1.0 mm), and twice more with 50 ml of benzene. Crystallization from 10 ml of chloroform at 0°C is productive of 700 mg of the white crystalline subject product.

### Example 76

Preparation of d,1-*erythro*-1-iodo-3,4-*bis*-trimethylsilyloxy-*trans*-1-octene

To a stirred solution of 700 mg (2.40 mmol.) of d,1 - *erythro* - iodo - 3,4 - dihydroxy - *trans* - 1 - octene (Example 75) and 800 mg (12.0 mmol.) of imidazole, in 10 ml of dry dimethylformamide at 0°C is added dropwise 1.20 g (11.0 mmol.) of trimethylchlorosilane. The ice bath is removed, and the mixture is stirred and heated at 50°C for five hours. It is then cooled, shaken with 50 ml of hexane and 50 ml of water, the organic layer separated and washed with 15 ml of 0.5M hydrochloric acid, 15 ml of a saturated solution of sodium bicarbonate, dried with magnesium sulfate, and evaporated. This crude product is fractionally distilled, bp 90°—92°C (0.40 mm) to yield 250 mg of a colourless oil.

Example 77

Preparation of d,1-*erythro*-3-trimethylsilyloxy-4-ethoxy-1-iodo-*trans*-1-octene

Following the procedure of Example 71, ethylation using iodoethane of d,1 - *erythro* - 3 - tetrahydropyranyloxy - 4 - hydroxy - 1 - octyne for a period of 22 hours is productive of the corresponding d,1 - *erythro* - 3 - tetrahydropyranyloxy - 4 - ethoxy - 1 - octyne. This intermediate is converted to d,1 - *erythro* - 3 - tetrahydropyranyloxy - 4 - ethoxy - 1 - iodo - *trans* - 1 - octene when treated successively with disiamylborane, trimethylamine oxide iodine, and sodium hydroxide solution after the procedure of Example 72. Acid hydrolysis by the method of Example 18 to d,1 - *erythro* - 3 - hydroxy - 4 - ethoxy - 1 - iodo - *trans* - 1 - octene, followed by treatment with chlorotrimethylsilane and imidazole in dimethylformamide using the procedure of Example 74, and subsequent distillation, is productive of the subject compound.

Examples 78—80

By the method of Example 58 reaction of 1 - trimethylsilyl - 3 - tetrahydropyranyloxy - 1 - propyne with *n*-butyllithium and subsequent treatment with the aldehydes listed in Table VII below, provides the d,1 - *erythro* - 1 - trimethylsilyl - 3 - tetrahydropyranyloxy - 4 - hydroxy - 1 - alkynes of the table.

TABLE VII

| Example | Starting Aldehyde | Product d,1-*erythro*-3-tetrahydropyranyloxy-4-hydroxy-1-trimethylsilyl-1-alkyne |
|---|---|---|
| 78 | *n*-butanal | d,1-*erythro*-1-trimethylsilyl-3-tetrahydropyranyloxy-4-hydroxy-1-heptyne |
| 79 | *n*-hexanal | d,1-*erythro*-1-trimethylsilyl-3-tetrahydropyranyloxy-4-hydroxy-1-nonyne |
| 80 | *n*-heptanal | d,1-*erythro*-1-trimethylsilyl-3-tetrahydropyranyloxy-4-hydroxy-1-decyne |

There are no Examples 81 and 82

Examples 83—87

Hydrolysis of the 3-tetrahydropyranyloxy group of the d,1 - *erythro* - 1 - trimethylsilyl - 3 - tetrahydropyranyloxy - 4 - hydroxy - 1 - alkynes listed in Table VIII below by the method described in Example 59, followed by conversion of the resulting d,1 - *erythro* - 1 - trimethylsilyl - 3,4 - dihydroxy - 1 - alkyne to the corresponding d,1 - *erythro* - 1 - trimethylsilyl - 3,4 - isopropylidenedioxy - 1 - alkyne by treatment with dimethoxypropane in the presence of perchloric acid by the method described in Example 60 followed by desilylation to the corresponding d,1 - *erythro* - 3,4 - isopropylidenedioxy - 1 - alkyne by the procedure of Example 61 followed by treatment with disiamylborane, trimethylamine oxide, iodine, and sodium hydroxide solution by the method described in Example 62 provides the product d,1 - *erythro* - 1 - iodo - 3,4 - isopropylidenedioxy - *trans* - 1 - alkenes of Table VIII below.

TABLE VIII

| Example | Starting d,1-*erythro*-1-trimethylsilyl-3-tetrahydropyranyloxy-4-hydroxy-1-alkyne of Example | Product d,1-*erythro*-1-iodo-3,4-isopropylidenedioxy-*trans*-1-alkene |
|---|---|---|
| 83 | 78 | d,1-*erythro*-1-iodo-3,4-isopropylidenedioxy-*trans*-1-heptene |
| 84 | 79 | d,1-*erythro*-1-iodo-3,4-isopropylidenedioxy-*trans*-1-nonene |
| 85 | 80 | d,1-*erythro*-1-iodo-3,4-isopropylidenedioxy-*trans*-1-decene |

There are no Examples 86 and 87

Examples 88—90

Acetylation of the 4-hydroxy group of the d,1 - *erythro* - 1 - trimethylsilyl - 3 - tetrahydropyranyloxy - 4 - hydroxy - 1 - alkynes listed in Table IX below by the method described in Example 63, followed by hydrolysis of the resulting d,1 - *erythro* - 1 - trimethylsilyl - 3 - tetrahydropyranyloxy - 4 - acetyloxy - 1 - alkynes to the corresponding d,1 - *erythro* - 1 - trimethylsilyl - 3 - hydroxy - 4 - acetyloxy - 1 - alkynes by the method of Example 64, followed by epimerization to d,1 - *threo* - 1 - trimethylsilyl - 3 - hydroxy - 4 - acetyloxy - 1 - alkynes by the method of Example 65 followed by hydrolysis by the method of Example 66 to give d,1 - *threo* - 3,4 - dihydroxy - 1 - alkynes are converted to the corresponding d,1 - *threo* - 3,4 - isopropylidenedioxy - 1 - alkynes by treatment with dimethoxypropane in the presence of perchloric acid by the method described in Example 68 followed by treatment with disiamylborane,, trimethylamine oxide, iodine, and sodium hydroxide solution by the method described in Example 62 provides the product, d,1 - *threo* - 3,4 - isopropylidenedioxy - *trans* - 1 - alkenes of Table IX below.

TABLE IX

| Example | Starting d,1-*erythro*-1-trimethylsilyl-3-tetrahydropyranyloxy-4-hydroxy-1-alkyne of Example | Product d,1-*threo*-1-iodo-3,4-isopropylidenedioxy-*trans*-1-alkene |
|---|---|---|
| 88 | 78 | d,1-*threo*-1-iodo-3,4-isopropylidenedioxy-*trans*-1-heptene |
| 89 | 79 | d,1-*threo*-1-iodo-3,4-isopropylidenedioxy-*trans*-1-nonene |
| 90 | 80 | d,1-*threo*-1-iodo-3,4-isopropylidenedioxy-*trans*-1-decene |

There are no Examples 91 and 92

Example 93

Preparation of 1-octyn-4-ol

A suspension of 24.3 g (1.0 mole) of magnesium in 90 ml of dry ether is stirred at room temperature under nitrogen with 100 mg of mercuric chloride. The reaction is initiated by the addition of 2 ml of propargyl bromide and maintained by the dropwise addition of a solution of 119.5 g (1.0 mole) of propargyl bromide and 107.7 g (1.25 mole) of valeraldehyde in 300 ml of dry ether. While the initial reaction is quite vigorous and is maintained at 30°C only by cooling in an ice bath it may become necessary to heat the mixture to reflux temperature after about a third of the ether solution is added in order to maintain the reaction. After the addition is complete the reaction mixture is refluxed until most of the magnesium is dissolved (several hours) and the reaction mixture is decanted from excess magnesium into 1500 ml of stirred ice-cold ammonium chloride solution. The ether layer is separated and the aqueous layer is extracted three times with 300 ml portions of ether. The combined ether extract is washed with saturated sodium chloride solution, dried over magnesium sulfate and filtered. Evaporation of the ether under vacuum leaves about 115 g of yellow oil, which is distilled through a 15 cm Vigreaux column at 18 mm. The fraction boiling at 81°—82°C is collected (36 g) and the higher-boiling and lower boiling distillates may be redistilled to yield additional product. The infrared absorption spectrum shows at most a trace of allene (5.1 $\mu$) and gas-liquid partition chromatrography shows a purity of about 98% for the main fraction.

Examples 94—96

The product 1 - alkyn - 4 - ols of Table X below are prepared by treatment of the aldehydes listed in Table X with propargyl magnesium bromide by the procedure described above in Example 93.

TABLE X

| Example | Starting Aldehyde | Product 1-alkyn-4-ol |
|---|---|---|
| 94 | *n*-hexaldehyde | 1-nonyn-4-ol |
| 95 | *n*-heptaldehyde | 1-decyn-4-ol |
| 96 | *n*-butyraldehyde | 1-heptyn-4-ol |

*M. Winter, Helv. Chim. Acta, *46*, 1792 (1963).

There is no Example 97

Example 98

Preparation of 4-triphenylmethoxy-1-octyne

A mixture of 10 g (0.08 moles) of 4-hydroxy-1-octyne (I. Crombie and A. G. Jacklin, J. Chem. Soc., 1632 (1957), also Example 93) and 30.75 g (0.09 moles) of triphenylmethyl bromide in 85 ml of dry pyridine is heated on the steam bath for 2 hours. The cooled mixture is treated with water and extracted with ether. The extract is washed successively with ice cold 2% hydrochloric acid, saturated sodium chloride solution, dried with magnesium sulfate, and taken to dryness. Column chromatography of the residue on Florisil afford an oil; $\lambda$ max 3.01, 4.72 (acetylenic hydrogen), 6.28, 9.65 and 14.25 $\mu$ (triphenylmethoxy group).

There are no Examples 99 and 100

Examples 101—104

The triphenylmethoxy substituted 1-alkynes listed in Table XI below are prepared by the method of Example 98 from triphenylmethyl bromide and the corresponding hydroxy substituted 1-alkynes, appropriate literature references to which are provided in the table. [There is no Example 102].

TABLE XI

| Example | Reference to Starting Hydroxy Substituted 1-Alkyne | Product Triphenylmethoxy Substituted 1-Alkyne |
|---|---|---|
| 101 | Reference 1 (Example 96) | 4-triphenylmethoxy--1-heptyne |
| 103 | Reference 2 (Example 94) | 4-triphenylmethoxy--1-nonyme |
| 104 | Reference 3 (Example 95) | 4-triphenylmethoxy--1-decyne |

References:

1. G. Fontaine, et al., Bull. Soc. Chem. France, 1447 (1963)
2. S. Abe and K. Sato, Bull. Soc. Chem. Japan, *29*, 88 (1956); Chem. Abstr., *50*, 13737 (1956);
3. L. Crombie and A. G. Jacklin, J. Chem. Soc., 1622 (1957);
4. Nobuharra, Akio, Chem. Abstr., 70, 3219 (1969).

There are no Examples 105 and 106

Example 107

Preparation of 1-iodo-4-triphenylmethoxy-*trans*-1-octene

To a stirred suspension of 1.78 g (0.074 mole) of sodium borohydride in 200 ml of dry glyme at −5°C under nitrogen is added 15.8 g (0.22 mole) of 2-methyl-2-butene and 16.2 g (0.11 mole) of boron trifluoride etherate, and the mixture is stirred for 2 hours at −5°C to 0°C. A solution of 37.5 g (0.10 mole) of 4-trityloxy-1-octyne (Example 98) in 50 ml of glyme is added to the cold solution during 5—10 minutes, and the solution is allowed to warm to 20°C during 1.5 hours. The reaction mixture is cooled to 0°C and 30 g (0.4 mole) of dry trimethylamine-N-oxide is added during 5 minutes. On removing the cooling bath the temperature rises to 40°C and the mixture is kept between 30°—40°C for 1.5 hours. The suspension is poured rapidly into one litre of ice cold 15% sodium hydroxide solution during good stirring and a solution of 80 g of iodine in 200 ml of tetrahydrofuran is added immediately.

Stirring is continued for 30 minutes without further cooling and the organic layer is separated. The aqueous layer is extracted with three 200 ml portions of ether and the combined organic layers are washed successively with water, 5% sodium thiosulfate solution and saturated sodium chloride, dried over magnesium sulfate, filtered and evaporated to yield 50 g of yellow oil. The bulk of the oil is dissolved in hexane and, after decantation from a gummy solid the hexane solution is percolated through a 5.1 cm diameter column of 1500 g of alumina with additional hexane. Fractions containing the desired product are concentrated to a pale yellow oil (33 g) which has n.m.r. and infrared spectra characteristics of the desired product.

There are no Examples 108 and 109

Examples 110—113

Treatment of the triphenylmethoxy substituted 1-alkynes listed in Table XII below with disiamylborane, prepared *in situ* from 2-methyl-2-butene, boron, trifluoride and sodium borohydride, followed by trimethylamine N-oxide and then sodium hydroxide and iodine — all by the procedure described in Example 107 above furnishes the product triphenylmethoxy substituted 1-iodo-1-*trans*-alkenes of the table. [There are no Examples 111 and 114]

TABLE XII

| Example | Starting Triphenylmethoxy Substituted 1-Alkyne of Example | Product 1-Iodo-triphenylmethoxy Substituted -1-trans-alkene |
|---|---|---|
| 110 | 101 | 1-iodo-4-triphenylmethoxy--1-*trans*-heptene |
| 112 | 103 | 1-iodo-4-triphenylmethoxy-1--*trans*-nonene |
| 113 | 104 | 1-iodo-4-triphenylmethoxy-1--*trans*-decene |

Examples 115—124a

The starting aldehydes or ketones of Table XIII below are converted to the product 1-alkyn-4-ols of the table by the procedure described in Example 93.

TABLE XIII

| Example | Starting Aldehyde or Ketone | Product 1-Alkyn-4-ol |
|---|---|---|
| 115 | 2-octanone | 4-methyl-4-hydroxy-1-decyne |
| 116 | *trans*-2-hexenal | 4-hydroxy-5-*trans*-nonen-1-yne |
| 117 | 2,2-dimethylhexanal | 5,5-dimethyl-4-hydroxy-1-nonyne |
| 118 | z-heptanone | 4-methyl-4-hydroxy-1-nonyne |
| 119 | 2,2-dimethylpentanal | 5,5-dimethyl-4-hydroxy-1-octyne |
| 120 | 2-methylpentanal | 5-methyl-4-hydroxy-1-octyne |
| 121 | 2-methylhexanal | 5-methyl-4-hydroxy-1-nonyne |
| 122 | 2-hexanone | 4-hydroxy-4-methyl-1-octyne |
| 123 | *trans*-3-hexen-2-one[a] | 4-hydroxy-4-methyl-5-*trans*--octen-1-yne |
| 124 | *trans*-2-pentenal[b] | 4-hydroxy-5-*trans*-octen-1-yne |
| 124a | *trans*-2-heptenal[b] | 4-hydroxy-5-*trans*-decen-1-yne |

a G. Sturtz, Bull. Soc. Chim. Fr., *1967,* 2477.

b R. I. Hoaglin and D. M. Hirsh, *U.S.* 2,628,257; Chem. Abstr., *48,* 1423e (1954).

Example 125

Preparation of 4-methyl-4-trimethylsilyloxy-1-octyne

To a stirred solution of 75.4 g (0.537 moles) of 4-hydroxy-4-methyl-1-octyne (Example 122), 104.9 g (1.54 moles) of imidazole, and 325 ml of dimethylformamide is added 65.2 g (0.60 moles) of chlorotrimethylsilane. After standing overnight the mixture is poured into 800 ml of hexane. The mixture is washed thoroughly with water followed by sodium bicarbonate solution and brine. The solution is dried over magnesium sulfate, filtered, and evaporated to give a liquid, p.m.r. spectrum, $\delta$1.26 (singlet, 3, $CH_3$), 1.92 (triplet, 1, *H*C), 2.30 (doublet, 2, $CH_2$).

Examples 126—129a

The 1-alkyn-4-ols of Table XIV are converted to the product trimethylsilyl ethers of the table by treatment with chlorotrimethylsilane according to the procedure described in Example 125.

TABLE XIV

| Example | Starting 1-Alkyn-4-ol | Product Trimethylsilyl Ether |
|---|---|---|
| 126 | 5,5-dimethyl-4-hydroxy-1--nonyne (Ex. 117) | 5,5-dimethyl-4-trimethylsilyloxy-1-nonyne |
| 127 | 4-methyl-4-hydroxy-1-nonyne (Ex. 118) | 4-methyl-4-trimethylsilyloxy-1-nonyne |
| 128 | 5,5-dimethyl-4-hydroxy-1-octyne (Ex. 119) | 5,5-dimethyl-4-trimethylsilyloxy-1-octyne |
| 129 | 4-hydroxy-4-methyl-5-*trans*-octen-1-yne (Ex. 123) | 4-methyl-4-trimethylsilyloxy-5-*trans*--octen-1-yne |
| 129a | 4-hydroxy-4-methyl-1-decyne (Ex. 115) | 4-methyl-4-trimethylsilyloxy-1-decyne |

Example 130

Preparation of 1-iodo-4-hydroxy-4-methyl-*trans*-1-octene

To a stirred solution of 400 ml of 0.5 M bis-(3-methyl-2-butyl)borane in glyme, prepared from sodium borohydride, 2-methyl-2-butene, and boron trifluoride etherate as in Example 107, is added 63.7 g (0.30 moles) of 4-methyl-4-trimethylsilyloxy-1-octyne (Example 125) at −10°C. The solution is stirred at ambient temperature for 2.5 hours, cooled to −10°C, and treated during 30 minutes with 158 g (2.1 moles) of solid trimethylamine oxide with cooling. The mixture is stirred at ambient temperature for 2 hours and then poured into a stirred, ice-cold solution of 15% aqueous sodium hydroxide; the stirred mixture is treated immediately with a solution of 426 g (1.68 moles) of iodine in 1100 ml of tetrahydrofuran. After 4 hours the mixture is extracted with ether. The extract is washed successively with water, aqueous sodium thiosulfate, and brine and dried over magnesium sulfate. The extract is concentrated, and the residue is subjected to chromatography on silica gel with hexane to provide an oil, p.m.r. ($CDCl_3$): $\delta$ 1.18 (singlet, 4-$CH_3$ group).

Examples 131—134a

The 4-trimethylsilyloxy-1-alkynes of Table XV are converted to the 4-hydroxy-1-iodo-trans-1-octenes of the table by the procedure described in Example 130.

TABLE XV

| Example | Starting 4-Trimethylsilyloxy-1-octyne of Example | Product 4-Hydroxy-1-iodo-*trans*-1-octene |
|---|---|---|
| 131 | 126 | 1-iodo-5,5-dimethyl-4-hydroxy-*trans*-1-nonene |
| 132 | 127 | 1-iodo-4-methyl-4-hydroxy-*trans*-1-nonene |
| 133 | 128 | 1-iodo-5,5-dimethyl-4-hydroxy-*trans*-1-octene |
| 134 | 129 | 1-iodo-4-methyl-4-hydroxy-*trans, trans*-1, 5-octadiene |
| 134a | 129a | 1-iodo-4-methyl-4-hydroxy-*trans*-1-decene |

Example 135

Preparation of 1-iodo-4-methyl-4-trimethylsilyloxy-*trans*-1-octene

To a stirred mixture of 24.5 g (55.6 mmoles) of 1-iodo-4-hydroxy-4-methyl-*trans*-1-octene (Example 130), 13.6 g (200 mmoles) of imidazole, and 75 ml of dimethylformamide is added 10.9 g (100 mmoles) of chlorotrimethylsilane. After standing overnight the mixture is poured into 250 ml of hexane. The mixture is washed thoroughly with water followed by brine and dried over magnesium sulfate. After removal of the solvent, the product is distilled to give a colourless liquid, bp 67.5°—68°C (0.07 mm).

Examples 136—139a

The 1-iodo-4-hydroxy-*trans*-1-alkenes of Table XVI are converted to the product trimethylsilyl ethers of the table according to the procedure described in Example 135.

TABLE XVI

| Example | Starting 1-Iodo-4-hydroxy-*trans*-1-alkene of Example | Product Trimethylsilyl Ether |
|---|---|---|
| 136 | 131 | 1-iodo-5,5-dimethyl-4-trimethylsilyloxy-*trans*-nonene |
| 137 | 132 | 1-iodo-4-methyl-4-trimethylsilyloxy-*trans*-1-nonene |
| 138 | 133 | 1-iodo-5,5-dimethyl-4-trimethylsilyloxy-*trans*-1-octene |
| 139 | 134 | 1-iodo-4-methyl-4-trimethylsilyloxy-*trans,-trans*-1,5-octadiene |
| 139a | 134a | 1-iodo-4-methyl-4-trimethylsilyloxy-*trans*-1-decene |

Example 140

Preparation of 4-benzoyloxy-1-octyne

To a stirred solution of 63. g (0.50 moles) of 1-octyn-4-ol (Example 93) in 500 ml of pyridine is added 77 g (0.55 moles) of benzoyl chloride. After stirring for 1.5 hours the mixture is treated with 10 ml of water, allowed to stand for 15 minutes, and concentrated. A solution of the residue in ether is washed successively with ice-cold hydrochloric acid, water, sodium bicarbonate solution, and brine. The solution is dried over magnesium sulfate, filtered through Celite, and concentrated to give an oil, $\lambda$ max. 3240 (terminal acetylene) and 1730 $cm^{-1}$ (benzyloxy group).

Example 141

Stereoselective Hydrolysis of Racemic 4-benzoyloxy-1-octyne by *Rhizopus arrhizus*

An agar slant of *R. arrhizus* (MUMF 1638) is used to inoculate 7 shake flasks (250 ml Erlenmeyer). Each flask contains 50 ml of a medium consisting of 2% Edamine, 2% glucose, and 0.72% corn steep liquor in water with pH adjusted to 7.0. A total of 14 such flasks are incubated on a rotary shaker at 28°C. After 72 hours incubation, 50 mg of racemic 4-benzoyloxy-1-octyne (Example 140) in 0.1 ml of acetone is added to each flask. After 28 hours the flasks are harvested and worked up by extraction of the whole mash with an equal volume of chloroform. The combined extracts are dried over magnesium sulfate and concentrated. The resulting oil is chromatographed on a column of silica gel with hexane progressively enriched in ethyl acetate.

From fractions 3—6 is obtained 150 mg of colourless oil, identical to 4-benzoyloxy-1-octyne, $(\alpha)_D^{25} = 5 \pm 1.0°$ (C=0.91, ethyl acetate). This compound has the (S)-configuration.

From fractions 13—20 is obtained 75 mg of colourless oil, identical to 4-hydroxy-1-octyne, $(\alpha)_D^{25} = -17 \pm 1.0°$ (C=0.77), ethyl acetate). This compound has the (R)-configuration.

The strain of *R. arrhizus* utilized in this experiment is a higher fungus which grows steadily on a variety of artificial media at 20°—25°C. In this study of the taxonomic aspects of the culture, Petri dishes of potato-dextrose, malt extract, and cornmeal agars were inoculated and incubated at ambient room temperature for 10 days. Observations of cultural and morphological characteristics are recorded in the description below:

Colonies on Petri dishes of potato-dextrose agar growing rapidly, covering the agar surface in 3—5 days and producing a thick, loose mat of greyish mycelium. Colony surface characterized by abundant black sporangia. Colony reverse greyish white. Colonies on malt extract agar growing rapidly, covering the agar surface in 3—5 days. Mycelial mat thick, greyish-yellow. Colony surface becoming brownish-black from masses of sporangia. Colony reverse yellowish. Colonies on cornmeal agar very thin, whitish; spreading across agar surface. Cultures transparent with relatively few sporangia produced. Visibility of micromorphology is good on this medium. Rhizoids produced sparingly along stoloniferous hyphae. Generally two to three sporangiophores arose from rhizoids. Walls of sporangiophores olive brown, 14.0—20.0 $\mu$m in width at base, tapering slightly to apex; 0.5—1.5 mm in length. Sporangiophores terminated by spherical sporangia, 130—225 $\mu$m in diameter. Columellae hemispherical, 3—50 $\mu$m high by 50—70 $\mu$m wide. Spores brownish when mature, 6.0—8.5 $\mu$m x 4.5—6.0 m. Spore walls conspicuously marked by longitudinal striations.

Example 142

Preparation of (S)-4-hydroxy-1-octyne

A solution of 1.15 g (5.0 mmoles) of (S)-4-benzoyl-oxy-1-octyne (Example 141) and 1.40 g (25 mmoles) of potassium hydroxide in 50 ml of 10:1 methanol-water is allowed to stand at room temperature for 24 hours. The bulk of the methanol is evaporated at room temperature, and the mixture is extracted with ether. The extract is washed with brine, dried over magnesium sulfate, and evaporated to give a colourless oil, identical to 4-hydroxy-1-octyne $(\alpha)_D^{25} = +17 \pm 1.0°$ (C=0.77, ethyl acetate). This compound has the (S)-configuration.

Examples 143—148a

The starting 1-alkyn-4-ols of Table XVII below are converted to the triphenylmethoxy substituted 1-alkynes by the method of Example 98.

TABLE XVII

| Example | Starting 1-Alkyn--4-ol of Example | Product Triphenylmethoxy Substituted 1-Alkyne |
|---|---|---|
| 143 | 116 | 4-triphenylmethoxy-5--*trans*-nonen-1-yne |
| 144 | 120 | 5-methyl-4-triphenylmethoxy-1-octyne |
| 145 | 121 | 5-methyl-4-triphenylmethoxy-1-nonyne |
| 146 | 124 | 4-triphenylmethoxy-5-*trans*-octen-1-yne |
| 147 | 141 | (R)-4-triphenylmethoxy-1-octyne |
| 148 | 142 | (S)-4-triphenylmethoxy-1-octyne |
| 148a | 124a | 4-triphenylmethoxy-5--*trans*-decen-1-yne |

Examples 149—154A

The product triphenylmethoxy substituted 1-iodo-1-*trans*-alkenes of Table XVIII below are prepared from the starting triphenylmethoxy substituted 1-alkynes of the table by the procedure described in Example 107.

TABLE XVIII

| Example | Starting Triphenylmethoxy Substituted 1-Alkyn of Example | Product Triphenylmethoxy Substituted 1-Iodo-*trans*-1-alkene |
|---|---|---|
| 149 | 143 | 1-iodo-4-triphenylmethoxy-*trans, trans*-1,5-nonadiene |
| 150 | 144 | 1-iodo-5-methyl-4-triphenylmethoxy-*trans*-1-octene |
| 151 | 145 | 1-iodo-5-methyl-4-triphenylmethoxy-*trans*-1-nonene |
| 152 | 146 | 1-iodo-4-triphenylmethoxy-*trans, trans*-1,5-octadiene |
| 153 | 147 | (R)-1-iodo-4-triphenylmethoxy-1-*trans*-octene |
| 154 | 148 | (S)-1-iodo-4-triphenylmethoxy-1-*trans*-octene |
| 154A | 148a | 1-iodo-4-triphenylmethoxy-*trans, trans*-1,5-decadiene |

Example 154B

Preparation of 4-Trimethylsiloxy-1-octyne

To a cold solution of 166 g of 4-hydroxy-1-octyne (Prostaglandins, *10*, 289 (1975)), and 240 g of imidazole in one litre of dimethylformamide is added dropwise 202 g of chlorotrimethylsilane. The mixture is allowed to stand at room temperature for 2 to 3 days. The mixture is partitioned with

water and hexane. The hexane layer is washed with brine, dried over magnesium sulfate, and concentrated. Distillation of the residue gives a colourless liquid, b.p. 38° (0.2 mm).

Example 154C

Preparation of 1-Iodo-4-trimethylsiloxy-*trans*-1-octene

To a stirred solution of 0.20 moles of freshly prepared bis-(3-methyl-2-butyl) borane in 300 ml of tetrahydrofuran at 0°—5°C is added dropwise a solution of 19.8 g of 4-trimethylsiloxy-1-octyne in 30 ml of tetrahydofuran. The resulting mixture is stirred at ambient temperature for several hours, cooled in an ice bath, and treated with 53 g of trimethylamine oxide. The mixture is stirred several hours at 25°—40°C and then poured into 2 litres of 15% sodium hydroxide. The resulting mixture is treated immediately with a solution of 140 g of iodine in 300 ml of tetrahydrofuran. After 0.5 hour the organic phase is separated and the aqueous gas is extracted with ether. The combined organic layers are washed with water, sodium thiosulfate solution, and brine; dried over magnesium sulfate; and concentrated to give an oil, p.m.r. spectrum ($CDCl_3$): 6.2 (d, IC*H*=) and 6.7 (quintuplet, =C*H*—).

Example 154D—E

Preparation of 4-Hydroxy-1-Iodo-*trans*-1-octene

A 23 g portion of 1-iodo-4-trimethylsiloxy-*trans*-1-octene is dissolved in a mixture of 200 ml of glacial acetic acid, 100 ml of tetrahydrofuran, and 50 ml of water. After solution occurs, toluene is added and the mixture is evaporated. The resulting oil is chromatographed on silica gel with hexane progressively enriched in benzene followed by acetone to give 16 g of an oil, p.m.r. spectrum (CDCl): 3.69 (m. C*H*OH) and 2.3 (s, O*H*).

Example 154F

Preparation of 4-Oxo-1-iodo-*trans*-1-octene

To a stirred suspension of 6.15 g of pyridinium chlorochromate (*Tetrahedron Letters, 1975,* 2647) in 20 ml of methylene chloride is added 450 mg of sodium acetate. After 5 minutes, a solution of 3.64 g of 4-hydroxy-1-iodo-*trans*-1-octene in 15 ml of methylene chloride is added in one portion. The dark mixture is stirred at room temperature for 75 minutes, diluted with 50 ml of ether, and decanted. The solid sludge is washed repeatedly with ether and decanted. The combined solutions are percolated through Florisil. The solution is concentrated to give an orange liquid, p.m.r. spectrum ($CDCl_3$): 3.20 (d,j = 7 cps;, = $CHCH_2CO$).

Example 154G

Preparation of 4-Hydroxy-4-(1-propyl)-1-iodo-*trans*-1-octene

To a stirred solution of propynyllithium at −25° is added a solution of 4-oxo-1-iodo-*trans*-1-octene in tetrahydrofuran. After the addition, the solution is stirred at −20° to −15°C for 30 minutes. The reaction is quenched with a mixture of hexane and ice. The aqueous phase is separated and extracted with additional hexane. The combined hexane extracts are washed successively with water and brine. The solution is dried over magnesium sulfate and concentrated. The residue is subjected to column chromatography on silica gel with toluene to provide the product as an oil.

Example 154H

Preparation of 4-Hydroxy-4(1-propynyl)-1-iodo-*trans*-1-decene

Treatment of 4-hydroxy-1-decyne (U.S. Pat. 3,950,406) by the procedures of Examples 154B, 154C, 154D, 154F and 154G is productive of the iodo-decene compound.

Example 154I—154J

Treatment of the iodoalkenes of Table IV with chlorotrimethylsilane by the procedure of Example 210H Infra is productive of the trimethylsilylether of the Table.

TABLE XVIIIA.

| Example | Starting Iodo Alkene | Product Silylether |
|---|---|---|
| 154I | 154G | 4-trimethylsilyloxy-4-(1-propynyl)-1-iodo-*trans*-1-octene |
| 154J | 154H | 4-trimethylsilyloxy-4-(1-propynyl)-1-iodo-*trans*-1-decane |

Example 155

Preparation of ethyl-*p*-fluorophenoxy-acetate

To a stirred solution of 50 g (0.29 moles) of *p*-fluorophenoxy acetic acid in one litre of absolute ethanol is added 10 ml of sulfuric acid. The mixture is heated to reflux for 18 hours, cooled to room temperature, and evaporated under vacuum. It is then poured onto 300 g of ice, extracted twice with 500 ml of ether, washed twice with 250 ml of a saturated solution of sodium bicarbonate, 100 ml of saturated sodium chloride solution, dried with magnesium sulfate, filtered and evaporated under vacuum giving 58 g of an oil. This is crystallized from 50 ml of hexane at −25°C to give 55 g (90%) of the subject product as colourless crystals, mp 32°—33°C.

Example 156

Preparation of *p*-fluorophenyoxy acetaldehyde

To a stirred solution of 1.98 g (10 mmoles) of ethyl-*p*-fluorophenoxy acetate (Example 155) in 15 ml of dry toluene, cooled to −78°C, under argon, is added dropwise over 30 minutes, 8 ml of a 1.4M solution of diisobutylaluminum hydride in toluene (11 mmoles). The mixture is stirred for 2 hours at −78°C, 1 ml of methanol is added, followed by 5 ml of water, dropwise. The gel formed is filtered through Celite and washed with 100 ml of ether, portionwise. The organic phase is separated, washed twice with 25 ml of a saturated brine solution, dried with magnesium sulfate, filtered, and evaporated. The oil obtained is distilled at 71°—73°C (0.1 mm) to give 600 mg (45%) of the subject product as a colourless liquid.

Example 157

Preparation of 3-hydroxy-4-*p*-fluorophenoxy-1-butyne

Acetylene gas, dried by passing through a trap containing sulfuric acid is bubbled at a moderate rate, through 5 ml of vigorously stirred tetrahydrofuran, for 15 minutes. To this acetylenic solution, is then added dropwise, with continued passage of acetylene, 3.5 ml of a 2.4M solution of *n*-butylmagnesium chloride in tetrahydrofuran (8.4 mmoles) over 45 minutes. The mixture is stirred a further 15 minutes and a solution of 580 mg (3.9 mmoles) of *p*-fluorophenoxy acetaldehyde (Example 156) in 3 ml of tetrahydrofuran is added dropwise over 15 minutes. This solution is stirred for 2 more hours, with pasage of acetylene, poured into 50 ml of a saturated solution of ammonium chloride, extracted twice with 50 ml of ether, washed with 10 ml of ammonium chloride solution, dried with magnesium sulfate, filtered and evaporated. The crude subject product is purified by sublimation at 75°C (0.1 mm) for 5 hours to give 330 mg (48%) of white crystals, mp 46°—47°C.

Example 158

Preparation of 4-*p*-fluorophenoxy-3-trimethylsilyloxy-1-butyne

To a 0°C solution of 10 g (55 mmoles) of 3-hydroxy-4-*p*-fluorophenoxy-1-butyne (Example 157) in 75 ml of dry dimethylformamide and 88 g (130 mmoles) of imidazole is added dropwise, with stirring, 7.5 g (68 mmoles) of chlorotrimethylsilane. The mixture, while under an argon atmosphere, is stirred at room temperature for 18 hours, and then poured into 150 ml of hexane and 100 ml of ice-water. The organic phase is separated, washed with 50 ml of a brine solution, dried with magnesium sulfate, and evaporated under vacuum. This crude product is distilled under vacuum at 0.1 mm (bp 73°—75°C), to give 12.2 g (91%) of the subject compound as a colourless oily liquid.

Example 159

Preparation of 1-tri-*n*-butylstannyl-4-*p*-fluorophenoxy-3-trimethylsilyloxy-*trans*-1-butene

A mixture of 2.52 g (10 mmoles) of 3-trimethylsilyloxy-4-*p*-fluorophenoxy-1-butyne (Example 158), 2.91 g (10 mmoles) of tri-*n*-butyl-tin hydride, and 10 mg of azobisisobutyronitrile is heated, under an argon atmosphere, with stirring, for 2 hours at 140°C. After cooling to room temperature, the crude reaction mixture is fractionally distilled at 180°—185°C (0.05 mm), to give 4.6 g (85%) of the subject product as a colourless liquid.

Examples 160—162

The product esters of Table XIX below are obtained by the procedure described in Example 155.

TABLE XIX

| Example | Starting Aryloxy Acid | Product Aryloxy Ethyl Ester |
|---|---|---|
| 160 | *m*-chlorophenoxy-acetic acid | ethyl-*m*-chlorophenoxy-acetate |
| 161 | 3,4-dichlorophen-oxyacetic acid | ethyl-3,4-dichlorophen-oxyacetate |
| 162 | phenoxyacetic acid | ethyl phenoxyacetate |

Example 163

Preparation of ethyl-*m*-trifluoromethylphenoxy-acetate

A mixture of 100 g (.618 mole) of $\alpha$, $\alpha$, $\alpha$-trifluoro-*m*-cresol, 106 g (.632 mole) of ethyl bromoacetate, 87.5 g (.632 mole) of potassium carbonate, and 1500 ml of acetone is stirred at reflux for 4 hours, and at room temperature for 18 hours. The mixture is filtered, evaporated under vacuum on a rotary evaporator at 45°C and at 85°C (0.1 mm) to remove excess ethyl bromoacetate. The reaction mixture is taken up in 500 ml of ether, washed three times with 100 ml each of 0.1M potassium carbonate, once with 100 ml of water, 100 ml of .01M hydrochloric acid, and 100 ml of water. It is then dried with magnesium sulfate, filtered and evaporated, giving 142 g of the crude product. This is fractionally distilled at 73°—75°C (0.1 mm) to give 124 g of the purified subject product as a colourless liquid.

Examples 164 and 166

The product esters of Table XX are obtained by treating the starting phenols with ethyl bromoacetate by the procedure of Example 163. [There is no Example 165].

TABLE XX

| Example | Starting Phenol | Product Ester |
|---|---|---|
| 164 | *p*-bromophenol | ethyl *p*-bromophenoxyacetate |
| 166 | *p*-methoxyphenol | ethyl *p*-methoxyphenoxyacetic acid |

Examples 167—173

Following the procedure of Example 156, the starting esters of Table XXI are treated with diisobutylaluminum hydride to provide the product aldehydes of the table. [There is no Example 169].

TABLE XXI

| Example | Starting Ester | Product Aldehyde |
|---|---|---|
| 167 | 163 | *m*-trifluoromethylphenoxy acetaldehyde |
| 168 | 164 | *p*-bromophenoxy acetaldehyde |
| 170 | 166 | *p*-methoxyphenoxy acetaldehyde |
| 171 | 160 | *m*-chlorophenoxy acetaldehyde |
| 172 | 161 | 3,4-dichlorophenoxy acetaldehyde |
| 173 | 162 | phenoxyacetaldehyde |

Examples 174—180d

Following the procedure of Example 157, treatment of the starting aldehyde of Table XXII with acetylene magnesium chloride provides the product alkynes of Table XXII. [There is no Example 176].

TABLE XXII

| Example | Starting Aldehyde | Product Aryloxy Alkyne |
|---|---|---|
| 174 | 167 | 3-hydroxy-4-*m*-trifluoromethylphenoxy-1-butyne |
| 175 | 168 | 3-hydroxy-4-*p*-bromophenoxy-1-butyne |
| 177 | 170 | 3-hydroxy-4-*p*-methoxyphenoxy-1-butyne |
| 178 | 171 | 3-hydroxy-4-*m*-chlorophenoxy-1-butyne |
| 179 | 172 | 3-hydroxy-4-(3,4-dichlorophenoxy)-1-butyne |
| 180 | 173 | 3-hydroxy-4-phenoxy-1-butyne |
| 180a | a | 3-hydroxy-5-phenyl-1-pentyne |
| 180b | b | 3-hydroxy-5-(*p*-chlorophenyl)-1-pentyne |
| 180c | c | 3-hydroxy-5-(*p*-methoxyphenyl)-1-pentyne |
| 180d | d | 3-hydroxy-5-(*m*-trifluoromethylphenyl)-1-pentyne |

a. hydrocinnamaldehyde[1]
b. *p*-chlorohydrocinnamaldehyde[1]
c. *p*-methoxyhydrocinnamaldehyde[1]
d. *m*-trifluoromethylhydrocinnamaldehyde[2]

1) Billmann, *et al.*, Synthetic Communications, *1*, 127—131 (1971).
2) Lednicer, Journ. Med. Chem., *11*, 1258 (1968).

Examples 180e—186e

Treatment of the starting alkynes of Table XXIII by the procedure of Example 158 followed by treatment of the procedure of Example 159 provides the product (E) 1-tri-*n*-butyltin-1-alkenes of the table. [There are no Examples 182, and 186a].

TABLE XXIII

| Example | Starting Alkyne | Product (E)-1-tri-*n*-butyltin-1-alkene |
|---|---|---|
| 180e | 174 | (E)-1-tri-*n*-butylstannyl-3-trimethylsilyloxy-4-*m*-trifluoromethylphenoxy-1-butene |
| 181 | 175 | (E)-1-tri-*n*-butylstannyl-3-trimethylsilyloxy-4-*p*-bromophenoxy-1-butene |
| 183 | 177 | (E)-1-tri-*n*-butylstannyl-3-trimethylsilyloxy-4-*p*-methoxyphenoxy-1-butene |
| 184 | 178 | (E)-1-tri-*n*-butylstannyl-3-trimethylsilyloxy-4-*m*-chlorophenoxy-1-butene |
| 185 | 179 | (E)-1-tri-*n*-butylstannyl-3-trimethylsilyloxy-3,4-dichlorophenoxy-1-butene |
| 186 | 180 | (E)-1-tri-*n*-butylstannyl-3-trimethylsilyloxy-4-phenoxy-1-butene |
| 186b | 180a | (E)-1-tri-*n*-butylstannyl-3-trimethylsilyloxy-5-phenyl-1-pentene |
| 186c | 180b | (E)-1-tri-*n*-butylstannyl-3-trimethylsilyloxy-5-(*p*-chlorophenyl)-1-pentene |
| 186d | 180c | (E)-1-tri-*n*-butylstannyl-3-trimethylsilyloxy-5-(*p*-methoxyphenyl)-1-pentene |
| 186e | 180d | (E)-1-tri-*n*-butylstannyl-3-trimethylsilyloxy-5-(*m*-trifluoromethylphenyl)-1-pentene |

Examples 187

Preparation of 1-chloro-1-octen-3-one

This compound is prepared according to the procedure of Price and Pappalardo (C. C. Price and J. A. Pappalardo, Org. Syn, *32*, 27 (1952)) from hexanoyl chloride, acetylene, and aluminum chloride in 94% yield, bp 51°—52°C (0.1 mm): $\lambda$ max 1680, 1595, 941 $cm^{-1}$.

Example 188

Preparation of 1-iodo-1-octen-3-one

A mixture of 25 g (0.16 moles) of 1-chloro-1-octen-3-one (Example 187) and 35 g (0.23 moles) of sodium iodide in 200 ml of reagent acetone is stirred at the reflux temperature for 18 hours. The cooled mixture is filtered and the mother liquor taken to dryness. The residual oil is dissolved in benzene and the solution is washed with 5% sodium thiosulfate solution, saturated sodium chloride solution, dried and taken to dryness. The residual oil is crystallized from hexane to give 26 g of a white solid, mp 35°—37°C; $\lambda$ max 1670, 950 $cm^{-1}$.

Example 189

Preparation of 3-hydroxy-1-iodo-3-methyl-1-octene

To a Grignard solution prepared from 1.05 g (0.41 moles) of magnesium and 6.2 g (0.435 moles) of methyl iodide in 30 ml of dry ether under argon is added dropwise 10 g of 1-iodo-1-octen-3-one (Example 188) in 45 ml of ether. The resulting solution is stirred at ambient temperature for one hour. After the addition of 75 ml of saturated ammonium chloride the ether layer is separated and the aqueous layer is separated and the aqueous layer is extracted several times with ether. The combined

ether extracts are washed successively with ammonium chloride and water, dried and taken to dryness to give 9.24 g of product as an oil; λ max 2.89, 3.23, 6.24 and 10.5.

Example 190

Preparation of 1-iodo-3-methyl-3-trimethylsilyloxy-1-octene

To a stirred solution of 11.7 g of 3-hydroxy-1-iodo-3-methyl-1-octene (Example 189) and 7.4 g of imidazole in 45 ml of dry dimethylformamide is added dropwise 5.98 g of trimethylsilylchloride at 0°C under argon atmosphere. After stirring at 0°C for an additional 15 minutes, the solution is stirred at ambient temperature for 18 hours. The reaction mixture is poured into 600 ml of hexane and the resulting solution washed with water, saturated sodium chloride solution, dried over anhydrous magnesium sulfate and taken to dryness to furnish 14.7 g of oil. Distillation affords 13.4 g of clear oil; bp 65°C (0.05 mm); λ max 6.21, 800, 9.90, 10.51, 11.90, 13.2μ.

Example 190a

Preparation of 1-iodo-3-methyl-3-trimethylsilyloxy-1-decene

Treatment of octanoylchloride by the procedures of Example 187 followed by treatment of the resulting 1-chloro-1-decen-3-one by the procedure of Example 188 followed by treatment according to Examples 189 and 190 is productive of the named compound.

There are no Examples 191—193

Example 194

Preparation of 4-trimethylsiloxy-4-vinyl-1-iodo-*trans*-1-octene

To a stirred solution of 456 mg of 4-hydroxy-4-vinyl-1-iodo-*trans*-1-octene and 320 mg of imidazole in 1.0 m of dimethylformamide is added 0.23 ml of chlorotrimethylsilane during 3 minutes. The mixture is stirred at room temperature for 22 hours and partitioned with a mixture of cold hexane and water. The hexane layer is washed repeatedly with water and then brine, dried over magnesium sulfate, and concentrated to give an oil, p.m.r. spectrum ($CDCl_3$): 0.13 (s, trimethylsiloxy group) and 2.32 (d, $=CHCH_2$).

Example 195

Preparation of *n*-butyl cyclopropyl ketone

To a vigorously-stirred solution of 31.0 g of cyclopropanecarboxylic acid in 330 ml of ether is added a solution of *n*-butyllithium (748 mmoles) in about 750 ml of 2:1 ether-hexane during one hour at 5°—10°C. The resulting suspension is diluted with 300 ml of ether and stirred at room temperature for 2 hours and at reflux for 2 hours. The mixture is cooled and poured into several portions of 1:1 ice: 4N hydrochloric acid. The ethereal phases are combined and washed with brine, sodium carbonate solution, and brine. The extract is dried over magnesium sulfate and concentrated. The residue is distilled to provide a liquid, bp 102°—104°C (80 mm) p.m.r. spectrum ($CDCl_3$): δ 2.55 (triplet, $—CH_2CO—$).

Example 196

Preparation of 4-cyclopropyl-4-hydroxy-1-octyne

To a stirred, refluxing suspension of amalgum prepared from 6.2 g of magnesium and 50 mg of mercuric chloride suspended in 60 ml of ether is added a solution of a mixture of 30.4 g of *n*-butyl cyclopropyl ketone (Example 195) and 29.8 g of propargyl bromide in 65 ml of ether during 60 minutes. After reaction at reflux temperature for an additional 30 minutes, the mixture is cooled to 0°C and treated with 35 ml of saturated ammonium chloride. The mixture is diluted with ether and filtered through Celite. The filtrate is washed with brine, dried over potassium carbonate, and concentrated. The residue is distilled to provide a liquid δ 0.43 (cyclopropyl hydrogens), 2.07 (triplet, $HC{\equiv}C$), and 2.44 (doublet, $C{\equiv}CCH_2$).

Example 197

Preparation of 4-cyclopropyl-4-trimethylsiloxy-1-octyne

To a stirred solution of 27.8 g of 4-cyclopropyl-4-hydroxy-1-octyne (Example 196) and 33.3 g of imidazole in 130 ml of dimethylformamide at 5°C is added 24 ml of chlorotrimethylsilane during 5 minutes. The solution is stirred at ambient temperature for 17 hours and then partitioned with 600 ml of hexane and 250 ml of ice water. The hexane phase is separated and washed successively with water and brine. The solution is dried over magnesium sulfate and evaporated to give a liquid, p.m.r. spectrum ($CDCl_3$): δ 0.12 (singlet, trimethylsiloxy group), 2.02 (triplet, $HC{\equiv}C$), and 2.45 (doublet, $C{\equiv}CH_2$).

Example 198

Preparation of 4-cyclopropyl-4-trimethylsiloxy-1-(tri-*n*-butylstannyl)-*trans*-1-octene

A stirred mixture of 23.8 g of 4-cyclopropyl-4-trimethylsiloxy-1-octyne (Example 197), 28 ml of tri-*n*-butyltin hydride, and 50 mg of azobisisobutyronitrile under nitrogen is heated to 85°C. After the

resulting exothermic reaction subsides the mixture is heated at 130°C for one hour. The crude product is evaporatively distilled to give a liquid, p.m.r. spectrum ($CDCl_3$): $\delta$ 0.10 (trimethylsiloxy group, 2.33 (doublet, $=CHCH_2$), and 6.02 (vinyl hydrogens).

Examples 199—204a

Treatment of the starting carboxylic acids of Table XXIV with the appropriate alkyllithium by the method of Example 195 provides the product ketones of the table.

TABLE XXIV

| Example | Starting Carboxylic Acid | Alkyl Lithium | Product Ketone |
|---|---|---|---|
| 199 | cyclopropane carboxylic acid | *n*-hexyllithium | *n*-hexylcyclopropyl ketone |
| 200 | cyclopropane carboxylic acid | *n*-propyllithium | *n*-propylcyclopropyl ketone |
| 201 | acrylic acid | *n*-hexyllithium | *n*-hexylvinyl ketone |
| 202 | acrylic acid | *n*-propyllithium | *n*-propylvinyl ketone |
| 203 | crotonic acid | *n*-butyllithium | *n*-butyl-1-propenyl ketone |
| 204 | crotonic acid | *n*-hexyllithium | *n*-hexyl-1-propenyl-ketone |
| 204a | acrylic acid | *n*-butyllithium | *n*-butylvinyl ketone |

Examples 205—210E

Treatment of the starting ketones of Table XXV with propargymagnesium bromide by the procedure of Example 196 followed by treatment with chlorotrimethylsilane by the procedure of Example 197 followed by treatment with tri-*n*-butyltin hydride by the method of Example 198 is productive of the vinylstannyl derivatives of the table.

TABLE XXV

| Example | Starting Ketone | Product Vinylstannyl Derivative |
|---|---|---|
| 205 | 199 | (E) 4-trimethylsilyloxy-4-cyclopropyl-1-tri-*n*-butylstannyl dec-1-ene |
| 206 | 200 | (E) 4-trimethylsilyloxy-4-cyclopropyl-1-tri-*n*-butylstannyl dec-1-ene |
| 207 | 201 | (E) 4-trimethylsilyloxy-4-vinyl-1-tri-*n*-butylstannyl dec-1-ene |
| 208 | 202 | (E) 4-trimethylsilyloxy-4-vinyl-1-tri-*n*-butylstannyl dec-1-ene |
| 209 | 203 | (E) 4-trimethylsilyloxy-4-(1-propenyl)-1-tri-*n*-butylstannyl-oct-1-ene |
| 210 | 204 | (E) 4-trimethylsilyloxy-4-(1-propenyl)-1-tri-*n*-butyl-1-decene |
| 210A | 204a | (E) 4-trimethylsilyloxy-4-vinyl-1-tri-*n*-butylstannyl-1-octene |
| 210B | 2-hexanone | (E) 4-trimethylsilyloxy-4-methyl-1-tri-*n*-butylstannyl-1-octene |
| 210C | 3-heptanone | (E) 4-trimethylsilyloxy-4-ethyl-1-tri-*n*-butylstannyl-1-octene |
| 210D | 3-octanone | (E) 4-trimethylsilyloxy-4-ethyl-1-tri-*n*-butylstannyl-1-nonene |
| 210E | 3-hexanone | (E) 4-trimethylsilyloxy-4-ethyl-1-tri-*n*-butylstannyl-1-heptene |

Example 210F

*n*-Butyl trimethylsilylethynyl ketone

To a stirred solution of 14.4 g of valeryl chloride and 20.4 g of bis-trimethylsilylacetylene in 300 ml of dry methylene chloride, cooled in an ice bath, is added powdered anhydrous aluminum chloride, portionwise, over a period of 20 minutes. The mixture is stirred for 5 minutes, then the cooling bath is removed and the mixture is stirred at room temperature for 4 hours. The mixture is poured into 500 ml of ice-water. The organic layer is separated, washed with water and brine, dried over anhydrous sodium sulfate and filtered through diatomaceous earth. The mother liquor is evaporated to dryness giving a brownish residue. This residue is Kugelrohr-distilled to give 16.56 g of colourless liquid at 45°C/0.3 mm which is essentially identical with the authentic product.

Example 210G

4-Trimethylsilylethynyl-1-octyn-4-ol

To a stirred suspension of 1.29 g of magnesium and 10 mg of mercuric chloride in 12 ml of ether is added 0.4 ml of propargyl bromide. The reaction is initiated after stirring at room temperature for a few minutes. The stirred mixture is cooled in an ice-water bath and a solution of 9.64 g of *n*-butyl trimethylsilylethynyl ketone and 3.51 ml of propargyl bromide in 13 ml of ether is added dropwise so that the mixture is very gently boiling during 40 minutes. After addition, the cooling bath is removed and the mixture is stirred at room temperature for 1.5 hours. The mixture is recooled in an ice bath and 10 ml of saturated ammonium chloride solution is added. The resulting white mixture is filtered through diatomaceous earth. The clear mother liquor is washed with saturated sodium chloride solution and dried over anhydrous magnesium sulfate. The solvent is evaporated to dryness giving 10.5 g of a red liquid. This liquid is Kugelrohr-distilled at 60°C/0.25—0.3 mm. The pale yellow liquid distillate which is the desired product weighs 8.5 g.

Example 210H

4-Trimethylsilylethynyl-1-octyn-4-ol trimethylsilyl ether

To a stirred mixture of 8.5 g of 4-trimethylsilyl-ethynyl-1-octyn-4-ol and 6.2 g of imidazole in 24 ml of dry dimethylformamide is added, under nitrogen, 5.7 ml of chlorotrimethylsilane, in a slow stream, via a syringe. The mixture is stirred in an ice bath for one hour and then at room temperature overnight. The mixture is poured into hexane, washed with saturated sodium bicarbonate solution, water and then brine and dried over sodium sulfate. The solvents are evaporated to dryness giving 11.1 g of the desired product.

Example 210I

4-trimethylsilylethynyl-4-trimethylsiloxy-1-tri-*n*-butylstannyl-1-octene

To a mixture of 10 mg of azobisisobutyronitrile and 2.94 g of 4-trimethylsilylethynyl-1-octyn-4-ol trimethylsilyl ether is added 2.65 ml of tri-*n*-butyl stannane via a syringe. The mixture is stirred and heated under nitrogen in an oil bath at 130°C for 3 hours and then cooled to room temperature. This mixture is vacuum-distilled through a short-path distillation apparatus to remove a forerun at 40°C/0.4 mm. The yellow oil (pot residue) comprises the desired product.

Examples 210L—210M

Treatment of the acid chlorides of Table I by the procedure of Example 210F with bis-trimethylsilylacetylene is productive of the ketones of Table XXVA.

TABLE XXVA

| Example | Starting Acid Chloride | Product Alkyl Trimethylsilylethynyl Ketone |
|---|---|---|
| 210L | butyryl chloride | *n*-propyl trimethylsilylethynyl ketone |
| 210M | heptanoyl chloride | *n*-hexyl trimethylsilylethynyl ketone |

Examples 210N—2100

Treatment of the ketones of Table XXVB by the procedure of Example 210G is productive of the 4-trimethylsilylethynyl-1-alkyn-4-ol's of the Table.

TABLE XXVB

| Example | Starting Ketone | Product 4-Trimethylsilylethynyl-1-alkyn-4-ol |
|---|---|---|
| 210N | 210L | 4-trimethylsilylethynyl-1-heptyn-4-ol |
| 2100 | 210M | 4-trimethylsilylethynyl-1-decyn-4-ol |

Examples 210P—210Q

Treatment of the alkyn-4-ol's of Table XXVB with chlorotrimethylsilane by the procedure of Example 210H followed by treatment of the resulting trimethylsilylether with tri-*n*-butylstannane by the procedure of Example 210I is productive of alkenes of Table XXVC.

TABLE XXVC

| Example | Starting Alkyn-4-ol | Product Alkene |
|---|---|---|
| 210P | 210N | (E) 4-trimethylsilylethynyl-4-trimethylsiloxy-1-tri-*n*-butylstannyl-1-heptene |
| 210Q | 2100 | (E) 4-trimethylethynyl-4-trimethylsilyloxy-1-tri-*n*-butylstannyl-1-decene |

Example 211

Preparation of *trans*-1-Hydroxy-2-(3-trifluoromethyl)phenoxycyclopentane

A mixture of 88 g of cyclopentene oxide, 150.7 g of 3-trifluoromethylphenol, 5.0 g of sodium hydroxide in 30 ml of water and 4.0 g of methyltricaprylyl ammonium chloride is stirred at 70°—80°C for 51 hours and at 25°C for 96 hours. The mixture is then diluted with methylene chloride and poured into water. The organic layer is washed with dilute sodium hydroxide solution and water. The solution is dried over magnesium sulfate. The solvent is removed giving 221.5 g of a liquid which is distilled (bp 110°—113°C 0.8 mm) giving *trans*-1-hydroxy-2-(3-trifluoromethyl)phenoxycyclopentane.

Example 212

In the manner described above in Example 211 from 4-fluorophenol and cyclopentane epoxide is prepared *trans*-1-hydroxy-2-(4-fluoro)phenoxycyclopentane.

Example 213

In the manner described above in Example 211 from 3-chlorophenol and cyclopentane epoxide is prepared *trans*-1-hydroxy-2-(3-chloro)phenoxycyclopentane.

Example 214

Preparation of 2-(3-Trifluoromethylphenoxy)cyclopentanone

To a suspension of 327.43 g of pyridinium chlorochromate in one litre of methylene chloride is added 220 g of *trans*-1-hydroxy-2-(3-trifluoromethylphenoxy) cyclopentane in 500 ml of methylene chloride. The mixture is stirred for 2 hours 15 minutes. Another 50 g of the oxidizing agent is added and the mixture is stirred for 4½ hours. The mixture is diluted with ether and decanted from a black residue which is washed with more ether. The combined solutions are filtered through silica gel. The solvent is removed. The residue is dissolved in ether and again filtered through silica-gel. The solvent is removed and the residue is distilled (bp 113°—116°C, 1.5 mm) to give 188 g of 2-(3-trifluoromethylphenoxy)cyclopentanone.

Example 215

In the manner described above for Example 214 is prepared from the product of Example 212; 2-(4-fluorophenoxy)cyclopentanone.

Example 216

In the manner described above for Example 214 is prepared from the product of Example 213; 2-(3-chlorophenoxy)cyclopentanone.

Example 217

Preparation of 1R, 2S (and 1S, 2R)-1-Ethynyl-1-hydroxy-2-butyl-cyclopentane and 1R, 2R (and 1S, 2S)-1-ethynyl-1-hydroxy-2-butylcyclopentane

Into 150 ml of dry tetrahydrofuran is bubbled purified acetylene, as a solution of 2.4M *n*-butyl magnesium chloride (92 ml) is added dropwise with stirring over a 2 hour period. To the resulting solution of acetylene magnesium chloride is added 21 g of 2-butylcyclopentanone in 50 ml of tetrahydrofuran dropwise over 15 minutes. The solution is stirred for 30 minutes and then is poured into an ice cold solution of saturated ammonium chloride. The mixture is acidified to pH 5 and extracted with ether. The ether solution is washed with brine and dried over magnesium chloride. The ether is removed and the residue is distilled giving 14.8 g of a colourless liquid. This is chromatographed on a dry column of silica-gel eluting with benzene-ethyl acetate (19:1) to separate isomers giving 1R, 2S (and 1S, 2R)-1-ethynyl-1-hydroxy-2-butylcyclopentane and 1R, 2R (and 1S, 2S)-1-ethynyl-1-hydroxy-2-butylcyclopentane.

Example 218

Preparation of 1-propargyl-1-hydroxycyclohexane

A stirred suspension of 121.6 g (5.0 mol) of magnesium in 1—1 of anhydrous ether is treated with 0.6 g of mercuric chloride and about 100 mg of iodine. After several minutes, 3 ml of propargyl bromide is added and if no exotherm is noted, a small amount of reacting propargyl bromide and magnesium in ether is added. When the reaction begins, a mixture of 5.0 mol of cyclohexanone and 595 g (5.0 mol) of propargyl bromide is added dropwise at a rate that produces vigorous refluxing of the solution. (The propargyl bromide must always be present in some excess otherwise the reaction will stop. If this happens, the addition of about 1 ml of propargyl bromide will restart the reaction.) After about half of the propargyl bromide-cyclohexanone mixture has been added, another 500—750 ml of ether is used to dilute the reaction mixture. At the end of the addition, the reaction mixture is refluxed for at least 0.5 hour, cooled and poured into 4 litres of saturated ammonium chloride during good stirring. The ethereal layer is separated and the aqueous layer is washed with ether several times and the combined extract is washed twice with saturated sodium chloride solution and dried over

anhydrous magnesium sulfate. Evaporation of the ether yields 583 g (630 g theory) of a dark oil which is distilled giving purified 1-propargyl-1-hydroxycyclohexane.

Examples 219—238

In the manner of Examples 217 and 218 described above the following acetylenic alcohols listed in Table XXVI were prepared from the acetylenic Grignard reagent and ketone specified.

TABLE XXVI

| Example | Grignard Reagent | Ketone | Acetylenic Alcohol |
|---|---|---|---|
| 219 | acetylene magnesium chloride | cyclohexanone | 1-ethynyl-1-hydroxycyclohexane |
| 220 | acetylene magnesium chloride | cyclopentanone | 1-ethynyl-1-hydroxycyclopentane |
| 221 | acetylene magnesium chloride | cycloheptanone | 1-ethynyl-1-hydroxycycloheptane |
| 222 | acetylene magnesium chloride | 3-propylcyclopentanone | 1R, 3S- (and 1S, 3R-) 1-ethynyl-1-hydroxy-3-propylcyclopentane |
| 223 | acetylene magnesium chloride | 3-propylcyclopentanone | 1R, 3R- (and 1S, 3S-) 1-ethynyl-1-hydroxy-3-propylcyclopentane |
| 224 | acetylene magnesium chloride | 2-butylcyclohexanone | 1R, 2S- (and 1S, 2R-) 1-ethynyl-1-hydroxy-2-butylcyclohexane |
| 225 | acetylene magnesium chloride | 2-butylcyclohexanone | 1R, 2R- (and 1S, 2S-) 1-ethynyl-1-hydroxy-2-butylcyclohexane |
| 226 | acetylene magnesium chloride | 2-(3-trifluoromethyl-phenoxy)cyclopentanone | 1R, 2S- (and 1S, 2R-) 1-ethynyl-1-hydroxy-2-(3-trifluoromethylphenoxy)cyclopentane |
| 227 | acetylene magnesium chloride | 2-(3-trifluoromethyl-phenoxy)cyclopentanone | 1R, 2R- (and 1S, 2S-) 1-ethynyl-1-hydroxy-2-(3-trifluoromethylphenoxy)cyclopentane |
| 228 | acetylene magnesium chloride | 2-(4-fluorophenoxy)-cyclopentanone | 1R, 2S- (and 1S, 2R-) 1-ethynyl-1-hydroxy-2-(4-fluorophenoxy)cyclopentane |
| 229 | acetylene magnesium chloride | 2-(4-fluorophenoxy)-cyclopentanone | 1R, 2R- (and 1S, 2S-) 1-ethynyl-1-hydroxy-2-(4-fluorophenoxy)cyclopentane |
| 230 | acetylene magnesium chloride | 2-(3-chlorophenoxy)-cyclopentanone | 1R, 2S- (and 1S, 2R-) 1-ethynyl-1-hydroxy-2-(3-chlorophenoxy)cyclopentane |
| 231 | acetylene magnesium chloride | 2-(3-chlorophenoxy)-cyclopentanone | 1R, 2R- (and 1S, 2S-) 1-ethynyl-1-hydroxy-2-(3-chlorophenoxy)cyclopentane |

TABLE XXVI (continued)

| Example | Grignard Reagent | Ketone | Acetylenic Alcohol |
|---|---|---|---|
| 232 | acetylene magnesium chloride | 3-methylcyclohexanone | 1R, 3S- (and 1S, 3R-) 1-ethynyl-1-hydroxy-3-methylcyclohexane |
| 233, 234 | acetylene magnesium chloride | 3-methylcyclohexanone | 1R, 3R- (and 1S, 3S-) 1-ethynyl-1-hydroxy-3-methylcyclohexane |
| 235 | propargyl magnesium bromide | 2-butylcyclopentanone | 1R, 2S- (and 1S, 2R-) 1-propargyl-1-hydroxy-2-butylcyclopentane |
| 236 | propargyl magnesium bromide | 2-butylcyclopentanone | 1R, 2R- (and 1S, 2S-) 1-propargyl-1-hydroxy-2-butylcyclopentane |
| 237 | propargyl magnesium bromide | 2-(3-trifluoromethyl-phenoxy)cyclopentanone | 1R, 2S- (and 1S, 2R-) 1-propargyl-1-hydroxy-2-(3-trifluoromethylphenoxy)cyclopentane |
| 238 | propargyl magnesium bromide | 2-(3-trifluoromethyl-phenoxy)cyclopentanone | 1R, 2R- (and 1S, 2S-) 1-propargyl-1-hydroxy-2-(3-trifluoromethylphenoxy)cyclopentane |

Example 239

Preparation of 1R,2S(and 1S,2R)-1-Ethynyl-1-trimethylsilyloxy-2-butylcyclopentane

To a solution of 29.4 g of 1R,2S(and 1S,2R)-1-ethynyl-1-hydroxy-2-butylcyclopentane and 30.2 g of imidazole in 180 ml of dimethylformamide is added at 0°C with stirring 24.1 g of trimethylsilylchloride. The mixture is stirred for 3 hours. The mixture is poured into 700 ml of hexane and washed twice with water and one with brine. The ether solution is dried over magnesium sulfate. The solvent is removed and the residue is distilled (bp 64°—72°C, 0.6 mm) to give 35.8 g of 1R,2S(and 1S,2R)-1-ethynyl-1-trimethylsilyloxy-2-butylcyclopentane.

Example 240

Preparation of 1R,2R(and 1S,2S)-1-Ethynyl-1-trimethylsilyloxy-2-butylcyclopentane

To a mixture of 45.0 g of 1R,2R(and 1S,2S)-ethynyl-1-hydroxy-2-butylcyclopentane and 46.2 g of imidazole in 255 ml of dimethylformamide at 0°C under nitrogen is added 36.9 g of trimethylsilylchloride. The mixture is stirred at room temperature for 3 hours and then poured into 700 ml of hexane. Water is added, the organic layer is separated and the water layer is extracted with hexane. The combined hexane solutions are washed twice with water and dried over magnesium sulfate. The solvent is removed and the residue is distilled giving the product as 53 g of a colourless oil.

Example 241

Preparation of 1-Ethynyl-1-trimethyl-silyloxycyclohexane

A 194 g portion of imidazole and 158.2 g of 1-ethynylcyclohexan-1-ol are mixed with 500 g of dimethylformamide with cooling in an ice bath. A 152 g portion of trimethylformamide is added with cooling and stirring in about one minute. The mixture is stirred for one hour and allowed to stand overnight. One litre of hexane is added. The lower layer is separated, diluted with water and extracted with hexane. The hexane layers are washed several times with water and then combined and dried over magnesium sulfate. Filtration and then evaporation of the hexane gives 198.5 g of product which is distilled giving 168 g of the desired product.

Example 242

Preparation of 1-Propargyl-1-trimethylsilyloxycyclohexane

To a stirred solution of 55.4 g of 1-(2-propyn-1-yl)cyclohexanol (H. Gutmann, *et al., Helv. Chim. Acta, 42,* 719 (1959)) and 79 g of imidazole in 240 ml of DMF at 10°C initially is added 56 ml of chlorodimethylsilane during 10 minutes. The cloudy yellow solution is stirred at room temperature for 26 hours. The resulting mixture is partitioned between 1000 ml of hexane and 400 ml of water at 0°—5°C. The hexane phase is washed successively with 6 x 200 ml of cold water and 200 ml of brine. The extract is dried over magnesium sulfate, filtered, and evaporated to give 85 g of colourless liquid, i.r. (film): 1240 and 830 $cm^{-1}$ (trimethylsilyloxy group).

Example 243

Preparation of 1R,2S(and 1S,2R) - 1 - (*trans* - 2 - Iodovinyl) - 1 - trimethylsilyloxy - 2 - butylcyclopentane

To a mixture of 9.2 g of sodium borohydride and 45.8 g of 2 - methy - 2 - butene in 350 ml of dry tetrahydrofuran at 0°C with stirring under nitrogen is added, over 20 minutes, 41.1 ml of boron trifluoride etherate. After 3 hours, to this resulting solution of diisoamylborane is added 38.8 g of 1R,2S(and 1S,2R) - 1 - ethynyl - 1 - trimethylsilyloxy - 2 - butylcyclopentane in 40 ml of tetrahydrofuran in 20 minutes. The mixture is stirred 2 hours and then stored at —20°C overnight. The mixture is allowed to warm to 0°C and at 0°C 85 g of dry trimethylamineoxide is added portionwise over 20 minutes. After stirring at 25°C for one hour, the mixture is filtered through diatomaceous earth. The filtrate is poured simultaneously with a solution of 230 g of iodine in 250 ml of tetrahydrofuran into a stirred, cold solution of 430 g of sodium hydroxide in 1900 ml of water. After stirring for 30 minutes, the organic layer is separated. The aqueous layer is extracted with ether. The combined organic solutions are washed twice with a saturated solution of sodium thiosulfate and once with brine. The solution is dried over magnesium sulfate, the solvent is removed and the residue is dissolved in hexane. The hexane solution is filtered through diatomaceous earth and silica gel. The hexane is removed and the residue is purified by dry column chromatography on silica gel eluting with hexane: 45.35 g of 1R,2S(and 1S,2R) - 1 - (*trans* - 1 - iodovinyl) - 1 - trimethylsilyloxy - 2 - butylcyclopentane is obtained.

Example 244

Preparation of 1R,2R(and 1S,2S) - (*trans* - 2 - Iodovinyl) - 1 - trimethylsilyloxy - 2 - butylcyclopentane

To a mixture of 12.22 g of sodium borohydride and 60.82 g of 2 - methyl - 2 - butene in 450 ml of tetrahydrofuran under nitrogen at 0°C, is added 54.6 ml of boron trifluoride etherate, dropwise over a 20 minute period. The solution is stirred at 0°C for 2 hours and then at room temperature for 30 minutes. This solution is cooled to 0°C and 55.5 g of 1R,2R(and 1S,2S) - 1 - ethynyl - 1 -

trimethylsilyloxy - 2 - butylcyclopentane in 50 ml of tetrahydrofuran is added. The mixture is allowed to stand in a cold room overnight. To this mixture at 0°C is added with stirring 112.8 g of trimethylamine oxide over a 20 minute period. The mixture is stirred at room temperature for 90 minutes and then filtered. To the filtrate is added simultaneously a solution of 565 g of sodium hydroxide in 2000 ml of water and a solution of 300 g of iodine in 300 ml of tetrahydrofuran. The mixture is stirred 30 minutes, the organic layer is separated and the aqueous layer is extracted with ether. The combined organic solutions are washed with saturated sodium thiosulfate solution and with saturated sodium chloride solution. The solution is dried with magnesium sulfate and filtered through a pad of silica gel. The solution is removed giving an orange liquid which is chromatographed on a dry column of silica gel giving 59.5 g of the product as a yellow liquid.

Example 245

Preparation of 1-(3-Tri-*n*-butylstannyl-2-*trans*-propenyl)-1-trimethylsilyloxycyclohexane

To a stirred mixture of 31.5 g of 1 - propargyl - 1 - trimethylsilyloxycyclohexane and 150 mg of azobisisobutyronitrile is added 41 ml of tri - *n* - butyltin hydride. The stirred mixture is heated to about 80°C. The initial exothermic reaction is moderated, and the temperature is subsequently maintained at 130°—135°C for one hour.

The product is distilled to afford 56 g of colourless liquid, bp 150°—160°C (0.15—0.3 mm), p.m.r. ($CDCl_3$): 6.0 (multiplet, vinyl protons).

Examples 246—265

Using the procedure outline above for Examples 239—242, the acetylenic alcohols listed in Table XXVII are converted to their corresponding acetylenic trimethylsilyloxy derivatives, these in turn using the procedure outlined above for Examples 243 and 244, were converted to their corresponding *trans*-2-iodovinyl derivatives or using the procedure outline above for Example 245, were converted to their corresponding *trans* - 2 - tri - *n* - butylstannyl derivatives (Table XXVII).

There is no Example 251

TABLE XXVII

| Example | Acetylene of Example | Method of Example | Vinyl Iodide or Vinyl Tin Compound |
|---|---|---|---|
| 246 | 219 | 244 | 1-(*trans*-2-iodovinyl)-1-trimethylsilyloxy-cyclohexane |
| 247 | 220 | 244 | 1-(*trans*-2-iodovinyl)-1-trimethylsilyloxy-cyclopentane |
| 248 | 221 | 245 | 1-(*trans*-2-tri-*n*-butylstannylvinyl)-1-trimethylsilyloxycycloheptane |
| 249 | 222 | 244 | 1R,3S-(and 1S,3R-) 1-(*trans*-2-iodovinyl)-1-trimethylsilyloxy-3-propylcyclopentane |
| 250 | 223 | 244 | 1R,3R-(and 1S,3S-) 1-(*trans*-2-iodovinyl)-1-trimethylsilyloxy-3-propylcyclopentane |
| 252 | 224 | 244 | 1R,2S-(and 1S,2R-) 1-(*trans*-2-iodovinyl)-1-trimethylsilyloxy-2-butylcyclohexane |
| 253 | 225 | 244 | 1R,2R-(and 1S,2S-) 1-(*trans*-2-iodovinyl)-1-trimethylsilyloxy-2-butylcyclohexane |
| 254 | 226 | 245 | 1R,2S-(and 1S,2R-) 1-(*trans*-2-tri-*n*-butyl stannylvinyl)-1-trimethylsilyloxy-2-(3-trifluoromethylphenoxy)cyclopentane |
| 255 | 227 | 245 | 1R,2R-(and 1S,2S-) 1-(*trans*-2-tri-*n*-butyl-stannylvinyl)-1-trimethylsilyloxy-2-(3-trifluoromethylphenoxy)cyclopentane |
| 256 | 228 | 244 | 1R,2S-(and 1S,2R-) 1-(*trans*-2-iodovinyl)-1-trimethylsilyloxy-2-(4-fluorophenoxy)-cyclopentane |
| 257 | 229 | 244 | 1R,2R-(and 1S,2S-) 1-(*trans*-2-iodovinyl)-1-trimethylsilyloxy-2-(4-fluorophenoxy)-cyclopentane |
| 258 | 230 | 245 | 1R,2S-(and 1S,2R-) 1-(*trans*-2-tri-*n*-butyl-stannylvinyl)-1-trimethylsilyloxy-2-(3-chlorophenoxy)cyclopentane |
| 259 | 231 | 245 | 1R,2R-(and 1S,2S-) 1-(*trans*-2-tri-*n*-butyl-stannylvinyl)-1-trimethylsilyloxy-2-(3-chlorophenoxy)cyclopentane |
| 260 | 232 | 244 | 1R,3S-(and 1S,3R-) 1-(*trans*-2-iodovinyl)-1-trimethylsilyloxy-3-methylcyclohexane |
| 261 | 234 | 244 | 1R,3R-(and 1S,3S-) 1-(*trans*-2-iodovinyl)-1-trimethylsilyloxy-3-methylcyclohexane |
| 262 | 235 | 245 | 1R,2S-(and 1S,2R-) 1-(3-tri-butylstannyl-2-*trans*-propenyl)-1-trimethylsilyloxy-2-butylcyclopentane |
| 263 | 236 | 245 | 1R,2R-(and 1S,2S-) 1-(3-tri-*n*-butylstannyl-2-*trans*-propenyl)-1-trimethylsilyloxy-2-butylcyclopentane |

TABLE XXVII (continued)

| Example | Acetylene of Example | Method of Example | Vinyl Iodide or Vinyl Tin Compound |
|---|---|---|---|
| 264 | 237 | 245 | 1R,2S-(and 1S,2R-) 1-(3-tri-*n*-butylstannyl-2-*trans*-propenyl)-1-trimethylsilyloxy-2-(3-trifluoromethylphenoxy)cyclopentane |
| 265 | 238 | 245 | 1R,2R-(and 1S,2S-) 1-(3-tri-*n*-butylstannyl-2-*trans*-propenyl)-1-trimethylsilyloxy-2-(3-trifluoromethylphenoxy)cyclopentane |

Example 266

Preparation of trimethylsilyl-2-trimethylsilyloxy acetate

To a solution of 15 g (0.197 mol) of glycolic acid in 50 ml of dry pyridine is poured 32.3 g (0.2 mol) of 1,1,1,3,3,3-hexamethyldisilazine. After stirring 15 minutes, 10.86 g (0.1 mol) of trimethylsilyl chloride is added dropwise. The mixture is stirred for one hour and then filtered from a white solid which is washed with petroleum ether. The filtrate and washings are concentrated at reduced pressure at 30 C. The residue is distilled (85°—86°, 15 minutes) to give 38 g of the title compound.

Example 267

Preparation of *tris*-trimethylsilyloxyethylene

To a solution of 50.98 g (0.316 mol) of 1,1,1,3,3,3-hexamethyldisilazine in 250 ml of tetrahydrofuran is added with stirring under argon at 0°C dropwise 133.3 ml (0.32 mol) of 2.4M *n*-butyl lithium in hexane. After addition is complete, the solution is maintained at 45°C for 30 minutes. The solution is cooled to —78° and 58.7 g of trimethylsilyl - 2 - trimethylsilyloxy acetate (Example 266) is added dropwise. After stirring 30 minutes, 43.2 g (0.4 mol) of trimethylsilylchloride is added over 10 minutes. The solution is allowed to warm to room temperature over 30 minutes. The solvent is removed at reduced pressure. The residue is mixed with an equal volume of petroleum ether and filtered from the suspended lithium chloride. The solvent is removed and the residue is distilled (70°—75°C, 1.4 minute) to give 64.65 g of the title compound.

Example 268

Preparation of 2-(6-chloroformyl)hexyl)cyclopent-2-en-1-one

To a suspension of 1.94 g (0.08 mol) of sodium hydride in 100 ml of tetrahydrofuran is added with stirring under argon dropwise a solution of 17 g (0.08 mol) of 2 - (6 - carboxyhexyl) - cyclopent - 2 - en - 1 - one in 160 ml of tetrahydrofuran. After the addition is complete, the mixture is stirred for 1 hour 15 minutes. The mixture is cooled to 0°C and 13 ml of oxalyl chloride is added. The mixture is stirred at 0°C for 30 minutes and at room temperature for 30 minutes. The solution is diluted with 300 ml of ether and filtered through Celite. The solvent is removed from the nitrate and the residue is extracted with hot hexane twice. The hexane is removed to give 16.0 g of the title compound.

Example 269

Preparation of 2-(8-hydroxy-7-oxo-octyl)cyclopent-2-en-1-one

A mixture of 6.3 g of 2-(6-(chloroformyl)hexyl)-cyclopent-2-en-1-one (Example 268) and 16 g of *tris*-trimethylsilyloxyethylene (Example 267) are stirred at 90° to 100°C under argon for one hour. To this mixture is added 25 ml of dioxane and 10 ml of 0.6N hydrochloric acid. The mixture is heated at 80°C for 30 minutes. The mixture is poured into brine and extracted with ether. The ether solution is washed with saturated sodium bicarbonate and dried over magnesium sulfate. The solvent is removed and the residue is chromatographed on a dry column of silica gel eluting with ether containing 2% acetic acid to give 1.7 g of the title compound ($R_f = 0.45$).

Example 270

Preparation of 2 - (6 - carbodimethyl - *t* - butylsilyloxyhex - 2 - *cis* - enyl) - 4 - dimethyl - *t* - butylsiloxy - cyclopent - 2 - en - 1 - one

To 5.0 g of 2-(6-carboxyhex-2-*cis*-enyl)-4-hydroxy-cyclopent-2-en-1-one and 7.5 g of imidazole in 24 ml of dimethylformamide is added 10.2 g of dimethyl-t-butylsilylchloride. The mixture is maintained at 37°C for 4 hours. The mixture is poured into ice water and extracted with hexane. The hexane solution is washed with saturated sodium bicarbonate and dried over magnesium sulfate. The solvent is removed. Toluene is added and removed. The residue is distilled in a Kugelrohr apparatus (165°C, 0.5—0.1 mm) to give 4.56 g of the title compound.

Example 271

Preparation of 1-(6-carboxyhex-2-*cis*-enyl)-4-dimethyl-*t*-butylsilyloxycyclopent-2-en-1-one

A solution of 1 - (6 - carbodimethyl - *t* - butylsilyloxyhex - 2 - *cis* - enyl) - 4 - dimethyl - *t* - butylsilyloxycyclopent - 2 - en - 1 - one (Example 270) in 40 ml of acetic acid - tetrahydrofuran - water (4:2:1) is stirred at room temperature for 1.5 hour. The solvents are removed at reduced pressure at 40°C. The residue is dissolved in ether. The ether solution is washed with water, brine, and dried over magnesium sulfate. The solvent is removed. Toluene is added and removed to give 3.1 g of the title compound.

Example 272

Preparation of 1-(6-chloroformyl)hex-2-*cis*-enyl)-4-dimethyl-*t*-butylsilyloxycyclopent-2-en-1-one

To 59.66 g of 1 - (6 - carboxyhex - 2 - *cis* - enyl) - 4 - dimethyl - *t* - butylsilyloxycyclopent - 2 - en - 1 - one (Example 271) in 300 ml of tetrahydrofuran containing 0.5 ml of dimethyformamide at 0°C under argon with stirring is added over 20 minutes 29.2 ml of oxalyl chloride in 40 ml of tetrahydrofuran. After 1.5 hours the solvent is removed at reduced pressure at 35°C. The residue is dissolved in petroleum ether and filtered through Celite. The solvent is removed to give 59.3 g of the title compound.

Example 273

Preparation of 1-(8-hydroxy-7-oxo-oct-2-*cis*-enyl)-4-hydroxycyclopent-2-en-1-one

A mixture of 59.3 g of 1 - (6 - (chloroformyl)hex - 2 - *cis* - enyl) - 4 - dimethyl - *t* - butylsilyloxycyclopent - 2 - en - 1 - one (Example 272) and 101.5 g of tris-trimethylsilyloxyethylene (Example 267) is heated under argon at 90°—95°C for 3 hours 10 minutes. The reaction mixture is poured into a mixture of 300 ml of tetrahydrofuran and 140 ml of 0.6N hydrochloric acid and the resulting mixture is stirred at 70°C for 2.5 hours. The mixture is poured in brine and extracted with ethyl acetate. The organic layer is washed with saturated sodium bicarbonate and dried of magnesium sulfate. The solvent is removed and the residue is chromatographed of a dry column of silica gel eluting with ethyl acetate. The product bond ($R_1 = 0.4$) is extracted to give 6.45 g of the title compound.

Example 274

Preparation of 5-bromopentanoylchloride

To a solution of 97 g of 5-bromopentanoic acid in 240 ml of methylene chloride containing 1 ml of dimethylformamide is added dropwise 76.2 g of oxalyl chloride. The mixture is stirred one hour at room temperature and 30 minutes at 50°C. The solvent is removed and the residue is distilled twice (75°C, 0.6 minute) to give 88.2 g of the title compound.

Example 275

Preparation of 6-bromo-1-hydroxy-2-hexanone

To 191.4 g of *tris*-trimethylsilyloxyethylene (Example 267) containing 15 drops of stannic tetrachloride under argon with stirring at 10°C is added 87 g of 5-bromopentanoyl chloride (Example 274) dropwise. After one-half of the acid chloride added, the mixture is stirred until an exotherm ensues. The remaining acid chloride is added dropwise maintaining the reaction exotherm at 65°C. The mixture is then stirred for 2.5 hours. The mixture is slowly poured into a stirred mixture of 100 ml of 0.6N hydrochloric acid and 200 ml of tetrahydrofuran. The mixture is stirred for 30 minutes and poured into brine. The mixture is extracted with ether. The ether solution is washed with saturated sodium bicarbonate and dried over magnesium sulfate. The solvent is removed. The residue is mixed with petroleum ether and cooled in dry-ice-acetone to induce crystallization. The petroleum ether is decanted and the solid is dried at reduced pressure to give 64.72 g of the title compound.

Example 276

Preparation of 6-bromo-1-dimethyl-*t*-butylsilyloxy-2-hexanone ethylene ketal

A mixture of 84 g of 6-bromo-1-hydroxy-2-hexanone (Example 275)), 240 ml of ethylene glycol, and 1.7 g of *p*-toluenesulfonic acid is refluxed in 1800 ml of toluene using a Dean-Stark trap for 1 hour 45 minutes. The mixture is cooled to room temperature and washed with saturated sodium bicarbonate, water, and brine. The solvent is removed giving 75.17 g cf a yellow oil. To a 27.36 g portion of this material and 16.2 g of imidazole in 57 ml of dimethylformamide at 0° with stirring is added 20.55 g of dimethyl-*t*-butylchlorosilane. The mixture is stirred at room temperature for 1.5 hour and then poured into water. The mixture is extracted with petroleum ether. The organic phase is washed with dilute hydrochloric acid, saturated sodium bicarbonate and dried over magnesium sulfate. The solvent is removed and the residue is distilled in a Klugrohr apparatus (0.5—0.2 mm, 100°—110°C) to give 35.25 g of the title compound.

Example 277

Preparation of 1-dimethyl-*t*-butylsilyloxy-2-hexanone ethylene ketal 6-triphenylphosphonium bromide

A mixture of 35.25 g of 6-bromo-1-dimethyl-*t*-butylsilyloxy-2-hexanone ethylene ketal (Example

276) and 26.2 g of triphenylphosphine in 68 ml of acetonitrile is refluxed 90 hours. The acetonitrile is removed at reduced pressure. The residue is washed three times with ether and dried at reduced pressure to give 53.8 g of the title compound.

Example 278

Preparation of 2,5, - dihydro - 2,5 - dimethoxy - 2 - (9 - dimethyl - *t* - butylsilyloxy - 8 - oxonon - 3 - *cis* - enyl)furan

A suspension of 2.3 g (0.096 mol) of oil free sodium hydride is stirred under argon at 65°C in 75 ml of dimethylsulfoxide. After gas evolution ceased (1 hour), at 0°C is added 53.8 g (0.086 mol of 1 - dimethyl - *t* - butylsilyloxy - 2 - hexanone ethylene ketal 6-triphenylphosphonium bromide (Example 277) in 160 ml of dimethylsulfoxide. After stirring 15 minutes at room temperature, 16.3 g (0.087 mol) of 2,5 - dihydro - 2,5 - dimethoxy - 2 - (3′ - oxopropyl)furan (U.S. Patent 3,952,033) in 40 ml of dimethylsulfoxide is added. After stirring one hour at room temperature, the solvent is removed at reduced pressure at 55°C. The solid residue is extracted with an ether-petroleum ether mixture. The solution is washed with water, saturated sodium bicarbonate and dried over magnesium sulfate. The solvent is removed and petroleum ether is added. After standing 30 minutes, the triphenylphosphine oxide is removed by filtration. The solvent is removed and the residue is chromatographed on a dry column of florisil eluting first with hexane and then with hexaneether 5:1 to give 13.2 g of the title compound.

Example 279

Preparation of 2 - (8 - dimethyl - *t* - butylsilyloxy - 7 - oxo - oct - 2 - *cis* - enyl) - 4 - hydroxy-cyclopent - 2 - en - 1 - one,7 - ethylene ketal

A mixture of 66.08 g of 2,5 - dihydro - 2,5, - dimethoxy - 2 - (9 - dimethyl - *t* - butylsilyloxy - 8 - oxonon - 3 - *cis* - enyl)furan (Example 278), 26.4 g of sodium dihydrogen phosphate, 5.2 g of sodium acetate and 0.5 g of hydroquinone in 1320 ml of dioxane and 660 ml of water is stirred at reflux under argon for 22 hours. The mixture is cooled to room temperature, saturated with sodium chloride, and the organic layer is separated. The aqueous layer is extracted with ether. The combined organic solutions are washed with brine and dried over magnesium sulfate. The solvent is removed to give 57.5 g of an oil. To this is added 300 ml of ether, 300 ml of petroleum ether and 22 g of anhydrous chloral. The solution is stirred under argon and 23 g of triethylamine is added. After 1 hour 40 minutes, the solution is washed with water, dilute hydrochloric acid, saturated sodium bicarbonate, brine, and dried over magnesium sulfate. The solvent is removed and the residue is chromatographed on a dry column of silica gel eluting with ethylacetate-hexane 1:1 to give 14.55 g of the title compound ($R_f = 0.4$).

Example 280

Preparation of 2 - (8 - dimethyl - *t* - butylsilyloxy - 7 - oxo - oct - 2 - *cis* - enyl) - 4 - trimethylsilyloxycyclopent - 2 - en - 1 - one, 7 - ethylene ketal

A mixture of 14.5 g (0.0367 mol) of 2 - (8 - dimethyl - *t* - butylsilyloxy - 7 - oxo - oct - 2 - *cis* - enyl) - 4 - hydroxycyclopent - 2 - en - 1 - one, 7 - ethylene ketal (Example 279) and 3.34 g (0.04 mol) of imidazole in 30 ml of dimethylformamide is stirred as 4.98 g (0.046 mol) of trimethylsilylchloride is added. After one hour, the mixture is poured into water and extracted with hexane. The hexane solution is washed with water, saturated sodium bicarbonate, and dried over magnesium sulfate. The solution is filtered through a pad of silica gel. The solvent is removed and the residue is dried at reduced pressure to give 12.5 g of the title compound.

Example 281

Preparation of 2 - (6 - (4 - methoxy - 2,2 - dimethyl - 1,3 - dioxolan - 4 - yl) hex - 2 - *cis* - enyl) - 4 - (2 - methoxypropyl - 2 - oxy)cyclopent - 2 - en - 1 - one

A mixture of 2 - (8 - hydroxy - 7 - oxo - oct - 2 - *cis* - enyl) - 4 - hydroxycyclopent - 2 - en - 1 - one (Example 273), 14 ml of 2-methoxy propene, and 0.23 g of ammonium nitrate in 35 ml of benzene is added 7 ml of dimethoxypropane. *P*-toluenesulfonic acid is added in very small portions until TLC indicates the reaction is initiated. The mixture is stirred 1.5 hour at room temperature, 40°C for 15 minutes, and another 30 minutes at room temperature. To the stirred solution is added 50 g of crushed 4A molecular sieve. After 15 minutes, the solution is filtered washed with saturated sodium bicarbonate, and dried over sodium sulfate. The solvent is removed and the residue is chromatographed on a dry column of silica gel eluting with etherhexane 1:1. The product bond ($R_f = 0.5$) is extracted to give 3.44 g of the tile compound.

Example 282

Preparation of 2-(8-hydroxy-7-oxo-octyl)cyclopent-2-en-1-one,7-ethylene ketal

A solution of 5.5 g of 2-(8-hydroxy-7-oxo-octyl)cyclopent-2-en-1-one (Example 269), 25 ml of ethylene glycol, and 0.1 g of *p*-toluenesulfonic acid in 200 ml of toluene is refluxed for 40 minutes using a Dean-Stark trap. The solution is poured into saturated sodium bicarbonate. The mixture is

extracted with benzene. The organic solution is washed three times with water and dried over magnesium sulfate. The solvent is removed to give 6.0 g of the title compound.

Example 283

Preparation of 2-(8-trimethylsilyloxy-7-oxo-octyl)cyclopent-2-en-1-one,7-ethylene ketal

To a solution of 2.2 g of 2 - (8 - hydroxy - 7 - oxo - octyl)cyclopent - 2 - en - 1 - one,7 - ethylene ketal (Example 282) in 20 ml of pyridine is added 4.6 ml of 1,1,1,3,3,3-hexamethyldisilazane and, dropwise, 2.3 ml of trimethylsilylchloride. After 15 minutes, the excess reagents and solvent is removed at reduced pressure. The residue is taken up in ether and filtered through a short pad of silica gel. The solvent is removed. Toluene is added and removed. The residue is dried at reduced pressure to give 2.77 g of the title compound.

Examples 284—296

By the sequence of reactions described hereinabove for Examples 268 and 269 or 270 to 273 and the protection reaction described hereinabove in Example 281, the protected cyclopent-2-en-1-one listed in Table XXVIII hereinbelow are prepared from the indicated carboxylic acids.

TABLE XXVIII

| Example | Carboxylic acid | Protected cyclopent-2-en-1-one |
|---|---|---|
| 284 | 2-(6-carboxyhexyl)cyclopent-2-en-1-one | 2-[6-(4-methoxy-2,2-dimethyl-1,3-dioxolan-4-yl)-hexylcyclopent-2-en-1-one |
| 285 | 2-(5-carboxypentyl)cyclopent-2-en-1-one | 2-[5-(4-methoxy-2,2-dimethyl-1,3-dioxolan-4-yl)-pentylcyclopent-2-en-1-one |
| 286 | 2-(7-carboxyheptyl)cyclopent-2-en-1-one | 2-[7-(4-methoxy-2,2-dimethyl-1,3-dioxolan-4-yl)-heptylcyclopent-2-en-1-one |
| 287 | 2-(6-carboxyhex-2-*cis*-enyl)-cyclopent-2-en-1-one | 2-[6-(4-methoxy-2,2-dimethyl-1,3-dioxolan-4-yl)-hex-2-*cis*-enyl]cyclopent-2-en-1-one |
| 288 | 2-(5-carboxypent-2-*cis*-enyl)-cyclopent-2-en-1-one | 2-[5-(4-methoxy-2,2-dimethyl-1,3-dioxolan-4-yl)-pent-2-*cis*-enyl]cyclopent-2-en-1-one |
| 289 | 2-(7-carboxyhept-2-*cis*-enyl)-cyclopent-2-en-1-one | 2-[7-(4-methoxy-2,2-dimethyl-1,3-dioxolan-4-yl)-hept-2-*cis*-enyl]cyclopent-2-en-1-one |
| 290 | 2-(4-carboxypent-2-*cis*-enyl)-4-hydroxycyclopent-2-en-1-one | 2-[5-(4-methoxy-2,2-dimethyl-1,3-dioxolan-4-yl)-pent-2-*cis*-enyl]4-(2-methoxypropyl-2-oxy)cyclopent-2-en-1-one |
| 291 | 2-(7-carboxyhept-2-*cis*-enyl)-4-hydroxycyclopent-2-en-1-one | 2-[7-(4-methoxy-2,2-dimethyl-1,3-dioxolan-4-yl)-hept-2-*cis*-enyl]4-(2-methoxypropyl-2-oxy)cyclopent-2-en-1-one |
| 292 | 2-(6-carboxyhexyl)-4-hydroxy-cyclopent-2-en-1-one | 2-[6-(4-methoxy-2,2-dimethyl-1,3-dioxolan-4-yl)-hexyl]-4-(2-methoxypropyl-2-oxy)cyclopent-2-en-1-one |
| 293 | 2-(5-carboxypentyl)-4-hydroxy-cyclopent-2-en-1-one | 2-[5-(4-methoxy-2,2-dimethyl-1,3-dioxolan-4-yl)-pentyl]-4-(2-methoxypropyl-2-oxy)cyclopent-2-en-1-one |
| 294 | 2-(7-carboxyheptyl)-4-hydroxy-cyclopent-2-en-1-one | 2-[7-(4-methoxy-2,2-dimethyl-1,3-dioxolan-4-yl)-heptyl]-4-(2-methoxypropyl-2-oxy)cyclopent-2-en-1-one |
| 295 | 2-(6-carboxyhex-2-*cis*-enyl)-4(R)-hydroxycyclopent-2-en-1-one | 2-[6-(4-methoxy-2,2-dimethyl-1,3-dioxolan-4-yl)-hex-2-*cis*-enyl]-4(R)-(2-methoxypropyl-2-oxy)cyclopent-2-en-1-one |
| 296 | 2-(6-carboxyhexyl)-4(R)-hydroxycyclopent-2-en-1-one | 2-[6-(4-methoxy-2,2-dimethyl-1,3-dioxolan-4-yl)-hexyl]-4(R)-(2-methoxypropyl-2-oxy)cyclopent-2-en-1-one |

Example 296A

Preparation of 1 - (1 - methoxy - 1 - methylethoxy) - 8 - (3 - (1 - methoxy - 1 - methylethoxy) - 5 - oxo - 1 - cyclopenten - 1 - yl - 2 - octanone

To a stirred solution of 31.2 g (130 mmol) of 1 - hydroxy - 8 - (3 - hydroxy - 5 - oxo - 1 - cyclopenten - 1 - yl) - 2 - octanone (Example 269) in 190 ml of sieve dried $CH_2Cl_2$ is added 47 ml of 2-methoxy propene (Eastman) followed by 0.1 ml of dichloroacetic acid. The resulting mild exotherm is maintained at 25°C by water bath cooling. After 30 min., TLC (ethyl acetate, 2,4-DNP) shows no starting material, minor spots at RF = 0.5 and 0.6, and a major spot at Rf = 0.78. After 1 hour total time, another 0.1 ml of dichloroacetic acid is added. After a total reaction time of 2 hours, the solution is diluted with 650 ml of hexane. The solution is washed with 50 ml of saturated $NaHCO_3$ and brine, the solution is dried ($K_2CO_3$,$MgSO_4$) and 0.05 ml of pyridine is added. The solvents are removed giving 48.4 g (97%) of Anal. Calcd for $C_{21}H_{36}O_6$: C, 65.60; H, 9.44. Found: C, 65.35; H, 9.45.

PMR: $\delta$ ($CDCl_3$), 7.06 (m, 1H, enone), 4.92 (m, 1H, C*H*O), 4.00 (s, 2H, $CH_2O$), 3.22 (s, 3H, $OCH_3$), 3.18 (s, 3H, $OCH_3$), 2.62, 2.40, 2.16 (m'ss, 6H, $CH_2$'s), 1.36 (m, 20H, $CH_2$'s, $CH_3$'s). IR: (neat) 5830 nm.

Examples 296B—296N

In accordance with the protection reaction described hereinabove in Example 296A and the reaction sequence of Examples 269—273, the protect cyclopent - 2 - en - 1 - ones listed in Table XXVIIIA hereinbelow are prepared from the indicated carboxylic acids.

TABLE XXVIIIA

| Example | Carboxylic acid | Protected cyclopent-2-en-1-one |
|---|---|---|
| 296B | 2-(6-carboxyhexyl)cyclopent-2-en-1-one | 1-(1-methoxy-1-methylethoxy)-8-(5-oxo-1-cyclopenten-1-yl)-2-octanone |
| 296C | 2-(5-carboxypentyl)cyclopent-2-en-1-one | 1-(1-methoxy-1-methylethoxy-7-(5-oxo-1-cyclopenten-1-yl)2-heptanone |
| 296D | 2-(7-carboxyheptyl)cyclopent-2-en-1-one | 1-(1-methoxy-1-methylethoxy-9-(5-oxo-1-cyclopenten-1-yl)2-nonanone |
| 296E | 2-(6-carboxyhex-2-*cis*-enyl)-cyclopent-2-en-1-one | 1-(1-methoxy-1-methylethoxy-8-(5-oxo-1-cyclopenten-1-yl)6-*cis*-octen-2-one |
| 296F | 2-(5-carboxypent-2-*cis*-enyl)-cyclopent-2-en-1-one | 1-(1-methoxy-1-methylethoxy-7-(5-oxo-1-cyclopenten-1-yl)-5-*cis*,hepten-2-one |
| 296G | 2-(7-carboxyhept-2-*cis*-enyl)-cyclopent-2-en-1-one | 1-(1-methoxy-1-methylethoxy-9-(5-oxo-1-cyclopenten-1-yl)-7-*cis*,nonen-2-one |
| 296H | 2-(5-carboxypent-2-*cis*-enyl)-4-hydroxycyclopent-2-en-1-one | 1-(1-methoxy-1-methylethoxy-7-[3-(1-methoxy-1-methylethoxy 5-oxo-1-cyclopent-1-yl]-5-*cis*,hepten-2-one |
| 296I | 2-(7-carboxyhept-2-*cis*-enyl)-4-hydroxycyclopent-2-en-1-one | 1-(1-methoxy-1-methylethoxy-9-[3-(1-methoxy-1 methylethoxy 5-oxo-1-cyclopenten-yl]-7-*cis*,nonen-2-one |
| 296J | 2-(6-carboxyhex-2-*cis*-enyl)-4-hydroxycyclopent-2-en-1-one | 1-(1-methoxy-1-methylethoxy-8-[3-(1-methoxy-1-methylethoxy)-5-oxo-1-cyclopenten-1-yl]-6-*cis*-octen-2-one |
| 296K | 2-(5-carboxypentyl)4-hydroxy-cyclopent-2-en-1-one | 1-(1-methoxy-1-methoxyethoxy)-7-[3-(1-methoxy-1-methylethoxy)-5-oxo-1-cyclopenten-1-yl]-2-heptanone |
| 296L | 2-(7-carboxyheptyl)-4-hydroxy-cyclopent-2-en-1-one | 1-(1-methoxy-1-methylethoxy)-9-[3-(1-methoxy-1-methoxyethoxy)-5-oxo-1-cyclopenten-1-yl]-2-nonanone |
| 296M | 2-(6-carboxyhex-2-*cis*-enyl)-4-(R)-hydroxycyclopent-2-en-1-one | 1-(1-methoxy-1-methoxyethoxy)-8-[3R-3-(1-methoxy-1-methylethoxy)-5-oxo-1-cyclopenten-1-yl]-6-*cis*,octen-2-one |
| 296N | 2-(6-carboxyhexyl)-4-(R)-hydroxycyclopent-2-en-1-one | 1-(1-methoxy-1-methylethoxy)-8-[3R-3-(1-methoxy-1-methylethoxy)-5-oxo-1-cyclopenten-1-yl]-2-octanone |

Examples 297—298

By the sequence of reaction given hereinabove in the Examples 274 to 280, the protected cyclopent - 2 - en - 1 - ones of Table XXIXa are prepared from indicated Bromocarboxylic acid.

TABLE XXIX

| Example | Bromo-carboxylic acid | Protected Cyclopent-2-en-1-one |
|---|---|---|
| 297 | 4-bromobutanoyl-chloride | 1-(7-dimethyl-*t*-butylsilyloxy-6-oxohept-2-*cis*-enyl)-4-trimethylsilyloxycyclopent-2-en-1-one,6-ethylene ketal |
| 298 | 6-bromohexanoyl chloride | 1-(9-dimethyl-*t*-butylsilyloxy-8-oxonon-2-*cis*-enyl)-4-trimethylsilyloxycyclopent-2-en-1-one,8-ethylene ketal |

Examples 299—308

By the methods hereinabove in Examples 268 and 269 and the ketalization reaction described in Example 282, the cyclopent - 2 - en -1 - ones in Table XXX are prepared from the indicated carboxylic acid. By the methods described hereinabove in the sequence of reactions shown in Examples 270—273 and the ketalization reaction described in Example 282 the 4 - hydroxy - cyclopent - 2 - en - 1 - ones in Table XXX are prepared from the indicated carboxylic acid.

TABLE XXX

| Example | Carboxylic acid | Cyclopent-2-en-1-one |
|---|---|---|
| 299 | 2-(5-carboxypentyl)cyclopent-2-en-1-one | 2-(7-hydroxy-6-oxoheptyl)cyclopent-2-en-1-one, 6-ethylene ketal |
| 300 | 2-(7-carboxyheptyl)cyclopent-2-en-1-one | 2-(9-hydroxy-8-oxononyl)cyclopent-2-en-1-one, 8-ethylene ketal |
| 301 | 2-(6-carboxyhexyl)-4-hydroxycyclopent-2-en-1-one | 2-(8-hydroxy-7-oxooctyl)-4-hydroxycyclopent-2-en-1-one,7-ethylene ketal |
| 302 | 2-(5-carboxypentyl)-4-hydroxycyclopent-2-en-1-one | 2-(7-hydroxy-6-oxoheptyl)-4-hydroxycyclopent-2-en-1-one,6-ethylene ketal |
| 303 | 2-(7-carboxyheptyl)-4-hydroxycyclopent-2-en-1-one | 2-(9-hydroxy-8-oxononyl)-4-hydroxycyclopent-2-en-1-one,8-ethylene ketal |
| 304 | 2-(6-carboxyhex-2-*cis*-enyl)cyclopent-2-en-1-one | 2-(8-hydroxy-7-oxooct-2-*cis*-enyl)cyclopent-2-en-1-one,7-ethylene ketal |
| 305 | 2-(5-carboxypent-2-*cis*-enyl)cyclopent-2-en-1-one | 2-(7-hydroxy-6-oxohept-2-*cis*-enyl)cyclopent-2-en-1-one,6-ethylene ketal |
| 306 | 2-(7-carboxyhept-2-*cis*-enyl)cyclopent-2-en-1-one | 2-(9-hydroxy-8-oxonon-2-*cis*-enyl)cyclopent-2-en-1-one,8-ethylene ketal |
| 307 | 2-(6-carboxyhex-2-*cis*-enyl)-4(R)-hydroxycyclopent-2-en-1-one | 2-(8-hydroxy-7-oxooct-2-*cis*-enyl)-4(R)-hydroxycyclopent-2-en-1-one,7-ethylene ketal |
| 308 | 2-(6-carboxyhexyl)-4(R)-hydroxycyclopent-2-en-1-one | 2-(8-hydroxy-7-oxooctyl)-4(R)-hydroxycyclopent-2-en-1-one,7-ethylene ketal |

Example 309

Preparation of 2 - (8 - trimethysilyloxy - 7 - octyl) - 4 - trimethylsilyloxycyclopent - 2 - en - 1 - one, 7 - ethylene ketal

To a solution of 2.2 g of 2 - (8 - hydroxy - 7 - oxo - octyl) - 4 - hydroxycyclopent - 2 - en - 1 - one, 7 - ethylene ketal (Example 301) in 40 ml of pyridine is added 9.2 ml 1,1,1,3,3,3-hexamethyldisilazine and, dropwise, 4.6 ml of trimethylsilylchloride. After 30 minutes, the excess reagents and pyridine are removed at reduced pressure. The residue is taken up in ether and filtered through a short pad of silica gel. The solvent is removed and the residue is dried at reduced pressure to give the title compound.

Examples 310—318

By the methods described hereinabove in Examples 283 and 309, the hydroxycyclopent - 2 - en - 1 - ones listed in Table XXXI are converted to their trimethylsilyl ethers shown.

TABLE XXXI

| Example | Hydroxycyclopent-2-en-1-one of Example | Protected cyclopent-2-en-1-one |
|---|---|---|
| 310 | 299 | 2-(7-trimethylsilyloxy-6-oxoheptyl)cyclopent-2-en-1-one,6-ethylene ketal |
| 311 | 300 | 2-(9-trimethylsilyloxy-8-oxononyl)cyclopent-2-en-1-one,8-ethylene ketal |
| 312 | 302 | 2-(7-trimethylsilyloxy-6-oxoheptyl)-4-trimethyl silyloxycyclopent-2-en-1-one,6-ethylene ketal |
| 313 | 303 | 2-(9-trimethylsilyloxy-8-oxononyl)-4-trimethyl-silyloxycyclopent-2-en-1-one,8-ethylene ketal |
| 314 | 304 | 2-(8-trimethylsilyloxy-7-oxooct-2-*cis*-enyl)-cyclopent-2-en-1-one,7-pentethylene ketal |
| 315 | 305 | 2-(7-trimethylsilyloxy-6-oxohept-2-*cis*-enyl)-cyclopent-2-en-1-one,6-ethylene ketal |
| 316 | 306 | 2-(9-trimethylsilyloxy-8-oxonon-2-*cis*-enyl)-cyclopent-2-en-1-one,8-ethylene ketal |
| 317 | 307 | 2-(8-trimethylsilyloxy-7-oxooct-2-*cis*-enyl)-4(R)-trimethylsilyloxycyclopent-2-en-1-one,7-ethylene ketal |
| 318 | 308 | 2-(8-trimethylsilyloxy-7-oxooctyl)-4(R)-tri-methylsilyloxycyclopent-2-en-1-one,7-ethylene ketal |

Example 319

Preparation of 1,9 - dioxo - 11$\alpha$,15 - dihydroxy - 1 - hydroxymethyl - 16,16 - trimethylene - 13 - *trans* - 5 - *cis* - prostadiene and 1,9 - dioxo - 11$\alpha$,15 - *epi* - dihydroxy - 1 - hydroxymethyl - 16,16 - trimethylene - 13 - *trans* - 5 - *cis* - prostadiene

To a solution of 1.95 g (0.00533 mol) of 1 - iodo - 4,4 - trimethylene - 3 - trimethylsilyloxy - 1 - *trans* - octene (Example 13) in 6.5 ml of ether is added at —78°C with stirring under argon 6.66 ml (0.0107 mol) of 1.6M *t*-butyllithium in pentane. After 15 minutes at —78°C, the mixture is stirred at —10° to —5°C for 90 minutes. The solution is cooled to —78°C and a solution of 0.704 g (0.00533 mol) of copper pentyne and 1.75 g (0.0106 mol) of hexamethylphosphoroustriamide in 20 ml of ether is added. After stirring for one hour, 1.7 g (0.0044 mol) of 2 - (6 - methoxy - 2,2 - dimethyl - 1,3 - dioxolan - 4 - yl)hex - 2 - *cis* - enyl) - 4 - (2-methoxypropyl - 2 - oxy)cyclopent - 2 - en - 1 - one (Example 281) in 13 ml of ether is added. The solution is maintained at —40° to —50°C for one hour and —30°C for 30 minutes. A solution of 1 ml of acetic acid in 5 ml of ether is added followed by a saturated solution of ammonium chloride and dilute hydrochloric acid. The solution is filtered and solids are washed with ether. The combined filtrate is extracted with ether. The ether solution is washed with saturated sodium bicarbonate and dried over magnesium sulfate. The ether is removed giving a yellow oil which is dissolved in 60 ml of acetic acid-tetrahydrofuran-water 4:2:1 and heated to 40°—50°C for 70 minutes. The solvent is removed at reduced pressure at 50°C. The residue is dissolved in ethyl acetate. The ethyl acetate solution is washed with saturated sodium bicarbonate, brine, and dried over magnesium sulfate. The solvent is removed. The residue is chromatographed on a dry column of silica gel eluting with ethyl acetate-benzene 3:2 containing 1% acetic acid to give 0.35 g of 1,9 - dioxo - 11$\alpha$,15 - dihydroxy - 1 - hydroxymethyl - 16,16 - trimethylene - 13 - *trans* - 5 - *cis* - prostadiene and 0.39 g of 1,9 - dioxo - 11$\alpha$,15 - *epi* - dihydroxy - 1 - hydroxymethyl - 16,16 - trimethylene - 13 - *trans* - 5 - *cis* - prostadiene.

Example 320

Preparation of 1,9 - dioxo - 11$\alpha$,16 - dihydroxy - 1 - hydroxymethyl - 16 - vinyl - 5 - *cis* - 13 - *trans* - prostadiene

To a solution of 2.9 g (3.6 mmol) of (E)4 - trimethylsilyloxy - 4 - vinyl - 1 - *tri* - *n* - butylstannyloctene (Example 210a) in 4 ml of tetrahydrofuran at −78°C under argon with stirring is added 2.4 ml of 2.4M *n*-butyllithium in hexane. The solution is stirred at −30° to −20°C for 2 hours. A solution of 0.74 g (5.6 mmol) of copper pentyne and 2.3 ml of hexamethylphosphorous triamide in 18 ml of ether is added at −78°C. The solution is stirred at −78°C for 1.5 hour. A soltution of 2.0 g (5.2 mmol) of 2 - (6 - methoxy - 2,2 - dimethyl - 1,3 - dioxolan - 4 - yl)hex - 2 - *cis* - enyl) - 4 - (2 - methoxypropyl - 2 - oxy)cyclopent - 2 - en - 1 - one (Example 281) in 20 ml of ether is added. The solution is stirred at −30° to −20°C for 1.5 hour. To the solution is added 100 ml of saturated ammonium chloride. The mixture is extracted with ether and the ether solution is washed with dilute hydrochloric acid, saturated sodium bicarbonate, and dried over magnesium sulfate. The solvent is removed and the residue is dissolved in 90 ml of acetic acid - tetrahydrofuran - water 4:2:1. The solution is stirred at room temperature for 2 hours. The solvents are removed at reduced pressure at 50°C. Toluene was added and removed. The residue is chromatographed on a dry column of silica gel eluting with ethyl acetate to give 0.5 g of the title compound.

Examples 321—422E

By the methods described hereinabove in Examples 319 and 320, the 1,9 - dioxo - 1 - hydroxymethylprostene derivatives shown in Table 32 are prepared by the indicated method from the indicated vinyl iodide or vinyl tin compound and the indicated cyclopent 2 - en - 1 - one. In those cases where isomers are obtained at the $C_{15}$ or $C_{16}$ positions, only the $C_{15}$ or $C_{16}$-normal isomers are listed in Table XXXII: it should be understood that the corresponding $C_{15}$ or $C_{16}$-*epi* isomers are also formed and are part of this invention.

In those cases where the initial conjugate addition product contains a triphenylmethoxy blocking group, deblocking is conducted in acetic acid - tetrahydrofuran - water 4:2:1 at 50°C for 5 hours.

There are no Examples 342, 343, 349, 350, 352, 353, 359 and 388

TABLE XXXII

| Example | Method of Example | Cyclopent-2-en-1-one | Vinyl Iodide or Vinyl Tin of Example | 1,9-dioxo-1-hydroxymethylprostene |
|---|---|---|---|---|
| 321 | 319 | 281 | 49 | 1,9-dioxo-11$\alpha$,15-dihydroxy-1-hydroxymethyl-16,16-trimethylene-20-nor-5-*cis*-13-*trans*-prostadiene |
| 322—332 | 319 | 281 | 50 | 1,9-dioxo-11$\alpha$,15-dihydroxy-1-hydroxymethyl-16,16-trimethylene-20-methyl-5-*cis*-13-*trans*-prostadiene |
| 333 | 319 | 281 | 51 | 1,9-dioxo-11$\alpha$,15-dihydroxy-1-hydroxymethyl-16,16-trimethylene-20-ethyl-5-*cis*-13-*trans*-prostadiene |
| 334 | 320 | 281 | 205 | 1,9-dioxo-11$\alpha$,16-dihydroxy-16-cyclopropyl-20-ethyl-1-hydroxymethyl-5-*cis*-13-*trans*-prostadiene |
| 335 | 320 | 281 | 207 | 1,9-dioxo-11$\alpha$,16-dihydroxy-16-vinyl-20-ethyl-1-hydroxymethyl-5-*cis*-13-*trans*-prostadiene |
| 336a | 320 | 281 | 198 | 1,9-dioxo-11$\alpha$,16-dihydroxy-16-cyclopropyl-1-hydroxymethyl-5-*cis*-13-*trans*-prostadiene |
| 336b | 320 | 281 | 210c | 1,9-dioxo-11$\alpha$,16-dihydroxy-16-ethyl-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 337 | 319 | 281 | 74 | *dl-erythro*-1,9-dioxo-11α,15-dihydroxy-16-methoxy-1-hydroxymethyl-5-*cis*-13-*trans*-prostadiene |
| 338 | 319 | 281 | 77 | *dl-erythro*-1,9-dioxo-11α,15-dihydroxy-16-ethoxy-1-hydroxymethyl-5-*cis*-13-*trans*-prostadiene |
| 339 | 319 | 281 | 83 | *dl-erythro*-1,9-dioxo-11α,15,16-trihydroxy-1-hydroxymethyl-20-nor-5-*cis*-13-*trans*-prostadiene |
| 340 | 319 | 281 | 84 | *dl-erythro*-1,9-dioxo-11α,15,16-trihydroxy-1-hydroxymethyl-20-methyl-5-*cis*-13-*trans*-prostadiene |
| 341 | 319 | 281 | 85 | *dl-erythro*-1,9-dioxo-11α,15,16-trihydroxy-1-hydroxymethyl-20-ethyl-5-*cis*-13-*trans*-prostadiene |
| 344 | 319 | 281 | 69 | *dl-threo*-1,9-dioxo-11α,15,16-trihydroxy-1-hydroxymethyl-5-*cis*-13-*trans*-prostadiene |
| 345 | 319 | 281 | 88 | *dl-threo*-1,9-dioxo-11α,15,16-trihydroxy-20-nor-1-hydroxymethyl-5-*cis*-13-*trans*-prostadiene |
| 346 | 319 | 281 | 76 | *dl-erythro*-1,9-dioxo-11α,15,16-trihydroxy-1-hydroxymethyl-5-*cis*-13-*trans*-prostadiene |
| 347 | 319 | 281 | 89 | *dl-threo*-1,9-dioxo-11α,15,16-trihydroxy-20-methyl-1-hydroxymethyl-5-*cis*-13-*trans*-prostadiene |
| 348 | 319 | 281 | 90 | *dl-threo*-1,9-dioxo-11α,15,16-trihydroxy-20-ethyl-1-hydroxymethyl-5-*cis*-13-*trans*-prostadiene |
| 351 | 319 | 281 | 107 | 1,9-dioxo-11α,16-dihydroxy-1-hydroxymethyl-5-*cis*-13-*trans*-prostadiene |
| 354 | 319 | 281 | 110 | 1,9-dioxo-11α,16-dihydroxy-1-hydroxymethyl-20-nor-5-*cis*-13-*trans*-prostadiene |
| 355 | 319 | 281 | 144 | 1,9-dioxo-11α,16-dihydroxy-17-methyl-1-hydroxymethyl-5-*cis*-13-*trans*-prostadiene |
| 356 | 319 | 281 | 112 | 1,9-dioxo-11α,16-dihydroxy-20-methyl-1-hydroxymethyl-5-*cis*-13-*trans*-prostadiene |
| 357 | 319 | 281 | 113 | 1,9-dioxo-11α,16-dihydroxy-20-ethyl-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 358 | 319 | 281 | 139a | 1,9-dioxo-11$\alpha$,16-dihydroxy-16-methyl-20-ethyl-1-hydroxymethyl-5-*cis*-13-*trans*-prostadiene |
| 360 | 319 | 281 | 135 | 1,9-dioxo-11$\alpha$,16-dihydroxy-16-methyl-1-hydroxymethyl-5-*cis*-13-*trans*-prostadiene |
| 361 | 319 | 281 | 136 | 1,9-dioxo-11$\alpha$,16-dihydroxy-17,17,20-trimethyl1-1-hydroxymethyl-5-*cis*-13-*trans*-prostadiene |
| 362 | 319 | 281 | 137 | 1,9-dioxo-11$\alpha$,16-dihydroxy-16,20-di-methyl-1-hydroxymethyl-13-*trans*,5-*cis*-prostadiene |
| 363 | 319 | 281 | 138 | 1,9-dioxo-11$\alpha$,16-dihydroxy-17,17-di-methyl-1-hydroxymethyl-5-*cis*-13-*trans*-prostadiene |
| 364 | 319 | 281 | 139 | 1,9-dioxo-11$\alpha$,16-dihydroxy-16-methyl-1-hydroxymethyl-13-*trans*-17-*trans*-5-*cis*-prostatriene |
| 365 | 319 | 281 | 149 | 1,9-dioxo-11$\alpha$,16-dihydroxy-20-methyl-1-hydroxymethyl-13-*trans*-17-*trans*-5-*cis*-prostatriene |
| 366 | 319 | 281 | 150 | 1,9-dioxo-11$\alpha$,16-dihydroxy-17-methyl-1-hydroxymethyl-5-*cis*-13-*trans*-prostadiene |
| 367 | 319 | 281 | 151 | 1,9-dioxo-11$\alpha$,16-dihydroxy-17,20-di-methyl-1-hydroxymethyl-5-*cis*-13-*trans*-prostadiene |
| 368 | 319 | 281 | 152 | 1,9-dioxo-11$\alpha$,16-dihydroxy-1-hydroxy-methyl-13-*trans*-17-*trans*-5-*cis*-prostatriene |
| 369 | 319 | 281 | 153 | 1,9-dioxo-11$\alpha$,16(R)-dihydroxy-1-hydroxy-methyl-5-*cis*-13-*trans*-prostadiene |
| 370 | 319 | 281 | 154 | 1,9-dioxo-11$\alpha$,16(S)-dihydroxy-1-hydroxy-methyl-5-*cis*-13-*trans*-prostadiene |
| 371 | 319 | 281 | 154a | 1,9-dioxo-11$\alpha$,16-dihydroxy-20-ethyl-1-hydroxymethyl-5-*cis*-13-*trans*-17-*trans*-prostatriene |
| 372 | 319 | 281 | 1-iodo-3-tri-phenylmethoxy-1-*trans*-octene U.S. Patent No. 3,873,607). | 1,9-dioxo-11$\alpha$,15$\alpha$-dihydroxy-1-hydroxy-methyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 373 | 319 | 281 | 1-iodo-3-triphenylmethoxy-1-*trans*-nonene (U.S. Patent No. 3,873,607). | 1,9-dioxo-11$\alpha$,15$\alpha$-dihydroxy-20-methyl-1-hydroxymethyl-5-*cis*-13-*trans*-prostadiene |
| 374 | 319 | 281 | 1-iodo-3-triphenylmethoxy-1-*trans*-decene (U.S. Patent No. 3,873,607). | 1,9-dioxo-11$\alpha$,15$\alpha$-dihydroxy-20-ethyl-1-hydroxymethyl-5-*cis*-13-*trans*-prostadiene |
| 375 | 319 | 281 | 1-iodo-3-triphenylmethoxy-4,4-dimethyl-1-trans-octene (U.S. Patent No. 3,873,607). | 1,9-dioxo-11$\alpha$,15$\alpha$-dihydroxy-16,16-dimethyl-1-hydroxymethyl-5-*cis*-13-*trans*- |
| 376 | 319 | 281 | 1-iodo-3-triphenylmethoxy-5,5-dimethyl-1-*trans*-octene (U.S. Patent No. 3,873,607). | 1,9-dioxo-11$\alpha$,15$\alpha$-dihydroxy-17,17-dimethyl-1-hydroxymethyl-5-*cis*-13-*trans*-prostadiene |
| 377 | 319 | 281 | 1-iodo-3-triphenylmethoxy-4-methyl-1-*trans*-octene (U.S. Patent No. 3,876,690). | 1,9-dioxo-11$\alpha$,15$\alpha$-dihydroxy-16-methyl-1-hydroxymethyl-5-*cis*-13-*trans*-prostadiene |
| 378 | 319 | 281 | 1-iodo-3-triphenylmethoxy-4-ethyl-1-*trans*-octene (U.S. Patent No. 3,876,690). | 1,9-dioxo-11$\alpha$,15$\alpha$-dihydroxy-16-ethyl-1-hydroxymethyl-5-*cis*-13-*trans*-prostadiene |
| 379 | 319 | 281 | 1-iodo-3-triphenylmethoxy-4-cyclopentyl-1-*trans*-butene-(U.S. Patent No. 3,884,969). | 1,9-dioxo-11$\alpha$,15$\alpha$-dihydroxy-17,20-tetranor-16-cyclopentyl-1-hydroxymethyl-5-*cis*-13-*trans*-prostadiene |
| 380 | 319 | 281 | 1-iodo-3-triphenylmethoxy-3-cyclohexyl-1-*trans*-propene (U.S. Patent No. 3,884,969). | 1,9-dioxo-11$\alpha$,15$\alpha$-dihydroxy-16,20-pentanor-15-cyclohexyl-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 381 | 319 | 281 | 1-iodo-3-triphenylmethoxy-5-cyclohexyl-1-*trans*-pentene (U.S. Patent No. 3,884,969). | 1,9-dioxo-11α,15α-dihydroxy-18,20-trinor-17-cyclohexyl-1-hydroxymethyl-5-*cis*-13-*trans*-prostadiene |
| 382 | 319 | 281 | 1-iodo-3-triphenylmethoxy-6-cyclopentyl-1-*trans*-hexene (U.S. Patent No. 3,884,969). | 1,9-dioxo-11α,15α-dihydroxy-19,20-dinor-18-cyclopentyl-1-hydroxymethyl-5-*cis*-13-*trans*-prostadiene |
| 383 | 319 | 281 | 1-iodo-3-methyl-3-trimethylsilyloxy-*trans*-1-octene (Example 190) | 1,9-dioxo-11α,15α-dihydroxy-15-methyl-1-hydroxymethyl-5-*cis*-13-*trans*-prostadiene |
| 384 | 319 | 281 | 1-iodo-3-methyl-3-trimethylsilyl oxy-*trans*-1-decene (Example 190a) | 1,9-dioxo-11α,15α-dihydroxy-15-methyl-20-ethyl-1-hydroxymethyl-5-*cis*-13-*trans*-prostadiene |
| 385 | 320 | 281 | 159 | 1,9-dioxo-11α,15α-dihydroxy-17,18,19,20-tetranor-16-*p*-fluorophenoxy-1-hydroxymethyl-5-*cis*-13-*trans*-prostadiene |
| 386 | 320 | 281 | 186 | 1,9-dioxo-11α,15α-dihydroxy-17,18,19,20-tetranor-16-phenoxy-1-hydroxymethyl-5-*cis*-13-*trans*-prostadiene |
| 387 | 320 | 281 | 181 | 1,9-dioxo-11α,15α-dihydroxy-17,18,19,20-tetranor-16-*p*-bromophenoxy-1-hydroxymethyl-5-*cis*-13-*trans*-prostadiene |
| 389 | 320 | 281 | 183 | 1,9-dioxo-11α,15α-dihydroxy-17,18,19,20-tetranor-16-*p*-methoxyphenoxy-1-hydroxymethyl-5-*cis*-13-*trans*-prostadiene |
| 390 | 320 | 281 | 184 | 1,9-dioxo-11α,15α-dihydroxy-17,18,19,20-tetranor-16-*m*-chlorophenoxy-1-hydroxymethyl-5-*cis*-13-*trans*-prostadiene |
| 391 | 320 | 201 | 182e | 1,9-dioxo-11α,15α-dihydroxy-17,18,19,20-tetranor-16-*m*-trifluorophenoxy-1-hydroxymethyl-5-*cis*-13-*trans*-prostadiene |
| 392 | 320 | 281 | 185 | 1,9-dioxo-11α,15α-dihydroxy-17,18,19,20-tetranor-16-(3,4-dichlorophenoxy)-1-hydroxymethyl-5-*cis*-13-*trans*-prostadiene |
| 393 | 320 | 281 | 186b | 1,9-dioxo-11α,15α-dihydroxy-18,19,20-trinor-17-phenyl-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 394 | 320 | 281 | 186e | 1,9-dioxo-11α,15α-dihydroxy-18,19,20-trinor-17-(*m*-trifluoromethylphenyl)-1-hydroxymethyl-5-*cis*-13-*trans*-prostadiene |
| 395 | 320 | 281 | 186d | 1,9-dioxo-11α,15α-dihydroxy-18,19,20-trinor-17-(*p*-methoxyphenyl)-1-hydroxymethyl-5-*cis*-13-*trans*-prostadiene |
| 396 | 319 | 290 | 1-iodo-3-triphenylmethoxy-1-*trans*-octene (U.S. Pat. 3,873,607) | 1,9-dioxo-11α,15α-dihydroxy-1-hydroxymethyl-2-nor-5-*cis*-13-*trans*-prostadiene |
| 397 | 319 | 290 | 190 | 1,9-dioxo-11α,15α-dihydroxy-15-methyl-1-hydroxymethyl-2-nor-5-*cis*-13-*trans*-prostadiene |
| 398 | 319 | 290 | 1-iodo-3-tri phenylmethoxy-5,5-dimethyl octene (U.S. Pat. 3,873,607) | 1,9-dioxo-11α,15α-dihydroxy-17,17-dimethyl-1-hydroxymethyl-2-nor-5-*cis*-13-*trans*-prostadiene |
| 399 | 319 | 290 | 13 | 1,9-dioxo-11α,15α-dihydroxy-16,16-trimethylene-1-hydroxymethyl-2-nor-5-*cis*-13-*trans*-prostadiene |
| 400 | 319 | 290 | 76 | *dl-erythro*-1,9-dioxo-11α,15α-16-trihydroxy-1-hydroxymethyl-2-nor-5-*cis*-13-*trans*-prostadiene |
| 401 | 319 | 290 | 69 | *dl-threo*-1,9-dioxo-11α,15α-16-trihydroxy-1-hydroxymethyl-2-nor-5-*cis*-13-*trans*-prostadiene |
| 402 | 319 | 290 | 74 | *dl-erythro*-1,9-dioxo-11α,15α-dihydroxy-16-methoxy-1-hydroxymethyl-2-nor-5-*cis*-13-*trans*-prostadiene |
| 403 | 319 | 290 | 107 | 1,9-dioxo-11α,16-dihydroxy-1-hydroxymethyl-2-nor-5-*cis*-13-*trans*-prostadiene |
| 404 | 319 | 290 | 130 | 1,9-dioxo-11α,16-dihydroxy-16-methyl-1-hydroxymethyl-2-nor-5-*cis*-13-*trans*-prostadiene |
| 405 | 319 | 290 | 134 | 1,9-dioxo-11α,16-dihydroxy-16-methyl-1-hydroxymethyl-2-nor-5-*cis*-13-*trans*-17-*trans*-prostatriene |
| 406 | 320 | 290 | 186 | 1,9-dioxo-11α,15α-dihydroxy-2,17,18-19,20-pentanor-16-phenoxy-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 407 | 320 | 290 | 180e | 1,9-dioxo-11α,15α-dihydroxy-2,17,18,-19,20-pentanor-16-*m*-trifluoromethyl-phenoxy-1-hydroxymethyl-5-*cis*-13-*trans*-prostadiene |
| 408 | 319 | 290 | 194 | 1,9-dioxo-11α,16-dihydroxy-16-vinyl-1-hydroxymethyl-2-nor-5-*cis*-13-*trans*-prostadiene |
| 409 | 320 | 290 | 198 | 1,9-dioxo-11α,16-dihydroxy-16-cyclo-propyl-1-hydroxymethyl-2-nor-5-*cis*-13-*trans*-prostadiene |
| 410 | 320 | 290 | 186b | 1,9-dioxo-11α,15α-dihydroxy-2,18,19-20-tetranor-17-phenyl-1-hydroxymethyl-5-*cis*-13-*trans*-prostadiene |
| 411 | 319 | 290 | 186e | 1,9-dioxo-11α,15α-dihydroxy-2,18,19,-20-tetranor-17-(*m*-trifluorophenyl)-1-hydroxymethyl-5-*cis*-13-*trans*-prostadiene |
| 412 | 319 | 290 | 186d | 1,9-dioxo-11α,15α-dihydroxy-2,18,19,-20-tetranor-17-(*p*-methoxyphenyl)-1-hydroxymethyl-5-*cis*-13-*trans*-prostadiene |
| 413 | 319 | 285 | 190 | 1,9-dioxo-15-hydroxy-15-methyl-1-hy-droxymethyl-2-nor-13-*trans*-prostene |
| 414 | 319 | 289 | 130 | 1,9-dioxo-11α,16-dihydroxy-16-methyl-1-homo-1-hydroxymethyl-5-*cis*-13-*trans*-prostadiene |
| 415 | 319 | 289 | 134 | 1,9-dioxo-11α-16-dihydroxy-16-methyl-1-homo-1-hydroxymethyl-5-*cis*-13-*trans*-17-*trans*-prostatriene |
| 416 | 320 | 289 | 186 | 1,9-dioxo-11α,15α-dihydroxy-1-homo-17,-18,19,20-tetranor-16-phenoxy-1-hydroxy-methyl-5-*cis*-13-*trans*-prostadiene |
| 417 | 320 | 289 | 180e | 1,9-dioxo-11α,15α-dihydroxy-1-homo-17,-18,19,20-tetranor-16-*m*-trifluoromethyl-phenoxy-1-hydroxymethyl-5-*cis*-13-*trans*-prostadiene |
| 418 | 319 | 289 | 194 | 1,9-dioxo-11α,16-dihydroxy-16-vinyl-1-homo-1-hydroxymethyl-5-*cis*-13-*trans*-prostadiene |
| 419 | 320 | 289 | 198 | 1,9-dioxo-11α,16-dihydroxy-16-cyclo-propyl-1-homo-1-hydroxymethyl-5-*cis*-13-*trans*-prostadiene |
| 420 | 320 | 289 | 186b | 1,9-dioxo-11α,15α-dihydroxy-1-homo-18,-19,20-trinor-17-phenyl-1-hydroxymethyl-5-*cis*-13-*trans*-prostadiene |

TABLE XXXII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 421 | 319 | 289 | 186e | 1,9-dioxo-11α,15α-dihydroxy-1-homo-18,-19,20-trinor-17-(*m*-trifluorophenyl)-13-*trans*-1-hydroxymethyl-5-*cis*-prostadiene |
| 422 | 319 | 289 | 186d | 1,9-dioxo-11α,15α-dihydroxy-1-homo-18,-19,20-trinor-17-(*p*-methoxyphenyl)-13-*trans*-1-hydroxymethyl-5-*cis*-prostadiene |
| 422A* | 320 | 281 | 210I | 1,9-dioxo-11α,16-dihydroxy-16-ethynyl-1-hydroxymethyl-5-*cis*,13-*trans*-prostadiene |
| 422B* | 320 | 281 | 210P | 1,9-dioxo-11α,16-dihydroxy-16-ethynyl-1-hydroxymethyl-20-nor-5-*cis*,13-*trans*-prostadiene |
| 422C* | 320 | 281 | 210Q | 1,9-dioxo-11α,16-dihydroxy-16-ethynyl-1-hydroxy-methyl-20-ethyl-5-*cis*,13-*trans*-prostadiene |
| 422D | 319 | 281 | 154I | 1,9-dioxo-11α,16-dihydroxy-16-(1-propynyl)-1-hydroxymethyl-5-*cis*,13-*trans*-prostadiene |
| 422E | 319 | 281 | 154J | 1,9-dioxo-11α,16-dihydroxy-16-(1-propynyl)-1-hydroxymethyl-20-ethyl-5-*cis*, 13-*trans*-prostadiene |

*For cleavage of the TMS—C≡C— group see, Example 772(B).

Examples 423—445

By the methods described hereinabove in Examples 319 and 320, the 1,9 - dioxo - 1 - hydroxymethyl prostene derivative shown in Table XXXIII are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XXXIII. It should be understood that the other diastereoisomer is also formed which in its *nat* and *ent* forms has an opposite (mirror image) configuration at the asymmetric carbon atoms on the β-chain (the chain containing $C_{13}$ ...$C_{14}$ etc.) to that of the respective *nat* and *ent* forms of the listed diastereoisomer; both of these diastereoisomers are claimed in this invention as well as their component enantiomers.

TABLE XXXIII

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 423 | 319 | 281 | 246 | *nat*(and *ent*)-1,9-dioxo-11α,15-dihydroxy-15,16-tetramethylene-17,18,19,20-tetranor-1-hydroxymethyl-5-*cis*,13-*trans*-prostadiene |
| 424 | 320 | 281 | 245 | *nat*(and *ent*)-1,9-dioxo-11α,16-dihydroxy-16,17-tetramethylene-18,19,20-trinor-1-hydroxymethyl-5-*cis*-13-*trans*-prostadiene |
| 425 | 319 | 281 | 243 | *nat*-15S,16R(and *ent*-15R,16S)-1,9-dioxo-11α,15-dihydroxy-15,16-trimethylene-1-hydroxymethyl-5-*cis*-13-*trans*-prostadiene |
| 426 | 319 | 281 | 243 | *nat*-15S,16R-(and *ent*-15R,16S)-1,9-dioxo-11α,15-dihydroxy-15,16-trimethylene-1-hydroxymethyl-13-*trans*-5-*cis*-prostadiene |
| 427 | 319 | 281 | 251 | *nat*-15S,16S-(and *ent*-15R,16R)-1,9-dioxo-11α,15-dihydroxy-15,16-trimethylene-1-hydroxymethyl-13-*trans*-5-*cis*-prostadiene |
| 428 | 319 | 281 | 247 | *nat*-(and *ent*)-1,9-dioxo-11α,15-dihydroxy-15,16-trimethylene-17,18,19,20-tetranor-1-hydroxymethyl-13-*trans*-5-*cis*-prostadiene |
| 429 | 320 | 281 | 248 | *nat*-(and *ent*)-1,9-dioxo-11α,15-dihydroxy-15,16-pentamethylene-17,18,19,20-tetranor-1-hydroxymethyl-13-*trans*-5-*cis*-prostadiene |
| 430 | 319 | 281 | 249 | *nat*-15S,17R-(and *ent*-15R,17S)-1,9-dioxo-11α,15-dihydroxy-15,17-dimethylene-1-hydroxymethyl-13-*trans*-5-*cis*-prostadiene |
| 431 | 319 | 281 | 250 | *nat*-15S,17S-(and *ent*-15R,17R)-1,9-dioxo-11α,15-dihydroxy-15,17-dimethylene-1-hydroxymethyl-13-*trans*-5-*cis*-prostadiene |
| 432 | 319 | 281 | 252 | *nat*-15S,16R-(and *ent*-15R,16S)-1,9-dioxo-11α,15-dihydroxy-15,16-tetramethylene-1-hydroxymethyl-13-*trans*-5-*cis*-prostadiene |
| 433 | 319 | 281 | 253 | *nat*-15S,16S-(and *ent*-15R,16R)-1,9-dioxo-11α,15-dihydroxy-15,16-tetramethylene-1-hydroxymethyl-13-*trans*-5-*cis*-prostadiene |
| 434 | 320 | 281 | 254 | *nat*-15R,16S-(and *ent*-15S,16R)-1,9-dioxo-11α,15-dihydroxy-15,16-trimethylene-16-(3-trifluoromethylphenoxy)-17,18,19,20-tetranor-1-hydroxymethyl-13-*trans*-5-*cis*-prostadiene |
| 435 | 320 | 281 | 255 | *nat*-15R,16R-(and *ent*-15S,16S)-1,9-dioxo-11α,15-dihydroxy-15,16-trimethylene-16-(3-trifluoromethylphenoxy)-17,18,19,20-tetranor-1-hydroxymethyl-13-*trans*-5-*cis*-prostadiene |

TABLE XXXIII (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 436 | 319 | 281 | 256 | *nat*-15R,16S-(and *ent*-15S,16R)-1,9-dioxo-11$\alpha$,15-dihydroxy-15,16-trimethylene-16-(4-fluorophenoxy)-17,18,19,20-tetranor-1-hydroxymethyl-13-*trans*-5-*cis*-prostadiene |
| 437 | 319 | 281 | 257 | *nat*-15R,16R-(and *ent*-15S,16S)-1,9-dioxo-11$\alpha$,15-dihydroxy-15,16-trimethylene-16-(4-fluorophenoxy)-17,18,19,20-tetranor-1-hydroxymethyl-13-*trans*-5-*cis*-prostadiene |
| 438 | 320 | 281 | 258 | *nat*-15R,16S-(and *ent*-15S,16R)-1,9-dioxo-11$\alpha$,15-dihydroxy-15,16-trimethylene-16-(3-chlorophenoxy)-17,18,19,20-tetranor-1-hydroxymethyl-13-*trans*-5-*cis*-prostadiene |
| 439 | 320 | 281 | 259 | *nat*-15R,16R-(and *ent*-15S,16S)-1,9-dioxo-11$\alpha$,15-dihydroxy-15,16-trimethylene-16-(3-chlorophenoxy)-17,18,19,20-tetranor-1-hydroxymethyl-13-*trans*-5-*cis*-prostadiene |
| 440 | 319 | 281 | 260 | *nat*-15S,17R-(and *ent*-15R,17S)-1,9-dioxo-11$\alpha$,15-dihydroxy-15,17-trimethylene-19,-20-dinor-1-hydroxymethyl-13-*trans*-5-*cis*-prostadiene |
| 441 | 319 | 281 | 261 | *nat*-15S,17S-(and *ent*-15R,17R)-1,9-dioxo-11$\alpha$,15-dihydroxy-15,17-trimethylene-19,-20-dinor-1-hydroxymethyl-13-*trans*-5-*cis*-prostadiene |
| 442 | 320 | 281 | 262 | *nat*-16R,17S-(and *ent*-16S,17R)-1,9-dioxo-11$\alpha$,16-dihydroxy-16,17-trimethylene-20-methyl-1-hydroxymethyl-13-*trans*-5-*cis*-prostadiene |
| 443 | 320 | 281 | 263 | *nat*-16R,17R-(and *ent*-16S,17S)-1,9-dioxo-11$\alpha$,16-dihydroxy-16,17-trimethylene-20-methyl-1-hydroxymethyl-13-*trans*-5-*cis*-prostadiene |
| 444 | 320 | 281 | 264 | *nat*-16R,17S-(and *ent*-16S,17R)-1,9-dioxo-11$\alpha$,16-dihydroxy-16,17-trimethylene-16-(3-trifluoromethylphenoxy)-18,19,20-tri-nor-1-hydroxymethyl-13-*trans*-5-*cis*-prostadiene |
| 445 | 320 | 281 | 265 | *nat*-16R,17R-(and *ent*-16S,17S)-1,9-dioxo-11$\alpha$,16-dihydroxy-16,17-trimethylene-16-(3-trifluoromethylphenoxy)-18,19,20-tri-nor-1-hydroxymethyl-13-*trans*-5-*cis*-prostadiene |

Examples 446—530E

By the methods described hereinabove in Examples 319 and 320 the 1,9-dioxo-1-hydroxymethyl prostene derivative shown in Table XXXIV are prepared by the indicated method from the indicated vinyl iodide or vinyl tin compound and the indicated cyclopent-2-en-1-one.

In those cases where isomers are obtained at the $C_{15}$ or $C_{16}$ positions, only the $C_{15}$ or $C_{16}$-normal isomers are listed in Table XXXIV; it should be understood that the corresponding $C_{15}$ or $C_{16}$-di-isomers are also formed and are part of this invention.

In those cases where the initial conjugate addition product contains a triphenylmethoxy blocking group, deblocking is conducted in acetic acid-tetrahydrofuran-water 4:2:1 at 50°C for 5 hours. [There are no Examples 459, 460, 466, 467, 469, 470, 476 and 506].

TABLE XXXIV

| Example | Method of Example | Cyclopent-2-en-1-one | Vinyl Iodide or Vinyl Tin of Example | 1,9-dioxo-L-hydroxymethylprostene |
|---|---|---|---|---|
| 446 | 319 | 292 | 49 | 1,9-dioxo-11α,15-dihydroxy-1-hydroxymethyl-16,16-trimethylene-20-nor-13-*trans*-prostene |
| 447 | 319 | 292 | 50 | 1,9-dioxo-11α,15-dihydroxy-1-hydroxymethyl-16,16-trimethylene-20-methyl-13-*trans*-prostene |
| 448 | 319 | 292 | 51 | 1,9-dioxo-11α,15-dihydroxy-1-hydroxymethyl-16,16-trimethylene-20-ethyl-13-*trans*-prostene |
| 449 | 320 | 292 | 205 | 1,9-dioxo-11α,16-dihydroxy-16-cyclopropyl-20-ethyl-1-hydroxymethyl-13-*trans*-prostene |
| 450 | 320 | 292 | 207 | 1,9-dioxo-11α,16-dihydroxy-16-vinyl-20-ethyl-1-hydroxymethyl-13-*trans*-prostene |
| 451 | 320 | 292 | 198 | 1,9-dioxo-11α,16-dihydroxy-16-cyclopropyl-1-hydroxymethyl-13-*trans*-prostene |
| 452 | 319 | 292 | 13 | 1,9-dioxo-11α,15-dihydroxy-1-hydroxymethyl-16,16-trimethylene-13-*trans*-prostene |
| 453 | 320 | 292 | 210a | 1,9-dioxo-11α,16-dihydroxy-1-hydroxymethyl-16-vinyl-13-*trans*-prostene |
| 454 | 319 | 292 | 74 | *dl-erythro*-1,9-dioxo-11α,15-dihydroxy-16-methoxy-1-hydroxymethyl-13-*trans*-prostene |
| 455a | 319 | 292 | 77 | *dl-erythro*-1,9-dioxo-11α,15-dihydroxy-16-ethoxy-1-hydroxymethyl-13-*trans*-prostene |
| 455b | 320 | 292 | 210c | 1,9-dioxo-11α,16-dihydroxy-16-ethyl-1-hydroxymethyl-13-*trans*-prostene |
| 456 | 319 | 292 | 83 | *dl-erythro*-1;9-dioxo-11α-15,16-trihydroxy-1-hydroxymethyl-20-nor-13-*trans*-prostene |
| 457 | 319 | 292 | 84 | *dl-erythro*-1,9-dioxo-11α,15,16-trihydroxy-1-hydroxymethyl-20-methyl-13-*trans*-prostene |

TABLE XXXIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 458 | 319 | 292 | 85 | *dl-erythro*-1,9-dioxo-11α,15,16-tri-hydroxy-20-ethyl-1-hydroxymethyl-13-*trans*-prostene |
| 461 | 319 | 292 | 69 | *dl-threo*-1,9,dioxo-11α,15,16-trihydroxy-1-hydroxymethyl-13-*trans*-prostene |
| 462 | 319 | 292 | 88 | *dl-threo*-1,9-dioxo-11α-15,16-trihydroxy-20-nor-1-hydroxymethyl-13-*trans*-prostene |
| 463 | 319 | 292 | 76 | *dl-erythro*-1,9-dioxo-11α,15,16-tri-hydroxy-1-hydroxymethyl-13-*trans*-prostene |
| 464 | 319 | 292 | 89 | *dl-threo*-1,9-dioxo-11α-15,16-trihydroxy-20-methyl-1-hydroxymethyl-13-*trans*-prostene |
| 465 | 319 | 292 | 90 | *dl-threo*-1,9-dioxo-11α,15,16-trihydroxy-20-ethyl-1-hydroxymethyl-13-*trans*-prostene |
| 468 | 319 | 292 | 107 | 1,9-dioxo-11α,16-dihydroxy-1-hydroxy-methyl-13-*trans*-prostene |
| 471 | 319 | 292 | 110 | 1,9-dioxo-11α,16-dihydroxy-1-hydroxy-methyl-20-nor-13-*trans*-prostene |
| 472 | 319 | 292 | 144 | 1,9-dioxo-11α,16-dihydroxy-17-methyl-1-hydroxymethyl-13-*trans*-prostene |
| 473 | 319 | 292 | 112 | 1,9-dioxo-11α,16-dihydroxy-20-methyl-1-hydroxymethyl-13-*trans*-prostene |
| 474 | 319 | 292 | 113 | 1,9-dioxo-11α,16-dihydroxy-20-ethyl-1-hydroxymethyl-13-*trans*-prostene |
| 475 | 319 | 292 | 139a | 1,9-dioxo-11α,16-dihydroxy-16-methyl-20-ethyl-1-hydroxymethyl-13-*trans*-prostene |
| 477 | 319 | 292 | 135 | 1,9-dioxo-11α,16-dihydroxy-16-methyl-1-hydroxymethyl-13-*trans*-prostene |
| 478 | 319 | 292 | 136 | 1,9-dioxo-11α,16-dihydroxy-17,17,20-tri-methyl-1-hydroxymethyl-13-*trans*-prostene |
| 479 | 319 | 292 | 137 | 1,9-dioxo-11α,16-dihydroxy-16,20-di-methyl-1-hydroxymethyl-13-*trans*-prostene |
| 480 | 319 | 292 | 138 | 1,9-dioxo-11α,16-dihydroxy-17,17-di-methyl-1-hydroxymethyl-13-*trans*-prostene |
| 481 | 319 | 292 | 139 | 1,9-dioxo-11α,16-dihydroxy-16-methyl-1-hydroxymethyl-13-*trans*-17-*trans*-prosta-diene |
| 482 | 319 | 292 | 149 | 1,9-dioxo-11α,16-dihydroxy-20-methyl-1-hydroxymethyl-13-*trans*-17-*trans*-prosta-diene |

TABLE XXXIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 483,484 | 319 | 292 | 150 | 1,9-dioxo-11α,16-dihydroxy-17-methyl-1-hydroxymethyl-13-*trans*-prostene |
| 485 | 319 | 292 | 151 | 1,9-dioxo-11α,16-dihydroxy-17,20-dimethyl-1-hydroxymethyl-13-*trans*-prostene |
| 486 | 319 | 292 | 152 | 1,9-dioxo-11α,16-dihydroxy-1-hydroxymethyl-13-*trans*-17-*trans*-prostadiene |
| 487 | 319 | 292 | 153 | 1,9-dioxo-11α,16(R)-dihydroxy-1-hydroxymethyl-13-*trans*-prostene |
| 488 | 319 | 292 | 154 | 1,9-dioxo-11α,16(S)-dihydroxy-1-hydroxymethyl-13-*trans*-prostene |
| 489 | 319 | 292 | 154a | 1,9-dioxo-11α,16-dihydroxy-20-ethyl-1-hydroxymethyl-13-*trans*-17-*trans*-prostadiene |
| 490 | 319 | 292 | 1-iodo-3-triphenylmethoxy-1-*trans*-octene (U.S. Pat. No. 3,873,607). | 1,9-dioxo-11α,15α-dihydroxy-1-hydroxymethyl-13-*trans*-prostadiene |
| 491 | 319 | 292 | 1-iodo-3-triphenylmethoxy-1-trans-nonene (U.S. Pat. No. 3,873,607). | 1,9-dioxo-11α,15α-dihydroxy-20-methyl-1-hydroxymethyl-13-*trans*-prostene |
| 492 | 319 | 292 | 1-iodo-3-triphenylmethoxy-1-*trans*-decene (U.S. Pat. No. 3,873,607). | 1,9-dioxo-11α-15α-dihydroxy-20-ethyl-1-hydroxymethyl-13-*trans*-prostene |
| 493 | 319 | 292 | 1-iodo-3-triphenylmethoxy-4,4-dimethyl 1-*trans*-octene (U.S. Pat. No. 3,873,607). | 1,9-dioxo-11α,15α-dihydroxy-16,16-dimethyl-1-hydroxymethyl-13-*trans*-prostene |
| 494 | 319 | 292 | 1-iodo-3-triphenylmethoxy-5,5-dimethyl-1-*trans*-octene (U.S. Pat. No. 3,873,607). | 1,9-dioxo-11α,15α-dihydroxy-17,17-dimethyl-1-hydroxymethyl-13-*trans*-prostene |
| 495 | 319 | 292 | 1-iodo-3-triphenylmethoxy-4-methyl-1-*trans*-octene (U.S. Pat. No. 3,876,690). | 1,9-dioxo-11α,15α-dihydroxy-16-methyl-1-hydroxymethyl-13-*trans*-prostene |

TABLE XXXIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 496 | 319 | 292 | 1-iodo-3-triphenylmethoxy-4-ethyl-1-*trans*-octene (U.S. Pat. No. 3,876,690). | 1,9-dioxo-11α,15α-dihydroxy-16-ethyl-1-hydroxymethyl-13-*trans*-prostene |
| 497 | 319 | 292 | 1-iodo-3-triphenylmethoxy-4-cyclopentyl-1-*trans*-butene-(U.S. Pat. No. 3,884,969). | 1,9-dioxo-11α,15α-dihydroxy-17,20-tetranor-16-cyclopentyl-1-hydroxymethyl-13-*trans*-prostene |
| 498 | 319 | 292 | 1-iodo-3-triphenylmethoxy-3-cyclohexyl-1-*trans*-propene (U.S. Pat. No. 3,884,969). | 1,9-dioxo-11α,15α-dihydroxy-16,20-pentanor-15-cyclohexyl-1-hydroxymethyl-13-*trans*-prostene |
| 499 | 319 | 292 | 1-iodo-3-triphenylmethoxy-5-cyclohexyl-1-*trans*-pentene (U.S. Pat. No. 3,884,969). | 1,9-dioxo-11α,15α-dihydroxy-18,20-trimor-17-cyclohexyl-1-hydroxymethyl-13-*trans*-prostene |
| 500 | 319 | 292 | 1-iodo-3-triphenylmethoxy-6-cyclopentyl-1-*trans*-hexene (U.S. Pat. No. 3,884,969). | 1,9-dioxo-11α,15α-dihydroxy-19,20-dinor-18-cyclopentyl-1-hydroxymethyl-13-*trans*-prostene |
| 501 | 319 | 292 | 1-iodo-3-trimethylsilyloxy-*trans*-1-octene (Example 190) | 1,9-dioxo-11α,15α-dihydroxy-15-methyl-1-hydroxymethyl-13-*trans*-prostene |
| 502 | 319 | 292 | 1-iodo-3-methyl-3-trimethylsilyloxy-*trans*-1 -decene (Example 190a) | 1,9-dioxo-11α,15α-dihydroxy-15-methyl-20-ethyl-1-hydroxymethyl-13-*trans*-prostene |
| 503 | 320 | 292 | 159 | 1,9-dioxo-11α,15α-dihydroxy-17,18,19,20-tetranor-16-*p*-fluorophenoxy-1-hydroxymethyl-13-*trans*-prostene |
| 504 | 320 | 292 | 186 | 1,9-dioxo-11α,15α-dihydroxy-17,18,19,20-tetranor-16-phenoxy-1-hydroxymethyl-13-*trans*-prostene |
| 505 | 320 | 292 | 181 | 1,9-dioxo-11α,15α-dihydroxy-17,18,19,20-tetranor-16-*p*-bromophenoxy-1-hydroxymethyl-13-*trans*-prostene |

TABLE XXXIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 507 | 320 | 292 | 183 | 1,9-dioxo-11α,15α-dihydroxy-17,18,19,20-tetranor-16-*p*-methoxyphenoxy-1-hydroxy-methyl-13-*trans*-prostene |
| 508 | 320 | 292 | 184 | 1,9-dioxo-11α,15α-dihydroxy-17,18,19,20-tetranor-16-*m*-chlorophenoxy-1-hydroxy-methyl-13-*trans*-prostene |
| 509 | 320 | 292 | 180 | 1,9-dioxo-11α,15α-dihydroxy-17,18,19,20-tetranor-16-*m*-trifluorophenoxy-1-hydroxymethyl-13-*trans*-prostene |
| 510 | 320 | 292 | 185 | 1,9-dioxo-11α,15α-dihydroxy-17,18,19,20-tetranor-16-(3,4-dichlorophenoxy)-1-hydroxymethyl-13-*trans*-prostene |
| 511 | 320 | 292 | 186b | 1,9-dioxo-11α,15α-dihydroxy-18,19,20-trinor-17-phenyl-1-hydroxymethyl-13-*trans*-prostene |
| 512 | 320 | 292 | 186e | 1,9-dioxo-11α,15α-dihydroxy-18,19,20-trinor-17-(*m*-trifluoromethylphenyl)-1-hydroxy-13-*trans*-prostene |
| 513 | 320 | 292 | 186d | 1,9-dioxo-11α,15α-dihydroxy-18,19,20-trinor-17-(*p*-methoxyphenyl)-1-hydroxy-methyl-13-*trans*-prostene |
| 514 | 319 | 293 | 1-iodo-3-triphenylmethoxy-1-*trans*-octene (U.S. Pat. No. 3,873,607). | 1,9-dioxo-11α15α-dihydroxy-1-hydroxy-methyl-*trans*-prostadiene |
| 515 | 319 | 293 | 190 | 1,9-dioxo-11α,15α-dihydroxy-15-methyl-1-hydroxymethyl-2-nor-13-*trans*-prostene |
| 516 | 319 | 293 | 1-iodo-3-triphenylmethoxy-5,5-dimethyl-octene (U.S. Pat. No. 3,873,607) | 1,9-dioxo-11α,15α-dihydroxy-17,17-dimethyl-1-hydroxymethyl-2-nor-13-*trans*-prostene |
| 517 | 319 | 293 | 13 | 1,9-dioxo-11α,15α-dihydroxy-16,16-trimethylene-1-hydroxymethyl-2-nor-13-*trans*-prostene |
| 518 | 319 | 293 | 76 | *dl-erythro*-1,9-dioxo-11α,15α,16-trihydroxy-1-hydroxymethyl-2-nor-13-*trans*-prostene |
| 519 | 319 | 293 | 69 | *dl-threo*-1,9-dioxo-11α,15α,16,trihydroxy-1-hydroxymethyl-2-nor-13-*trans*-prostene |
| 520 | 293 | 319 | 74 | *dl-erythro*-1,9-dioxo-11α,15α-dihydroxy-16-methoxy-1-hydroxymethyl-2-nor-13-*trans*-prostene |

TABLE XXXIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 521 | 319 | 293 | 107 | 1,9-dioxo-11α,16-dihydroxy-1-hydroxymethyl-2-nor-13-*trans*-prostene |
| 522 | 319 | 293 | 130 | 1,9-dioxo-11α,16-dihydroxy-16-methyl-1-hydroxymethyl-2-nor-13-*trans*-prostene |
| 523 | 319 | 293 | 134 | 1,9-dioxo-11α,16-dihydroxy-16-methyl-1-hydroxymethyl-2-nor-13-*trans*-17-*trans*-prostadiene |
| 524 | 320 | 293 | 186 | 1,9-dioxo-11α,15α-dihydroxy-2,17,18,19-20-pentanor-16-phenoxy-1-hydroxymethyl-13-*trans*-prostene |
| 525 | 320 | 293 | 180e | 1,9-dioxo-11α,15α-dihydroxy-2,17,18,19-20-pentanor-16-*m*-trifluoromethylphenoxy-1-hydroxymethyl-13-*trans*-prostene |
| 530A* | 320 | 292 | 210I | 1,9-dioxo-11α,16-dihydroxy-16-ethynyl-1-hydroxymethyl-13-*trans*-prostene |
| 530B* | 320 | 292 | 210P | 1,9-dioxo-11α,16-dihydroxy-16-ethynyl-1-hydroxymethyl-20-nor-13-*trans*-prostene |
| 530C* | 320 | 292 | 210Q | 1,9-dioxo-11α,16-dihydroxy-16-ethynyl-1-hydroxymethyl-20-ethyl-13-*trans*-prostene |
| 530D | 319 | 292 | 154I | 1,9-dioxo-11α,16-dihydroxy-16-(1-propynyl)-1-hydroxymethyl-13-*trans*-prostene |
| 530E | 319 | 292 | 154J | 1,9-dioxo-11α,16-dihydroxy-16-(1-propynyl)-1-hydroxymethyl-20-ethyl-13-*trans*-prostene |

*For the cleavage of the TMS—C≡C— bond see Example 772(B).

Examples 531—553

The methods described hereinabove in Examples 319 and 320, the 1,9-dioxo-1-hydroxymethyl prostene derivatives shown in Table XXXV are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XXXV. It should be understood that the other diastereoisomer is also formed which in its *nat* and *ent* forms has an opposite (mirror image) configuration at the asymmetric carbon atoms on the β-chain (the chain containing $C_{13}...C_{14}$ etc) to that of the respective *nat* and *ent* forms of the listed diastereoisomer; both of these diastereoisomers are claimed in this invention as well as their component enantiomers.

[There is no Example 534].

TABLE XXXV

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 531 | 319 | 292 | 246 | *nat*(and *ent*)-1,9-dioxo-11α,15-dihydroxy-15,16-tetramethylene-17,18,19,20-tetranor-1-hydroxymethyl-13-*trans*-prostene |
| 532 | 320 | 292 | 245 | *nat*(and *ent*)-1,9-dioxo-11α,16-dihydroxy-16,17-tetramethylene-18,19,20-trinor-1-hydroxymethyl-13-*trans*-prostene |
| 533 | 319 | 292 | 243 | *nat*-15S,16R(and *ent*-15R,16S)-1,9-dioxo-11α,15-dihydroxy-15,16-trimethylene-1-hydroxymethyl-13-*trans*-prostene |
| 535 | 319 | 292 | 244 | *nat*-15S,16S-(and *ent*-15R,16R)-1,9-dioxo-11α,15-dihydroxy-15,16-trimethylene-1-hydroxymethyl-13-*trans*-prostene |
| 536 | 319 | 292 | 247 | *nat*-(and *ent*)-1,9-dioxo-11α,15-dihydroxy-15,16-trimethylene-17,18,19,20-tetranor-1-hydroxymethyl-13-*trans*-prostene |
| 537 | 320 | 292 | 248 | *nat*-(and *ent*)-1,9-dioxo-11α,15-dihydroxy-15,16-pentamethylene-17,18,19,20-tetranor-1-hydroxymethyl-13-*trans*-prostene |
| 538 | 319 | 292 | 249 | *nat*-15S,17R-(and *ent*-15R,17S)-1,9-dioxo-11α15-dihydroxy-15,17-dimethylene-1-hydroxymethyl-13-*trans*-prostene |
| 539 | 319 | 292 | 250 | *nat*-15S,17S-(and *ent*-15R,17R)-1,9-dioxo-11α,15-dihydroxy-15,17-dimethylene-1-hydroxymethyl-13-*trans*-prostene |
| 540 | 319 | 292 | 252 | *nat*-15S,16R-(and *ent*-15R,16S)-1,9-dioxo-11α,15-dihydroxy-15,16-tetramethylene-1-hydroxymethyl-13-*trans*-prostene |
| 541 | 319 | 292 | 253 | *nat*-15S,16S-(and *ent*-15R,16R)-1,9-dioxo-11α,15-dihydroxy-15,16-tetramethylene-1-hydroxymethyl-13-*trans*-prostene |
| 542 | 320 | 292 | 254 | *nat*-15R,16S-(and *ent*-15S,16R)-1,9-dioxo-11α,15-dihydroxy-15,16-trimethylene-16-(3-trifluoromethylphenoxy)-17,18,19,20-tetranor-1-hydroxymethyl-13-*trans*-prostene |
| 543 | 320 | 292 | 255 | *nat*-15R,16R-(and *ent*-15S,16S)-1,9-dioxo-11α,15-dihydroxy-15,16-trimethylene-16-(3-trifluoromethylphenoxy)-17,18,19,20-tetranor-1-hydroxymethyl-13-*trans*-prostene |
| 544 | 319 | 292 | 256 | *nat*-15R,16S-(and *ent*-15S,16R)-1,9-dioxo-11α,15-dihydroxy-15,16-trimethylene-16-(4-fluorophenoxy)-17,18,19,20-tetranor-1-hydroxymethyl-13-*trans*-prostene |

TABLE XXXV (continued)

| Example | Method of Example | Cyclopent-2-en-1-one of Example | Vinyl Iodide or Vinyl Tin of Example | 1,9-dioxo-1-hydroxymethylprostene and its diasteromer |
|---|---|---|---|---|
| 545 | 319 | 292 | 257 | *nat*-15R,16R-(and *ent*-15S, 16)-1,9-dioxo-11$\alpha$,15-dihydroxy-15,16-trimethylene-16-(4-fluorophenoxy)-17,18,19,20-tetranor-1-hydroxymethyl-13-*trans*-prostene |
| 546 | 320 | 292 | 258 | *nat*-15R,16S-(and *ent*-15S,16R)-1,9-dioxo-11$\alpha$,15-dihydroxy-15,16-trimethylene-16-(3-chlorophenoxy)-17,18,19,20-tetranor-1-hydroxymethyl-13-*trans*-prostene |
| 547 | 320 | 292 | 259 | *nat*-15R,16R-(and *ent*-15S,16S)-1,9-dioxo-11$\alpha$,15-dihydroxy-15,16-trimethylene-16-(3-chlorophenoxy)-17,18,19,20-tetranor-1-hydroxymethyl-13-*trans*-prostene |
| 548 | 319 | 292 | 260 | *nat*-15S,17R-(and *ent*-15R,17S)-1,9-dioxo-11$\alpha$,15-dihydroxy-15,17-trimethylene-19,-20-dinor-1-hydroxymethyl-13-*trans*-prostene |
| 549 | 319 | 292 | 261 | *nat*-15S,17S-(and *ent*-15R,17R)-1,9-dioxo-11$\alpha$,15-dihydroxy-15,17-trimethylene-19,-20-dinor-1-hydroxymethyl-13-*trans*-prostene |
| 550 | 320 | 292 | 262 | *nat*-16R,17S-(and *ent*-16S,17R)-1,9-dioxo-11$\alpha$,16-dihydroxy-16,17-trimethylene-20-methyl-1-hydroxymethyl-13-*trans*-prostene |
| 551 | 320 | 292 | 263 | *nat*-16R,17R-(and *ent*-16S,17S)-1,9-dioxo-11$\alpha$,16-dihydroxy-16,17-trimethylene-20-methyl-1-hydroxymethyl-13-*trans*-prostene |
| 552 | 320 | 292 | 264 | *nat*-16R,17S-(and *ent*-16S,17R)-1,9-dioxo-11$\alpha$,16-dihydroxy-16,17-trimethylene-16-(3-trifluoromethylphenoxy)-18,19,20-trinor-1-hydroxymethyl-13-*trans*-prostene |
| 553 | 320 | 292 | 265 | *nat*-16R,17R-(and *ent*-16S,17S)-1,9-dioxo 11$\alpha$,16-dihydroxy-16,17-trimethylene-16-(3-trifluoromethylphenoxy)-18,19,20-trinor-1- hydroxymethyl-13-*trans*-prostene |

Examples 554—637G

By the methods described hereinabove in Examples 319 and 320, the 1,9 - dioxo - 1 - hydroxymethyl prostene derivatives shown in Table XXXVI are prepared by the indicated method from the indicated vinyl iodide or vinyl tin compound and the indicated cyclopent - 2 - en - 1 - one.

In those cases where isomers are obtained at the $C_{15}$ or $C_{16}$ positions, only the $C_{15}$ or $C_{16}$-normal isomers are listed in Table XXXVI; it should be understood that the corresponding $C_{15}$ or $C_{16}$-*epi* isomer is also formed and is part of this invention.

In those cases where the initial conjugate addition product contains a triphenylmethoxy blocking group, deblocking is conducted in acetic acid-tetrahydrofuran-water 4:2:1 at 50°C for 5 hours. [There are no Examples 567, 568, 574, 575, 577, 578, 584 and 613].

TABLE XXXVI

| Example | Method of Example | Cyclopent-2-en-1-one | Vinyl Iodide or Vinyl Tin of Example | 1,9-dioxo-1-hydroxymethylprostene |
|---|---|---|---|---|
| 554 | 319 | 287 | 49 | 1,9-dioxo-15-hydroxy-1-hydroxymethyl-16,16- trimethylene-20-nor-5-*cis*-13-*trans*-prostadiene |
| 555 | 319 | 287 | 50 | 1,9-dioxo-15-hydroxy-1-hydroxymethyl-16,-16-trimethylene-20-methyl-5-*cis*-13-*trans*-prostadiene |
| 556 | 319 | 287 | 51 | 1,9-dioxo-15-hydroxy-1-hydroxymethyl-16,-16-trimethylene-20-ethyl-5-*cis*-13-*trans*-prostadiene |
| 557 | 320 | 287 | 205 | 1,9-dioxo-16-hydroxy-16-cyclopropyl-20-ethyl-1-hydroxymethyl-5-*cis*-13-*trans*-prostadiene |
| 558 | 320 | 287 | 207 | 1,9-dioxo-16-hydroxy-16-vinyl-20-ethyl-1-hydroxymetyl-5-*cis*-13-*trans*-prostadiene |
| 559 | 320 | 287 | 198 | 1,9-dioxo-16-hydroxy-16-cyclopropyl-1-hydroxymethyl-5-*cis*-13-*trans*-prostadiene |
| 560 | 319 | 287 | 13 | 1,9-dioxo-15-hydroxy-1-hydroxymethyl-16,-16-trimethylene-13-*trans*-5-*cis*-prostadiene |
| 561 | 320 | 287 | 210a | 1,9-dioxo-16-hydroxy-1-hydroxymethyl-16-vinyl-5-*cis*-13-*trans*-prostadiene |
| 562 | 319 | 287 | 74 | *dl-erythro*-1,9-dioxo-15-hydroxy-16-methoxy-1-hydroxymethyl-5-*cis*-13-*trans*-prostadiene |
| 563 | 319 | 287 | 77 | *dl-erythro*-1,9-dioxo-15-hydroxy-16-ethoxy-1-hydroxymethyl-5-*cis*-13-*trans*-prostadiene |
| 564 | 319 | 287 | 83 | *dl-erythro*-1,9-dioxo-15,16-dihydroxy-1-hydroxymethyl-20-nor-5-*cis*-13-*trans*-prostadiene |
| 565 | 319 | 287 | 84 | *dl-erythro*-1,9-dioxo-15,16-dihydroxy-1-hydroxymethyl-20-methyl-5-*cis*-13-*trans*-prostadiene |
| 566 | 319 | 287 | 85 | *dl-erythro*-1,9-dioxo-15,16-dihydroxy-20-ethyl-1-hydroxymethyl-5-*cis*-13-*trans*-prostadiene |
| 569 | 319 | 287 | 69 | *dl-threo*-1,9-dioxo-15,16-dihydroxy-1-hydroxymethyl-5-*cis*-13-*trans*-prostadiene |
| 570 | 319 | 287 | 88 | *dl-threo*-1,9-dioxo-15,16-dihydroxy-20-nor-1-hydroxymethyl-5-*cis*-13-*trans*-prostadiene |
| 571 | 319 | 287 | 76 | *dl-erythro*-1,9-dioxo-15,16-dihydroxy-1-hydroxymethyl-5-*cis*-13-*trans*-prostadiene |

TABLE XXXVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 572 | 319 | 287 | 89 | *dl-threo*-1,9-dioxo-15,16-dihydroxy-20-methyl-1-hydroxymethyl-5-*cis*-13-*trans*-prostadiene |
| 573 | 319 | 287 | 90 | *dl-threo*-1,9-dioxo-15,16-dihydroxy-20-ethyl-1-hydroxymethyl-5-*cis*-13-*trans*-prostadiene |
| 576 | 319 | 287 | 107 | 1,9-dioxo-16-hydroxy-1-hydroxymethyl-5-*cis*-13-*trans*-prostadiene |
| 579 | 319 | 287 | 110 | 1,9-dioxo-16-hydroxy-1-hydroxymethyl-20-nor-5-*cis*-13-*trans*-prostadiene |
| 580 | 319 | 287 | 144 | 1,9-dioxo-16-hydroxy-17-methyl-1-hydroxymethyl-5-*cis*-13-*trans*-prostadiene |
| 581 | 319 | 287 | 112 | 1,9-dioxo-16-hydroxy-20-methyl-1-hydroxymethyl-5-*cis*-13-*trans*-prostadiene |
| 582 | 319 | 287 | 113 | 1,9-dioxo-16-hydroxy-20-ethyl-1-hydroxymethyl-5-*cis*-13-*trans*-prostadiene |
| 583 | 319 | 287 | 139a | 1,9-dioxo-16-hydroxy-16-methyl-20-ethyl-1-hydroxymethyl-5-*cis*-13-*trans*-prostadiene |
| 585 | 319 | 287 | 135 | 1,9-dioxo-16-hydroxy-16-methyl-1-hydroxymethyl-5-*cis*-13-*trans*-prostadiene |
| 586 | 319 | 287 | 136 | 1,9-dioxo-16-hydroxy-17,17,20-trimethyl-1-hydroxymethyl-5-*cis*-13-*trans*-prostadiene |
| 587 | 319 | 287 | 137 | 1,9-dioxo-16-hydroxy-16,20-dimethyl-1-hydroxymethyl-13-*trans*-5-*cis*-prostadiene |
| 588 | 319 | 287 | 138 | 1,9-dioxo-16-hydroxy-17,17-dimethyl-1-hydroxymethyl-5-*cis*-13-*trans*-prostadiene |
| 589 | 319 | 287 | 139 | 1,9-dioxo-16-hydroxy-16-methyl-1-hydroxymethyl-13-*trans*-17-*trans*-5-*cis*-prostatriene |
| 590 | 319 | 287 | 149 | 1,9-dioxo-16-hydroxy-20-methyl-1-hydroxymethyl-13-*trans*-17-*trans*-5-*cis*-prostatriene |
| 591 | 319 | 287 | 150 | 1,9-dioxo-16-hydroxy-17-methyl-1-hydroxymethyl-5-*cis*-13-*trans*-prostadiene |
| 592 | 319 | 287 | 151 | 1,9-dioxo-16-hydroxy-17,20-dimethyl-1-hydroxymethyl-5-*cis*-13-*trans*-prostadiene |
| 593 | 319 | 287 | 152 | 1,9-dioxo-16-hydroxy-1-hydroxymethyl-13-*trans*-17-*trans*-5-*cis*-prostatriene |

TABLE XXXVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 594 | 319 | 287 | 153 | 1,9-dioxo-16(R)-hydroxy-1-hydroxymethyl-5-*cis*-13-*trans*-prostadiene |
| 595 | 319 | 287 | 154 | 1,9-dioxo-16(S)-hydroxy-1-hydroxymethyl-5-*cis*-13-*trans*-prostadiene |
| 596 | 319 | 287 | 154a | 1,9-dioxo-16-hydroxy-20-ethyl-1-hydroxymethyl-5-*cis*-13-*trans*-17-*trans*-prostatriene |
| 597 | 319 | 287 | 1-iodo-3-triphenylmethoxy-1-*trans*-octene (U.S. Pat. No. 3,873,607). | 1,9-dioxo-15α-hydroxy-1-hydroxymethyl-5-*cis*-13-*trans*-prostadiene |
| 598 | 319 | 287 | 1-iodo-3-triphenylmethoxy-1-*trans*-nonene (U.S. Pat. No. 3,873,607). | 1,9-dioxo-15α-hydroxy-20-methyl-1-hydroxymethyl-5-*cis*-13-*trans*-prostadiene |
| 599 | 319 | 287 | 1-iodo-3-triphenylmethoxy-1-*trans*-decene (U.S. Pat. No. 3,873,607). | 1,9-dioxo-15α-hydroxy-20-ethyl-1-hydroxymethyl-5-*cis*-13-*trans*-prostadiene |
| 600 | 319 | 287 | 1-iodo-3-triphenylmethoxy-4,4-dimethyl-1-*trans*-octene (U.S. Pat. No. 3,873,607). | 1,9-dioxo-15α-hydroxy-16,16-dimethyl-1-hydroxymethyl-5-*cis*-13-*trans*-prostadiene |
| 601 | 319 | 287 | 1-iodo-3-triphenylmethoxy-5,5-dimethyl-1-*trans*-octene (U.S. Pat. No. 3,873,607). | 1,9-dioxo-15α-hydroxy-17,17-dimethyl-1-hydroxymethyl-5-*cis*-13-*trans*-prostadiene |
| 602 | 319 | 287 | 1-iodo-3-triphenylmethoxy-4-methyl-1-*trans*-octene (U.S. Pat. No. 3,876,690). | 1,9-dioxo-15α-hydroxy-16-methyl-1-hydroxymethyl-5-*cis*-13-*trans*-prostadiene |
| 603 | 319 | 287 | 1-iodo-3-triphenylmethoxy 4-ethyl-1-*trans*-octene (U.S. Pat. No. 3,876,690). | 1,9-dioxo-15α-hydroxy-16-ethyl-1-hydroxymethyl-5-*cis*-13-*trans*-prostadiene |

TABLE XXXVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 604 | 319 | 287 | 1-iodo-3-triphenylmethoxy-4-cyclopentyl 1-*trans*-butene (U.S. Pat. No. 3,884,969). | 1,9-dioxo-15α-hydroxy-17,20-tetranor-16-cyclopentyl-1-hydroxymethyl-5-*cis*-13-*trans*-prostadiene |
| 605 | 319 | 287 | 1-iodo-3-triphenylmethoxy-3-cyclohexyl-1-*trans*-propene (U.S. Pat. No. 3,884,969). | 1,9-dioxo-15α-hydroxy-16,20-pentanor-15-cyclohexyl-1-hydroxymethyl-5-*cis*-13-*trans*-prostadiene |
| 606 | 319 | 287 | 1-iodo-3-triphenylmethoxy-5-cyclohexyl-1-*trans*-pentene (U.S. Pat. No. 3,884,969). | 1,9-dioxo-15α-hydroxy-18,20-trinor-17-cyclohexyl-1-hydroxymethyl-5-*cis*-13-*trans*-prostadiene |
| 607 | 319 | 287 | 1-iodo-3-triphenylmethoxy-6-cyclopentyl-1-*trans*-hexene (U.S. Pat. No. 3,884,969). | 1,9-dioxo-15α-hydroxy-19,20-dinor-18-cyclopentyl-1-hydroxymethyl-5-*cis*-13-*trans*-prostadiene |
| 608 | 319 | 287 | 1-iodo-3-methyl-3-trimethylsilyl-oxy-*trans*-1-octene (Example 190) | 1,9-dioxo-15α-hydroxy-15-methyl-1-hydroxymethyl-5-*cis*-13-*trans*-prostadiene |
| 609 | 319 | 287 | 1-iodo-3-methyl-3-trimethylsilyloxy *trans*-1-decene (Example 190a) | 1,9-dioxo-15α-hydroxy-15-methyl-20-ethyl-1-hydroxymethyl-5-*cis*-13-*trans*-prostadiene |
| 610 | 320 | 287 | 159 | 1,9-dioxo-15α-hydroxy-17,18,19,20-tetranor-16-*p*-fluorophenoxy-1-hydroxymethyl-5-*cis*-13-*trans*-prostadiene |
| 611 | 320 | 287 | 186 | 1,9-dioxo-15α-hydroxy-17,18,19,20-tetranor-16-phenoxy-1-hydroxymethyl-5-*cis*-13-*trans*-prostadiene |
| 612 | 320 | 287 | 181 | 1,9-dioxo-15α-hydroxy-17,18,19,20-tetranor-16-*p*-bromophenoxy-1-hydroxymethyl-5-*cis*-13-*trans*-prostadiene |
| 614 | 320 | 287 | 183 | 1,9-dioxo-15α-hydroxy-17,18,19,20-tetranor-16-*p*-methoxyphenoxy-1-hydroxymethyl-5-*cis*-13-*trans*-prostadiene |
| 615 | 320 | 287 | 184 | 1,9-dioxo-15α-hydroxy-17,18,19,20-tetranor-16-*p*-chlorophenoxy-1-hydroxymethyl-5-*cis*-13-*trans*-prostadiene |

TABLE XXXVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 616 | 320 | 287 | 180e | 1,9-dioxo-11α,15α-dihydroxy-17,18,19,20-tetranor-*m*-trifluorophenoxy-1-hydroxymethyl-5-*cis*-13-*trans*-prostadiene |
| 617 | 320 | 287 | 185 | 1,9-dioxo-15α-hydroxy-17,18,19,20-tetranor-16-(3,4-dichlorophenoxy)-1-hydroxymethyl-5-*cis*-13-*trans*-prostadiene |
| 618 | 320 | 287 | 186b | 1,9-dioxo-15α-hydroxy-18,19,20-trinor-17-phenyl-1-hydroxymethyl-5-*cis*-13-*trans*-prostadiene |
| 619 | 320 | 287 | 186e | 1,9-dioxo-15α-hydroxy-18,19,20-trinor-17-(*m*-trifluoromethylphenyl)-1-hydroxymethyl-5-*cis*-13-*trans*-prostadiene |
| 620 | 320 | 287 | 186d | 1,9-dioxo-15α-hydroxy-18,19,20-trinor-17-(*p*-methoxyphenyl)-1-hydroxymethyl-5-*cis*-13-*trans*-prostadiene |
| 621 | 319 | 288 | 1-iodo-3-triphenylmethoxy-1-*trans*-octene (U.S. Pat. No. 3,873,607). | 1,9-dioxo-15α-dihydroxy-1-hydroxymethyl-2-nor-5-*cis*-13-*trans*-prostadiene |
| 622 | 319 | 288 | 190 | 1,9-dioxo-15α-hydroxy-15-methyl-1-hydroxymethyl-2-nor-5-*cis*-13-*trans*-prostadiene |
| 623 | 319 | 288 | 1-iodo-3-triphenylmethoxy-5,5-dimethyl-octene (U.S. Pat. No. 3,873,607). | 1,9-dioxo-15α-hydroxy-16,16-dimethyl-1-hydroxymethyl-2-nor-5-*cis*-13-*trans*-prostadiene |
| 624 | 319 | 288 | 13 | 1,9-dioxo-15α-hydroxy-16,16-trimethylene-1-hydroxymethyl-2-nor-5-*cis*-13-*trans*-prostadiene |
| 625 | 319 | 288 | 76 | *dl-erythro*-1,9-dioxo-15α,16-dihydroxy-1-hydroxymethyl-2-nor-5-*cis*-13-*trans*-prostadiene |
| 626 | 319 | 288 | 69 | *dl-threo*-1,9-dioxo-15α,16-dihydroxy-1-hydroxymethyl-2-nor-5-*cis*-13-*trans*-prostadiene |
| 627 | 319 | 288 | 74 | *dl-erythro*-1,9-dioxo-15α-hydroxy-16-methoxy-1-hydroxymethyl-2-nor-5-*cis*-13-*trans*-prostadiene |
| 628 | 319 | 288 | 107 | 1,9-dioxo-16-hydroxy-1-hydroxymethyl-2-nor-5-*cis*-13-*trans*-prostadiene |
| 629 | 319 | 288 | 135 | 1,9-dioxo-16-hydroxy-16-methyl-1-hydroxymethyl-2-nor-5-*cis*-13-*trans*-prostadiene |

TABLE XXXVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 630 | 319 | 288 | 134 | 1,9-dioxo-16-hydroxy-16-methyl-1-hydroxymethyl-2-nor-5-*cis*-13-*trans*-17-*trans*-prostatriene |
| 631 | 320 | 288 | 186 | 1,9-dioxo-15$\alpha$-hydroxy-2,17,18,19,20-pentanor-16-phenoxy-1-hydroxymethyl-5-*cis*-13-*trans*-prostadiene |
| 632 | 320 | 288 | 180e | 1,9-dioxo-15$\alpha$-hydroxy-2,17,18,19,20-pentanor-16-*m*-trifluoromethylphenoxy-1-hydroxymethyl-5-*cis*-13-*trans*-prostadiene |
| 633 | 319 | 288 | 194 | 1,9-dioxo-16-hydroxy-16-vinyl-1-hydroxymethyl-2-nor-5-*cis*-13-*trans*-prostadiene |
| 634 | 320 | 288 | 198 | 1,9-dioxo-16-hydroxy-16-cyclopropyl-1-hydroxymethyl-2-nor-5-*cis*-13-*trans*-prostadiene |
| 635 | 320 | 288 | 186b | 1,9-dioxo-15$\alpha$-hydroxy-2,18,19,20-tetranor-17-phenyl-1-hydroxymethyl-5-*cis*-13-*trans*-prostadiene |
| 636 | 319 | 288 | 186e | 1,9-dioxo-15$\alpha$-hydroxy-2,18,19,20-tetranor-17-(*m*-trifluorophenyl)-1-hydroxymethyl-5-*cis*-13-*trans*-prostadiene |
| 637A | 319 | 288 | 186d | 1,9-dioxo-15$\alpha$-hydroxy-2,18,19,20-tetranor-17-(*p*-methoxyphenyl)-1-hydroxymethyl-5-*cis*-13-*trans*-prostadiene |
| 637B | 320 | 288 | 210c | 1,9-dioxo-16-hydroxy-16-ethyl-1-hydroxymethyl-2-nor-5-*cis*-13-*trans*-prostadiene |
| 637C* | 320 | 287 | 210l | 1,9-dioxo-16-hydroxy-16-ethynyl-1-hydroxymethyl-5-*cis*,13-*trans*-prostadiene |
| 637D* | 320 | 287 | 210P | 1,9-dioxo-16-hydroxy-16-ethynyl-1-hydroxymethyl-20-nor-5-*cis*-13-*trans*-prostadiene |
| 637E* | 320 | 287 | 210Q | 1,9-dioxo-16-hydroxy-16-ethynyl-1-hydroxymethyl-20-ethyl-5-*cis*,13-*trans*-prostadiene |
| 637F | 319 | 287 | 154l | 1,9-dioxo-16-hydroxy-16-(1-propynyl)-1-hydroxymethyl-5-*cis*,13-trans-prostadiene |
| 637G | 319 | 287 | 154J | 1,9-dioxo-16-hydroxy-16-(1-propynyl)-1-hydroxy-20-ethyl-5-cis,13-*trans*-prostadiene |

*For the cleavage of the TMS—C=C—bond see Example 772(B).

Examples 638—660

By the methods described hereinabove in Examples 319 and 320, the 1,9-dioxo-1-hydroxymethyl prostene derivatives shown in Table XXXVII are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XXXVII. It should be understood that the other diastereoisomer is also formed which in its *nat* and *ent* forms has an opposite (mirror image) configuration at the asymmetric carbon atoms on the β-chain (the chain containing $C_{13}$...$C_{14}$ etc.) to that of the respective *nat* and *ent* forms of the listed diastereoisomer; both of these diastereoisomers are claimed in this invention as well as their component enantiomers. [There is no Example 641]

TABLE XXXVII

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 638 | 319 | 287 | 246 | *nat*-(and *ent*)-1,9-dioxo-15-hydroxy-15,16-tetramethylene-17,18,19,20-tetranor-1-hydroxymethyl-5-*cis*-13-*trans*-prostadiene |
| 639 | 320 | 287 | 245 | *nat*-(and *ent*)-1,9-dioxo-16-hydroxy-16,17-tetramethylene-18,19,20-trinor-1-hydroxymethyl-5-*cis*-13-*trans*-prostadiene |
| 640 | 319 | 287 | 243 | *nat*-15S,16R(and *ent*-15R,16S)-1,9-dioxo-15-hydroxy-15,16-trimethylene-1-hydroxymethyl-5-*cis*-13-*trans*-prostadiene |
| 642 | 319 | 287 | 244 | *nat*-15S,16S-(and *ent*-15R,16R)-1,9-dioxo-15-hydroxy-15,16-trimethylene-1-hydroxymethyl-13-*trans*-5-*cis*-prostadiene |
| 643 | 319 | 287 | 247 | *nat*-(and *ent*)-1,9-dioxo-15-hydroxy-15,16-trimethylene-17,18,19,20-tetranor-1-hydroxymethyl-13-*trans*-5-*cis*-prostadiene |
| 644 | 320 | 287 | 248 | *nat*-(and *ent*)-1,9-dioxo-15-hydroxy-15,16-pentamethylene-17,18,19,20-tetranor-1-hydroxymethyl-13-*trans*-5-*cis*-prostadiene |
| 645 | 319 | 287 | 249 | *nat*-15S,17R-(and *ent*-15R,17S)-1,9-dioxo-15-hydroxy-15,17-dimethylene-1-hydroxymethyl-13-*trans*-5-*cis*-prostadiene |
| 646 | 319 | 287 | 250 | *nat*-15S,17S-(and *ent*-15R,17R)-1,9-dioxo-15-hydroxy-15,17-dimethylene-1-hydroxymethyl-13-*trans*-5-*cis*-prostadiene |
| 647 | 319 | 287 | 252 | *nat*-15S,16R-(and *ent*-15R,16S)-1,9-dioxo-15-hydroxy-15,16-tetramethylene-1-hydroxymethyl-13-*trans*-5-*cis*-prostadiene |
| 648 | 319 | 287 | 253 | *nat*-15S,16S-(and *ent*-15R,16R)-1,9-dioxo-15-hydroxy-15,16-tetramethylene-1-hydroxymethyl-13-*trans*-5-*cis*-prostadiene |
| 649 | 320 | 287 | 254 | *nat*-15R,16S-(and *ent*-15S,16R)-1,9-dioxo-15-hydroxy-15,16-trimethylene-16-(3-trifluoromethylphenoxy)-17,18,19,20-tetranor-1-hydroxymethyl-13-*trans*-5-*cis*-prostadiene |

TABLE XXXVII (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 650 | 320 | 287 | 255 | *nat*-15R,16R-(and *ent*-15S,16S)-1,9-dioxo-15-hydroxy-15,16-trimethylene-16-(3-trifluoromethylphenoxy)-17,18,19,20-tetranor-1-hydroxymethyl-13-*trans*-5-*cis*-prostadiene |
| 651 | 319 | 287 | 256 | *nat*-15R,16S-(and *ent*-15S,16R)-1,9-dioxo-15-hydroxy-15,16-trimethylene-16-(4-fluorophenoxy)-17,18,19,20-tetranor-1-hydroxymethyl-13-*trans*-5-*cis*-prostadiene |
| 652 | 319 | 287 | 257 | *nat*-15R,16R-(and *ent*-15S,16S)-1,9-dioxo-15-hydroxy-15,16-trimethylene-16-(4-fluorophenoxy)-17,18,19,20-tetranor-1-hydroxymethyl-13-*trans*-5-*cis*-prostadiene |
| 653 | 320 | 287 | 258 | *nat*-15R,16S-(and *ent*-15S,16R)-1,9-dioxo-15-hydroxy-15,16-trimethylene-16-(3-chlorophenoxy)-17,18,19,20-tetranor-1-hydroxymethyl-13-*trans*-5-*cis*-prostadiene |
| 654 | 320 | 287 | 259 | *nat*-15R,16R-(and *ent*-15S,16S)-1,9-dioxo-15-hydroxy-15,16-trimethylene-16-(3-chlorophenoxy)-17,18,19,20-tetranor-1-hydroxymethyl-13-*trans*-5-*cis*-prostadiene |
| 655 | 319 | 287 | 260 | *nat*-15S,17R-(and *ent*-15R,17S)-1,9-dioxo-15-hydroxy-15,17-trimethylene-19,20-dinor-1-hydroxymethyl-13-*trans*-5-*cis*-prostadiene |
| 656 | 319 | 287 | 261 | *nat*-15S,17S-(and *ent*-15R,17R)-1,9-dioxo-15-hydroxy-15,17-trimethylene-19,20-dinor-1-hydroxymethyl-13-*trans*-5-*cis*-prostadiene |
| 657 | 320 | 287 | 262 | *nat*-16R,17S-(and *ent*-16S,17R)-1,9-dioxo-16-hydroxy-16,17-trimethylene-20-methyl-1-hydroxymethyl-13-*trans*-5-*cis*-prostadiene |
| 658 | 320 | 287 | 263 | *nat*-16R,17R-(and *ent*-16S,17S)-1,9-dioxo-16-hydroxy-16,17-trimethylene-20-methyl-1-hydroxymethyl-13-*trans*-5-*cis*-prostadiene |
| 659 | 320 | 287 | 264 | *nat*-16R,17S-(and *ent*-16S,17R)-1,9-dioxo-16-hydroxy-16,17-trimethylene-16-(3-trifluoromethylphenoxy)-18,19,20-trinor-1-hydroxymethyl-13-*trans*-5-*cis*-prostadiene |
| 660 | 320 | 287 | 265 | *nat*-16R,17R-(and *ent*-16S,17S)-1,9-dioxo-16-hydroxy-16,17-trimethylene-16-(3-trifluoromethylphenoxy)-18,19,20-trinor-1-hydroxymethyl-13-*trans*-5-*cis*-prostadiene |

Examples 661—746E

By the methods described hereinabove in Examples 319 and 320, the 1,9-dioxo-1-hydroxymethyl prostene derivatives shown in Table XXXVIII are prepared by the indicated method from the indicated vinyl iodide or vinyl tin compound and the indicated cyclopent-2-en-1-one.

In those cases where isomers are obtained at the $C_{15}$ or $C_{16}$ positions, only the $C_{15}$ or $C_{16}$-normal isomers are listed in Table XXXVIII: it should be understood that the corresponding $C_{15}$ or $C_{16}$-*epi* isomers are also formed and are part of this invention.

In those cases where the initial conjugate-addition product contains a triphenylmethoxy blocking group, deblocking is conducted in acetic acid-tetrahydrofuran-water 4:2:1 at 50°C for 5 hours.

There are no Examples 675, 676, 682, 683, 685—688, 693 and 722.

TABLE XXXVIII

| Example | Method of Example | Cyclopent-2-en-1-one | Vinyl Iodide or Vinyl Tin of Example | 1,9-diox-1-hydroxymethylprostene |
|---|---|---|---|---|
| 661,662 | 319 | 284 | 49 | 1,9-dioxo-15-hydroxy-1-hydroxymethyl-16,-16-trimethylene-20-nor-13-*trans*-prostene |
| 663 | 319 | 284 | 50 | 1,9-dioxo-15-hydroxy-1-hydroxymethyl-16,-16-trimethylene-20-methyl-13-*trans*-prostene |
| 664 | 319 | 284 | 51 | 1,9-dioxo-15-hydroxy-1-hydroxymethyl-16,-16-trimethylene-20-ethyl-13-*trans*-prostene |
| 665 | 320 | 284 | 205 | 1,9-dioxo-16-hydroxy-16-cyclopropyl-20-ethyl-1-hydroxymethyl-13-*trans*-prostene |
| 666 | 320 | 284 | 207 | 1,9-dioxo-16-hydroxy-16-vinyl-20-ethyl-1-hydroxymethyl-13-*trans*-prostene |
| 667 | 320 | 284 | 198 | 1,9-dioxo-16-hydroxy-16-cyclopropyl-1-hydroxymethyl-13-*trans*-prostene |
| 668 | 319 | 284 | 13 | 1,9-dioxo-15-hydroxy-1-hydroxymethyl-16,-16-trimethylene-13-*trans*-prostene |
| 669 | 320 | 284 | 210a | 1,9-dioxo-16-hydroxy-1-hydroxoymethyl-16-vinyl-13-*trans*-prostene |
| 670 | 319 | 284 | 74 | *dl-erythro*-1,9-dioxo-15-hydroxy-16-methoxy-1- hydroxymethyl-13-*trans*-prostene |
| 671 | 319 | 284 | 77 | *dl-erythro*-1,9-dioxo-16-hydroxy-16-ethoxy-1-hydroxymethyl-13-*trans*-prostene |
| 672 | 319 | 284 | 83 | *dl-erythro*-1,9-dioxo-15,16-dihydroxy-1-hydroxymethyl-20-nor-13-*trans*-prostene |
| 673 | 319 | 284 | 84 | *dl-erythro*-1,9-dioxo-15,16-dihydroxy-1-hydroxymethyl-20-methyl-13-*trans*-prostene |
| 674 | 319 | 284 | 85 | *dl-erythro*-1,9-dioxo-15,16-dihydroxy-20-ethyl-1-hydroxymethyl-13-*trans*-prostene |
| 677 | 319 | 284 | 69 | *dl-threo*-1,9-doxo-15,16-dihydroxy-1-hydroxymethyl-13-*trans*-prostene |
| 678 | 319 | 284 | 88 | *dl-threo*-1,9-dioxo-15,16-dihydroxy-20-nor-1-hydroxymethyl-13-*trans*-prostene |
| 679 | 319 | 284 | 76 | *dl-erythro*-1,9-dioxo-15,16-dihydroxy-1-hydroxymethyl-13-*trans*-prostene |

TABLE XXXVIII (continued)

| Example | Method of Example | Cyclopent-2-en-1-one | Vinyl Iodide or Vinyl Tin of Example | 1,9-diox-1-hydroxymethylprostene |
|---|---|---|---|---|
| 680 | 319 | 284 | 89 | *dl-threo*-1,9-dioxo-15,16-dihydroxy-20-methyl-1-hydroxymethyl-13-*trans*-prostene |
| 681 | 319 | 284 | 90 | *dl-threo*-1,9-dioxo-15,16-dihydroxy-20-ethyl-1-hydroxymethyl-13-*trans*-prostene |
| 684 | 319 | 284 | 107 | 1,9-dioxo-16-hydroxy-1-hydroxymethyl-13-*trans*-prostene |
| 688 | 319 | 284 | 110 | 1,9-dioxo-16-hydroxy-1-hydroxymethyl-20-nor-13-*trans*-prostene |
| 689 | 319 | 284 | 144 | 1,9-dioxo-16-hydroxy-17-methyl-1-hydroxymethyl-13-*trans*-prostene |
| 690 | 319 | 284 | 112 | 1,9-dioxo-16-hydroxy-20-methyl-1-hydroxymethyl-13-*trans*-prostene |
| 691 | 319 | 284 | 113 | 1,9-dioxo-16-hydroxy-20-ethyl-1-hydroxymethyl-13-*trans*-prostene |
| 692 | 319 | 284 | 139a | 1,9-dioxo-16-hydroxy-16-methyl-20-ethyl-1-hydroxymethyl-13-*trans*-prostene |
| 694 | 319 | 284 | 135 | 1,9-dioxo-16-hydroxy-16-methyl-1-hydroxymethyl-13-*trans*-prostene |
| 695 | 319 | 284 | 136 | 1,9-dioxo-16-hydroxy-17,17,20-trimethyl-1-hydroxymethyl-13-*trans*-prostene |
| 696 | 319 | 284 | 137 | 1,9-dioxo-16-hydroxy-16,20-dimethyl-1-hydroxymethyl-13-*trans*-prostene |
| 697 | 319 | 284 | 138 | 1,9-dioxo-16-hydroxy-17,17-dimethyl-1-hydroxymethyl-13-*trans*-prostene |
| 698 | 319 | 284 | 139 | 1,9-dioxo-16-hydroxy-16-methyl-1-hydroxymethyl-13-*trans*-17-*trans*-prostadene |
| 699 | 319 | 284 | 149 | 1,9-dioxo-16-hydroxy-20-methyl-1-hydroxymethyl-13-*trans*-17-*trans*-prostadiene |
| 700 | 319 | 284 | 150 | 1,9-dioxo-16-hydroxy-17-methyl-1-hydroxymethyl-13-*trans*-prostene |
| 701 | 319 | 284 | 151 | 1,9-dioxo-16-hydroxy-17,20-dimethyl-1-hydroxymethyl-13-*trans*-prostene |
| 702 | 319 | 284 | 152 | 1,9-dioxo-16-hydroxy-1-hydroxymethyl-13-*trans*-17-*trans*-prostadiene |
| 703 | 319 | 284 | 153 | 1,9-dioxo-16(R)-hydroxy-1-hydroxymethyl-13-*trans*-prostene |
| 704 | 319 | 284 | 154 | 1,9-dioxo-16(S)-hydroxy-1-hydroxymethyl-13-*trans*-prostene |
| 705 | 319 | 284 | 154a | 1,9-dioxo-16-hydroxy-20-ethyl-1-hydroxymethyl-13-*trans*-17-*trans*-prostadiene |

TABLE XXXVIII (continued)

| Example | Method of Example | Cyclopent-2-en-1-one | Vinyl Iodide or Vinyl Tin of Example | 1,9-diox-1-hydroxymethylprostene |
|---|---|---|---|---|
| 706 | 315 | 284 | 1-iodo-3 triphenylmethoxy-1-*trans*-oxtene (U.S. Pat. No. 3,873,607). | 1,9-dioxo-15α-hydroxy-1-hydroxymethyl-13-*trans*-prostene |
| 707 | 319 | 284 | 1-iodo-3-triphenylmethoxy-1-*trans*-nonene (U.S. Pat. No. 3,873,607). | 1,9-dioxo-15α-hydroxy-20-methyl-1-hydroxymethyl-13-*trans*-prostene |
| 708 | 319 | 284 | 1-iodo-3-triphenylmethoxy-1-*trans*-decene (U.S. Pat. No. 3,873,607). | 1,9-dioxo-15α-hydroxy-20-ethyl-1-hydroxymethyl-13-*trans*-prostene |
| 709 | 319 | 284 | 1-iodo-3-tri phenylmethoxy-4, 4-dimethyl-1-*trans*- octene (U.S. Pat. No. 3,873,607). | 1,9-dioxo-15α-hydroxy-16,16-dimethyl-1-hydroxymethyl-13-*trans*-prostene |
| 710 | 319 | 284 | 1-iodo-3-tri-phenylmethoxy-5, 5-dimethyl-1-*trans*-octene (U.S. Pat. No. 3,873,607). | 1,9-dioxo-15α-hydroxy-17,17-dimethyl-1--hydroxymethyl-13-*trans*-prostene |
| 711 | 319 | 284 | 1-iodo-3-tri-phenylmethoxy-4-methyl-1-*trans*-octene (U.S. Pat. No.3,876,690). | 1,9-dioxo-15α-hydroxy-16-methyl-1-hydroxy methyl-13-*trans*-prostene |
| 712 | 319 | 284 | 1-iodo-3-tri-phenylmethoxy-4-ethyl-1-*trans*-octene (U.S. Pat. No. 3,876,690). | 1,9-dioxo-15α-hydroxy-16-ethyl-1-hydrooxymethyl-13-*trans*-prostene |
| 713 | 319 | 284 | 1-iodo-3-tri-phenylmethoxy-4-cyclopentyl-1-*trans*-butene (U.S. Pat. No. 3,884,969). | 1,9-dioxo-15α-hydroxy-17,20-tetranor-16-cyclopentyl-1-hydroxymethyl-13-*trans*-prostene |
| 714 | 319 | 284 | 1-iodo-3-tri-phenylmethoxy-3-cyclohexyl-1-*trans*-propene (U.S. Pat. No. 3,884,969). | 1,9-dioxo-15α-hydroxy-16,20-pentanor-15-cyclohexyl-1-hydroxymethyl-13-*trans*-prostene |
| 715 | 319 | 284 | 1-iodo-3-tri phenylmethoxy-5-cyclohexyl-1-*trans*-pentene U.S. Pat. No. 3,884,969). | 1,9-dioxo-15α-hydroxy-18,20-trinor-17-cyclohexyl-1-hydroxymethyl-13-*trans*-prostene |

TABLE XXXVIII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 716 | 319 | 284 | 1-iodo-3-triphenylmethoxy-6-cyclopentyl-1-*trans*-hexene (U.S. Pat. No. 3,884,969). | 1,9-dioxo-15α-hydroxy-19,20-dinor-18-cyclopentyl-1-hydroxymethyl-13-*trans*-prostene |
| 717 | 319 | 284 | 1-iodo-3-methyl-3-trimethylsilyloxy-*trans*-1-octene (Example 190). | 1,9-dioxo-15α-hydroxy-15-methyl-1-hydroxymethyl-13-*trans*-prostene |
| 718 | 319 | 284 | 1-iodo-3-methyl-3-trimethylsilyloxy-*trans*-1-decene (Example 190a). | 1,9-dioxo-15α-hydroxy-15-methyl-20-ethyl-1-hydroxymethyl-13-*trans*-prostene |
| 719 | 320 | 284 | 159 | 1,9-dioxo-15α-hydroxy-17,18,19,20-tetranor-16-*p*-fluorophenoxy-1-hydroxymethyl-13-*trans*-prostene |
| 720 | 320 | 284 | 186 | 1,9-dioxo-15α-hydroxy-17,18,19,20-tetranor-16-phenoxy-1-hydromethyl-13-*trans*-prostene |
| 721 | 320 | 284 | 181 | 1,9-dioxo-15α-hydroxy-17,18,19,20-tetranor-16-*p*-bromophenoxy-1-hydroxymethyl-13-*trans*-prostene |
| 723 | 320 | 284 | 183 | 1,9-dioxo-15α-hydroxy-17,18,19,20-tetranor-16-*p*-methoxyphenoxy-1-hydroxymethyl-13-*trans*-prostene |
| 724 | 320 | 284 | 184 | 1,9-dioxo-15α-hydroxy-17,18,19,20-tetranor-16-*m*-chlorophenoxy-1-hydroxymethyl-13-*trans*-prostene |
| 725 | 320 | 284 | 180e | 1,9-dioxo-15α-hydroxy-17,18,19,20-tetranor-16-*m*-trifluorophenoxy-1-hydroxymethyl-13-*trans*-prostene |
| 726 | 320 | 284 | 185 | 1,9-dioxo-15α-hydroxy-17,18,19,20-tetranor-16-(3,4-dichlorophenoxy)-1-hydroxy methyl-13-*trans*-prostene |
| 727 | 320 | 284 | 186b | 1,9-dioxo-15α-hydroxy-18,19,20-trinor-17-phenyl-1-hydroxymethyl-13-*trans*-prostene |
| 728 | 320 | 284 | 186e | 1,9-dioxo-15α-hydroxy-18,19,20-trinor-17-(m-trifluoromethylphenyl)-1-hydroxymethyl-13-*trans*-prostene |
| 729 | 320 | 284 | 186d | 1,9-dioxo-15α-hydroxy-18,19,20-trinor-17-(*p*-methoxyphenyl)-1-hydroxymethyl-13-*trans*-prostene |

TABLE XXXVIII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 730 | 319 | 285 | 1-iodo-3-triphenylmethoxy-1-*trans*-octene (U.S. Pat. No. 3,873,607). | 1,9-dioxo-15α-hydroxy-1-hydroxymethyl-2-nor-13-*trans*-prostene |
| 731 | 319 | 285 | 190 | 1,9-dioxo-15α-hydroxy-15-methyl-1-hydroxymethyl-2-nor-13-*trans*-prostene |
| 732 | 319 | 285 | 1-iodo-3-triphenylmethoxy-5,5-dimethyl-octene (U.S.Pat. No. 3,873,607) | 1,9-dioxo-15α-hydroxy-17,17-dimethyl-1-hydroxymethyl-2-nor-13-*trans*-prostene |
| 733 | 319 | 285 | 13 | 1,9-dioxo-15α-hydroxy-16,16-trimethylene-1-hydroxymethyl-2-nor-13-*trans*-prostene |
| 734 | 319 | 285 | 76 | *dl-erythro*-1,9-dioxo-15α,16-dihydroxy-1-hydroxymethyl-2-nor-13-*trans-prostene* |
| 735 | 319 | 285 | 69 | *dl-threo*-1,9-dioxo-15α,16-dihydroxy-1-hydroxymethyl-2-nor-13-*trans*-prostene |
| 736 | 319 | 285 | 74 | *dl-erythro*-1,9-dioxo-15α-hydroxy-16-methoxy-1-hydroxymethyl-2-nor-13-*trans*-prostene |
| 737 | 319 | 285 | 107 | 1,9-dioxo-16-hydroxy-1-hydroxymethyl-2-nor-13-*trans*-prostene |
| 738 | 319 | 285 | 130 | 1,9-dioxo-16-hydroxy-16-methyl-1-hydroxymethyl-2-nor-13-*trans*-prostene |
| 739 | 319 | 285 | 134 | 1,9-dioxo-16-hydroxy-16-methyl-1-hydroxymethyl-2-nor-13-*trans*-17-*trans*-prostadiene |
| 740 | 320 | 285 | 186 | 1,9-dioxo-15α-hydroxy-2,17,18,19,20-pentanor-16-phenoxy-1-hydroxymethyl-13-*trans*-prostene |
| 741 | 320 | 285 | 180e | 1,9-dioxo-15α-hydroxy-2,17,18,19,20-pentanor-16-*m*-trifluoromethylphenoxy-1-hydroxymethyl-13-*trans*-prostene |
| 742 | 319 | 285 | 194 | 1,9-dioxo-16-hydroxy-16-vinyl-1-hydroxymethyl-2-nor-13-*trans*-prostene |
| 743 | 320 | 285 | 198 | 1,9-dioxo-16-hydroxy-16-cyclopropyl-1-hydroxymethyl-2-nor-13-*trans*-prostene |
| 744 | 320 | 285 | 186b | 1,9-dioxo-15α-hydroxy-2,18,19,20-tetranor-17-phenyl-1-hydroxymethyl-13-*trans*-prostene |
| 745 | 319 | 285 | 186e | 1,9-dioxo-15α-hydroxy-2,18,19,20-tetranor-17-(*m*-trifluorophenyl)-1-hydroxymethyl-13 *trans*-prostene |
| 746 | 319 | 285 | 186d | 1,9-dioxo-15α-hydroxy-2,18,19,20-tetranor-17-(*p*-methoxyphenyl)-1-hydroxymethyl-13-*trans*-prostene |

TABLE XXXVIII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 746A* | 320 | 284 | 210I | 1,9-dioxo-16-hydroxy-16-ethynyl-1-hydroxymethyl-13-*trans*-prostene |
| 746B* | 320 | 284 | 210P | 1,9-dioxo-16-hydroxy-16-ethynyl-1-hydroxymethyl-20-nor-13-*trans*-prostene |
| 746C* | 320 | 284 | 210Q | 1,9-dioxo-16-hydroxy-16-ethynyl-1-hydroxymethyl-20-ethyl-13-*trans*-prostene |
| 746D | 319 | 284 | 154I | 1,9-dioxo-16-hydroxy-16-(1-propynyl)1-hydroxy-methyl-13-*trans*-prostene |
| 746E | 319 | 284 | 154J | 1,9-dioxo-16-hydroxy-16-(1-propynyl)-1-hydroxymethyl-20-ethyl-13-*trans*-prostene |

*For cleavage of the TMS—CEC see Example 772B.

Examples 747—769

By the methods described hereinabove in Examples 319 and 320, the 1,9-dioxo-1-hydroxymethyl prostene derivatives shown in Table XXXIX are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XXXIX. It should be understood that the other diastereoisomers is also formed which in its *nat* and *ent* forms has an opposite (mirror image) configuration at the asymmetric carbon atoms on the β-chain (the chain containing $C_{13}\ldots C_{14}$ etc.) to that of the respective *nat* and *ent* forms of the listed diastereoisomer; both of these diastereoisomers are claimed in the invention as well as their component enantiomers.

[There is no Example 750]

TABLE XXXIX

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 747 | 319 | 284 | 246 | *nat*-(and *ent*)-1,9-dioxo-15-hydroxy-15,-16-tetramethylene-17,18,19,20-tetranor-1-hydroxymethyl-13-*trans*-prostene |
| 748 | 320 | 284 | 245 | *nat*-(and *ent*)-1,9-dioxo-16-hydroxy-16,17-tetramethylene-18,19,20-trinor-1-hydroxy-methyl-13-*trans*-prostene |
| 749 | 319 | 284 | 243 | *nat*-15S,16R(and *ent*-15R,16S)-1,9-dioxo-15-hydroxy-15,16-trimethylene-1-hydroxy-methyl-13-*trans*-prostene |
| 751 | 319 | 284 | 244 | *nat*-15S,16S(and *ent*-15R,16R)-1,9-dioxo-15-hydroxy-15,16-trimethylene-1-hydroxy-methyl-13-*trans*-prostene |
| 752 | 319 | 284 | 247 | *nat*-(and *ent*)-1,9-dioxo-15-hydroxy-15,16-trimethylene-17,18,19,20-tetranor-1-hydroxymethyl-13-*trans*-prostene |
| 753 | 320 | 284 | 248 | *nat*-(and *ent*)-1,9-dioxo-15-hydroxy-15,16-pentamethylene-17,18,19,20-tetranor-1-hydroxymethyl-13-*trans*-prostene |
| 754 | 319 | 284 | 249 | *nat*-15S,17R(and *ent*-15R,17S)-1,9-dioxo-15-hydroxy-15,17-dimethylene-1-hydroxy-methyl-13-*trans*-prostene |
| 755 | 319 | 284 | 250 | *nat*-15S,17S(and *ent*-15R,17R)-1,9-dioxo-15-hydroxy-15,17-dimethylene-1-hydroxy-methyl-13-*trans*-prostene |
| 756 | 319 | 284 | 252 | *nat*-15S,16R-(and *ent*-15R,16S)-1,9-dioxo-15-hydroxy-15,16-tetramethylene-1-hydroxy-methyl-13-*trans*-prostene |
| 757 | 319 | 284 | 253 | *nat*-15S,16S-(and *ent*-15R,16R)-1,9-dioxo-15-hydroxy-15,16-tetramethylene-1-hydroxy-methyl-13-*trans*-prostene |
| 758 | 320 | 284 | 254 | *nat*-15R,16S-(and *ent*-15S,16R)-1,9-dioxo-15-hydroxy-15,16-trimethylene-16-(3-tri-fluoromethylphenoxy)-17,18,19,20-tetra-nor-1-hydroxymethyl-13-*trans*-prostene |
| 759 | 320 | 284 | 255 | *nat*-15R,16R-(and *ent*-15S,16S)-1,9-dioxo-15-hydroxy-15;16-trimethylene-16-(3-tri-fluoromethylphenoxy)-17,18,19,20-tetra-nor-1-hydroxymethyl-13-*trans*-prostene |
| 760 | 319 | 284 | 256 | *nat*-15R,16S-(and *ent*-15S,16R)-1,9-dioxo-15-hydroxy-15,16-trimethylene-16-(4-fluorophenoxy)17,18,19,20-tetranor-1-hydroxymethyl-13-*trans*-prostene |
| 761 | 319 | 284 | 257 | *nat*-15R,16R-(and *ent*-15S,16S)-1,9-dioxo-15-hydroxy-15,16-trimethylene-16-(4-fluorophenoxy)-17,18,19,20-tetranor-1-hydroxymethyl-13-*trans*-prostene |

TABLE XXXIX (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 762 | 320 | 284 | 258 | *nat*-15R,16S-(and *ent*-15S,16R)-1,9-dioxo-15-hydroxy-15,16-trimethylene-16-(3-chlorophenoxy)-17,18,19,20-tetranor-1-hydroxymethyl-13-*trans*-prostene |
| 763 | 320 | 284 | 259 | *nat*-15R,16R-(and *ent*-15S,16S)-1,9-dioxo-15-hydroxy-15,16-trimethylene-16-(3-chlorophenoxy)-17,18,19,20-tetranor-1-hydroxymethyl-13-*trans*-prostene |
| 764 | 319 | 284 | 260 | *nat*-15S,17R-(and *ent*-15R,17S)-1,9-dioxo-15-hydroxy-15,17-trimethyl-19,20-di-nor-1-hydroxymethyl-13-*trans*-prostene |
| 765 | 319 | 284 | 261 | *nat*-15S,17S-(and *ent*-15R,17R)-1,9-dioxo-15-hydroxy-15,17-trimethylene-19,20-di nor-1-hydroxymethyl-13-*trans*-prostene |
| 766 | 320 | 284 | 262 | *nat*-16R,17S-(and *ent*-16S,17R)-1,9-dioxo-16-hydroxy-16,17-trimethylene-20-methyl-1-hydroxymethyl-13-*trans*-prostene |
| 767 | 320 | 284 | 263 | *nat*-16R,17R-(and *ent*-16S,17S)-1,9-dioxo-16-hydroxy-16,17-trimethylene-20-methyl-1-hydroxymethyl-13-*trans*-prostene |
| 768 | 320 | 284 | 264 | *nat*-16R,17S-(and *ent*-16S,17R)-1,9-dioxo-16-hydroxy-16,17-trimethylene-16-(3-tri-fluoromethylphenoxy)-18,19,20-trinor-1-hydroxymethyl-13-*trans*-prostene |
| 769 | 320 | 284 | 265 | *nat*-16R,17R-(and *ent*-16S,17S)-1,9-dioxo-16-hydroxy-16,17-trimethylene-16-(3-tri fluoromethylphenoxy)-18,19,20-trinor-1-hydroxymethyl-13-*trans*-prostene |

Examples 769A—769H

Conjugate addition of the vinylstannane of Example 210(a) by the procedure of Example 320 or 772A is productive of the optically active or racemic prostanoid of Table XXXIXA.

The listed compounds are separable by silica gel dry-column chromatography or H.P.L.C. techniques into the individual 16$\alpha$- or 16$\beta$-isomers.

TABLE XXXIXA

| Example | Cyclopentenone of Example | Product Prostanoid |
|---|---|---|
| 769A | 296N | *nat.*-1,9-dioxo-11$\alpha$,16(R)-dihydroxy-1-hydroxymethyl-16-vinyl-13-*trans*-prostene |
| 769B | 296N | *nat.*-1,9-dioxo-11$\alpha$,16(S)-dihydroxy-1-hydroxymethyl-16-vinyl-13-*trans*-prostene |
| 769C | 296M | *nat.*-1,9-dioxo-11$\alpha$,16(R)-dihydroxy-1-hydroxymethyl-16-vinyl-5-*cis*,-13-*trans*-prostadiene |
| 769D | 296M | *nat.*-1,9-dioxo-11$\alpha$,16(R)-dihydroxy-1-hydroxymethyl-16-vinyl-5-*cis*,-13-*trans*-prostadiene |
| 769E | 296—A | *dl*-1,9-dioxo-11$\alpha$,16(R)-dihydroxy-1-hydroxymethyl-16-vinyl 13-*trans*-prostene |
| 769F | 296—A | *dl*-1,9-dioxo-11$\alpha$,16(R)-dihydroxy-1-hydroxymethyl-16-vinyl 13-*trans*-prostene |
| 769G | 296A | *dl*-1,9-dioxo-11$\alpha$,16(R)-dihydroxy-1-hydroxymethyl-16-vinyl-5-*cis*, 13-*trans*-prostadiene |
| 769H | 296J | *dl*-1,9-dioxo-11$\alpha$,16(S)-dihydroxy-1-hydroxymethyl-16-vinyl-5-*cis*-13-*trans*-prostadiene |

Example 770

Preparation of *l*-9-oxo-15(S)-acetoxy prostanoyl chloride

To a solution of 0.5 g of *l*-9-oxo-15(S)-acetoxy prostanoic acid in 10 ml of benzene at 0°C with stirring is added 0.5 ml of oxalyl chloride. The mixture is stirred at room temperature for 2½ hours. The solvent and excess oxalyl chloride is removed at reduced pressure. The residue is dissolved in hexane and filtered. The solvent is removed giving the title compound.

Example 771

Preparation of *l*-1,9-dioxo-15(S)-acetoxy-1-hydroxymethyl-prostane

To a solution of 11.9 mmol of diazomethane in ether at 0°C with stirring is added dropwise a solution of 2.99 mmol of *l*-9-oxo-15(S)-acetoxy prostanoyl chloride (Example 770) in 10 ml of ether. After 10 minutes the solution is warmed to room temperature and the solvent and excess diazomethane is removed in a stream of nitrogen. The residue is stirred at 55°C in 15 ml of tetrahydrofuran containing 5.75 ml of 2M sulfuric acid for 30 minutes. The mixture is poured into water and extracted with ether. The ether solution is washed with saturated sodium bicarbonate, brine, and dried over magnesium sulfate. The ether is removed and the residue is chromatographed on a dry column of silica gel eluting with hexane-ethyl acetate 3:2 to give the title compound.

Example 772

Preparation of *l*-1,9-dioxo-15(S)-hydroxy-1-hydroxymethyl prostane

A solution of 0.41 g of *l*-1,9-dioxo-15(S)-acetoxy-1-hydroxymethyl prostane (Example 771) in 8b ml of methanol containing 1 ml of water and 0.19 ml of concentrated sulfuric acid is refluxed for 5 hours. The methanol is removed at reduced pressure and water and tetrahydrofuran is added. The mixture is then refluxed for 1 hour. The mixture is poured into dilute sodium bicarbonate and extracted with ether. The ether solution is washed with and dried over magnesium sulfate. The solvent is removed giving 0.34 g of the title compound.

Example 772A

Preparation of 1,9-dioxo-11$\alpha$, 16-dihydroxy-16-trimethylsilylethynyl-1-hydroxymethyl-13-*trans*-prostene

To a solution of 9.2 g of *E* 1-tri-*n*-butylstannyl-4-trimethylsilylethynyl-4-trimethylsiloxy-1-octene (Example 210 I) in 8.2 ml of distilled tetrahydrofuran (from $LiA1H_4$) at —78°C is added, dropwise, 5.2 ml of 2.0 *M* *n*-butyllithium (in hexane). After 15 minutes at —78°C, the bath temperature is raised to —40°C to —30°C for 1.5 hours. The solution is cooled to —78°C and a solution of 1.5 g of pentynyl copper in 4.2 ml of distilled hexamethyl phosphoramide and 3 ml of dry ether is added. The solution is stirred at —78°C for 1 hour.

To the above cuprate solution at −78°C is added a solution of 2.0 g 1-(methoxy-1-methylethoxy)-8-(5-oxo-1-cyclopenten-1-yl)-2-octanone (Example 296A) in 5 ml of dry ether. After stirring at −78°C for 10 minutes the solution is stirred at −40°C to −30°C for 1½ hours. The solution is briefly warmed to −25°C and then cooled to −78°C and 1.1 ml of gl. acetic acid is added dropwise. The solution is poured into ether (70 ml) and saturated aq. ammonium chloride ($NH_4Cl$) (70 ml) and stirred for 15 minutes at ambient temperature.

The organic phase is separated and the aqueous layer is extracted twice again with ether (50 ml). The organic phases are combined, washed with 5% cold aq.HCl (50 ml), three times with saturated NaCl (50 ml), dried over $MgSO_4$, and concentrated *in vacuo* to a viscous oil.

The oily residue is diluted with acetic acid tetrahydrofuran-water (4:2:1) (70 ml) and this solution is stirred vigorously at 40°C to 44°C for 1 hour. Toluene (50 ml) is added and the solution is concentrated *in vacuo.* An additional portion of toluene (50 ml) is added and concentrated. The toluene addition and concentration is repeated two or more times to remove traces of acetic acid.

The residue is dissolved in methanol (40 ml) and the lower phase (mostly tetrabutylstannane) is discarded. The methanol solution is extracted twice with heptane (40 ml). The combined heptane solution is extracted with methanol (10 ml) and the methanol fractions are combined and concentrated *in vacuo* to an oily residue.

This residue is dissolved in ethyl acetate-hexane-acetic acid (60:40:1) (10 ml) and applied to a dry-column of silica gel (Woelm: 550 g—69″ x 1½″). The dry column is developed with the above solution and a 200 ml fraction is collected.

The column is cut into 1″ segments and 540 mg of the title compound is isolated.

### Example 772B

Preparation of 1,9-dioxo-11α,16-dihydroxy-16-ethynyl-1-hydroxymethyl-13-*trans*-prostene

To a solution of 1,9-dioxo-11α,16-dihydroxy-16-trimethylsilyl-ethynyl-1-hydroxymethyl-13-*trans*-prostene (400 mg) in dimethyl formamide (11 ml) at ambient temperature is added 400 mg of potassium fluoride dihydrate. After 90 minutes the solution is diluted with ether (60 ml) and washed three times with 30 ml water and once with 30 ml of brine.

The aqueous extracts are acidified with dilute HCl and extracted with ethyl acetate (2 x 30 ml). The organic phases are combined, washed with water (30 ml), brine (30 ml), dried over $MgSO_4$ and concentrated *in vacuo* to provide 303 mg of a viscous oil.

The viscous oil is dissolved in a small amount of ethyl acetatehexane (3:2) and applied to a dry column (of silica-gel (Woelm: 225 g, 54″ x 1″)). The column is developed with ethyl acetate-hexane (3:2). The column is cut into 1″ segments and the portions containing the product are eluted off of the silica-gel with ethyl acetate to provide 160 mg of the title compound.

### Example 772C

Preparation of 1,9-dioxo-11α,16-dihydroxy-16-methyl-1-hydroxymethyl-13-*trans*-prostene

A. To a stirred solution of 75.5 g (150 mmol) total vinylstannane; estimated 120 mmol of *trans*-isomer by CMR) of *E*-1-tri-*n*-butylstannyl-4-methyl-4-trimethylsilyloxy-1-octane in 120 ml of THF at −78° initially was added 60 ml of 2.0 M *n*-butyllithium in hexane during 5 minutes so that the temperature was < −60°C. The light amber solution was warmed to −40°C during 10 minutes and maintained at that temperature for 2 hours. The solution was recooled to −78°C for use in paragraph *C.*

B. To a well-stirred 17.25 g (132 mmol) sample of copper pentyne was added 48 ml (about 43.1 g, or 264 mmol) of freshly distilled hexamethyl-phosphorous triamide. After 20 minutes 300 ml of ether was added. The resulting clear, light yellow solution was cooled to −78°C for 60 minutes.

C. To the vinyllithium solution prepared in paragraph *A* at −78°C, initially was added the precooled solution of copper complex prepared in paragraph *B via* double tip needle technique during 10 minutes so that the temperature was < −65°C. The resulting light amber solution was stirred at −78°C for 60 minutes.

D. To the stirred solution prepared in paragraph *C* at −78°C initially was added a solution of 23.07 of (60.0 mmol) of 1-(1-methoxy-1-methylethoxy)-8-(3-(1-methoxy-1-methylethoxy)-5-oxo-1-cyclopenten-1-yl)-2-octanone in 50 ml of ether during 10 minutes so that the temperature was < −65°. The syringe and septum bottle were washed with 10 ml ether. After 5 minutes the light amber solution was warmed to −40°C during 10 minutes. The solution was stirred at −40° for 1.5 hr and then allowed to warm to −20°C during 30 minutes and then recorderd to −78°C. The reaction was quenched by addition of a solution of 14.4 ml (about 240 mmol) of gl HOAc in 100 ml ether. The precipitate which formed was stirrable with a magnetic stirrer on this scale.

E. The mixture above was transferred with the aid of washing with 750 ml of ether into a stirred, ice-cold mixture of 48 ml of N/l HCl and 240 ml of satd $NH_4Cl$. The mixture was stirred vigorously at 0—5° for 5 minutes. The aqueous phase was separated and extracted with 350 ml of ether. The combined organic phases were washed successively with 2 x 240 ml of ice-cold N/l HCl, 240 ml of half-satd brine, 240 ml of 1:1 satd brine-satd $NaHCO_3$, 240 ml of half-satd brine, and 3 x 240 ml satd

brine. The solution was dried over $MgSO_4$, filtered through a small pad of Celite, and concentrated *in vacuo* at *ca* 30°C to give 108 g of mobile light yellow liquid.

F. The materials from paragraph *E* resulting from this run (60 mmol-scale, 108 g) and a similar run (57.7 mmol-scale, 103 g) were combined. This material (211 g) was treated with a solution prepared from 940 ml of gl.HOAc, 470 ml of THF, and 235 ml of water. The resulting solution was stirred at 40—43°C for 60 minutes. Some $(n\text{-Bu})_4$ Sn was not dissolved. The mixture was diluted with 600 ml of toluene. Then 600 ml of distillate was removed via rotary evaporator at about 30°. The flask which contained the solution was set up for standard vacuum distillate (dry-ice acetone cooled receiver). The solution was diluted with 600 ml of toluene. Then 600 ml of distillate was removed (both at about 30°C, 0.1 mm). The solution was diluted with 600 ml of toluene. Then the volume of the solution was reduced to about 1000 ml. The solution was diluted with 600 ml of toluene. Then the volume was reduced to about 500 ml. The solution was diluted with 300 ml of toluene (total toluene used was 2700 ml). This solution was evaporated to give 194 g of a mixture of colourless $(n\text{-Bu})_4Sn$ and a dark amber oil.

G. The material from paragraph *F* was placed, with the aid of repeated hexane wash, on the top of a pad of 385 g of "Mallinckrodt" (registered Trade Mark) Silic Ar CC—7 silica gel contained in a glass column; dimensions 5.8 x 30 cm. The column was washed with 2500 ml of hexane to remove stannane material. The column was then washed with 4000 ml of ethyl acetate, taking care to wash all hexane-insoluble material in flask and on column sides onto the top of the silica gel. The first 3250 cc of ethyl acetate eluate yielded 77.4 g of amber oil on evaporation. The last 750 ml of eluate yielded 0.1 g of amber oil (total = 77.5 g).

H. *Chromatography* To a 5.4 cm diameter column filled with 2:1 heptaneethyl acetate was added 970 g of Mallinckrodt SilicAr CC—7 silica gel. The column stood overnight; dimensions 5.4 × 98 cm. This column was used to purify most of the material from paragraph *G* (71.0 g).

The material (71.0 g) was dissolved in 250 ml of 2:1 heptaneethyl acetate and 50 ml of ethyl acetate (required to produce solution). The solution was developed on the above-described column. Elution was carried out under slight nitrogen pressure to produce a flow rate of about 3—4 1 /hour.

Fractions which emerged from the column were examined by TLC with the solvent system 20:1 EtOAc—MeOH. Plates were developed by spraying first with 2,4—DNP solution and then cupric acetate solution and charring.

The column was eluted with heptane-ethyl acetate block gredients according to the following schedule:

| Batch | Volume (1) | Solvent Ratio |
|---|---|---|
| 1 | 7 | 2:1 heptane-EtOAc |
| 2 | 7 | 5:3 " |
| 3 | 12 | 3:2 " |
| 4 | 2 | 4:3 " |
| 5 | 2 | 10:9 " |
| 6 | 4 | 1:1 " |
| 7 | 2 | 3:2 EtOAc-heptane |
| 8 | 2 | 2:1 " |
| 9 | 6 | 3:1 " |
| 10 | 12 | 3:1 " increased to pure EtOAc. |

Fractions collected were of appropriate size (about 1500—200 ml) and were pooled according to TLC as above. The product emerged from the column corresponding to solvent batches 6—10 above to provide 16.9 g of product.

Examples 772D—772AAA

Conjugate addition of the indicated vinylstannane or vinyuliodide with the indicated cyclopentanone of the following table by the procedure of Examples 772A, 772B, 772C or the

procedure of Examples 319—320 is productive of the optically active or recemic prostanoid of Table XXXIXA.

The listed compounds are separable by silica gel dry-column chromatography or H.P.L.C. techniques into the individual 16$\alpha$ and 16$\beta$-isomers.

TABLE XXXIXA

| Example | Cyclopentenone | Vinylstannane or Vinyl Iodide | Product Prostanoid |
|---|---|---|---|
| 772D | 1-(1-methoxy-1-methylethoxy)-8[3R-3-(1-methoxy-1-methylethoxy)-5-oxo-1-cyclopenten-1-yl]-6-*cis*-octen-2-one | 4(S)-4-methyl-4-triethylsilyloxy-*trans*-1-octenyl iodide (U.S. Pat. 4,060,091-Ex. 11—C, 11—E) | *nat.* 1,9-dioxo-11$\alpha$,16(S)-dihydroxy-1-hydroxymethyl-16-methyl-5-*cis*, 13-*trans*-prostadiene |
| 772E | 1-(1-methoxy-1-methylethoxy)-8[3R-3-(1-methoxy-1-methylethoxy)-5-oxo-1-cyclopenten-1-yl]-6-*cis*-octen-2-one | 4(R)-4-methyl-4-triethylsilyloxy-*trans*-1-octenyl-iodide (U.S. Pat. 4,060,091-Ex-11—D) | *nat.* 1,9-dioxo-11$\alpha$,16(R)-dihydroxy-1-hydroxymethyl-16-methyl-5-*cis* 13-*trans*-prostadiene |
| 772F | 1-(1-methoxy-1-methylethoxy)-8[3R-3-(1-methoxy-1-methylethoxy)-5-oxo-1-cyclopenten-1-yl]-2-octanone | 4(S)-4-methyl-4-triethylsilyloxy-*trans*-1-octenyl-iodide (U.S. Pat. 4,060,091-Ex. 11C, 11E) | *nat.* 1,9-dioxo-11$\alpha$,16(S)-dihydroxy-1-hydroxymethyl-16-methyl-13-*trans*-prostene |
| 772G | 1-(1-methoxy-1-methylethoxy)-8[3R-3-(1-methoxy-1-methylethoxy)-5-oxo-1-cyclopenten-1-yl]-2-octanone | 4(R)-methyl-4-triethylsilyloxy-*trans*-1-octenyl-iodide (U.S. Pat. 4,060,091 Ex.11—D) | *nat.* 1,9-dioxo-11$\alpha$,16(R)-dihydroxy-1-hydroxymethyl-16-methyl-13-*trans*-prostene |
| 772H | 1-(1-methoxy-1-methylethoxy)-8[3-(1-methoxy-1-methylethoxy)-5-oxo-1-cyclopenten-1-yl]-6-*cis*-octen-2-one | 4(S)-4-methyl-4-triethylsilyloxy-*trans*-1-octenyl-iodide (U.S. Pat. 4,060,091-Ex. 11—C, 11—E) | 1,9-dioxo-11$\alpha$,16(S)-dihydroxy-1-hydroxy-methyl-16-methyl-5-*cis*, 13-*trans*-prostadiene |
| 772I | 1-(1-methoxy-1-methylethoxy)-8[3-(1-methoxy-1-methylethoxy)-5-oxo-1-cyclopenten-1-yl]-6-*cis*-octen-2-one | 4(R)-4-methyl-4-triethylsilyloxy-*trans*-1-octenyl-iodide (U.S. Pat. 4,060,091-Ex. 11—D) | 1,9-dioxo-11$\alpha$,16(R)-dihydroxy-1-hydroxy-methyl-16-methyl-5-*cis*, 13-*trans*-prostadiene |
| 772J | 1-(1-methoxy-1-methylethoxy)-8[3-(1-methoxy-1-methylethoxy)-5-oxo-1-cyclopenten-1-yl]-2-octanone | 4(S)-4-methyl-4-triethylsilyloxy-*trans*-1-octenyl-iodide (U.S. Pat. 4,060,091-Ex 11—C, 11—E) | 1,9-dioxo-11$\alpha$,16(S)-dihydroxy-1-hydroxy-methyl-16-methyl-13-*trans*-prostene |
| 772K | 1-(1-methoxy-1-methylethoxy)-8[3-(1-methoxy-1-methylethoxy)-5-oxo-1-cyclopenten-1-yl]-2-octanone | 4(R)-4-methyl-4-triethylsilyloxy-*trans*-1-octenyl-iodide (U.S. Pat. 4,060,091 Ex. 11—D) | 1,9-dioxo-11$\alpha$,16(R)-dihydroxy-1-hydroxymethyl-16-methyl-13-*trans*-prostene |

TABLE XXXIXA (continued)

| Example | Cyclopentenone | Vinylstannane or Vinyl Iodide | Product Prostanoid |
|---|---|---|---|
| 772L | 1-(1-methoxy-1-methylethoxy)-8[3R-3-(1-methoxy-1-methylethoxy)-5-oxo-1-cyclopenten-1-yl]-6-*cis*-octen-2-one | 4(R/S)-4-methyl-4-triethylsilyloxy-*trans*-1-octenyl-iodide (U.S. Pat. 4,060,091-Ex. 11—C, 11—E) | *nat.* 1,9-dioxo-11$\alpha$,16(R/S)-dihydroxy-1-hydroxymethyl-16-methyl-5-*cis*-13-*trans* prostadiene |
| 772M | 1-(1-methoxy-1-methylethoxy)-8[3R-3-(1-methoxy-1-methylethoxy)-5-oxo-1-cyclopenten-1-yl]-2-octanone | 4(R/S)-4-methyl-4-triethylsilyloxy-*trans*-1-octenyl-iodide (U.S. Pat. 4,060,091-Ex. 11—C, 11—E) | *nat.* 1,9-dioxo-11$\alpha$,16(R/S)-dihydroxy-1-hydroxymethyl-16-methyl-13-*trans*-prostadiene |
| 772N* | 1-(1-methoxy-1-methylethoxy)-8-[3R-3-(1-methoxy-1-methylethoxy)-5-oxo-cyclopenten-1-yl]-6-*cis*-octen-2-one | 4-trimethylsilylethynyl-4-tri methylsilyloxy-tri-*n*-butyl-stannyl-1-heptene (Example 210P) | *nat.* 1,9-dioxo-11$\alpha$,16(R)-dihydroxy-ethynyl-1-hydroxymethyl-20-nor-5-*cis*,13-*trans*-prostadiene |
| 772O* | 1-(1-methoxy-1-methylethoxy)-8-[3R-3-(1-methoxy-1-methylethoxy)-5-oxo-cyclopenten-1-yl]-6-*cis*-octen-2-one | 4-trimethylsilylethynyl-4-tri methylsilyloxy-tri-*n*-butyl-stannyl-1-decene (Example 210Q) | *nat.* 1,9-dioxo-11$\alpha$,16(R)-dihydroxy-16-ethynyl-1-hydroxymethyl-20-ethyl-5-*cis*,13-*trans*-prostadiene |
| 772P* | 1-(1-methoxy-1-methylethoxy)-8[3R-3-(1-methoxy-1-methylethoxy)-5-oxo-1-cyclopenten-1-yl]-6-*cis*-octen-2-one | 4-tri-*n*-butylstannyl-4-trimethyl-silylethynyl-4-trimethylsilyloxy-1-octene (Example 210I) | *nat.* 1,9-dioxo-11$\alpha$,16(R)-dihydroxy-16-ethynyl-1-hydroxymethyl-5-*cis*,13-*trans*-prostadiene |
| 772Q* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-tri methylsilyloxy-tri-*n*-butyl stannyl-1-heptene (Example 210P) | *nat.* 1,9-dioxo-11$\alpha$16(R)-dihydroxy-16-ethynyl-1-hydroxymethyl-20-nor-13-*trans*-prostene |
| 772R* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-tri methylsilyloxy-1-tri-*n*-butyl-stannyl-1-decene (Example 210Q) | *nat.* 1,9-dioxo-11$\alpha$16(R)-dihydroxy-16-ethynyl-1-hydroxymethyl-20-ethyl-13-*trans*-prostene |

TABLE XXXIXA (continued)

| Example | Cyclopentenone | Vinylstannane or Vinyl Iodide | Product Prostanoid |
|---|---|---|---|
| 772S* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-tri methylsilyloxy-1-tri-*n*-butyl-stannyl-1-octene (Example 210I) | *nat.*1,9-dioxo-11$\alpha$16(R)-dihydroxy-16-ethynyl-1-hydroxymethyl-13-*trans*-prostene |
| 772T | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-octene (Example 154I) | *nat.*1,9-dioxo-11$\alpha$,16(R)-dihydroxy-16-(1-propynyl)-1-hydroxymethyl-5-*cis*, 13-*trans*-prostadiene |
| 772U | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilyloxy-4-(1-propynyl)-1-iodo-*trans*--1-decene (Example 154J) | *nat.*1,9-dioxo-11$\alpha$,16(R)-dihydroxy-16-(1-propynyl)-1-hydroxymethyl-20-ethyl-5-*cis*,13-*trans*-prostadiene |
| 772V | 1-(1-methoxy-1-methylethoxy)-8[3R-3-(1-methoxy-1-methylethoxy)-5-oxo-1-cyclopenten-1-yl]-2-octanone | 4-trimethylsilyloxy-4-(1-propy-nyl)-1-iodo-*trans*-1-octene (Example 154I) | *nat.*1,9-dioxo-11$\alpha$,16(R)-dihydroxy-16-(1-propynyl)-1-hydroxymethyl-13-*trans*-prostene |
| 772W | 1-(1-methoxy-1-methylethoxy)-8-[3R-3-(1-methoxy-1-methylethoxy)-5-oxo-1-cyclopenten-1-yl]-2-octanone | 4-trimethylsilyloxy-4-(1-propnyl)-1-iodo-*trans*-decene (Example 154J) | *nat.*1,9-dioxo-11$\alpha$,16(R)-dihydroxy-16-(1-propynyl)-1-hydroxymethyl-20-ethyl-13-*trans*-prostene |
| 772X* | 1-(1-methoxy-1-methylethoxy)-8-[-3-(methoxy-1-methylethoxy)-5-oxo-1-cyclopenten-1-yl]-6-*cis*-octen-2-one | 4-trimethylsilylethynyl-4-trimethyl-silyloxy-1-tri-*n*-butylstannyl-1-heptene (Example 210P) | 1,9-dioxo-11$\alpha$,16(R)-dihydroxy-16-ethynyl-1-hydroxymethyl-20-*nor*-5-*cis*,13-*trans*-prostadiene |
| 772Y* | 1-(1-methoxy-1-methylethoxy)-8-[-3-(methoxy-1-methylethoxy)-5-oxo-1-cyclopenten-1-yl]-6-*cis*-octen-2-one | 4-trimethylsilylethynyl-4-trimethyl-silyloxy-1-tri-*n*-butylstannyl-1-decene (Example 210Q) | 1,9-dioxo-11$\alpha$,16(R)-dihydroxy-16-ethynyl-1-hydroxymethyl-20-ethyl-5-*cis*,13-*trans*-prostadiene |
| 772Z* | 1-(1-methoxy-1-methylethoxy)-8-[-3-(1-methoxy-1-methylethoxy)-5-oxo-1-cyclopenten-1-yl]-6-*cis*-octen-2-one | tri-*n*-butylstannyl-4-trimethyl-silylethynyl-4-trimethylsilyloxy-1-octene (Example 210I) | 1,9-dioxo-11$\alpha$,16(R)-dihydroxy-16-ethynyl-1-hydroxymethyl-5-*cis*,13-*trans*-prostadiene |

TABLE XXXIXA (continued)

| Example | Cyclopentenone | Vinylstannane or Vinyl Iodide | Product Prostanoid |
|---|---|---|---|
| 772AA* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-heptene (Example 210P) | 1,9-dioxo-11$\alpha$,16(R)-dihydroxy-16-ethynyl-1-hydroxymethyl-20-nor-13-*trans*-prostene |
| 772BB* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-decene (Example 210Q) | 1,9-dioxo-11$\alpha$,16(R)-dihydroxy-16-ethynyl-1-hydroxymethyl-20-ethyl-13-*trans*-prostene |
| 772CC* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-octene (Example 210I) | 1,9-dioxo-11$\alpha$,16(R)-dihydroxy-16-ethynyl-1-hydroxymethyl-13-trans-prostene |
| 772DD | 1-(1-methoxy-1-methylethoxy)-8-[-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-octene (Example 154I) | 1,9-dioxo-11$\alpha$,16(R)-dihydroxy-16-(1-propynyl)-1-hydroxymethyl-5-*cis*, 13-*trans*-prostadiene |
| 772EE | 1-(1-methoxy-1-methylethoxy)-8-[-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-decene (Example 154J) | 1,9-dioxo-11$\alpha$,16(R)-dihydroxy-16-(1-propynyl)-1-hydroxymethyl-20-ethyl-5-*cis*-13-*trans*-prostadiene |
| 772FF | 1-(1-methoxy-1-methylethoxy)-8-[-3-(1-methoxy-1-methylethoxy)-5-oxo-1-cyclopenten-1-yl]-2-octanone | 4-trimethylsilyloxy-4-(1-propynyl)-1-iodo-*trans*-1-octene (Example 154I) | 1,9-dioxo-11$\alpha$,16(R)-dihydroxy-16-(1-propynyl)-1-hydroxymethyl-13-*trans*-prostene |
| 772GG | 1-(1-methoxy-1-methylethoxy)-8-[-3-(1-methoxy-1-methylethoxy)-5-oxo-1-cyclopenten-1-yl]-2-octanone | 4-trimethylsilyloxy-4-(1-propynyl)-1-iodo-*trans*-1-decene (Example 154J) | 1,9-dioxo-11$\alpha$,16(R)-dihydroxy-16-(1-propynyl)-1-hydroxymethyl-20-ethyl-13-*trans*prostene |
| 772HH* | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilylethynyl-4-trimethylsilyloxy-1-tri-*n*-butylstannyl-1-heptene (Example 210P) | *nat.*1,9-dioxo-11$\alpha$,16(S)-dihydroxy-16-ethynyl-1-hydroxymethyl-20-nor-5-*cis*,13-*trans*-prostadiene |

TABLE XXXIXA (continued)

| Example | Cyclopentenone | Vinylstannane or Vinyl Iodide | Product Prostanoid |
|---|---|---|---|
| 772II* | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilylethynyl-4-trimethylsilyloxy-1-tri-*n*-butylstannyl-1-decene (Example 210O) | *nat.*1,9-dioxo-11α,16(S)-dihydroxy-16-ethynyl-1-hydroxymethyl-20-ethyl-5-*cis*,13-*trans*-prostadiene |
| 772JJ* | 1-(1-methoxy-1-methylethoxy)-8[3R-3-(1-methoxy-1-methylethoxy)-5-oxo-1-cyclopenten-1-yl]-6-*cis*-octen-2-one | tri-*n*-butylstannyl-4-trimethylsilylethynyl-4-trimethylsilyloxy-1-octene (Example 210I) | *nat.*1,9-dioxo-11α,16(S)-dihydroxy-16-ethynyl-1-hydroxymethyl-5-*cis*-13-*trans*-prostadiene |
| 772KK* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-heptene (Example 210P) | *nat.*1,9-dioxo-11α,16(S)-dihydroxy-16-ethynyl-1-hydroxymethyl-20-nor-13-*trans*-prostene |
| 772LL* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-decene (Example 210Q) | *nat.*1,9-dioxo-11α,16(S)-dihydroxy-16-ethynyl-1-hydroxymethyl-20-ethyl-13-*trans*-prostene |
| 772MM* | 1-(1-methoxy-1-methylethoxy)-8-[3R-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-octene (Example 210I) | *nat.*1,9-dioxo-11α,16(S)-dihydroxy-16-ethynyl-1-hydroxymethyl-13-*trans*-prostene. |
| 772NN | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-octene (Example 154I) | *nat.*1,9-dioxo-11α,16(S)-dihydroxy-16-(1-propynyl)-1-hydroxymethyl-5-*cis*,13-*trans*-prostadiene |
| 772OO | 1-(1-methoxy-1-methylethoxy)-8-[3R-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-decene (Example 154J) | *nat.*1,9-dioxo-11α,16(S)-dihydroxy-16-(1-propynyl-1-hydroxymethyl-20-ethyl-5-*cis*,13-*trans*-prostadiene |
| 772PP | 1-(1-methoxy-1-methylethoxy)-8-[3R-3-(1-methoxy-1-methylethoxy)-5-oxo-1-cyclopenten-1-yl]-2-octanone | 4-trimethylsilyloxy-4-(1-propynyl)-1-iodo-*trans*-1-octene (Example 154I) | *nat.*1,9-dioxo-11α,16(S)-dihydroxy-16-(1-propynyl-1-hydroxymethyl-13-*trans*-prostadiene |

TABLE XXXIXA (continued)

| Example | Cyclopentenone | Vinylstannane or Vinyl Iodide | Product Prostanoid |
|---|---|---|---|
| 772QQ | 1-(1-methoxy-1-methylethoxy)-8-[3R-3-(1-methoxy-1-methylethoxy)-5-oxo-1-cyclopenten-1-yl]-2-octanone | 4-trimethylsilyloxy-4-(1-propynyl)-1-iodo-*trans*-1-decene (Example 154J) | *nat.*1,9-dioxo-11$\alpha$,16(S)-dihydroxy-16-(1-propynyl-1-hydroxymethyl-20-ethyl-13-*trans*-prostene |
| 772RR* | 1-(1-methoxy-1-methylethoxy)-8-[-3-(methoxy-1-methylethoxy)-5-oxo-1-cyclopenten-1-yl]-6-*cis*-octen-2-one | 4-trimethylsilylethynyl-4-trimethylsilyloxy-1-tri-*n*-butylstannyl-1-heptene (Example 210P) | 1,9-dioxo-11$\alpha$,16(S)-dihydroxy-16-ethynyl-1-hydroxymethyl-20-nor-5-*cis*,13-*trans*-prostadiene |
| 772SS* | 1-(1-methoxy-1-methylethoxy)-8-[-3-(methoxy-1-methylethoxy)-5-oxo-1-cyclopenten-1-yl]-6-*cis*-octen-2-one | 4-trimethylsilylethynyl-4-trimethylsilyloxy-1-tri-*n*-butylstannyl-1-decene (Example 210Q) | 1,9-dioxo-11$\alpha$,16(S)-dihydroxy-16-ethynyl-1-hydroxymethyl-20-ethyl-5-*cis*,13-*trans*-prostadiene |
| 772TT* | 1-(1-methoxy-1-methylethoxy)-8-[-3-(1-methoxy-1-methylethoxy)-5-oxo-1-cyclopenten-1-yl]-6-*cis*-octen-2-one | tri-*n*-butylstannyl-4-trimethylsilylethynyl-4-trimethylsilyloxy 1-octene (Example 210I) | 1,9-dioxo-11$\alpha$,16(S)-dihydroxy-16-ethynyl-1-hydroxymethyl-5-*cis*,13-*trans*-prostadiene |
| 772UU* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-heptene (Example 210P) | 1,9-dioxo-11$\alpha$,16(S)-dihydroxy-16-ethynyl-1-hydroxymethyl-20-nor-13-*trans*-prostene |
| 772VV* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-decene (Example 210O) | 1,9-dioxo-11$\alpha$,16(S)-dihydroxy-16-ethynyl-1-hydroxymethyl-20-ethyl-13-*trans*-prostene |
| 772WW* | 1-(1-methoxy-1-methylethoxy)-8-[-3-(1-methoxy-1-methylethoxy)-5-oxo-1-cyclopenten-1-yl]-2-octanone | 4-trimethylsilylethynyl-4-trimethylsilyloxy-1-tri-*n*-butylstannyl-1-octene (Example 210I) | 1,9-dioxo-11$\alpha$,16(S)-dihydroxy-16-ethynyl-1-hydroxymethyl-13-*trans*-prostene |
| 772XX | 1-(1-methoxy-1-methylethoxy)-8-[-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-octene (Example 154I) | 1,9-dioxo-11$\alpha$,16(S)-dihydroxy-16-(1-propynyl)-1-hydroxymethyl-5-*cis*,13-*trans*-prostadiene |

TABLE XXXIXA (continued)

| Example | Cyclopentenone | Vinylstannane or Vinyl Iodide | Product Prostanoid |
|---|---|---|---|
| 772YY | 1-(1-methoxy-1-methylethoxy)-8-[-3-(methoxy-1-methylethoxy)-5-oxo-1-cyclopenten-1-yl]-6-*cis*-octen-2-one | 4-trimethylsilyloxy-4-(1-propynyl)-1-iodo-*trans*-1-decene (Example 154J) | 1,9-dioxo-11$\alpha$,16(S)-dihydroxy-16-(1-propynyl)-1-hydroxymethyl-20-ethyl-5-*cis*,13-*trans*-prostadiene |
| 772ZZ | 1-(1-methoxy-1-methylethoxy)-8-[-3-(1-methoxy-1-methylethoxy)-5-oxo-1-cyclopenten-1-yl]-2-octanone | 4-trimethylsilyloxy-4-(1-propynyl)-1-iodo-*trans*-1-octene (Example 154I) | 1,9-dioxo-11$\alpha$,16(S)-dihydroxy-16-(1-propynyl)-1-hydroxymethyl-13-*trans*-prostadiene |
| 772AAA | 1-(1-methoxy-1-methylethoxy)-8-[-3-(1-methoxy-1-methylethoxy)-5-oxo-1-cyclopenten-1-yl]-2-octanone | 4-trimethylsilyloxy-4-(1-propynyl)-1-iodo-*trans*-1-decene (Example 154J) | 1,9-dioxo-11$\alpha$,16(S)-dihydroxy-16-(1-propynyl)-1-hydroxymethyl-20-ethyl-13-*trans*-prostene |

* For cleavage of the TMS—C=C— bond see Example 772 (B).

Example 773

Preparation of 1,9-dioxo-11$\alpha$,16-dihydroxy-1-hydroxymethyl-20-methyl-5-*cis*-13-*trans*-prostadiene, 1-ethylene ketal

To a solution of 3.58 g (7.0 mmol) of 1-iodo-4-triphenylmethoxy-1-*trans*-nonene (Example 112) in 8.0 ml of ether is added 8.9 ml of 1.6M *t*-butyllithium solution (14.1 mmol) with stirring under argon at —78°C. The solution is allowed to warm to —30°C over a 2 hour period. The solution is recooled to —78°C and a solution of 7.0 mmol of copper pentyne and 2.9 ml of hexamethylphosphorous triamide in 20 ml of ether is added. After 1 hour, 3.28 g (7.0 mmol) of 2 - (8 - dimethyl - *t* - butylsilyloxy - 7 - oxo - oct - 2 - *cis* - enyl) - 4 - trimethylsilyloxycyclopent - 2 - en - 1 - one, 7 - ethylene ketal (Example 280) in 5 ml of ether is added. The mixture is stirred at —30°C for 2 hours and at 0°C for 30 minutes. The mixture is poured into 150 ml of saturated ammonium chloride and extracted with ether. The ether solution is washed with dilute hydrochloric acid and dried over magnesium sulfate to give 6.0 g of an oil. A 3.0 g portion of this oil is stirred in 40 ml of tetrahydrofuran-0.6N Hydrochloric acid 5:1 for 5 hours at room temperature.

The mixture is poured into water and extracted with ether. The ether is removed and the residue is heated in 70 ml of acetic acid-tetrahydrofuran-water 4:2:1 at 60°C for 4 hours. The solvent is removed at reduced pressure. Toluene is added and removed. The residue is chromatographed on a silica gel column eluting with ethyl acetate giving 0.1 g of the title compound.

Example 774

Preparation of 1,9 - dioxo - 11$\alpha$,16 - dihydroxy - 16 - methyl - 1 - hydroxymethyl - 5 - *cis* - 13 - *trans* - prostadiene, 1 - ethylene ketal

To a solution of 3.4 g (6.75 mmol) of (E) - 4 - trimethylsilyloxymethyl - 1 - tri - *n* - butylstannyl - 1 - octene (Example 210b) in 3.5 ml of tetrahydrofuran with stirring at —78°C under argon is added 2.8 ml (6.75 mmol) of 2.4M *n*-butyllithium in hexane. The solution is maintained at —20° to —15°C for 2½ hours. A solution of 0.89 g (6.75 mmol) of copper pentyne and 2.2 g of hexamethylphosphorous triamide in 25 ml of ether is added at —78°C. After 1 hour, a solution of 2.3 g (5.0 mmol of 2 - (8 - dimethyl - *t* - butylsilyloxy - 7 - oxo - oct - 2 - *cis* - enyl) - 4 - trimethylsilyloxycyclopent - 2 - en - 1 - one, 7 - ethylene ketal (Example 280) in 20 ml of ether is added. The solution is stirred at —45°C for 30 minutes and at —45° to —20°C over 30 minutes. To the solution is added 3 ml of acetic acid followed by saturated ammonium chloride. The mixture is poured into water and extracted with ether. The ether solution is washed with dilute hydrochloric acid, saturated sodium bicarbonate and dried over magnesium sulfate. The solvent is removed and the residue is stirred in 75 ml of tetrahydrofuran containing 15 ml of 0.6N hydrochloric acid and 1 ml of acetic acid at room temperature for 5 hours. The solution is saturated with sodium chloride and extracted with ethyl acetate. The ethyl acetate solution is washed with saturated sodium bicarbonate and dried over magnesium sulfate. The solvent is removed and the residue is chromatographed on a dry column of silica gel eluting with ethyl acetate containing 0.5% acetic acid (1000 ml) to give 0.65 g of the title compound.

Examples 775—868E

By the methods described hereinabove in Examples 773 and 774 the 1,9-dioxo-1-hydroxymethylprostene derivatives shown in Table XL are prepared by the indicated method from the indicated cyclopent-2-en-1-one.

In those cases where isomers are obtained at the $C_{15}$ or $C_{16}$ positions, only the $C_{15}$ or $C_{16}$-normal isomers are listed in Table XL; it should be understood that the corresponding $C_{15}$ or $C_{16}$-*epi* isomers are also formed and are part of this invention.

[There are no Examples 788, 789, 795, 798, 799, 805 and 834.]

TABLE XL

| Example | Method of Example | Cyclopent-2-en-1-one | Vinyl Iodide or Vinyl Tin of Example | 1,9-dioxo-1-hydroxymethylprostene |
|---|---|---|---|---|
| 775 | 773 | 280 | 49 | 1,9-dioxo-11α,15-dihydroxy-1-hydroxymethyl-16,16-trimethylene-20-nor-5-*cis*-13-*trans*-prostadiene,1-ethylene ketal |
| 776 | 773 | 280 | 50 | 1,9-dioxo-11α-15-dihydroxy-1-hydroxymethyl-16,16-trimethylene-20-methyl-5-*cis*-13-*trans*-prostadiene,1-ethylene ketal |
| 777 | 773 | 280 | 51 | 1,9-dioxo-11α,15-dihydroxy-1-hydroxymethyl-16,16-trimethylene-20-ethyl-5-*cis*-13-*trans*-prostadiene,1-ethylene ketal |
| 778 | 773 | 280 | 13 | 1,9-dioxo-11α15-dihydroxy-1-hydroxy-Methyl-16,16-trimethylene-13-*trans*-5-*cis*-prostadiene,1-ethylene ketal |
| 779 | 774 | 280 | 210a | 1,9-dioxo-11α16-dihydroxy-1-hydroxymethyl-16-vinyl-5-*cis*-13-*trans*-prostadiene, 1-ethylene ketal |
| 780 | 774 | 280 | 205 | 1,9-dioxo-11α16-dihydroxy-16-cyclopropyl-20 ethyl-1-hydroxymethyl-5-*cis*-13-*trans*-prostadiene 1-ethylene ketal |
| 781 | 774 | 280 | 207 | 1,9-dioxo-11α16-dihydroxy-16-vinyl-20-ethyl-1-hydroxymethyl-5-*cis*-13-*trans*-prostadiene 1-ethylene ketal |
| 782 | 774 | 280 | 198 | 1,9-dioxo-11α16-dihydroxy-16-cyclopropyl-1-hydroxymethyl-5-*cis*-13-*trans*-prostadiene, 1-ethylene ketal |
| 783a | 773 | 280 | 774 | *dl-erythro*-1,9-dioxo-11α,15-dihydroxy-16-methoxy-1-hydroxymethyl-5-*cis*-13-*trans*-prostadiene,1-ethylene ketal |
| 783b | 774 | 280 | 210c | 1,9-dioxo-11α16-dihydroxy-16-ethyl-1-hydroxymethyl-5-*cis*-13-*trans*-prostadiene,1-ethylene ketal |
| 784 | 773 | 280 | 77 | *dl-erythro*-1,9-dioxo-11α,15-trihydroxy-16-ethoxy-1-hydroxymethyl-5-*cis*-13-*trans*-prostadiene,1-ethylene ketal |
| 785 | 773 | 280 | 83 | *dl-erythro*-1,9-dioxo-11α,15,16-trihydroxy-1-hydroxymethyl-20-nor-5-*cis*-13-*trans*-prostadiene,1-ethylene ketal |
| 786 | 773 | 280 | 84 | *dl-erythro*-1,9-dioxo-11α,15,16-trihydroxy-20-methyl-1-hydroxymethyl-5-*cis*-13-*trans*-prostadiene,1-ethylene ketal |
| 787 | 773 | 280 | 85 | *dl-erythro*-1,9-dioxo-11α,15,16-trihydroxy-20-ethyl-1-hydroxymethyl-5-*cis*-13-*trans-prostadiene,1-ethylene ketal* |
| 790 | 773 | 280 | 69 | *dl-threo*-1,9-dioxo-11α,15,16-trihydroxy-1-hydroxymethyl-5-*cis*-13-*trans*-hydroxymethyl,1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 791 | 773 | 280 | 88 | *dl-threo*-1,9-dioxo-11α,15,16-trihydroxy-20-nor-1-hydroxymethyl-5-*cis*-13-*trans*-prostadiene,1-ethylene ketal |
| 792 | 773 | 280 | 76 | *dl-erythro*-1,9-dioxo-11α,15,16-trihydroxy-1-hydroxymethyl-5-*cis*-13-*trans*-prostadiene,1-ethylene ketal |
| 793 | 773 | 280 | 89 | *dl-threo*-1,9-dioxo-11α,15,16-trihydroxy-20-methyl-1-hydroxymethyl-5-*cis*-13-*trans*-prostadiene,1-ethylene ketal |
| 794 | 773 | 280 | 90 | *dl-threo*-1,9-dioxo-11α,15,16-trihydroxy-20-ethyl-1-hydroxymethyl-5-*cis*-13-*trans*-prostadiene,1-ethylene ketal |
| 796 | 773 | 280 | 92 | *dl-threo*-1,9-dioxo-11α,15,16-trihydroxy-1-hydroxymethyl-5-*cis*-13-*trans*-17-*trans*-prostatriene,1-ethylene ketal |
| 797 | 773 | 280 | 107 | 1,9-dioxo-11α,16-dihydroxy-1-hydroxymethyl-5-*cis*-13-*trans*-prostadiene,1-ethylene ketal |
| 800 | 773 | 280 | 110 | 1,9-dioxo-11α,16-dihydroxy-1-hydroxymethyl-20-nor-5-*cis*-13-*trans*-prostadiene, 1-ethylene ketal |
| 801 | 773 | 280 | 144 | 1,9-dioxo-11α,16-dihydroxy-17-methyl-1-hydroxymethyl-5-*cis*-13-*trans*-prostadiene,1-ethylene ketal |
| 802 | 773 | 280 | 112 | 1,9-dioxo-11α,16-dihydroxy-20-methyl-1-hydroxymethyl-5-*cis*-13-*trans*-prostadiene,1-ethylene ketal |
| 803 | 773 | 280 | 113 | 1,9-dioxo-11α,16-dihydroxy-20-ethyl-1-hydroxymethyl-5-*cis*-13-*trans*-prostadiene,1-ethylene ketal |
| 804 | 773 | 280 | 139a | 1,9-dioxo-11α,16-dihydroxy-16-methyl-20-ethyl-1-hydroxymethyl-5-*cis*-13-*trans*-prostadiene,1-ethylene ketal |
| 806 | 773 | 280 | 135 | 1,9-dioxo-11α,16-dihydroxy-16-methyl-1-hydroxymethyl-5-*cis*-13-*trans*-prostadiene,1-ethylene ketal |
| 807 | 773 | 280 | 136 | 1,9-dioxo-11α,16-dihydroxy-17,17,20-tri methyl-1-hydroxymethyl-5-*cis*-13-*trans*-prostadiene,1-ethylene ketal |
| 808 | 773 | 280 | 137 | 1,9-dioxo-11α,16-dihydroxy-16,20-dimethyl-1-hydroxymethyl-13-*trans*-5-*cis*-prostadiene,1-ethylene ketal |
| 809 | 773 | 280 | 138 | 1,9-dioxo-11α,16-dihydroxy-17,17-dimethyl--1-hydroxymethyl-5-*cis*-13-*trans*-prostadiene,1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 810 | 773 | 280 | 139 | 1,9-dioxo-11α,16-dihydroxy-16-methyl-1-hydroxymethyl-13-*trans*-17-*trans*-5-*cis*-prostatriene,1-ethylene ketal |
| 811 | 773 | 280 | 149 | 1,9-dioxo-11α,16-dihydroxy-20-methyl-1-hydroxymethyl-13-*trans*-17-*trans*-5-*cis*-prostatriene,1-ethylene ketal |
| 812 | 773 | 280 | 150 | 1,9-dioxo-11α,16-dihydroxy-17-methyl-1-hydroxymethyl-5-*cis*-13-*trans*-prostadiene,1-ethylene ketal |
| 813 | 773 | 280 | 151 | 1,9-dioxo-11α,16-dihydroxy-17,20-dimethyl-1-hydroxymethyl-5-*cis*-13-*trans*-prostadiene,1-ethylene ketal |
| 814 | 773 | 280 | 152 | 1,9-dioxo-11α,16-dihydroxy-1-hydroxymethyl-13-*trans*-17-*trans*-5-*cis*-prostatriene 1-ethylene ketal |
| 815 | 773 | 280 | 153 | 1,9-dioxo-11α,16(R)-dihydroxy-1-hydroxy-methyl-5-*cis*-13-*trans*-prostadiene, 1-ethylene ketal |
| 816 | 773 | 280 | 154 | 1,9-dioxo-11α,16(S)-dihydroxy-1-hydroxy-methyl-5-*cis*-13-*trans*-prostadiene, 1-ethylene ketal |
| 817 | 773 | 280 | 154a | 1,9-dioxo-11α,16-dihydroxy-20-ethyl-1-hydroxymethyl-5-*cis*-13-*trans*-17-trans-prostatriene,1-ethylene ketal |
| 818 | 773 | 280 | 1-iodo-3-triphen-ylmethoxy-1-*trans*-octene (U.S. Pat. No.3,873,607). | 1,9-dioxo-11α,15α-dihydroxy-1-hydroxy methyl-5-*cis*-13-*trans*-prostadiene, 1-ethylene ketal |
| 819 | 773 | 280 | 1-iodo-3-triphen-ylmethoxy-1-*trans*-nonene (U.S. Pat. No. 3,873,607. | 1,9-dioxo-11α,15α-dihydroxy-20-methyl-1-hydroxymethyl-5-*cis*-13-*trans*-prostadiene,1-ethylene ketal |
| 820 | 773 | 280 | 1-iodo-3-triphen-ylmethoxy-1-*trans*-decene (U.S. Pat. No. 3,873,607). | 1,9-dioxo-11α,15α-dihydroxy-20-ethyl-1-hydroxymethyl-5-*cis*-13-*trans*-prostadiene,1-ethylene ketal |
| 821 | 773 | 280 | 1-iodo-3-triphen-ylmethoxy-4,4-di-methyl-1-*trans*-octene (U.S. Pat. No. 3,873,607). | 1,9-dioxo-11α,15α-dihydroxy-16,16-dimethyl-1-hydroxymethyl-5-*cis*-13-*trans*-prostadiene,1-ethylene ketal |
| 822 | 773 | 280 | 1-iodo-3-triphen-ylmethoxy-5,5-di methyl-1-*trans*-octene (U.S. Pat. No. 3,873,607). | 1,9-dioxo-11α,15α-dihydroxy-17,17-dimethyl 5-*cis*-13-*trans*-prostadiene,1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 823 | 773 | 280 | 1-iodo-3-triphenylmethoxy-4-methyl-1-*trans*-octene (U.S. Pat. No. 3,876,690). | 1,9-dioxo-11$\alpha$,15$\alpha$-dihydroxy-16-methyl-1-hydroxymethyl-5-*cis*-13-*trans*-prostadiene,1-ethylene ketal |
| 824 | 773 | 280 | 1-iodo-3-triphenylmethoxy-4-ethyl-1-*trans*-octene (U.S. Pat. No. 3,876,690. | 1,9-dioxo-11$\alpha$,15$\alpha$-dihydroxy-16-ethyl-1-hydroxymethyl-5-*cis*-13-*trans*-prostadiene,1-ethylene ketal |
| 825 | 773 | 280 | 1-iodo-3-triphenylmethoxy-3-cyclopentyl-1-*trans*-butene (U.S. Pat. No. 3,884,969). | 1,9-dioxo-11$\alpha$,15$\alpha$-dihydroxy-17,20-tetranor-16-cyclopentyl-1-hydroxymethyl-5-*cis*-13-*trans*-prostadiene,1-ethylene ketal |
| 826 | 773 | 280 | 1-iodo-3-triphenylmethoxy-3-cyclohexyl-1-*trans*-propene (U.S. Pat. No. 3,884,969). | 1,9-dioxo-11$\alpha$,15$\alpha$-dihydroxy-16,20-pentanor-15-cyclohexyl-1-hydroxymethyl-5-*cis*-13-*trans*-prostadiene,1-ethylene ketal |
| 827 | 773 | 280 | 1-iodo-3-triphenylmethoxy-5-cyclohexyl-1-*trans*-pentene (U.S. Pat. No. 3,884,969). | 1,9-dioxo-11$\alpha$,15$\alpha$-dihydroxy-18,20-trinor-17-cyclohexyl-1-hydroxymethyl-5-*cis*-13-*trans*-prostadiene,1-ethylene ketal |
| 828 | 773 | 280 | 1-iodo-3-triphenylmethoxy-6-cyclopentyl-1-*trans*-hexene (U.S. Pat. No. 3,884,969). | 1,9-dioxo-11$\alpha$,15$\alpha$-dihydroxy-19,20-dinor-18-cyclopentyl-1-hydroxymethyl-5-*cis*-13-*trans*-prostadiene,1-ethylene ketal |
| 829 | 773 | 280 | 1-iodo-3-methyl-3-trimethylsilyloxy-*trans*-1-octene (Example 190) | 1,9-dioxo-11$\alpha$,15$\alpha$-dihydroxy-15-methyl-1-hydroxymethyl-5-*cis*-13-*trans*-prostadiene,1-ethylene ketal |
| 830 | 773 | 280 | 1-iodo-3-methyl-3-trimethylsilyloxy-*trans*-1-decene (Example 190a) | 1,9-dioxo-11$\alpha$,15$\alpha$-dihydroxy-15-methyl-20-ethyl-1-hydroxymethyl-5-*cis*-13-trans-prostadiene,1-ethylene ketal |
| 831 | 774 | 280 | 159 | 1,9-dioxo-11$\alpha$,15$\alpha$-dihydroxy-17,18,19,20-tetranor-16-*p*-fluorophenoxy-1-hydroxymethyl-5-*cis*-13-*trans*-prostadiene, 1-ethylene ketal |
| 832 | 774 | 200 | 186 | 1,9-dioxo-11$\alpha$,15$\alpha$-dihydroxy-17,18,19,20-tetranor-16-phenoxy-1-hydroxymethyl-5-*cis*-13-*trans*-prostadiene,1-ethylene ketal |
| 833 | 774 | 280 | 181 | 1,9-dioxo-11$\alpha$,15$\alpha$-dihydroxy-17,18,19,20 tetranor-16-*p*-bromophenoxy-1-hydroxymethyl-5-*cis*-13-*trans*-prostadiene, 1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 835 | 774 | 280 | 183 | 1,9-dioxo-11α,15α-dihydroxy-17,18,19,20-tetranor-16-*p*-methoxyphenoxy-1-hydroxymethyl-5-*cis*-13-*trans*-prostadiene, 1-ethylene ketal |
| 836 | 774 | 280 | 184 | 1,9-dioxo-11α,15α-dihydroxy-17,18,19,20-tetranor-16-*p*-chlorophenoxy-1-hydroxymethyl-5-*cis*-13-*trans*-prostadiene,1-ethylene ketal |
| 837 | 774 | 280 | 180e | 1,9-dioxo-11α,15α-dihydroxy-17,18,19,20-tetranor-*m*-trifluorophenoxy-1-hydroxymethyl-5-*cis*-13-*trans*-prostadiene,1-ethylene ketal |
| 838 | 774 | 280 | 185 | 1,9-dioxo-11α,15α-dihydroxy-17,18,19,20-tetranor-16-(3,4-dichlorophenoxy)-1-hydroxymethyl-5-*cis*-13-*trans*-prostadiene, ethylene ketal |
| 839 | 774 | 280 | 186b | 1,9-dioxo-11α,15α-dihydroxy-18,19,20-trinor-17-phenyl-1-hydroxymethyl-5-*cis*-13-*trans*-prostadiene,1-ethylene ketal |
| 840 | 774 | 280 | 186e | 1,9-dioxo-11α,15α-dihydroxy-18,19,20-trinor-17-(*m*-trifluoromethylphenyl)-1-hydroxymethyl-5-*cis*-13-*trans*-prostadiene,1-ethylene ketal |
| 841 | 774 | 280 | 186d | 1,9-dioxo-11α,15α-dihydroxy-18,19,20-trinor-17-(*p*-methoxyphenyl)-1-hydroxymethyl-5-*cis*-13-*trans*-prostadiene,1-ethylene ketal |
| 842 | 773 | 297 | 1-iodo-3-triphenylmethoxy-1-*trans*-octene (U.S. Pat. No. 3,873,607). | 1,9-dioxo-11α,15α-dihydroxy-1-hydroxymethyl-2-nor-5-*cis*-13-*trans*-prostadiene,1-ethylene ketal |
| 843 | 773 | 297 | 190 | 1,9-dioxo-11α,15α-dihydroxy-15-methyl-1-hydroxymethyl-2-nor-5-*cis*-13-*trans*-prostadiene,1-ethylene ketal |
| 844 | 773 | 297 | 1-iodo-3-triphenylmethoxy-5,5-dimethyl octene (U.S. Pat. No. 3,873,607). | 1,9-dioxo-11α,15α-dihydroxy-17,17-dimethyl-1-hydroxymethyl-2-nor-5-*cis*-13-*trans*-prostadiene,1-ethylene ketal |
| 845 | 773 | 297 | 13 | 1,9-dioxo-11α,15α-dihydroxy-16,16-trimethylene-1-hydroxymethyl-2-nor-5-*cis*-13-*trans*-prostadiene,1-ethylene ketal |
| 846 | 773 | 297 | 76 | *dl-erythro*-1,9-dioxo-11α,15α,16-trihydroxy-1-hydroxymethyl-2-nor-5-*cis*-13-*trans*-prostadiene,1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 847 | 773 | 297 | 69 | *dl-threo*-1,9-dioxo-11α,15α,16-trihydroxy-1-hydroxymethyl-2-nor-5-*cis*-13-*trans*-prostadiene,1-ethylene ketal |
| 848 | 773 | 297 | 74 | *dl-erythro*-1,9-dioxo-11α,15α,16-dihydroxy-16-methoxy-1-hydroxymethyl-5-*cis*-13-*trans*-prostadiene,1-ethylene ketal |
| 849 | 773 | 297 | 107 | 1,9-dioxo-11α,16-dihydroxy-1-hydroxymethyl-2-nor-5-*cis*-13-*trans*-prostadiene,1-ethylene ketal |
| 850 | 773 | 297 | 135 | 1,9-dioxo-11α,16-dihydroxy-16-methyl-1-hydroxymethyl-2-nor-5-*cis*-13-*trans*-prostadiene,1-ethylene ketal |
| 851 | 773 | 297 | 134 | 1,9-dioxo-11α,16-dihydroxy-16-methyl-1-hydroxymethyl-2-nor-5-*cis*-13-*trans*-17-*trans*-prostadiene,1-ethylene ketal |
| 852 | 774 | 297 | 186 | 1,9-dioxo-11α,15α-dihydroxy-2,17,18,19,-20-pentanor-16-phenoxy-1-hydroxymethyl-5-*cis*-13-*trans*-prostadiene,1-ethylene ketal |
| 853 | 774 | 297 | 180e | 1,9-dioxo-11α,15α-dihydroxy-2,17,18,19,-20-pentanor-16-*m*-trifluoromethylphenoxy-1-hydroxymethyl-5-*cis*-13-*trans*-prostadiene,1-ethylene ketal |
| 854 | 773 | 297 | 194 | 1,9-dioxo-11α,16-dihydroxy-16-vinyl-1-hydroxymethyl-2-nor-5-*cis*-13-*trans*-prostadiene,1-ethylene ketal |
| 855 | 774 | 297 | 198 | 1,9-dioxo-11α,16-dihydroxy-16-cyclopropyl-1-hydroxymethyl-2-nor-5-*cis*-13-*trans*-prostadiene,1-ethylene ketal |
| 856 | 774 | 297 | 186b | 1,9-dioxo-11α,15α-dihydroxy-2,18,19,20-tetranor-17-phenyl-1-hydroxymethyl-5-*cis*-13-*trans*-prostadiene,1-ethylene ketal |
| 857 | 773 | 297 | 186e | 1,9-dioxo-11α,15α-dihydroxy-2,18,19,20-tetranor-17-(*m*-trifluorophenyl)-1-hydroxymethyl-5-*cis*-13-*trans*-prostadiene,1-ethylene ketal |
| 858 | 773 | 297 | 186d | 1,9-dioxo-11α,15α-dihydroxy-2,18,19,20-tetranor-17-(*p*-methoxyphenyl)-1-hydroxymethyl-5-*cis*-13-*trans*-prostadiene,1-ethylene ketal |
| 859 | 773 | 310 | 190 | 1,9-dioxo-15-hydroxy-15-methyl-1-hydroxymethyl-2-nor-13-*trans*-prostene,1-ethylene ketal |
| 860 | 773 | 298 | 135 | 1,9-dioxo-11α,16-dihydroxy-16-methyl-1-homo-1-hydroxymethyl-5-*cis*-13-*trans*-prostadiene, 1-ethylene ketal |

TABLE XL (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 861 | 773 | 298 | 134 | 1,9-dioxo-11α,16-dihydroxy-16-methyl-1-homo-1-hydroxymethyl-5-*cis*-13-*trans*-17-*trans*-prostatriene,1-ethylene ketal |
| 862 | 774 | 298 | 186 | 1,9-dioxo-11α,15α-dihydroxy-17,18,19,20-tetranor-16-phenoxy-1-hydroxymethyl-5-*cis*-13-*trans*-prostadiene,1-ethylene ketal |
| 863 | 774 | 298 | 180e | 1,9-dioxo-11α,15α-dihydroxy-17,18,19,20-tetranor-16-*m*-trifluoromethylphenoxy-1-hydroxymethyl-5-*cis*-13-*trans*-prostadiene,1-ethylene ketal |
| 864 | 773 | 298 | 194 | 1,9-dioxo-11α,16-dihydroxy-16-vinyl-1-homo-1-hydroxymethyl-5-*cis*-13-*trans*-prostadiene,1-ethylene ketal |
| 865 | 774 | 298 | 198 | 1,9-dioxo-11α,16-dihydroxy-16-cyclopropyl-1-homo-1-hydroxymethyl-5-*cis*-13-*trans*-prostadiene,1-ethylene ketal |
| 866 | 774 | 298 | 186b | 1,9-dioxo-11α,15α-dihydroxy-1-homo-18,19,-20-trinor-17-phenyl-1-hydroxymethyl-5-*cis*-13-*trans*-prostadiene,1-ethylene ketal |
| 867 | 773 | 298 | 186e | 1,9-dioxo-11α,15α-dihydroxy-1-homo-18,19,-20-trinor-17-(*m*-trifluorophenyl)-1-hydroxymethyl-13-*trans*-5-*cis*-prostadiene,1-ethylene ketal |
| 868 | 773 | 298 | 186d | 1,9-dioxo-11α,15α-dihydroxy-1-homo-18,19,-20-trinor-17-(*p*-methoxyphenyl)-1-hydroxymethyl-13-*trans*-5-*cis*-prostadiene,1-ethylene ketal |
| 868A* | 774 | 280 | 210I | 1,9-dioxo-11α,16-dihydroxy-16-ethynyl-1-hydroxymethyl-5-*cis*,13-*trans*-prostadiene,1-ethylene ketal |
| 868B* | 774 | 280 | 210P | 1,9-dioxo-11α,16-dihydroxy-16-ethynyl-1-hydroxymethyl-20-nor-5-*cis*,13-*trans*-prostadiene,1-ethylene ketal |
| 868C* | 774 | 280 | 210Q | 1,9-dioxo-11α,16-dihydroxy-16-ethynyl-1-hydroxymethyl-20-ethyl-5-*cis*,13-*trans*-prostadiene,1-ethylene ketal |
| 868D | 773 | 280 | 154I | 1,9-dioxo-11α,16-dihydroxy-16-(1-propynyl)-1-hydroxymethyl-5-*cis*,13-*trans*-prostadiene,1-ethylene ketal |
| 868E | 773 | 280 | 154J | 1,9-dioxo-11α,16-dihydroxy-16-(1-propynyl)-1-hydroxymethyl-20-ethyl-5-*cis*,13-*trans*-prostadiene,1-ethylene ketal |

Examples 869—891

By the methods described hereinabove in Examples 773 and 774, the 1,9-dioxo-1-hydroxymethyl prostene derivatives shown in Table XLI are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XLI. It should be understood that the other diastereoisomer is also formed which in its *nat* and *ent* forms has an opposite (mirror image) configuration at the asymmetric carbon atoms on the β-chain (the chain containing $C_{13}$ .... $C_{14}$ etc.) to that of the respective *nat* and *ent* forms of the listed diastereoisomer; both of these diastereoisomers are claimed in this invention as well as their component enantiomers.

TABLE XLI

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 869 | 773 | 280 | 246 | *nat*-(and *ent*)-1,9-dioxo-11α,15-dihydroxy-15,16-tetramethylene-17,18,19,20-tetranor-1-hydroxymethyl-5-*cis*-13-*trans*-prostadiene,1-ethylene ketal |
| 870 | 774 | 280 | 245 | *nat*-(and *ent*)-1,9-dioxo-11α,16-dihydroxy-16,17-tetramethylene-18,19,20-trinor-1-hydroxymethyl-5-*cis*-13-*trans*-prostadiene,1-ethylene ketal |
| 871 | 773 | 280 | 243 | *nat*-15S,16R-(and *ent*-15R,16S)-1,9-dioxo-11α,15-dihydroxy-15,16-trimethylene-1-hydroxymethyl-5-*cis*-13-*trans*-prostadiene,1-ethylene ketal |
| 873 | 773 | 280 | 244 | *nat*-15S,16S-(and *ent*-15R,16R)-1,9-dioxo-11α,15-dihydroxy-15,16-trimethylene-1-hydroxymethyl-13-*trans*-5-*cis*-prostadiene,1-ethylene ketal |
| 874 | 773 | 280 | 247 | *nat*-(and *ent*)-1,9-dioxo-11α,15-dihydroxy-15,16-trimethylene-17,18,19,20-tetranor-1-hydroxymethyl-13-*trans*-5-*cis*-prostadiene,1-ethylene ketal |
| 875 | 774 | 280 | 248 | *nat*-(and *ent*)-1,9-dioxo-11α,15-dihydroxy-15,16-pentamethylene-17,18,19,20-tetranor-1-hydroxymethyl-13-*trans*-5-*cis*-prostadiene,1-ethylene ketal |
| 876 | 773 | 280 | 249 | *nat*-15S,17R-(and *ent*-15R,17S)-1,9-dioxo-11α,15-dihydroxy-15,17-dimethylene-1-hydroxymethyl-13-*trans*-5-*cis*-prostadiene,1-ethylene ketal |
| 877 | 773 | 280 | 250 | *nat*-15S,17S-(and *ent*-15R,17R)-1,9-dioxo-11α,15-dihydroxy-15,17-dimethylene-1-hydroxymethyl-13-*trans*-5-*cis*-prostadiene,1-ethylene ketal |
| 878 | 773 | 280 | 252 | *nat*-15S,16R-(and *ent*-15R,16S)-1,9-dioxo-11α,15-dihydroxy-15,16-tetramethylene-1-hydroxymethyl-13-*trans*-5-*cis*-prostadiene,1-ethylene ketal |

TABLE XLI (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 879 | 773 | 280 | 253 | *nat*-15S,16S-(and *ent*-15R,16R)-1,9-dioxo-11α,15-dihydroxy-15,16-tetramethylene-1-hydroxymethyl-13-*trans*-5-*cis*-prostadiene,1-ethylene ketal |
| 880 | 774 | 280 | 254 | *nat*-15R,16S-(and *ent*-15S,16R)-1,9-dioxo-11α,15-dihydroxy-15,16-trimethylene-16-(3-trifluoromethylphenoxy)-17,18,19,20-tetranor-1-hydroxymethyl-13-*trans*-5-*cis*-prostadiene,1-ethylene ketal |
| 881 | 774 | 280 | 255 | *nat*-15R,16R-(and *ent*-15S,16S)-1,9-dioxo-11α,15-dihydroxy-15,16-trimethylene-16-(3-trifluoromethylphenoxy)-17,18,19,20-tetranor-1-hydroxymethyl-13-*trans*-5-*cis*-prostadiene,1-ethylene ketal |
| 882 | 773 | 280 | 256 | *nat*-15R,16S-(and *ent*-15S,16R)-1,9-dioxo-11α,15-dihydroxy-15,16-trimethylene-16-(4-fluorophenoxy)-17,18,19,20-tetranor-1-hydroxymethyl-13-*trans*-5-*cis*-prostadiene,1-ethylene ketal |
| 883 | 773 | 280 | 257 | *nat*-15R,16R-(and *ent*-15S,16S)-1,9-dioxo-11α,15-dihydroxy-15,16-trimethylene-16-(4-fluorophenoxy)-17,18,19,20-tetranor-1-hydroxymethyl-13-*trans*-5-*cis*-prostadiene,1-ethylene ketal |
| 884 | 774 | 280 | 258 | *nat*-15R,16S-(and *ent*-15S,16R)-1,9-dioxo-11α,15-dihydroxy-15,16-trimethylene-16-(3-chlorophenoxy)-17,18,19,20-tetranor-1-hydroxymethyl-13-*trans*-5-*cis*-prostadiene,1-ethylene ketal |
| 885 | 774 | 280 | 259 | *nat*-15R,16R-(and *ent*-15S,16S)-1,9-dioxo-11α,15-dihydroxy-15,16-trimethylene-16-(3-chlorophenoxy)-17,18,19,20-tetranor-1-hydroxymethyl-13-*trans*-5-*cis*-prostadiene,1-ethylene ketal |
| 886 | 773 | 280 | 260 | *nat*-15S,17R-(and *ent*-15R,17S)-1,9-dioxo-11α,15-dihydroxy-15,17-trimethylene-19,-20-dinor-1-hydroxymethyl-13-*trans*-5-*cis*-prostadiene,1-ethylene ketal |
| 887 | 773 | 280 | 261 | *nat*-15S,17S-(and *ent*-15R,17R)-1,9-dioxo-11α,15-dihydroxy-15,17-trimethylene-19,-20-dinor-1-hydroxymethyl-13-*trans*-5-*cis*-prostadiene,1-ethylene ketal |
| 888 | 774 | 280 | 262 | *nat*-16R,17S-(and *ent*-16S,17R)-1,9-dioxo-11α,16-dihydroxy-16,17-trimethylene-20-methyl-1-hydroxymethyl-13-*trans*-5-*cis*-prostadiene,1-ethylene ketal |
| 889 | 774 | 280 | 263 | *nat*-16R,17R-(and *ent*-16S,17S)-1,9-dioxo-11α,16-dihydroxy-16,17-trimethylene-20-methyl-1-hydroxymethyl-13-*trans*-5-*cis*-prostadiene,1-ethylene ketal |

TABLE XLI (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 890 | 774 | 280 | 264 | *nat*-16R,17S-(and *ent*-16S,17R)-1,9-dioxo-11$\alpha$,16-dihydroxy-16,17-trimethylene-16-(3-trifluoromethylphenoxy)-18,19,20-tri-nor-1-hydroxymethyl-13-*trans*-5-*cis*-prostadiene,1-ethylene ketal |
| 891 | 774 | 280 | 265 | *nat*-16R,17R-(and *ent*-16S,17S)-1,9-dioxo-11$\alpha$,16-dihydroxy-16,17-trimethylene-16-(3-trifluoromethylphenoxy)-18,19,20-tri-nor-1-hydroxymethyl-13-*trans*-5-*cis*-prostadiene,1-ethylene ketal |

### Examples 892—975E

By the methods described hereinabove in Examples 773 and 774, the 1,9-dioxo-hydroxymethyl prostene derivatives shown in Table XLII are prepared by the indicated method from the indicated vinyl iodide or vinyl tine compound and the indicated cyclopent-2-en-1-one.

In those cases where isomers are obtained at the $C_{15}$ or $C_{16}$ positions, only the $C_{15}$ or $C_{16}$-normal isomers are listed in Table XLII; it should be understood that the corresponding $C_{15}$ or $C_{16}$-*epi* isomers are also formed and are part of this invention.

In those cases where the initial conjugate-addition product contains a triphenylmethoxy blocking group, deblocking is conducted in acetic acid-tetrahydrofuran-water 4:2:1 at 50°C for 5 hours.

[There are no Examples 905, 906, 912, 913, 915, 916 and 951]

TABLE XLII

| Example | Method of Example | Cyclopent-2-en-1-one | Vinyl Iodide or Vinyl Tin of Example | 1,9-dioxo-1-hydroxymethylprostene |
|---|---|---|---|---|
| 892 | 773 | 309 | 49 | 1,9-dioxo-11α,15-dihydroxy-1-hydroxymethyl-16,16-trimethylene-20-nor-13-*trans*-prostene,1-ethylene ketal |
| 893 | 773 | 309 | 50 | 1,9-dioxo-11α,15-dihydroxy-1-hydroxymethyl-16,16-trimethylene-20-methyl-13-*trans*-prostene,1-ethylene ketal |
| 894 | 773 | 309 | 51 | 1,9-dioxo-11α,15-dihydroxy-1-hydroxymethyl-16,16-trimethylene-20-ethyl-13-*trans*-prostene,1-ethylene ketal |
| 895 | 774 | 309 | 205 | 1,9-dioxo-11α,16-dihydroxy-16-cyclopropyl-20-ethyl-1-hydroxymethyl-13-*trans*-prostene,1-ethylene ketal |
| 896 | 774 | 309 | 207 | 1,9-dioxo-11α,16-dihydroxy-16-vinyl-20-ethyl-1-hydroxymethyl-13-*trans*-prostene,-1-ethylene ketal |
| 897 | 774 | 309 | 198 | 1,9-dioxo-11α,16-dihydroxy-16-cyclopropyl-1-hydroxymethyl-13-*trans*-prostene,1-ethylene ketal |
| 898 | 773 | 309 | 13 | 1,9-dioxo-11α,15-dihydroxy-1-hydroxymethyl-16,16-trimethylene-13-*trans*-prostene,1-ethylene ketal |
| 899 | 773 | 309 | 210a | 1,9-dioxo-11α,16-dihydroxy-1-hydroxymethyl-16-vinyl-13-*trans*-prostene,1-ethylene ketal |
| 900 | 773 | 309 | 74 | *dl-erythro*-1,9-dioxo-11α,15-dihydroxy-16-methoxy-1-hydroxymethyl-13-*trans*-prostene,1-ethylene ketal |
| 901 | 773 | 309 | 77 | *dl-erythro*-1,9-dioxo-11α,15-dihydroxy-16-ethoxy-1-hydroxymethyl-13-*trans*-prostene,1-ethylene ketal |
| 902 | 773 | 309 | 83 | *dl-erythro*-1,9-dioxo-11α,15,16-trihydroxy-1-hydroxymethyl-20-nor-13-*trans*-prostene,1-ethylene ketal |
| 903 | 773 | 309 | 84 | *dl-erythro*-1,9-dioxo-11α,15,16-trihydroxy-1-hydroxymethyl-20-methyl-13-*trans*-prostene, 1-ethylene ketal |
| 904 | 773 | 309 | 85 | *dl-erythro*-1,9-dioxo-11α,15,16-trihydroxy-1-hydroxymethyl-20-ethyl-13-*trans*-prostene,1-ethylene ketal |
| 907 | 773 | 309 | 69 | *dl-threo*-1,9-dioxo-11α,15,16-trihydroxy-1-hydroxymethyl-13-*trans*-prostene,1-ethylene ketal |
| 908 | 773 | 309 | 88 | *dl-threo*-1,9-dioxo-11α,16-trihydroxy-20-nor-1-hydroxymethyl-13-*trans*-prostene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 909 | 773 | 309 | 76 | *dl-erythro*-1,9-dioxo-11α,15,16-trihydroxy-1-hydroxymethyl-13-*trans*-prostene,1-ethylene ketal |
| 910 | 773 | 309 | 76 | *dl-threo*-1,9-dioxo-11α,15,16-trihydroxy-20 methyl-1-hydroxymethyl-13-*trans*-prostene, 1-ethylene ketal |
| 911 | 773 | 309 | 90 | *dl-threo*-1,9-dioxo-11α,15,16-trihydroxy-20-ethyl-1-hydroxymethyl-13-*trans*-prostene,1-ethylene ketal |
| 914 | 773 | 309 | 107 | 1,9-dioxo-11α,16-dihydroxy-1-hydroxy-methyl-13-*trans*-prostene,1-ethylene ketal |
| 917 | 773 | 309 | 110 | 1,9-dioxo-11α,16-dihydroxy-1-hydroxy-methyl-20-nor-13-*trans*-prostene,1-ethylene ketal |
| 918 | 773 | 309 | 144 | 1,9-dioxo-11α,16-dihydroxy-17-methyl-1-hydroxymethyl-13-*trans*-prostene,1-ethylene ketal |
| 919 | 773 | 309 | 112 | 1,9-dioxo-11α,16-dihydroxy-20-methyl-1-hydroxymethyl-13-*trans*-prostene,1-ethylene ketal |
| 920 | 773 | 309 | 113 | 1,9-dioxo-11α,16-dihydroxy-20-ethyl-1-hydroxymethyl-13-*trans*-prostene,1-ethylene ketal |
| 921 | 773 | 309 | 139a | 1,9-dioxo-11α,16-dihydroxy-16-methyl-20-ethyl-1-hydroxymethyl-13-*trans*-prostene,-1-ethylene ketal |
| 923 | 773 | 309 | 135 | 1,9-dioxo-11α,16-dihydroxy-16-methyl-1-hydroxymethyl-13-*trans*-prostene,1-ethylene ketal |
| 924 | 773 | 309 | 136 | 1,9-dioxo-11α,16-dihydroxy-17,17,20-tri-methyl-1-hydroxymethyl-13-*trans*-prostene,-1-ethylene ketal |
| 925 | 773 | 309 | 137 | 1,9-dioxo-11α,16-dihydroxy-16,20-methyl-1-hydroxymethyl-13-*trans*-prostene,1-ethylene ketal |
| 926 | 773 | 309 | 138 | 1,9-dioxo-11α,16-dihydroxy-17,17-dimethyl-1-hydroxymethyl-13-*trans*-prostene,1-ethylene ketal |
| 927 | 773 | 309 | 139 | 1,9-dioxo-11α,16-dihydroxy-16-methyl-1-hydroxymethyl-13-*trans*-17-*trans*-prosta-diene,1-ethylene ketal |
| 928 | 773 | 309 | 149 | 1,9-dioxo-11α,16-dihydroxy-20-methyl-1-hydroxymethyl-13-*trans*-17-*trans*-prosta-diene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 929 | 773 | 309 | 150 | 1,9-dioxo-11α,16-dihydroxy-17-methyl-1-hydroxymethyl-13-*trans*-prostene,1-ethylene ketal |
| 930 | 773 | 309 | 151 | 1,9-dioxo-11α,16-dihydroxy-17,20-methyl-1-hydroxymethyl-13-*trans*-prostene,1-ethylene ketal |
| 931 | 773 | 309 | 152 | 1,9-dioxo-11α,16-dihydroxy-1-hydroxymethyl-13-*trans*-17-*trans*-prostadiene, 1-ethylene ketal |
| 932 | 773 | 309 | 153 | 1,9-dioxo-11α,16(R)-dihydroxy-1-hydroxy-methyl-13-*trans*-prostene,1-ethylene ketal |
| 933 | 773 | 309 | 154 | 1,9-dioxo-11α,16(S)-dihydroxy-1-hydroxy-methyl-13-*trans*-prostene,1-ethylene ketal |
| 934 | 773 | 309 | 154a | 1,9-dioxo-11α,16-dihydroxy-20-ethyl-1-hydroxy-methyl-13-*trans*-17-*trans*-prostadiene,1-ethylene ketal |
| 935 | 773 | 309 | 1-iodo-3-triphenylmethoxy-1-*trans*-octene (U.S. Pat. No. 3,873,607). | 1,9-dioxo-11α,15α-dihydroxy-1-hydroxy-methyl-13-*trans*-prostadiene,1-ethylene ketal |
| 936 | 773 | 309 | 1-iodo-3-triphenylmethoxy-1-*trans*-nonene (U.S. Pat. No. 3,873,607). | 1,9-dioxo-11α,15α-dihydroxy-20-methyl-1-hydroxymethyl-13-*trans*-prostene,1-ethylene ketal |
| 937 | 773 | 309 | 1-iodo-3-triphenylmethoxy-1-*trans*-decene (U.S. Pat. No. 3,873,607). | 1,9-dioxo-11α,15α-dihydroxy-20-ethyl-1-hydroxymethyl-13-*trans*-prostene,1-ethylene ketal |
| 938 | 773 | 309 | 1-iodo-3-triphenylmethoxy-4,4-dimethyl-1-*trans*-octene (U.S. Pat. No. 3,873,607). | 1,9-dioxo-11α,15α-dihydroxy-16,16-dimethyl-1-hydroxymethyl-13-*trans*-prostene,1-ethylene ketal |
| 939 | 773 | 309 | 1-iodo-3-triphenylmethoxy-5,5-dimethyl-1-*trans*-octene (U.S. Pat. No. 3,873,607). | 1,9-dioxo-11α,15α-dihydroxy-17,17-dimethyl-1-hydroxymethyl-13-*trans*-prostene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 940 | 773 | 309 | 1-iodo-3-triphenylmethoxy-4-methyl-1-*trans*-octene (U.S. Pat. No. 3,876,690). | 1,9-dioxo-11α,15α-dihydroxy-16-methyl-1-hydroxymethyl-13-*trans*-prostene,1-ethylene ketal |
| 941 | 773 | 309 | 1-iodo-3-triphenylmethoxy-4-ethyl-1-*trans*-octene (U.S. Pat. No. 3,876,690). | 1,9-dioxo-11α,15α-dihydroxy-16-ethyl-1-hydroxymethyl-13-*trans*-prostene,1-ethylene ketal |
| 942 | 773 | 309 | 1-iodo-3-triphenylmethoxy-4-cyclopentyl-1-*trans*-butene (U.S. Pat. No. 3,884,969). | 1,9-dioxo-11α,15α-dihydroxy-17,20-tetra-nor-16-cyclopentyl-1-hydroxymethyl-13-*trans*-prostene,1-ethylene ketal |
| 943 | 773 | 309 | 1-iodo-3-triphenylmethoxy-3-cyclohexyl-1-*trans*-propone (U.S. Pat. No. 3,884,969). | 1,9-dioxo-11α,15α-dihydroxy-16,20-penta-nor-15-cyclohexyl-1-hydroxymethyl-13-*trans*-prostene,1-ethylene ketal |
| 944 | 773 | 309 | 1-iodo-3-triphenylmethoxy-5-cyclohexyl-1-*trans*-pentene (U.S. Pat. No. 3,884,969). | 1,9-dioxo-11α,15α-dihydroxy-18,20-trinor-17-cyclohexyl-1-hydroxymethyl-13-*trans*-prostene,1-ethylene ketal |
| 945 | 773 | 309 | 1-iodo-3-triphenylmethoxy-6-cyclopentyl-1-*trans*-hexene (U.S. Pat. No. 3,884,969). | 1,9-dioxo-11α,15α-dihydroxy-19,20-dinor-18-cyclopentyl-1-hydroxymethyl-13-*trans*-prostene,1-ethylene ketal |
| 946 | 773 | 309 | 1-iodo-3-methyl-3-tri-methylsilyloxy-*trans*-1-octene (Example 190) | 1,9-dioxo-11α,15α-dihydroxy-15-methyl-1-hydroxymethyl-13-*trans*-prostene,1-ethylene ketal |
| 947 | 773 | 309 | 1-iodo-3-methyl-3-tri-methylsilyloxy-*trans*-1-decene (Example 190a) | 1,9-dioxo-11α,15α-dihydroxy-15 methyl-20-ethyl-1-hydroxymethyl-13-*trans*-prostene,-1-ethylene ketal |
| 948 | 774 | 309 | 159 | 1,9-dioxo-11α,15α-dihydroxy-17,18,19,20-tetranor-16-*p*-fluorophenoxy-1-hydroxy-methyl-13-*trans*-prostene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 949 | 774 | 309 | 186 | 1,9-dioxo-11α,15α-dihydroxy-17,18,19,20-tetranor-16-phenoxy-1-hydroxymethyl-13-*trans*-prostene,1-ethylene ketal |
| 950 | 774 | 309 | 181 | 1,9-dioxo-11α,15α-dihydroxy-17,18,19,20-tetranor-16-*p*-bromophenoxy-1-hydroxy-methyl-13-*trans*-prostene,1-ethylene ketal |
| 952 | 774 | 309 | 183 | 1,9-dioxo-11α,15α-dihydroxy-17,18,19,20-tetranor-16-*p*-methoxyphenoxy-1-hydroxy-methyl-13-*trans*-prostene,1-ethylene ketal |
| 953 | 774 | 309 | 184 | 1,9-dioxo-11α,15α-dihydroxy-17,18,19,20-tetranor-16-*p*-chlorophenoxy-1-hydroxy-methyl-13-*trans*-prostene,1-ethylene ketal |
| 954 | 774 | 309 | 180 | 1,9-dioxo-11α,15α-dihydroxy-17,18,19,20-tetranor-*m*-trifluorophenoxy-1-hydroxy-methyl-13-*trans*-prostene,1-ethylene ketal |
| 955 | 774 | 309 | 185 | 1,9-dioxo-11α,15α-dihydroxy-17,18,19,20-tetranor-16-(3,4-dichlorophenoxy)-1-hydroxymethyl-13-*trans*-prostene,1-ethylene ketal |
| 956 | 774 | 309 | 186b | 1,9-dioxo-11α,15α-dihydroxy-18,19,20-tri-nor-17-phenyl-1-hydroxymethyl-13-*trans*-prostene,1-ethylene ketal |
| 957 | 774 | 309 | 186e | 1,9-dioxo-11α,15α-dihydroxy-18,19,20-trinor-17-(*m*-trifluoromethylphenyl)-1-hydroxymethyl-13-*trans*-prostene,1-ethylene ketal |
| 958 | 774 | 309 | 186d | 1,9-dioxo-11α,15α-dihydroxy-18,19,20-tri-nor-17-(*p*-methoxyphenyl)-1-hydroxymethyl-13-*trans*-prostene,1-ethylene ketal |
| 959 | 773 | 312 | 1-iodo-3-triphenylmethoxy-1-*trans*-octene (U.S. Pat. No. 3,873,607). | 1,9-dioxo-11α,15α-dihydroxy-1-hydroxy-methyl-2-nor-13-*trans*-prostadiene,1-ethylene ketal |
| 960 | 773 | 312 | 190 | 1,9-dioxo-11α,15α-dihydroxy-15-methyl-1-hydroxymethyl-2-nor-13-*trans*-prostene,1-ethylene ketal |
| 961 | 773 | 312 | 1-iodo-3-triphenylmethoxy-5,5-dimethyl-octene-(U.S. Pat. No. 3,873,607). | 1,9-dioxo-11α,15α-dihydroxy-17,17-dimethyl-1-hydroxymethyl-2-nor-13-*trans*-prostene,-1-ethylene ketal |
| 962 | 773 | 312 | 13 | 1,9-dioxo-11α,15α-dihydroxy-16,16-tri-methylene-1-hydroxymethyl-2-nor-13-*trans*-prostene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 963 | 773 | 312 | 76 | *dl-erythro*-1,9-dioxo-11$\alpha$,15$\alpha$,16-trihydroxy-1-hydroxymethyl-2-nor-13-*trans*-prostene,-1-ethylene ketal |
| 964 | 773 | 312 | 69 | *dl-threo*-1,9-dioxo-11$\alpha$,15$\alpha$,16-trihydroxy-1-hydroxymethyl-2-nor-13-*trans*-prostene,-1-ethylene ketal |
| 965 | 773 | 312 | 74 | *dl-erythro*-1,9-dioxo-11$\alpha$,15$\alpha$-dihydroxy-16-methoxy-1-hydroxymethyl-2-nor-13-*trans*-prostene,1-ethylene ketal |
| 966 | 773 | 312 | 107 | 1,9-dioxo-11$\alpha$,16-dihydroxy-1-hydroxymethyl-2-nor-13-*trans*-prostene,1-ethylene ketal |
| 967 | 774 | 312 | 135 | 1,9-dioxo-11$\alpha$,16-dihydroxy-16-methyl-1-hydroxymethyl-2-nor-13-*trans*-prostene,-1-ethylene ketal |
| 968 | 774 | 312 | 134 | 1,9-dioxo-11$\alpha$-16-dihydroxy-16-methyl-1-hydroxymethyl-2-nor-13-*trans*-17-*trans*-prostadiene,1-ethylene ketal |
| 969 | 774 | 312 | 186 | 1,9-dioxo-11$\alpha$,15$\alpha$-dihydroxy-2,17,18,19,-20-pentanor-16-phenoxy-1-hydroxymethyl-13-*trans*-prostene,1-ethylene ketal |
| 970 | 774 | 312 | 186e | 1,9-dioxo-11$\alpha$,15$\alpha$-dihydroxy-2,17,18,19,-20-pentanor-16-*m*-trifluoromethylphenoxy-1-hydroxymethyl-13-*trans*-prostene,1-ethylene ketal |
| 971 | 773 | 312 | 194 | 1,9-dioxo-11$\alpha$,16-dihydroxy-16-vinyl-1-hydroxymethyl-2-nor-13-*trans*-prostene,1-ethylene ketal |
| 972 | 774 | 312 | 198 | 1,9-dioxo-11$\alpha$-16-dihydroxy-16-cyclopropyl-1-hydroxymethyl-2-nor-13-*trans*-prostene,1-ethylene ketal |
| 973 | 774 | 312 | 186b | 1,9-dioxo-11$\alpha$,15$\alpha$-dihydroxy-2,18,19,20-tetranor-17-phenyl-1-hydroxymethyl-13-*trans*-prostene,1-ethylene ketal |
| 974 | 773 | 312 | 186e | 1,9-dioxo-11$\alpha$,15$\alpha$-dihydroxy-2,18,19,20-tetranor-17-(*m*-trifluorophenyl)-1-hydroxymethyl-13-*trans*-prostene,1-ethylene ketal |
| 975 | 773 | 312 | 186d | 1,9-dioxo-11$\alpha$,15$\alpha$-dihydroxy-2,18,19,20-tetranor-17-(*p*-methoxyphenyl)-1-hydroxymethyl-13-*trans*-prostene,1-ethylene ketal |
| 975A* | 774 | 309 | 210l | 1,9-dioxo-11$\alpha$-16-dihydroxy-16-ethynyl-1-hydroxymethyl-13-*trans*-prostene,1-ethylene ketal |

TABLE XLII (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 975B* | 774 | 309 | 210P | 1,9-dioxo-11α-16-dihydroxy-16-ethynyl-1-hydroxymethyl-20-nor-13-*trans*-prostene,-1-ethylene ketal |
| 975C* | 774 | 309 | 210Q | 1,9-dioxo-11α-16-dihydroxy-16-ethynyl-1-hydroxymethyl-20-ethyl-13-*trans*-prostene,-1-ethylene ketal |
| 975D | 773 | 309 | 154I | 1,9-dioxo-11α-16-dihydroxy-16-(1-propynyl)-1-hydroxymethyl-13-*trans*-prostene,1-ethylene ketal |
| 975E | 773 | 309 | 154J | 1,9-dioxo-11α-16-dihydroxy-16-(1-propynyl)-1-hydroxymethyl-20-ethyl-13-*trans*-prostene,1-ethylene ketal |

*For the cleavage of the TMS—C=C-linkage see, Example 772(B)

Examples 976—998

By the methods described hereinabove in Examples 773 and 774, the 1,9-dioxo-1-hydroxymethyl prostene derivatives shown in Table XLIII are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XLIII. It should be understood that the other diastereoisomer is also formed which in its *nat* and *ent* forms has an opposite (mirror image) configuration at the asymmetric carbon atoms on the β-chain (the chain containing $C_{13}$ .... $C_{14}$ etc.) to that of the respective *nat* and *ent* forms of the listed diastereoisomer; both of these diastereoisomers are claimed in this invention as well as their component enantiomers.

TABLE XLIII

| Example | Method of Example | Cyclopent-2-en-1-one of Example | Vinyl Iodide or Vinyl Tin of Example | 1,9-dioxo-hydroxymethylprostene and its diastereomer |
|---|---|---|---|---|
| 976 | 773 | 309 | 246 | *nat*-(and *ent*)-1,9-dioxo-11$\alpha$,15-dihydroxy-15,16-tetramethylene-17,18,19,20-tetranor-1-hydroxymethyl-13-*trans*-prostene,1-ethylene ketal |
| 977 | 774 | 309 | 245 | *nat*-(and *ent*)-1,9-dioxo-11$\alpha$,16-dihydroxy-16,17-tetramethylene-18,19,20-trinor-1-hydroxymethyl-13-*trans*-prostene,1-ethylene ketal |
| 978 | 773 | 309 | 243 | *nat*-15S,16R-(and *ent*-15R,16S)-1,9-dioxo-11$\alpha$,15-dihydroxy-15,16-trimethylene-1-hydroxymethyl-13-*trans*-prostene,1-ethylene ketal |
| 980 | 773 | 309 | 244 | *nat*-15S,16S-(and *ent*-15R,16R)-1,9-dioxo-11$\alpha$,15-dihydroxy-15,16-trimethylene-1-hydroxymethyl-13-*trans*-prostene,1-ethylene ketal |
| 981 | 773 | 309 | 247 | *nat*-(and *ent*)-1,9-dioxo-11$\alpha$,15-dihydroxy-15,16-trimethylene-17,18,19,20-tetranor-1-hydroxymethyl-13-*trans*-prostene,1-ethylene ketal |
| 982 | 774 | 309 | 248 | *nat*-(and *ent*)-1,9-dioxo-11$\alpha$,15-dihydroxy-15,16-pentamethylene-17,18,19,20-tetranor-1-hydroxymethyl-13-*trans*-prostene,1-ethylene ketal |
| 983 | 773 | 309 | 249 | *nat*-15S,17R-(and *ent*-15R,17S)-1,9-dioxo-11$\alpha$,15-dihydroxy-15,17-dimethylene-1-hydroxymethyl-13-*trans*-prostene,1-ethylene ketal |
| 984 | 773 | 309 | 250 | *nat*-15S,17S-(and *ent*-15R,17R)-1,9-dioxo-11$\alpha$,15-dihydroxy-15,17-dimethylene-1-hydroxymethyl-13-*trans*-prostene,1-ethylene ketal |
| 985 | 773 | 309 | 252 | *nat*-15S,16R-(and *ent*-15R,16S)-1,9-dioxo-11$\alpha$,15-dihydroxy-15,16-trimethylene-1-hydroxymethyl-13-*trans*-prostene,1-ethylene ketal |
| 986 | 773 | 309 | 253 | *nat*-15S,16S-(and *ent*-15R,16R)-1,9-dioxo-11$\alpha$,15-dihydroxy-15,16-tetramethylene-1-hydroxymethyl-13-*trans*-prostene,1-ethylene ketal |
| 987 | 774 | 309 | 254 | *nat*-15R,16S-(and *ent*-15S,16R)-1,9-dioxo-11$\alpha$,15-dihydroxy-15,16-trimethylene-16-(3-trifluoromethylphenoxy)-17,18,19,20-tetranor-1-hydroxymethyl-13-*trans*-prostene,1-ethylene ketal |
| 988 | 774 | 309 | 255 | *nat*-15R,16R-(and *ent*-15S,16S)-1,9-dioxo-11$\alpha$,15-dihydroxy-15,16-trimethylene-16 (3-trifluoromethylphenoxy)-17,18,19,20-tetranor-1-hydroxymethyl-13-*trans*-prostene,1-ethylene ketal |

TABLE XLIII (continued)

| Example | Method of Example | Cyclopent-2-en-1-one of Example | Vinyl Iodide or Vinyl Tin of Example | 1,9-dioxo-hydroxymethylprostene and its diastereomer |
|---|---|---|---|---|
| 989 | 773 | 309 | 256 | *nat*-15R,16S-(and *ent*-15S,16R)-1,9-dioxo-11$\alpha$,15-dihydroxy-15,16-trimethylene-16-(4-fluorophenoxy)-17,18,19,20-tetranor-1-hydroxymethyl-13-*trans*-prostene,1-ethylene ketal |
| 990 | 773 | 309 | 257 | *nat*-15R,16R-(and *ent*-15S,16S)-1,9-dioxo-11$\alpha$,15-dihydroxy-15,16-trimethylene-16-(4-fluorophenoxy)-17,18,19,20-tetranor-1-hydroxymethyl-13-*trans*-prostene,1-ethylene ketal |
| 991 | 774 | 309 | 258 | *nat*-15R,16S-(and *ent*-15S,16R)-1,9-dioxo-11$\alpha$,15-dihydroxy-15,16-trimethylene-16-(3-chlorophenoxy)-17,18,19,20-tetranor-1-hydroxymethyl-13-*trans*-prostene,1-ethylene ketal |
| 992 | 774 | 309 | 259 | *nat*-15R,15R-(and *ent*-15S,16S)-1,9-dioxo-11$\alpha$,15-dihydroxy-15,16-trimethylene-16-(3-chlorophenoxy)-17,18,19,20-tetranor-1-hydroxymethyl-13-*trans*-prostene,1-ethylene ketal |
| 993 | 773 | 309 | 260 | *nat*-15S,17R-(and *ent*-15R,17S)-1,9-dioxo-11$\alpha$,15-dihydroxy-15,17-trimethylene-19,20-dinor-1-hydroxymethyl-13-*trans*-prostene,1-ethylene ketal |
| 994 | 773 | 309 | 261 | *nat*-15S,17S-(and *ent*-15R,17R)-1,9-dioxo-11$\alpha$,15-dihydroxy-15,17-trimethylene-19,20-dinor-1-hydroxymethyl-13-*trans*-prostene,1-ethylene ketal |
| 995 | 774 | 309 | 262 | *nat*-16R,17S-(and *ent*-16S,17R)-1,9-dioxo-11$\alpha$,16-dihydroxy-16,17-trimethylene-20-methyl-1-hydroxymethyl-13-*trans*-prostene,1-ethylene ketal |
| 996 | 774 | 309 | 263 | *nat*-16R,17R-(and *ent*-16S,17S)-1,9-dioxo-11$\alpha$,16-dihydroxy-16,17-trimethylene-20-methyl-1-hydroxymethyl-13-*trans*-prostene,1-ethylene ketal |
| 997 | 774 | 309 | 264 | *nat*-16R,17S-(and *ent*-16S,17R)-1,9-dioxo-11$\alpha$,16-dihydroxy-16,17-trimethylene-16-(3-trifluoromethylphenoxy)-18,19,20-tri-nor-1-hydroxymethyl-13-*trans*-prostene,1-ethylene ketal |
| 998 | 774 | 309 | 265 | *nat*-16R,17R-(and *ent*-16S,17S)-1,9-dioxo-11$\alpha$,16-dihydroxy-16,17-trimethylene-16-(3-trifluoromethylphenoxy)-18,19,20-tri-nor-1-hydroxymethyl-13-*trans*-prostene,1-ethylene ketal |

Examples 999—1082E

By the methods described hereinabove in Examples 773 and 774, the 1,9-dioxo-1-hydroxymethyl prostene derivative shown in Table XLIV are prepared by the indicated method from the indicated vinyl iodide or vinyl tin compound and the indicated cyclopent-2-en-1-one.

In those cases where isomers are obtained at the $C_{15}$ or $C_{16}$ positions, only the $C_{15}$ or $C_{16}$-normal isomers are listed in Table XLIV: it should be understood that the corresponding $C_{15}$ or $C_{16}$-*epi* isomer is also formed and is part of this invention.

In those cases where the initial conjugate-addition product contains a triphenylmethoxy blocking group, deblocking is conducted in acetic acid-tetrahydrofuran-water 4:2:1 at 50°C for 5 hours.

TABLE XLIV

| Example | Method of Example | Cyclopent-2-en-1-one | Vinyl Iodide or Vinyl Tin of Example | 1,9-dioxo-1-hydroxymethylprostene |
|---|---|---|---|---|
| 999 | 773 | 314 | 49 | 1,9-dioxo-15-hydroxy-1-hydroxymethyl-16,-16-trimethylene-20-nor-5-*cis*-13-*trans*-prostadiene,1-ethylene ketal |
| 1000 | 773 | 314 | 50 | 1,9-dioxo-15-hydroxy-1-hydroxymethyl-16,-16-trimethylene-20-methyl-5-*cis*-13-*trans*-prostadiene,1-ethylene ketal |
| 1001 | 773 | 314 | 51 | 1,9-dioxo-15-hydroxy-1-hydroxymethyl-16,-16-trimethylene-20-ethyl-5-*cis*-13-*trans*-prostadiene,1-ethylene ketal |
| 1002 | 774 | 314 | 205 | 1,9-dioxo-16-hydroxy-16-cyclopropyl-20-ethyl-1-hydroxymethyl-5-*cis*-13-*trans*-prostadiene,1-ethylene ketal |
| 1003 | 774 | 314 | 207 | 1,9-dioxo-16-hydroxy-16-vinyl-20-ethyl-1-hydroxymethyl-5-*cis*-13-*trans*-prostadiene,1-ethylene ketal |
| 1004 | 774 | 314 | 198 | 1,9-dioxo-16-hydroxy-16-cyclopropyl-1-hydroxymethyl-5-*cis*-13-*trans*-prostadiene,-1-ethylene ketal |
| 1005 | 773 | 314 | 13 | 1,9-dioxo-15-hydroxy-1-hydroxymethyl-16,-16-trimethylene-13-*trans*-5-*cis*-prostadiene,1-ethylene ketal |
| 1006 | 774 | 314 | 210a | 1,9-dioxo-16-hydroxy-1-hydroxymethyl-16,-vinyl-5-*cis*-13-*trans*-prostadiene,1-ethylene ketal |
| 1007 | 773 | 314 | 74 | *dl-erythro*-1,9-dioxo-15-hydroxy-16-methoxy-1-hydroxymethyl-5-*cis*-13-*trans*-prostadiene,1-ethylene ketal |
| 1008 | 773 | 314 | 77 | *dl-erythro*-1,9-dioxo-15-hydroxy-16-ethoxy-hydroxymethyl-5-*cis*-13-*trans*-prostadiene,1-ethylene ketal |
| 1009 | 773 | 314 | 83 | *dl-erythro*-1,9-dioxo-15,16-dihydroxy-1-hydroxymethyl-20-nor-5-*cis*-13-*trans*-prostadiene,1-ethylene ketal |
| 1010 | 773 | 314 | 84 | *dl-erythro*-1,9-dioxo-15,16-dihydroxy-1-hydroxymethyl-20-methyl-5-*cis*-13-*trans*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1011 | 773 | 314 | 85 | *dl-erythro*-1,9-dioxo-15,16-dihydroxy-1-hydroxymethyl-20-ethyl-5-*cis*-13-*trans*-prostadiene,1-ethylene ketal |
| 1014 | 773 | 314 | 69 | *dl-threo*-1,9-dioxo-15,16-dihydroxy-1-hydroxymethyl-5-*cis*-13-*trans*-prostadiene,1-ethylene ketal |
| 1015 | 773 | 314 | 88 | *dl-threo*-1,9-dioxo-15,16-dihydroxy-20-nor-1-hydroxymethyl-5-*cis*-13-*trans*-prostadiene,1-ethylene ketal |
| 1016 | 773 | 314 | 76 | *dl-erythro*-1,9-dioxo-15,16-dihydroxy-1-hydroxymethyl-5-*cis*-13-*trans*-prostadiene,1-ethylene ketal |
| 1017 | 773 | 314 | 89 | *dl-threo*-1,9-dioxo-15,16-dihydroxy-20-methyl-1-hydroxymethyl-5-*cis*-13-*trans*-prostadiene,1-ethylene ketal |
| 1018 | 773 | 314 | 90 | *dl-threo*-1,9-dioxo-15,16-dihydroxy-20-ethyl-1-hydroxymethyl-5-*cis*-13-*trans*-prostadiene,1-ethylene ketal |
| 1021 | 773 | 314 | 107 | 1,9-dioxo-16-hydroxy-1-hydroxymethyl-5-*cis*-13-*trans*-prostadiene,1-ethylene ketal |
| 1024 | 773 | 314 | 110 | 1,9-dioxo-16-hydroxy-1-hydroxymethyl-20-nor-5-*cis*-13-*trans*-prostadiene,1-ethylene ketal |
| 1025 | 773 | 314 | 144 | 1,9-dioxo-16-hydroxy-17-methyl-1-hydroxymethyl-5-*cis*-13-*trans*-prostadiene,1-ethylene ketal |
| 1026 | 773 | 314 | 112 | 1,9-dioxo-16-hydroxy-20-methyl-1-hydroxymethyl-5-*cis*-13-*trans*-prostadiene,1-ethylene ketal |
| 1027 | 773 | 314 | 113 | 1,9-dioxo-16-hydroxy-20-ethyl-1-hydroxymethyl-5-*cis*-13-*trans*-prostadiene,1-ethylene ketal |
| 1028 | 773 | 314 | 139a | 1,9-dioxo-16-hydroxy-16-methyl-20-ethyl-1-hydroxymethyl-5-*cis*-13-*trans*-prostadiene,1-ethylene ketal |
| 1030 | 773 | 314 | 135 | 1,9-dioxo-16-hydroxy-16-methyl-1-hydroxymethyl-5-*cis*-13-*trans*-prostadiene,1-ethylene ketal |
| 1031 | 773 | 314 | 136 | 1,9-dioxo-16-hydroxy-17,17,20-trimethyl-1-hydroxymethyl-5-*cis*-13-*trans*-prostadiene,1-ethylene ketal |
| 1032 | 773 | 314 | 137 | 1,9-dioxo-16-hydroxy-16,20-dimethyl-1-hydroxymethyl-13-*trans*-5-*cis*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1033 | 773 | 314 | 138 | 1,9-dioxo-16-hydroxy-17,17-dimethyl-1-hydroxymethyl-5-*cis*-13-*trans*-prostadiene,1-ethylene ketal |
| 1034 | 773 | 314 | 139 | 1,9-dioxo-16-hydroxy-16-methyl-1-hydroxymethyl-13-*trans*-17-*trans*-5-*cis*-prostatriene, 1-ethylene ketal |
| 1035 | 773 | 314 | 149 | 1,9-dioxo-16-hydroxy-20-methyl-1-hydroxymethyl-13-*trans*-17-*trans*-5-*cis*-prostatriene,1-ethylene ketal |
| 1036 | 773 | 314 | 150 | 1,9-dioxo-16-hydroxy-17-methyl-1-hydroxymethyl-5-*cis*-13-*trans*-prostadiene,1-ethylene ketal |
| 1037 | 773 | 314 | 151 | 1,9-dioxo-16-hydroxy-17,20-dimethyl-1-hydroxymethyl-5-*cis*-13-*trans*-prostadiene,1-ethylene ketal |
| 1038 | 773 | 314 | 152 | 1,9-dioxo-16-hydroxy-1-hydroxymethyl-13-*trans*-17-*trans*-5-*cis*-prostatriene,1-ethylene ketal |
| 1039 | 773 | 314 | 153 | 1,9-dioxo-16(R)-hydroxy-1-hydroxymethyl-5-*cis*-13-*trans*-prostadiene,1-ethylene ketal |
| 1040 | 773 | 314 | 154 | 1,9-dioxo-16(S)-hydroxy-1-hydroxymethyl-5-*cis*-13-*trans*-prostadiene,1-ethylene ketal |
| 1041 | 773 | 314 | 154a | 1,9-dioxo-16-hydroxy-20-ethyl-1-hydroxymethyl-5-*cis*-13-*trans*-17-*trans*-prostatriene,1-ethylene ketal |
| 1042 | 773 | 314 | 1-iodo-3-triphenylmethoxy-1-*trans*-octene (U.S. Pat. No. 3,873,607). | 1,9-dioxo-15α-hydroxy-1-hydroxymethyl-5-*cis*-13-*trans*-prostadiene,1-ethylene ketal |
| 1043 | 773 | 314 | 1-iodo-3-triphenylmethoxy-1-*trans*-nonene (U.S. Pat. No. 3,873,607). | 1,9-dioxo-15α-hydroxy-20-methyl-1-hydroxymethyl-5-*cis*-13-*trans*-prostadiene,1-ethylene ketal |
| 1044 | 773 | 314 | 1-iodo-3-triphenylmethoxy-1-*trans*-decene (U.S. Pat. No. 3,873,607). | 1,9-dioxo-15α-hydroxy-20-ethyl-1-hydroxymethyl-5-*cis*-13-*trans*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1045 | 773 | 314 | 1-iodo-3-triphenylmethoxy-4,4-dimethyl-1-*trans*-octene (U.S. Pat. No. 3,873,607). | 1,9-dioxo-15α-hydroxy-16,16-dimethyl-1-hydroxymethyl-5-*cis*-13-*trans*-prostadiene,1-ethylene ketal |
| 1046 | 773 | 314 | 1-iodo-3-triphenylmethoxy-5,5-dimethyl-1-*trans*-octene (U.S. Pat. No. 3,873,607). | 1,9-dioxo-15α-hydroxy-17,17-dimethyl-1-hydroxymethyl-5-*cis*-13-*trans*-prostadiene,1-ethylene ketal |
| 1047 | 773 | 314 | 1-iodo-3-triphenylmethoxy-4-methyl-1-*trans*-octene (U.S. Pat. No. 3,876,690). | 1,9-dioxo-15α-hydroxy-16-methyl-1-hydroxymethyl-5-*cis*-13-*trans*-prostadiene,1-ethylene ketal |
| 1048 | 773 | 314 | 1-iodo-3-triphenylmethoxy-4-ethyl-1-*trans*-octene (U.S. Pat. No. 3,876,690). | 1,9-dioxo-15α-hydroxy-16-ethyl-1-hydroxymethyl-5-*cis*-13-*trans*-prostadiene,1-ethylene ketal |
| 1049 | 773 | 314 | 1-iodo-3-triphenylmethoxy-4-cyclopentyl-1-*trans*-butene (U.S. Pat. No. 3,884,969). | 1,9-dioxo-15α-hydroxy-17,20-tetranor-16-cyclopentyl-1-hydroxymethyl-5-*cis*-13-*trans*-prostadiene,1-ethylene ketal |
| 1050 | 773 | 314 | 1-iodo-3-triphenylmethoxy-3-cyclohexyl-1-*trans*-propene (U.S. Pat. No. 3,884,969). | 1,9-dioxo-15α-hydroxy-16,20-pentanor-15-cyclohexyl-1-hydroxymethyl-5-*cis*-13-*trans*-prostadiene,1-ethylene ketal |
| 1051 | 773 | 314 | 1-iodo-3-triphenylmethoxy-5-cyclohexyl-1-*trans*-pentene (U.S. Pat. No. 3,884,969). | 1,9-dioxo-15α-hydroxy-18,20-trinor-17-cyclohexyl-1-hydroxymethyl-5-*cis*-13-*trans*-prostadiene,1-ethylene ketal |
| 1052 | 773 | 314 | 1-iodo-3-triphenylmethoxy-6-cyclopentyl-1-*trans*-hexene (U.S. Pat. No. 3,884,969). | 1,9-dioxo-15α-hydroxy-19,20-dinor-18-cyclopentyl-1-hydroxymethyl-5-*cis*-13-*trans*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1053 | 773 | 314 | 1-iodo-3-methyl-3-trimethylsilyloxy-*trans*-1-octene (Example 190) | 1,9-dioxo-15α-hydroxy-15-methyl-1-hydroxymethyl-5-*cis*-13-*trans*-prostadiene,1-ethylene ketal |
| 1054 | 773 | 314 | 1-iodo-3-methyl-3-trimethylsilyloxy-*trans*-1-decene (Example 190a) | 1,9-dioxo-15α-hydroxy-15-methyl-20-ethyl-1-hydroxymethyl-5-*cis*-13-*trans*-prostadiene,1-ethylene ketal |
| 1055 | 774 | 314 | 159 | 1,9-dioxo-15α-hydroxy-17,18,19,20-tetranor-16-*p*-fluorophenoxy-1-hydroxymethyl-5-*cis*-13-*trans*-prostadiene,1-ethylene ketal |
| 1056 | 774 | 314 | 186 | 1,9-dioxo-15α-hydroxy-17,18,19,20-tetranor-16-phenoxy-1-hydroxymethyl-5-*cis*-13-*trans*-prostadiene,1-ethylene ketal |
| 1057 | 774 | 314 | 181 | 1,9-dioxo-15α-hydroxy-17,18,19,20-tetranor-16-*p*-bromophenoxy-1-hydroxymethyl-5-*cis*-13-*trans*-prostadiene,1-ethylene ketal |
| 1059 | 774 | 314 | 183 | 1,9-dioxo-15α-hydroxy-17,18,19,20-tetranor-16-*p*-methoxyphenoxy-1-hydroxymethyl-5-*cis*-13-*trans*-prostadiene,1-ethylene ketal |
| 1060 | 774 | 314 | 184 | 1,9-dioxo-15α-hydroxy-17,18,19,20-tetranor-16-*p*-chlorophenoxy-1-hydroxymethyl-5-*cis*-13-*trans*-prostadiene,1-ethylene ketal |
| 1061 | 774 | 314 | 180e | 1,9-dioxo-15α-hydroxy-17,18,19,20-tetranor-*m*-trifluorophenoxy-1-hydroxymethyl-5-*cis*-13-*trans*-prostadiene,1-ethylene ketal |
| 1062 | 774 | 314 | 185 | 1,9-dioxo-15α-hydroxy-17,18,19,20-tetranor-16-(3,4-dichlorophenoxy)-1-hydroxymethyl-5-*cis*-13-*trans*-prostadiene,1-ethylene ketal |
| 1063 | 774 | 314 | 186b | 1,9-dioxo-15α-hydroxy-18,19,20-trinor-17-phenyl-1-hydroxymethyl-5-*cis*-13-*trans*-prostadiene,1-ethylene ketal |
| 1064 | 774 | 314 | 186e | 1,9-dioxo-15α-hydroxy-18,19,20-trinor-17-(*m*-trifluoromethylphenyl)-1-hydroxymethyl-5-*cis*-13-*trans*-prostadiene,1-ethylene ketal |
| 1065 | 774 | 314 | 186d | 1,9-dioxo-15α-hydroxy-18,19,20-trinor-17-(*p*-methoxyphenyl)-1-hydroxymethyl-5-*cis*-13-*trans*-prostadiene,1-ethylene ketal |
| 1066 | 773 | 315 | 1-iodo-3-triphenylmethoxy-1-*trans*-octene (U.S. Pat. No. 3,873,607). | 1,9-dioxo-15α-hydroxy-1-hydroxymethyl-2-nor-5-*cis*-13-*trans*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1067 | 773 | 315 | 190 | 1,9-dioxo-15α-hydroxy-15-methyl-1-hydroxymethyl-2-nor-5-*cis*-13-*trans*-prostadiene,1-ethylene ketal |
| 1068 | 773 | 315 | 1-iodo-3-triphenylmethoxy-5,5-dimethyl-octene (U.S. Pat. No. 3,873,607). | 1,9-dioxo-15α-hydroxy-17,17-dimethyl-1-hydroxymethyl-2-nor-5-*cis*-13-*trans*-prostadiene,1-ethylene ketal |
| 1069 | 773 | 315 | 13 | 1,9-dioxo-15α-hydroxy-16,16-trimethylene-1-hydroxymethyl-2-nor-5-*cis*-13-*trans*-prostadiene,1-ethylene ketal |
| 1070 | 773 | 315 | 76 | *dl-erythro*-1,9-dioxo-15α,16-dihydroxy-1-hydroxymethyl-2-nor-5-*cis*-13-*trans*-prostadiene,1-ethylene ketal |
| 1071 | 773 | 315 | 69 | *dl-threo*-1,9-dioxo-15α,16-dihydroxy-1-hydroxymethyl-2-nor-5-*cis*-13-*trans*-prostadiene,1-ethylene ketal |
| 1072 | 773 | 315 | 74 | *dl-erythro*-1,9-dioxo-15α-hydroxy-16-methoxy-1-hydroxymethyl-2-nor-5-*cis*-13-*trans*-prostadiene,1-ethylene ketal |
| 1073 | 773 | 315 | 107 | 1,9-dioxo-16-hydroxy-1-hydroxymethyl-2-nor-5-*cis*-13-*trans*-prostadiene,1-ethylene ketal |
| 1074 | 773 | 315 | 135 | 1,9-dioxo-16-hydroxy-16-methyl-1-hydroxymethyl-2-nor-5-*cis*-13-*trans*-prostadiene,-1-ethylene ketal |
| 1075 | 773 | 315 | 134 | 1,9-dioxo-16-hydroxy-16-methyl-1-hydroxymethyl-2-nor-5-*cis*-13-*trans*-17-*trans*-prostatriene,1-ethylene ketal |
| 1076 | 774 | 315 | 186 | 1,9-dioxo-15α-hydroxy-2,17,18,19,20-pentanor-16-phenoxy-5-*cis*-13-*trans*-prostadiene,1-ethylene ketal |
| 1077 | 774 | 315 | 180e | 1,9-dioxo-15α-hydroxy-2,17,18,19,20-pentanor-16-*m*-trifluoromethylphenoxy-1-hydroxy-5-*cis*-13-*trans*-prostadiene,1-ethylene ketal |
| 1078 | 774 | 315 | 194 | 1,9-dioxo-16-hydroxy-16-vinyl-1-hydroxymethyl-2-nor-5-*cis*-13-*trans*-prostadiene,-1-ethylene ketal |
| 1079 | 774 | 315 | 198 | 1,9-dioxo-16-hydroxy-16-cyclopropyl-1-hydroxymethyl-2-nor-5-*cis*-13-*trans*-prostadiene,1-ethylene ketal |
| 1080 | 774 | 315 | 186b | 1,9-dioxo-15α-hydroxy-2,18,19,20-tetranor-17-phenyl-1-hydroxymethyl-5-*cis*-13-*trans*-prostadiene,1-ethylene ketal |

TABLE XLIV (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1081 | 773 | 315 | 186e | 1,9-dioxo-15α-hydroxy-2,18,19,20-tetranor-17-(*m*-trifluorophenyl)-1-hydroxymethyl-5-*cis*-13-*trans*-prostadiene,1-ethylene ketal |
| 1082 | 773 | 315 | 186d | 1,9-dioxo-15α-hydroxy-2,18,19,20-tetranor-17-(*p*-methoxyphenyl)-1-hydroxymethyl-5-*cis*-13-*trans*-prostadiene,1-ethylene ketal |
| 1082A* | 774 | 314 | 210I | 1,9-dioxo-16-hydroxy-16-ethynyl-1-hydroxymethyl-5-*cis*,13-*trans*-prostadiene,1-ethylene ketal |
| 1082B* | 774 | 314 | 210P | 1,9-dioxo-16-hydroxy-16-ethynyl-1-hydroxymethyl-20-nor-5-*cis*-13-*trans*-prostadiene,1-ethylene ketal |
| 1082C* | 774 | 314 | 210Q | 1,9-dioxo-16-hydroxy-16-ethynyl-1-hydroxymethyl-20-ethyl-5-*cis*-13-*trans*-prostadiene,-1-ethylene ketal |
| 1082D | 773 | 314 | 154I | 1,9-dioxo-16-hydroxy-16-(1-propynyl)-hydroxymethyl-5-*cis*-13-*trans*-prostadiene,1-ethylene ketal |
| 1082E | 773 | 314 | 154J | 1,9-dioxo-16-hydroxy-16-(1-propynyl)-hydroxymethyl-20-ethyl-5-*cis*-13-*trans*-prostadiene,-1-ethylene ketal |

*For cleavage of the TMS—C≡C— linkage see, Example 772(B).

Examples 1083—1105

By the methods described hereinabove in Examples 773 and 774, the 1,9-dioxo-1-hydroxymethyl prostene derivative shown in Table XLV are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XLV. It should be understood that the other diastereoisomer is also formed which in its *nat* and *ent* forms has an opposite (mirror image) configuration at the asymmetric carbon atoms on the β-chain (the chain containing $C_{13}$ .... $C_{14}$ etc.) to that of the respective *nat* and *ent* forms of the listed diastereoisomer; both of these diastereoisomers are claimed in this invention as well as their component enantiomers.

[There is no Example 1086]

TABLE XLV

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 1083 | 773 | 314 | 246 | *nat*-(and *ent*)-1,9-dioxo-15-hydroxy-15,16-tetramethylene-17,18,19,20-tetranor-1-hydroxymethyl-5-*cis*-13-*trans*-prostadiene, 1-ethylene ketal |
| 1084 | 774 | 314 | 245 | *nat*-(and *ent*)-1,9-dioxo-16-hydroxy-16,17-tetramethylene -18,19,20-trinor-1-hydroxymethyl-5-*cis*-13-*trans*-prostadiene,1-ethylene ketal |
| 1085 | 773 | 314 | 243 | *nat*-15S,16R-(and *ent*-15R,16S)-1,9-dioxo-15-hydroxy-15,16-trimethylene-1-hydroxymethyl-5-*cis*-13-*trans*-prostadiene,1-ethylene ketal |
| 1087 | 773 | 314 | 244 | *nat*-15S,16S-(and *ent*-15R,16R)-1,9-dioxo-15-hydroxy-15,16-trimethylene-1-hydroxymethyl-13-*trans*-5-*cis*-prostadiene,1-ethylene ketal |
| 1088 | 773 | 314 | 247 | *nat*-(and *ent*)-1,9-dioxo-15-hydroxy-15,16-trimethylene-17,18,19,20-tetranor-1-hydroxymethyl-13-*trans*-5-*cis*-prostadiene, -1-ethylene ketal |
| 1089 | 774 | 314 | 248 | *nat*-(and *ent*)-1,9-dioxo-15-hydroxy-15,16-pentamethylene-17,18,19,20-tetranor-1-hydroxymethyl-13-*trans*-5-*cis*-prostadiene,1-ethylene ketal |
| 1090 | 773 | 314 | 249 | *nat*-15S,17R-(and *ent*-15R,17S)-1,9-dioxo-15-hydroxy-15,17-dimethylene-1-hydroxymethyl-13-*trans*-5-*cis*-prostadiene,1-ethylene ketal |
| 1091 | 773 | 314 | 250 | *nat*-15S,17S-(and *ent*-15R,17R)-1,9-dioxo-15-hydroxy-15,17-dimethylene-1-hydroxymethyl-13-*trans*-5-*cis*-prostadiene,1-ethylene ketal |
| 1092 | 773 | 314 | 252 | *nat*-15S,16R-(and *ent*-15R,16S)-1,9-dioxo-15-hydroxy-15,16-tetramethylene-1-hydroxymethyl-13-*trans*-5-*cis*-prostadiene,1-ethylene ketal |
| 1093 | 773 | 314 | 253 | *nat*-15S,16S-(and *ent*-15R,16R)-1,9-dioxo-15-hydroxy-15,16-tetramethylene-1-hydroxymethyl-13-*trans*-5-*cis*-prostadiene,1-ethylene ketal |
| 1094 | 774 | 314 | 254 | *nat*-15R,16S-(and *ent*-15S,16R)-1,9-dioxo-15-hydroxy-15,16-trimethylene-16-(3-trifluoromethylphenoxy)-17,18,19,20-tetranor-1-hydroxymethyl-13-*trans*-5-*cis*-prostadiene,1-ethylene ketal |
| 1095 | 774 | 314 | 255 | *nat*-15R,16R-(and *ent*-15S,16S)-1,9-dioxo-15-hydroxy-15,16-trimethylene-16-(3-trifluoromethylphenoxy)-17,18,19,20-tetranor-1-hydroxymethyl-13-*trans*-5-*cis*-prostadiene,1-ethylene ketal |

TABLE XLV (Continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 1096 | 774 | 314 | 256 | *nat*-15R,16S-(and *ent*-15S,16R)-1,9-dioxo-15-hydroxy-15,16-trimethylene-16-(4-fluorophenoxy)-17,18,19,20-tetranor-1-hydroxymethyl-13-*trans*-5-*cis*-prostadiene,1-ethylene ketal |
| 1097 | 774 | 314 | 257 | *nat*-15R,16R-(and *ent*-15S,16S)-1,9-dioxo-15-hydroxy-15,16-trimethylene-16-(4-fluorophenoxy)-17,18,19,20-tetranor-1-hydroxymethyl-13-*trans*-5-*cis*-prostadiene,1-ethylene ketal |
| 1098 | 774 | 314 | 258 | *nat*-15R,16S-(and *ent*-15S,16R)-1,9-dioxo-15-hydroxy-15,16-trimethylene-16-(3-chlorophenoxy)-17,18,19,20-tetranor-1-hydroxymethyl-13-*trans*-5-*cis*-prostadiene,1-ethylene ketal |
| 1099 | 774 | 314 | 259 | *nat*-15R,16R-(and *ent*-15S,16S)-1,9-dioxo-15-hydroxy-15,16-trimethylene-16-(3-chlorophenoxy)-17,18,19,20-tetranor-1-hydroxymethyl-13-*trans*-5-*cis*-prostadiene, 1-ethylene ketal |
| 1100 | 773 | 314 | 260 | *nat*-15S,17R-(and *ent*-15R,17S)-1,9-dioxo-15-hydroxy-15,17-trimethylene-19,20-dinor-1-hydroxymethyl-13-*trans*-5-*cis*-prostadiene,1-ethylene ketal |
| 1101 | 773 | 314 | 261 | *nat*-15S,17S-(and *ent*-15R,17R)-1,9-dioxo-15-hydroxy-15,17-trimethylene-19,20-dinor-1-hydroxymethyl-13-*trans*-5-*cis*-prostadiene,1-ethylene ketal |
| 1102 | 774 | 314 | 262 | *nat*-16R,17S-(and *ent*-16S,17R)-1,9-dioxo-16-hydroxy-16,17-trimethylene-20-methyl-1-hydroxymethyl-13-*trans*-5-*cis*-prostadiene,1-ethylene ketal |
| 1103 | 774 | 314 | 263 | *nat*-16R,17R-(and *ent*-16S,17S)-1,9-dioxo-16-hydroxy-16,17-trimethylene-20-methyl-1-hydroxymethyl-13-*trans*-5-*cis*-prostadiene,1-ethylene ketal |
| 1104 | 774 | 314 | 264 | *nat*-16R,17S-(and *ent*-16S,17R)-1,9-dioxo-16-hydroxy-16,17-trimethylene-16-(3-trifluoromethylphenoxy)-18,19,20-trinor-1-hydroxymethyl-13-*trans*-5-*cis*-prostadiene,1-ethylene ketal |
| 1105 | 774 | 314 | 265 | *nat*-16R,17R-(and *ent*-16S,17S)-1,9-dioxo-16-hydroxy-16,17-trimethylene-16-(3-trifluoromethylphenoxy)-18,19,20-trinor-1-hydroxymethyl-13-*trans*-5-*cis*-prostadiene,1-ethylene ketal |

Examples 1106—1189E

By the methods described hereinabove in Examples 773 and 774, the 1,9-dioxo-1-hydroxymethylprostene derivatives shown in Table XLVI are prepared by the indicated method from the indicated vinyl iodide or vinyl tin compound and the indicated cyclopent-2-en-1-one.

In those cases where isomers are obtained at the $C_{15}$ or $C_{16}$ positions, only the $C_{15}$ or $C_{16}$-normal isomers are listed in Table XLVI; it should be understood that the corresponding $C_{15}$ or $C_{16}$-*epi* isomers are also formed and are part of this invention.

In those cases where the initial conjugate-addition product contains a triphenylmethoxy blocking group, deblocking is conducted in acetic acid-tetrahydrofuran-water 4:2:1 at 50°C for 5 hours.

[There are no Examples 1119, 1120, 1126, 1127, 1129, 1130, 1136 and 1165].

TABLE XLVI

| Example | Method of Example | Cyclopent-2-en-1-one | Vinyl Iodide or Vinyl Tin of Example | 1,9-dioxo-1-hydroxymethylprostene |
|---|---|---|---|---|
| 1106 | 773 | 283 | 49 | 1,9-dioxo-15-hydroxy-1-hydroxymethyl-16,-16-trimethylene-20-nor-13-*trans*-prostadiene,1-ethylene ketal |
| 1107 | 773 | 283 | 50 | 1,9-dioxo-15-hydroxy-1-hydroxymethyl-16,-16-trimethylene-20-methyl-13-*trans*-prostadiene,1-ethylene ketal |
| 1108 | 773 | 283 | 51 | 1,9-dioxo-15-hydroxy-1-hydroxymethyl-16,-16-trimethylene-20-ethyl-13-*trans*-prostadiene,1-ethylene ketal |
| 1109 | 774 | 283 | 205 | 1,9-dioxo-16-hydroxy-16-cyclopropyl-20-ethyl-1-hydroxymethyl-13-*trans*-prostene, 1-ethylene ketal |
| 1110 | 774 | 283 | 207 | 1,9-dioxo-16-hydroxy-16-vinyl-20-ethyl-1-hydroxymethyl-13-*trans*-prostene,1-ethylene ketal |
| 1111 | 774 | 283 | 198 | 1,9-dioxo-16-hydroxy-16-cyclopropyl-1-hydroxymethyl-13-*trans*-prostene,1-ethylene ketal |
| 1112 | 773 | 283 | 13 | 1,9-dioxo-15-hydroxy-1-hydroxymethyl-16,-16-trimethylene-13-*trans*-prostene,1-ethylene ketal |
| 1113 | 774 | 283 | 210a | 1,9-dioxo-16-hydroxy-1-hydroxymethyl-16-vinyl-13-*trans*-prostene,1-ethylene ketal |
| 1114 | 773 | 283 | 74 | *dl-erythro*-1,9-dioxo-15-hydroxy-16-methoxy-1-hydroxymethyl-13-*trans*-prostene,1-ethylene ketal |
| 1115 | 773 | 283 | 77 | *dl-erythro*-1,9-dioxo-15-hydroxy-16-ethoxy-1-hydroxymethyl-13-*trans*-prostene, 1-ethylene ketal |
| 1116 | 773 | 283 | 83 | *dl-erythro*-1,9-dioxo-15,16-dihydroxy-1-hydroxymethyl-20-nor-13-*trans*-prostene, 1-ethylene ketal |
| 1117 | 773 | 283 | 84 | *dl-erythro*-1,9-dioxo-15,16-dihydroxy-1-hydroxymethyl-20-methyl-13-*trans*-prostene,1-ethylene ketal |

TABLE XLVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1118 | 773 | 283 | 85 | *dl-erythro*-1,9-dioxo-15,16-dihydroxy-1-hydroxymethyl-20-ethyl-13-*trans*-prostene,1-ethylene ketal |
| 1121 | 773 | 283 | 69 | *dl-threo*-1,9-dioxo-15,16-dihydroxy-1-hydroxymethyl-13-*trans*-prostene,1-ethylene ketal |
| 1122 | 773 | 283 | 88 | *dl-threo*-1,9-dioxo-15,16-dihydroxy-20-nor-1-hydroxymethyl-13-*trans*-prostene, 1-ethylene ketal |
| 1123 | 773 | 283 | 76 | *dl-erythro*-1,9-dioxo-15,16-dihydroxy-1-hydroxymethyl-13-*trans*-prostene,1-ethylene ketal |
| 1124 | 773 | 283 | 89 | *dl-threo*-1,9-dioxo-15,16-dihydroxy-20-methyl-1-hydroxymethyl-13-*trans*-prostene,1-ethylene ketal |
| 1125 | 773 | 283 | 90 | *dl-threo*-1,9-dioxo-15,16-dihydroxy-20-ethyl-1-hydroxymethyl-13-*trans*-prostene,1-ethylene ketal |
| 1128 | 773 | 283 | 107 | 1,9-dioxo-16-hydroxy-1-hydroxymethyl-13-*trans*-prostene,1-ethylene ketal |
| 1131 | 773 | 283 | 110 | 1,9-dioxo-16-hydroxy-1-hydroxymethyl-20-nor-13-*trans*-prostene,1-ethylene ketal |
| 1132 | 773 | 283 | 144 | 1,9-dioxo-16-hydroxy-17-methyl-1-hydroxymethyl-13-*trans*-prostene,1-ethylene ketal |
| 1133 | 773 | 283 | 112 | 1,9-dioxo-16-hydroxy-20-methyl-1-hydroxymethyl-13-*trans*-prostene,1-ethylene ketal |
| 1134 | 773 | 283 | 113 | 1,9-dioxo-16-hydroxy-20-ethyl-1-hydroxymethyl-13-*trans*-prostene,1-ethylene ketal |
| 1135 | 773 | 283 | 139a | 1,9-dioxo-16-hydroxy-16-methyl-20-ethyl-1-hydroxymethyl-13-*trans*-prostene,1-ethylene ketal |
| 1137 | 773 | 283 | 135 | 1,9-dioxo-16-hydroxy-16-methyl-1-hydroxymethyl-13-*trans*-prostene, 1-ethylene ketal |
| 1138 | 773 | 283 | 136 | 1,9-dioxo-16-hydroxy-17,17,20-trimethyl-1-hydroxymethyl-13-*trans*-prostene,1-ethylene ketal |
| 1139 | 773 | 283 | 137 | 1,9-dioxo-16-hydroxy-16,20-dimethyl-1-hydroxymethyl-13-*trans*-prostene,1-ethylene ketal |
| 1140 | 773 | 283 | 138 | 1,9-dioxo-16-hydroxy-17,17-dimethyl-1-hydroxymethyl-13-*trans*-prostene,1-ethylene ketal |

TABLE XLVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1141 | 773 | 283 | 139 | 1,9-dioxo-16-hydroxy-16-methyl-1-hydroxymethyl-13-*trans*-17-*trans*-prostadiene,1-ethylene ketal |
| 1142 | 773 | 283 | 149 | 1,9-dioxo-16-hydroxy-20-methyl-1-hydroxymethyl-13-*trans*-17-*trans*-prostadiene,1-ethylene ketal |
| 1143 | 773 | 283 | 150 | 1,9-dioxo-16-hydroxy-17-methyl-1-hydroxymethyl-13-trans-prostene,1-ethylene ketal |
| 1144 | 773 | 283 | 151 | 1,9-dioxo-16-hydroxy-17,20-dimethyl-1-hydroxymethyl-13-*trans*-prostene,1-ethylene ketal |
| 1145 | 773 | 283 | 152 | 1,9-dioxo-16-hydroxy-1-hydroxymethyl-13-*trans*-17-*trans*-prostadiene,1-ethylene ketal |
| 1146 | 773 | 283 | 153 | 1,9-dioxo-16(R)-hydroxy-1-hydroxymethyl-13-*trans*-prostene,1-ethylene ketal |
| 1147 | 773 | 283 | 154 | 1,9-dioxo-16(S)-hydroxy-1-hydroxymethyl-13-*trans*-prostene,1-ethylene ketal |
| 1148 | 773 | 283 | 154a | 1,9-dioxo-16-hydroxy-20-ethyl-1-hydroxymethyl-13-*trans*-17-*trans*-prostadiene,1-ethylene ketal |
| 1149 | 773 | 283 | 1-iodo-3-triphenylmethoxy-1-*trans*-octene (U.S. Pat. No. 3,873,607). | 1,9-dioxo-15α-hydroxy-1-hydroxymethyl-13-*trans*-prostadiene,1-ethylene ketal |
| 1150 | 773 | 283 | 1-iodo-3-triphenylmethoxy-1-*trans*-nonene (U.S. Pat. No. 3,873,607). | 1,9-dioxo-15α-hydroxy-20-methyl-1-hydroxymethyl-13-*trans*-prostene,1-ethylene ketal |
| 1151 | 773 | 283 | 1-iodo-3-triphenylmethoxy-1-*trans*-decene (U.S. Pat. No. 3,873,607). | 1,9-dioxo-15α-hydroxy-20-ethyl-1-hydroxymethyl-13-*trans*-prostene,1-ethylene ketal |
| 1152 | 773 | 283 | 1-iodo-3-triphenylmethoxy-4,4-dimethyl-1-*trans*-octene (U.S. Pat. No.3,873,607). | 1,9-dioxo-15α-hydroxy-16,16-dimethyl-1-hydroxymethyl-13-*trans*-prostene,1-ethylene ketal |
| 1153 | 773 | 283 | 1-iodo-3-triphenylmethoxy-5,5-dimethyl-1-*trans*-octene (U.S. Pat. No. 3,873,607). | 1,9-dioxo-15α-hydroxy-17,17-dimethyl-1-hydroxymethyl-13-*trans*-prostene,1-ethylene ketal |

TABLE XLVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1154 | 773 | 283 | 1-iodo-3-triphenylmethoxy-4-methyl-1-*trans*-octene (U.S. Pat. No. 3,876,690). | 1,9-dioxo-15α-hydroxy-16-methyl-1-hydroxymethyl-13-*trans*-prostene,1-ethylene ketal |
| 1155 | 773 | 283 | 1-iodo-3-triphenylmethoxy-4-ethyl-1-*trans*-octene (U.S. Pat. No. 3,876,690). | 1,9-dioxo-15α-hydroxy-16-ethyl-1-hydroxymethyl-13-*trans*-prostene,1-ethylene ketal |
| 1156 | 773 | 283 | 1-iodo-3-triphenylmethoxy-4-cyclopentyl-1-*trans*-butene (U.S. Pat. No. 3,884,969). | 1,9-dioxo-15α-hydroxy-17,20-tetranor-16-cyclopentyl-1-hydroxymethyl-13-*trans*-prostene,1-ethylene ketal |
| 1157 | 773 | 283 | 1-iodo-3-triphenylmethoxy-3-cyclohexyl-1-*trans*-propene (U.S. Pat. No. 3,884,969). | 1,9-dioxo-15α-hydroxy-16,20-pentanor-15-cyclohexyl-1-hydroxymethyl-13-*trans*-prostene,1-ethylene ketal |
| 1158 | 773 | 283 | 1-iodo-3-triphenylmethoxy-5-cyclohexyl-1-*trans*-pentene (U.S. Pat. No. 3,884,969). | 1,9-dioxo-15α-hydroxy-18,20-trinor-17-cyclohexyl-1-hydroxymethyl-13-*trans*-prostene,1-ethylene ketal |
| 1159 | 773 | 283 | 1-iodo-3-triphenylmethoxy-6-cyclopentyl-1-*trans*-hexene (U.S. Pat. No. 3,884,969). | 1,9-dioxo-15α-hydroxy-19,20-dinor-18-cyclopentyl-1-hydroxymethyl-13-*trans*-prostene,1-ethylene ketal |
| 1160 | 773 | 283 | 1-iodo-3-methyl-3-trimethylsilyloxy-*trans*-1-octene (Example 190). | 1,9-dioxo-15α-hydroxy-15-methyl-1-hydroxymethyl-13-*trans*-prostene,1-ethylene ketal |
| 1161 | 773 | 283 | 1-iodo-3-methyl-3-trimethylsilyloxy-*trans*-1-decene (Example 190a). | 1,9-dioxo-15α-hydroxy-15-methyl-20-ethyl-1-hydroxymethyl-13-*trans*-prostene, 1-ethylene ketal |
| 1162 | 774 | 283 | 159 | 1,9-dioxo-15α-hydroxy-17,18,19,20-tetranor-16-*p*-fluorophenoxy-1-hydroxymethyl-13-*trans*-prostene,1-ethylene ketal |
| 1163 | 774 | 283 | 186 | 1,9-dioxo-15α-hydroxy-17,18,19,20-tetranor-16-phenoxy-1-hydroxymethyl-13-*trans*-prostene,1-ethylene ketal |
| 1164 | 774 | 283 | 181 | 1,9-dioxo-15α-hydroxy-17,18,19,20-tetranor-16-*p*-bromophenoxy-1-hydroxymethyl-13-*trans*-prostene,1-ethylene ketal |

TABLE XLVI (continued)

| Example | Method of Example | Cyclopent-2-en-1-one | Vinyl Iodide or Vinyl Tin of Example. | 1,9-dioxo-1-hydroxymethylprostene |
|---|---|---|---|---|
| 1166 | 774 | 283 | 183 | 1,9-dioxo-15α-hydroxy-17,18,19,20-tetra-nor-16-*p*-methoxyphenoxy-1-hydroxymethyl-13-*trans*-prostene,1-ethylene ketal |
| 1167 | 774 | 283 | 184 | 1,9-dioxo-15α-hydroxy-17,18,19,20-tetra-nor-16-*p*-chlorophenoxy-1-hydroxymethyl-13-*trans*-prostene,1-ethylene ketal |
| 1168 | 774 | 283 | 180e | 1,9-dioxo-15α-hydroxy-17,18,19,20-tetra-nor-*m*-trifluorophenoxy-1-hydroxymethyl-13-*trans*-prostene,1-ethylene ketal |
| 1169 | 774 | 283 | 185 | 1,9-dioxo-15α-hydroxy-17,18,19,20-tetra-nor-16-(3,4-dichlorophenoxy)-1-hydroxy-methyl-13-*trans*-prostene,1-ethylene ketal |
| 1170 | 774 | 283 | 186b | 1,9-dioxo-15α-hydroxy-18,19,20-trinor-17-phenyl-1-hydroxymethyl-13-*trans*-prost-ene,1-ethylene ketal |
| 1171 | 774 | 283 | 186e | 1,9-dioxo-15α-hydroxy-18,19,20-trinor-17-(*m*-trifluoromethylphenyl)-1-hydroxymethyl-13-*trans*-prostene,1-ethylene ketal |
| 1172 | 774 | 283 | 186d | 1,9-dioxo-15α-hydroxy-18,19,20-trinor-17-(*p*-methoxyphenyl)-1-hydroxymethyl-13-*trans*-prostene,1-ethylene ketal |
| 1173 | 773 | 310 | 1-iodo-3-tri-phenylmethoxy-1-*trans*-octene (U.S. Pat. No. 3,873,607). | 1,9-dioxo-15α-hydroxy-1-hydroxymethyl-2-nor-13-*trans*-prostene,1-ethylene ketal |
| 1174 | 773 | 310 | 190 | 1,9-dioxo-15α-hydroxy-15-methyl-1-hydr-oxymethyl-2-nor-13-*trans*-prostene,1-eth-ylene ketal |
| 1175 | 773 | 310 | 1-iodo-3-triphen-ylmethoxy-5,5-di-methyl-octene (U.S. Pat. No. 3,873,607). | 1,9-dioxo-15α-hydroxy-17,17-dimethyl-1-hydroxymethyl-2-nor-13-*trans*-prostene, 1-ethylene ketal |
| 1176 | 773 | 310 | 13 | 1,9-dioxo-15α-hydroxy-16,16-trimethylene-1-hydroxymethyl-2-nor-13-*trans*-prostene, 1-ethylene ketal |
| 1177 | 773 | 310 | 76 | *dl-erythro*-1,9-dioxo-15α,16-dihydroxy-1-hy-droxymethyl-2-nor-13-*trans*-prostene,1-ethylene ketal |
| 1178 | 773 | 310 | 69 | *dl-threo*-1,9-dioxo-15α,16-dihydroxy-1-hydroxymethyl-2-nor-13-*trans*-prostene, 1-ethylene ketal |
| 1179 | 773 | 310 | 74 | *dl-erythro*-1,9-dioxo-15α-hydroxy-16-methoxy-1-hydroxymethyl-2-nor-13-*trans*-prostene,1-ethylene ketal |

TABLE XLVI (continued)

| Example | Method of Example | Cyclopent-2-en-1-one | Vinyl Iodide or Vinyl Tin of Example | 1,9-dioxo-1-hydroxymethylprostene |
|---|---|---|---|---|
| 1180 | 773 | 310 | 107 | 1,9-dioxo-16-hydroxy-1-hydroxymethyl-2-nor-13-*trans*-prostene,1-ethylene ketal |
| 1181 | 773 | 310 | 135 | 1,9-dioxo-16-hydroxy-16-methyl-1-hydroxy-methyl-2-nor-13-*trans*-prostene,1-ethylene ketal |
| 1182 | 773 | 310 | 134 | 1,9-dioxo-16-hydroxy-16-methyl-1-hydroxy-methyl-2-nor-13-*trans*-17-*trans*-prostadi-ene,1-ethylene ketal |
| 1183 | 774 | 310 | 186 | 1,9-dioxo-15α-hydroxy-2,17,18,19,20-pentanor-16-phenoxy-1-hydroxymethyl-13-*trans*-prostene,1-ethylene ketal |
| 1184 | 774 | 310 | 180e | 1,9-dioxo-15α-hydroxy-2,17,18,19,20-pentanor-16-m-trifluoromethylphenoxy-1-hydroxymethyl-13-*trans*-prostene,1-ethylene ketal |
| 1185 | 773 | 310 | 194 | 1,9-dioxo-16-hydroxy-16-vinyl-1-hydroxy-methyl-2-nor-13-*trans*-prostene,1-ethylene ketal |
| 1186 | 774 | 310 | 198 | 1,9-dioxo-16-hydroxy-16-cyclopropyl-1-hydroxymethyl-2-nor-13-*trans*-prostene, 1-ethylene ketal |
| 1187 | 774 | 310 | 186b | 1,9-dioxo-15α-hydroxy-2,18,19,20-tetranor-17-phenyl-1-hydroxymethyl-13-*trans*-pro-stene,1-ethylene ketal |
| 1188 | 773 | 310 | 186e | 1,9-dioxo-15α-hydroxy-2,18,19,20-tetranor-17-(*m*-trifluorophenyl)-1-hydroxymethyl-13-*trans*-prostene,1-ethylene ketal |
| 1189 | 773 | 310 | 186d | 1,9-dioxo-15α-hydroxy-2,18,19,20-tetranor 17-(*p*-methoxyphenyl)-1-hydroxymethyl-13 *trans*-prostene,1-ethylene ketal |
| 1189A* | 774 | 283 | 210l | 1,9-dioxo-16-hydroxy-16-ethynyl-1-hydroxymethyl-13-*trans*-prostene, 1-ethylene ketal |
| 1189B* | 774 | 283 | 210P | 1,9-dioxo-16-hydroxy-16-ethynyl-1-hydroxymethyl-20-nor-13-*trans*-prostene, 1-ethylene ketal |
| 1189C* | 774 | 283 | 210Q | 1,9-dioxo-16-hydroxy-16-ethynyl-1-hydroxymethyl-20-ethyl-13-*trans*-prostene,1-ethylene ketal |
| 1189D | 773 | 283 | 154I | 1,9-dioxo-16-hydroxy-16-(1-propynyl)-1-hydroxymethyl-13-*trans*-prostene, 1-ethylene ketal |
| 1189E | 773 | 283 | 154J | 1,9-dioxo-16-hydroxy-16-(1-propynyl)-1-hydroxymethyl-20-ethyl-13-*trans*-prostene, 1-ethylene ketal |

For cleavage of the TMS—C≡C — bond see, Example 772(B).

Examples 1190—1212

By the methods described hereinabove in Examples 773 and 774, the 1,9-dioxo-1-hydroxymethyl prostene derivatives shown in Table XLVII are prepared by the indicated method from the indicated vinyl iodide or vinyl tin compound and the indicated cyclopent-2-en-1-one.

In those cases where two diastereoisomers are formed in the conjugate-addition, only one of the diastereoisomers is listed in Table XLVII. It should be understood that the other diastereoisomer is also formed which in its *nat* and *ent* forms has an opposite (mirror image) configuration at the asymmetric carbon atoms on the β-chain (the chain containing $C_{13}$ . . . $C_{14}$ etc.) to that of the respective *nat* and *ent* forms of the listed diastereoisomer; both of these diastereoisomers are claimed in this invention as well as their component enantiomers.

[There is no Example 1193].

TABLE XLVII

| Example | Method of Example | Cyclopent-2-en-1-one of Example | Vinyl Iodine or Vinyl Tin of Example | 1,9-dioxo-1-hydroxymethylprostene and its diastereomer |
|---|---|---|---|---|
| 1190 | 773 | 283 | 246 | *nat*-(and *ent*)-1,9-dioxo-16-hydroxy-15,16-tetramethylene-17,18,19,20-tetranor-1-hydroxymethyl-13-*trans*-prostene,1-ethylene ketal |
| 1191 | 774 | 283 | 245 | *nat*-(and *ent*)-1,9-dioxo-16-hydroxy-16,17-tetramethylene-18,19,20-trinor-1-hydroxymethyl-13-*trans*-prostene,1-ethylene ketal |
| 1192 | 773 | 283 | 243 | *nat*-15S,16R-(and *ent*-15R,16S)-1,9-dioxo-15-hydroxy-15,16-trimethylene-1-hydroxymethyl-13-*trans*-prostene,1-ethylene ketal |
| 1194 | 773 | 283 | 244 | *nat*-15S,16S-(and *ent*-15R,16R)-1,9-dioxo-15-hydroxy-15,16-trimethylene-1-hydroxymethyl-13-*trans*-prostene,1-ethylene ketal |
| 1195 | 773 | 283 | 247 | *nat*-(and *ent*)-1,9-dioxo-15-hydroxy-15,16-trimethylene-17,18,19,20-tetranor-1-hydroxymethyl-13-*trans*-prostene,1-ethylene ketal |
| 1196 | 773 | 283 | 248 | *nat*-(and *ent*)-1,9-dioxo-15-hydroxy-15,16-pentamethylene-17,18,19,20-tetranor-1-hydroxymethyl-13-*trans*-prostene,1-ethylene ketal |
| 1197 | 773 | 283 | 249 | *nat*-15S,17R-(and *ent*-15R,17S)-1,9-dioxo-15-hydroxy-15,17-dimethylene-1-hydroxymethyl-13-*trans*-prostene,1-ethylene ketal |
| 1198 | 773 | 283 | 250 | *nat*-15S,17S-(and *ent*-15R,17R)-1,9-dioxo-15-hydroxy-15,17-dimethylene-1-hydroxymethyl-13-*trans*-prostene,1-ethylene ketal |
| 1199 | 773 | 283 | 252 | *nat*-15S,16R-(and *ent*-15R,16S)-1,9-dioxo-15-hydroxy-15,16-tetramethylene-1-hydroxymethyl-13-*trans*-prostene,1-ethylene ketal |
| 1200 | 773 | 283 | 253 | *nat*-15S,16S-(and *ent*-15R,16R)-1,9-dioxo-15-hydroxy-15,16-tetramethylene-1-hydroxymethyl-13-*trans*-prostene,1-ethylene ketal |

TABLE XLVII (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 1201 | 774 | 283 | 254 | *nat*-15R,16S-(and *ent*-15S,16R)-1,9-dioxo-15-hydroxy-15,16-trimethylene-16-(3-trifluoromethylphenoxy)-17,18,19,20-tetranor-1-hydroxymethyl-13-*trans*-prostene,1-ethylene ketal |
| 1202 | 774 | 283 | 255 | *nat*-15R,16R-(and *ent*-15S,16S)-1,9-dioxo-15-hydroxy-15,16-trimethylene-16-(3-trifluoromethylphenoxy)-17,18,19,20-tetranor-1-hydroxymethyl-13-*trans*-prostene,1-ethylene ketal |
| 1203 | 773 | 283 | 256 | *nat*-15R,16S-(and *ent*-15S,16R)-1,9-dioxo-15-hydroxy-15,16-trimethylene-16-(4-fluorophenoxy)-17,18,19,20-tetranor-1-hydroxymethyl-13-*trans*-prostene,1-ethylene ketal |
| 1204 | 773 | 283 | 257 | *nat*-15R,16R-(and *ent*-15S,16S)-1,9-dioxo-15-hydroxy-15,16-trimethylene-16-(4-fluorophenoxy)-17,18,19,20-tetranor-1-hydroxymethyl-13-*trans*-prostene,1-ethylene ketal |
| 1205 | 774 | 283 | 258 | *nat*-15R,16S-(and *ent*-15S,16R)-1,9-dioxo-15-hydroxy-15,16-trimethylene-16-(3-chlorophenoxy)-17,18,19,20-tetranor-1-hydroxymethyl-13-*trans*-prostene,1-ethylene ketal |
| 1206 | 774 | 283 | 259 | *nat*-15R,16R-(and *ent*-15S,16S)-1,9-dioxo-15-hydroxy-15,16-trimethylene-16-(3-chlorophenoxy)-17,18,19,20-tetranor-1-hydroxymethyl-13-*trans*-prostene,1-ethylene ketal |
| 1207 | 773 | 283 | 260 | *nat*-15S,17R-(and *ent*-15R,17S)-1,9-dioxo-15-hydroxy-15,17-trimethylene-19,20-dinor-1-hydroxymethyl-13-*trans*-prostene,1-ethylene ketal |
| 1208 | 773 | 283 | 261 | *nat*-15S,17S-(and *ent*-15R,17R)-1,9-dioxo-15-hydroxy-15,17-trimethylene-19,20-dinor-1-hydroxymethyl-13-*trans*-prostene,1-ethylene ketal |
| 1209 | 774 | 283 | 262 | *nat*-16R,17S-(and *ent*-16S,17R)-1,9-dioxo-16-hydroxy-16,17-trimethylene-20-methyl-1-hydroxymethyl-13-*trans*-prostene,1-ethylene ketal |
| 1210 | 774 | 283 | 263 | *nat*-16R,17R-(and *ent*-16S,17S)-1,9-dioxo-16-hydroxy-16,17-trimethylene-20-methyl-1-hydroxymethyl-13-*trans*-prostene,1-ethylene ketal |
| 1211 | 774 | 283 | 264 | *nat*-16R,17S-(and *ent*-16S,17R)-1,9-dioxo-16-hydroxy-16,17-trimethylene-16-(3-trifluoromethylphenoxy)-18,19,20-trinor-1-hydroxymethyl-13-*trans*-prostene,1-ethylene ketal |

TABLE XLVII (continued)

| Example | Method of Example | Cyclopent-2-en-1-one of Example | Vinyl Iodide or Vinyl Tin of Example | 1,9-dioxo-1-hydroxymethylprostene and its diastereomer |
|---|---|---|---|---|
| 1212 | 774 | 283 | 265 | *nat*-16R,17R-(and *ent*-16S,17S)-1,9-dioxo-16-hydroxy-16,17-trimethylene-16-(3-trifluoromethylphenoxy)-18,19,20-trinor-1-hydroxymethyl-13-*trans*-prostene,1-ethylene ketal |

Example 1213

1,9-Dioxo-11$\alpha$,16-dihydroxy-16-vinyl-1-acetoxymethyl-5-*cis*-13-*trans*-prostadiene

To a solution of 0.1 g of 1,9-dioxo-11$\alpha$,16-dihydroxy-16-vinyl-1-hydroxy-5-*cis*-13-*trans*-prostadiene in 0.75 ml of pyridine is added 0.026 g of acetic anhydride. After standing overnight, the pyridine is removed at reduced pressure. The residue is chromatographed on a dry column of silica gel eluting with benzene-ethylacetate 1:1 to give 0.049 g of the product.

In accordance with the above Example 1213 the 1-hydroxymethyl analogs of Examples 319—772AAA and are treated with acetic anhydride, propionic anhydride, *n*-butyric anhydride and *n*-valeric anhydride to give the respective 1-acetoxymethyl-1-propanoyloxy, 1-*n*-butanoyloxymethyl, and 1-*n*-pentanoyloxymethyl analogs.

Example 1214

Preparation of 1-oxo-9$\beta$,11$\alpha$,16-trihydroxy-1-hydroxymethyl-16-vinyl-13 *trans*-prostene and 1-oxo-9$\alpha$,11$\alpha$,16-trihydroxy-1-hydroxymethyl-16-vinyl-13-*trans*-prostene

To a solution of 2.9 g (5.6 mmol) of (E) 4-trimethylsilyloxy-4-vinyl-1-tri-*n*-butylstannyl-1-octene (Example 210A) in 4 ml of tetrahydrofuran at 78°C under argon with stirring is added 2.4 ml of 2.4M *n*-butyllithium in hexane. The solution is stirred at —30° to —20°C for 2 hours. A solution of .74 g (5.6 mmol) of copper pentyne and 2.3 ml of hexamethylphosphoroustriamide in 18 ml of ether is added at —78°C. The solution is stirred at —78°C for 1.5 hours. A solution of 2.0 g (5.2 mmol) of 1-(6-(4-methoxy-2,2-dimethyl-1,3-dioxolan-4-yl)hexyl)-4-(2-methoxypropyl-2-oxy)cyclopent-2-en-1-one in 20 ml of ether is added. The solution is stirred at —30° to —20°C for 1.5 hours. To the solution is added 100 ml of saturated ammonium chloride. The mixture is extracted with ether and the ether solution is washed with dilute hydrochloric acid, saturated sodium bicarbonate, and dried over magnesium sulfate. Dry solvent is removed and the residue is dissolved in 30 ml of ethanol and .37 g of sodium borohydride is added. The mixture is stirred for 8 hours. The solvent is removed. The residue is dissolved in 90 ml of acetic acid-tetrahydrofuran — water 4:2:1. The solution is stirred at room temperature for 2 hours. The solvents are removed at reduced pressure. The residue is chromatographed on silica gel to give 1-oxo-9$\beta$,11$\alpha$,16-trihydroxy-1-hydroxymethyl-16-vinyl-13-*trans*-prostene and 1-oxo-9$\alpha$, 11$\alpha$,16-trihydroxy-1-hydroxymethyl-16-vinyl-13-*trans*-prostene.

By the methods described hereinabove in Example 1214 the vinyl iodides or vinyl tin compounds used in Examples 319- 772AAA are exchanged with *t*-butyl lithium or *n*-butyllithium respectively to give the vinyl lithium reagents which on reaction with copper pentyne and hexamethyl phosphorous triamide give the cuprate reagents. Addition of the cyclopent-2-en-1-ones used in Examples 319—772GG at —78°C followed by warming to —20°C as described hereinabove in Example 1214 gives the 9-oxo analogs in which the 1-oxo feature is still protected. The reaction of these with sodium borohydride in ethanol followed by deblocking with acetic acid tetrahydrofuran-water 4:2:1 or with dilute hydrochloric acid in tetrahydrofuran gives 1-oxo-9$\beta$-hydroxy and 1-oxo-9$\alpha$-hydroxy analogs corresponding to the 1,9-dioxo analogs of Examples 319—772AAA.

Example 1215

Preparation of 1-oxo-9$\alpha$,11$\alpha$, 16-trihydroxy-1-hydroxymethyl-16-methyl-13-*trans*-prostene

To a solution of 3.4 (0.00675 mol) of (E) 4-trimethylsilyloxy-4-methyl-1-tri-*n*-butylstannyl-1-octene (Example 210b) in 3.5 ml of tetrahydrofuran at —78°C under argon, with stirring is added 2.8 ml (0.00675 mol) of 2.4 M *n*-butyllithium in hexane. The solution is maintained at —15° to —20°C for 2.5 hours. A solution of 0.89 g (0.00675 mol) of copper pentyne and 2.2 g of hexamethyl phosphorus triamide in 25 ml of ether is added at —78°C. After 1 hour, a solution of 2.3 g (0.005 mol) of 1-(8-dimethyl-t-butylsilyloxy-7-oxo-octenyl)-4-trimethylsilyloxycyclopent-2-en-1-one, 7-ethylene ketal in 10 ml of ether is added. The mixture is stirred at —45°C for 0.5 hours and allowed to warm to —20°C over 40 minutes. The solution is recooled to —30°C and 2.5 ml of acetic acid followed by saturated ammonium chloride is added. The mixture is extracted with ether. The ether layer is worked with dilute hydrochloric acid followed by saturated sodium bicarbonate. The solution is dried over magnesium sulfate. The solvent is removed. The residue is dissolved in 10 ml of ether at —78°C under

argon with stirring is added 20 ml (0.01 mol) of 0.5M lithium perhydro-9b-borophenolyhydride in tetrahydrofuran. The solution is allowed to warm to 0° over 35 minutes. At 0° is added 8 g of sodium carbonate in 60 ml of water followed by 13 ml of 30% hydrogen peroxide. The mixture is stirred for 10 minutes and then extracted with ether. The ether solution is washed with water, saturated sodium bisulfite, and saturated sodium bicarbonate. The solution is dried by magnesium sulfate. The residue is dissolved in 60 ml of tetrahydrofuran containing 9.6 ml of water and 1.2 ml of concentrated hydrochloric acid. The solution is stirred at 55—60°C for four hours 45 minutes. The mixture is saturated with sodium chloride and extracted with ethyl acetate. The organic layer is washed with saturated sodium bicarbonate and dried over magnesium sulfate. The solvent is removed. The residue is chromatographed on a dry column of silica gel eluting with ethyl acetate containing 4% of methanol to give the title compound.

By the methods described hereinabove in Example 1215 the vinyl iodides or vinyl tin compounds used in Examples 319—772AAA are exchanged with *t*-butyllithium or *n*-butyllithium respectively to give the vinyl lithium reagents which on reaction with copper pentyne and hexamethyl phosphorous triamide give the cuprate reagents. Addition of the cyclopent-2-en-1-ones used in Examples 319—772AAA at −78°C followed by warming to −20°C as described hereinabove in Example 1214 gives the 9-oxo analogs in which the 1-oxo feature is still protected. The reaction of these with lithium perhydro-9b-borophenylhydride at −78°C followed by warming to 0°C, quenching with sodium carbonate and hydrogen peroxide and deblocking with acetic acid-tetrahydrofuran-water 4:2:1 or with dilute hydrochloric acid in tetrahydrofuran gives the 1-oxo-9α-hydroxy analogs corresponding to the 1,9-dioxo analogs of Examples 319—772AAA.

## Example 1216

Preparation of 1-oxo-9α,11α,16-trihydroxy-hydroxymethyl-16-methyl-13-*trans* prostene, 1-ethylene ketal and 1-oxo-9β,11α,16-trihydroxy-1-hydroxymethyl-16-methyl-13-*trans* prostene,1-ethylene ketal

To a solution of 0.1 g of 1,9-dioxo-11α, 16-dihydroxy-1-hydroxymethyl-16-methyl-5-*cis*, 13-*trans*-prostadiene, 1-ethylene ketal (Example 774) in 25 ml of ethanol is added 0.1 g of sodium borohydride. After stirring 3 hours the mixture is poured into water and extracted with ethyl acetate. The ethyl acetate solution is dried over magnesium sulfate. The solvent is removed and the title compounds are separated by silica gel chromatography.

By the method described hereinabove in Example 1216 the 1,9-dioxo-1-ethylene ketal analogs of Examples 775—1212 are reacted with an excess of sodium borohydride in ethanol to give the corresponding 9α-hydroxy-1-ethylene ketal and 9β-hydroxy-1-ethylene ketal analogs.

## Example 1217

Preparation of 1,9-dioxo-16-hydroxy-1-hydroxymethyl-16-methyl-10,13-*trans* prostadiene

To a solution of 0.1 g of 1,9-dioxo-11α,16-dihydroxy-1-hydroxymethyl-16-methyl-13-*trans* prostene in 4 ml of tetrahydrofuran is added 2 ml of 1.5N hydrochloric acid. After 2 days the mixture is poured into water and extracted with ether. The ether solution is dried over magnesium sulfate and the ether is removed to give the title compound.

## Example 1218

Preparation of 1,9-dioxo-16-hydroxy-1-hydroxymethyl-16-methyl-10,13-*trans*-prostadiene-1-ethylene ketal

To a solution of 0.1 g of 1,9-dioxo-11α,16-dihydroxy-1-hydroxymethyl-16-methyl-13-*trans*-prostene-1-ethylene ketal in 4 ml of tetrahydrofuran is added 2 ml of 1.5 N hydrochloric acid. After 2 days the mixture is poured into water and extracted with ether. The ether solution is dried over magnesium sulfate and the ether is removed to give the title compound.

## Example 1219

Preparation of 1,9-dioxo-16-hydroxy-1-acetoxymethyl-16-methyl-10,13-*trans*-prostadiene

To a solution of 0.1 g of 1,9-dioxo-16-hydroxy-1-hydroxymethyl-16-methyl-10,13-*trans*-prostadiene (Example 1217) in 0.75 ml of pyridine is added 0.026 g of acetic anhydride. After 18 hours at room temperature, the solvent is removed at reduced pressure and the residue is chromatographed on silica gel to give the title compound.

By the method described hereinabove in Examples 1218 and 1219 the 11α-hydroxy analogs of Examples 319—1213 are treated with 1.5N hydrochloric acid in tetrahydrofuran for 2 days to give the corresponding Δ10 compounds of this invention.

## Example 1220

Preparation of 1-oxo-9β,11α,16-trihydroxy-1-hydroxymethyl-16-vinyl-5-*cis*-13-*trans*-prostadiene and 1-oxo-9α,11α,16-trihydroxy-1-hydroxymethyl-16-vinyl-5-cis-13-*trans* prostadiene

To a solution of 2.9 g (5.6 mmol) of (E) 4 - trimethylsilyloxy - 4 - vinyl - 1 - tri - *n* - butylstannyloctene (Example 210A) in 4 ml of tetrahydrofuran at −78°C under argon with stirring

is added 2.4 ml of 2.4M *n*-butyllithium in hexane. The solution is stirred at –30° to –20°C for 2 hours. A solution of .74 g (5.6 mmol) of copper pentyne and 2.3 ml of hexaethylphosphorous-triamide in 18 ml of ether is added at –78°C. The solution is stirred at –78°C for 1.5 hours. A solution of 2.0 g (5.2 mmol) of 1 - (6 - (4 - methoxy - 2,2 - dimethyl - 1,3 - dioxolan-4 - yl(hex - 2 - enyl) - 4 - (2 - methoxypropyl - 2 - oxy)cyclopent - 2 - en - 1 - one in 20 ml of ether is added. The solution is stirred at –30° to –20°C for 1.5 hours. To the solution is added 100 ml of saturated ammonium chloride. The mixture is extracted with ether and the ether solution is washed with dilute hydrochloric acid, saturated sodium bicarbonate, and dried over magnesium sulfate. Dry solvent is removed and the residue is dissolved in 30 ml of ethanol and .37 g of sodium borohydride is added. The mixture is stirred for 8 hours. The solvent is removed. The residue is dissolved in 90 ml of acetic acid-tetrahydrofuran — water 4:2:1. The solution is stirred at room temperature for 2 hours. The solvents are removed at reduced pressure. The residue is chromatographed on silica gel to give 1 - oxo - 9β,11α,16 - trihydroxy - 1 - hydroxy-methyl 16 - vinyl - *cis* - 3 - *trans* - prostadiene and 1 - oxo - 9α,11α,16 - trihydroxy - 1 - hydroxy-methyl - 16 - vinyl - 5 - *cis* - 13 - *trans* - prostadiene.

By the methods described hereinabove in Example 1220 the vinyl iodides or vinyl tin compounds used in Examples 319—772AAA are exchanged with *t*-butyllithium or *n*-butyllithium respectively to give the vinyl lithium reagents which on reaction with copper pentyne and hexamethyl phosphorous triamide give the cuprate reagents. Addition of the cyclopent-2-en-1-ones used in Example 319—772AAA at –78°C followed by warming to –20°C as described hereinabove in Example 1214 gives the 9-oxo-analogs in which the 1-oxo feature is still protected. The reaction of these with sodium borohydride in ethanol followed by deblocking with acetic acid tetrahydrofuran-water 4:2:1 or with dilute hydrochloric acid in tetrahydrofuran gives 1-oxo-9β-hydroxy and 1-oxo-9α-hydroxy analogs corresponding to the 1,9-dioxo analogs of Examples 319—772AAA.

Example 1221

Preparation of 1 - oxo - 9α,11α,16 - trihydroxy - 1 - hydroxymethyl - 16 - methyl - 5 - *cis* - 13 - *trans* prostadiene

To a solution of 3.4 (0.00675 mol) of (E) 4-trimethylsilyloxy - 4 - methyl - 1 - tri - *n* - butylstannyl - 1 - octene (Example 210b) in 3.5 ml of tetrahydrofuran at –78°C under argon, with stirring is added 2.8 ml (0.00675 mol) of 2.4 M *n*-butyllithium in hexane. The solution is maintained at –15°C to –20°C for 2.5 hours. A solution of 0.89 g (0.00675 mol) of copper pentyne and 2.2 g of hexamethyl phosphorous triamide in 25 ml of ether is added at –78°C. After 1 hour, a solution of 2.4 g (0.005 mol) of 1 - (8 - dimethyl - t - butylsilyloxy - 7 - oxo - oct - 2 - enyl) - 4 - trimethylsilyloxycyclopent - 2 - en - 1 - one, 7 - ethylene ketal in 10 ml of ether is added. The mixture is stirred at –45°C for 0.5 hours and allowed to warm to –20°C over 40 minutes. The solution is recooled to –30°C and 2.5 ml of acetic acid followed by saturated ammonium chloride is added. The mixture is extracted with ether. The ether layer is worked with dilute hydrochloric acid followed by saturated sodium bicarbonate. The solution is dried over magnesium sulfate. The solvent is removed. The residue is dissolved in 10 ml of ether at –78°C under argon with stirring is added 20 ml (0.01 mol) of 0.5M lithium perhydro-9b-borophenolylhydride in tetrahydrofuran. The solution is allowed to warm to 0°C over 35 minutes. At 0°C is added 8 g of sodium carbonate in 60 ml of water followed by 13 ml of 30% hydrogen peroxide. The mixture is stirred for 10 minutes and then extracted with ether. The ether solution is washed with water, saturated sodium bisulfite, and saturated sodium bicarbonate. The solution is dried of magnesium sulfate. The residue is dissolved in 60 ml of tetrahydrofuran containing 9.6 ml of water and 1.2 ml of concentrated hydrochloric acid. The solution is stirred at 55°—60°C for 4 hours 45 minutes. The mixture is saturated with sodium chloride and extracted with ethyl acetate. The organic layer is washed with saturated sodium bicarbonate and dried over magnesium sulfate. The solvent is removed. The residue is chromatographed on a dry column of silica gel eluting with ethyl acetate containing 4% of methanol to give the title compound.

By the methods described hereinabove in Example 1221 the vinyl iodides or vinyl tin compounds used in Examples 319—772AAA are exchanged with *n*-butyllithium or *t*-butyl lithium respectively to give the vinyllithium reagents which on reaction with copper pentyne and hexamethyl phosphorous triamide give the cuprate reagents. Addition of the cyclopent-2-en-1-ones used in Examples 319—772AAA at –78°C followed by warming to –20°C as described hereinabove in Example 1214 gives the 9-oxo analogs in which the 1-oxo feature is still protected. The reaction of these with lithium perhydro-9b-borophenolylhydride at –78°C followed by warming to 0°, quenching with sodium carbonate and hydrogen peroxide and deblocking with acetic acid-tetrahydrofuran-water 4:2:1 or with dilute hydrochloric acid in tetrahydrofuran gives the 1-oxo-9 hydroxy analogs corresponding to the 1,9-dioxo analogs of Examples 319—772AAA.

Example 1222

Preparation of 1-oxo-9α,11α,16-trihydroxy-1-hydroxymethyl-16-methyl-5-*cis*-13-*trans* prostadiene, 1-ethylene ketal and 1-oxo-9β,11α,16-trihydroxy-1-hydroxymethyl-16-methyl-5-*cis*-13-*trans* prostadiene, 1-ethylene ketal

To a solution of 0.1 g of 1,9-dioxo-11α,16-dihydroxy-1-hydroxymethyl-16-methyl-5-*cis*-13-*trans* prostadiene, 1-ethylene ketal (Example 774) in 25 ml of ethanol is added 0.1 g of sodium borohydride. After stirring 3 hours the mixture is poured into water and extracted with ethyl acetate. The ethyl acetate solution is dried over magnesium sulfate. The solvent is removed and the title compounds are separated by silica gel chromatography.

By the method described hereinabove in Example 1222 the 1,9-dioxo-1-ethylene ketal analogs of Examples 773—1212 are reacted with an excess of sodium borohydride in ethanol to give the corresponding 9α-hydroxy-1-ethylene ketal and 9β-hydroxy-1-ethylene ketal analogs.

Example 1223

Preparation of 1,9-dioxo-16-hydroxy-1-hydroxymethyl-16-methyl-5-*cis*-10,13-*trans*-prostatriene

To a solution of 0.1 g of 1,9-dioxo-11α,16-dihydroxy-1-hydroxymethyl-16-methyl-5-*cis*-13-*trans*-prostadiene in 4 ml of tetrahydrofuran is added 2 ml of 1.5N hydrochloric acid. After 2 days the mixture is poured into water and extracted with ether. The ether solution is dried over magnesium sulfate and the ether is removed to give the title compound.

Example 1224

Preparation of 1,9-dioxo-16-hydroxy-1-hydroxymethyl-16-methyl-5-*cis*-10.13 *trans*-prostatriene-1-ethylene ketal

To a solution of 0.1 g of 1,9-dioxo-11α, 16-dihydroxy-1-hydroxymethyl-16-methyl-5-*cis*-13-*trans* prostadiene-1-ethylene ketal in 4 ml of tetrahydrofuran is added 2 ml of 1.5N hydrochloric acid. After 2 days the mixture is poured into water and extracted with ether. The ether solution is dried over magnesium sulfate and the ether is removed to give the title compound.

Example 1225

Preparation of 1,9-dioxo-16-hydroxy-1-acetoxymethyl-16-methyl-5-cis-10,13-*trans*-prostatriene

To a solution of 0.1 g of 1,9 - dioxo - 16 - hydroxy - 1 - hydroxymethyl - 16 - methyl - 5 - *cis*-10,13 - *trans* - prostatriene in 0.75 ml of pyridine is added 0.026 g of acetic anhydride. After 18 hours at room temperature, the solvent is removed at reduced pressure and the residue is chromatographed on silica gel to give the title compound.

By the method described hereinabove in Examples 1223 and 1224 the 11α-hydroxy analogs of Examples 319—1213 are treated with 1.5N hydrochloric acid in tetrahydrofuran for 2 days to give the corresponding Δ10 compounds of this invention.

Example 1226

Preparation of 1,9 - dioxo - 11α,16 - dihydroxy - 16 - methyl - 1 - hydroxymethyl - 13 - *trans*-prostene by hydrogenation of 1,9 - dioxo - 11α,16 - dihydroxy - 16 - methyl - 1 - hydroxymethyl - 5 - *cis*, 13 - *trans* - prostadiene

A solution of 1,9 - dioxo - 11α,16 - dihydroxy - 16 - methyl - 1 - hydroxymethyl - 5 - *cis*, 13 - *trans* - prostadiene in $CH_2Cl_2$ at 0°C is treated with 3.3 equivalents of dihydropyran and a catalytic amount of chloroacetic acid. After 2 hours, solid $KHCO_3$ is added, the solution is filtered, and concentrated *in vacuo* to provide *1,9 - dioxo - 11α,16 - bistetrahydropyranyloxy - 1 - tetrahydropyranyloxymethyl - 16 - methyl - 5* - cis, *13* - trans - *prostadiene.*

The tritetrahydropyanyl derivative is dissolved in methanol and hydrogenated at −15 to −20°C under 1 atm of hydrogen with 5% palladium - on - carbon as described by Corey, *J.A.C.S., 92,* 2586 (1970).

The reaction product is isolated and subjected to hydrolysis in acetic acid - water - THF (20:10:3) at 40°C for 4 hours. Reisolation and chromatography on silica-gel (impregnated with $AgNO^3$ if necessary), provides *1,9 - dioxo - 11α,16 - dihydroxy - 16 - methyl - 1 - hydroxymethyl - 13* - trans *prostene.*

Other suitable protecting groups are derived from dihydrofuran, ethylvinylether, methoxy-5,6-dihydro-2H-pyran, chloromethylmethylether, chloro-trilowerälkyl-silane, and the like.

Example 1227

In accordance with the partial hydrogenation procedure of Example 1226, the corresponding $E_1$ series compound may be prepared from the $E_2$ series compounds of Examples 319—1213. Also, in accordance with procedure of Example 1226, the $F_2$ series compounds described in Examples 1220—1222, may be partially hydrogenated to provide the corresponding $F_1$ series compound.

### Example 1228

Preparation of 1,9 - dioxo - 11$\alpha$,16 - dihydroxy - 16 - methyl - 1 hydromethyl - 13 - *trans*-prostene from the corresponding $F_{1}\alpha$ congener

To a solution of 1 - oxo - 9$\alpha$,11$\alpha$,16 - trihydroxy - 1 - hydroxymethyl - 16 - methyl - 13 - *trans*-prostene (Example 1215) in dry acetone at —40°C is added N-trimethylsilyldiethylamine according to the procedure of Yankee, Lin and Fried, *J. Chem. Soc.*, 1121 (1972).

The reaction mixture is diluted with water and extracted with ether; the ether is dried and concentrated *in vacuo* to provide *1 - oxo - 11$\alpha$ trimethylsilyloxy - 9$\alpha$,16 - dihydroxy - 1 - trimethylsilyloxymethyl - 13 -* trans *- prostene.*

The bissilylated material is oxidized with Collens reagent *in situ* (prepared from $CrO_3$ and pyridine in $CH_2CL_2$) according to the above reference for 5 minutes at 25° followed by desilylation with a mixture of methanol, water and acetic acid (1:0.1:0.05) for 1 hour.

The solvent is removed *in vacuo* and the product is purified on silica-gel, to provide 1,9 - dioxo-11$\alpha$,16 - dihydroxy - 16 - methyl - 1 - hydroxymethyl - 13 - *trans* - prostene.

### Example 1229

Preparation of 1,9 - dioxo - 11$\alpha$,16 - dihydroxy - 1 - hydroxymethyl - 13 - *trans* prostene from 1 - oxo - 9$\alpha$, - 11$\alpha$,16 - trihydroxy - 1 - hydroxymethyl - 16 - methyl - 13 - *trans* prostene, 1 - ethylene ketal

To a solution of 1 - oxo - 9$\alpha$,11$\alpha$,16 - trihydroxy - 1 - hydroxymethyl - 16 - methyl - 13 - *trans*-prostene, 1 - ethylene ketal, (Example 1216) in dry acetone at —40° is added N. trimethylsilyldiethylamine according to the procedure of Yankee, Lin and Fried, J. Chem. Soc., 1121 (1972).

The reaction mixture is diluted with water and extracted into ether; the ether is dried and concentrated *in vacuo* to provide *1 - oxo - 9$\alpha$,16 - trihydroxy - 11$\alpha$ - trimethylsilyloxy - 1 - trimethylsilyloxymethyl - 16 - methyl - 13 -* trans *- prostene, 1 - ethylene ketal.*

This 9$\alpha$ - hydroxy derivative is oxidized by the procedure of the above reference utilizing Collins reagent (prepared in situ from $CrO_3$, and pyridine in $CH_2Cl_2$). After 5 minutes at 25°, desilylation is accomplished with a mixture of methanol, water, acetic acid (1:0.01:0.05) containing a catalytic amount of HCl, for 1 hour.

The solvents are concentrated in vacuo and the residue is purified by silica-gel chromatography to provide *1,9 - dioxo - 11$\alpha$,16 - dihydroxy - 16 - methyl - 1 - hydroxymethyl - 13 -* trans *- prostene, 1-ethylene ketal.*

### Example 1230

In accordance with the procedure of Example 1228 the corresponding $E_1$-series compound may be prepared from the $F_1$-series compounds described in Examples 1214—1216.

Also in accordance with the procedure of Example 1228, the corresponding $E_2$-series compounds may be prepared from the $F_2$-series compounds described in Examples 1220—1222.

### Example 1231

In accordance with the procedure of Example 1229, the corresponding $E_1$-series ethylene ketal or dioxolan compound may be prepared from the $F_1$-series ethylene ketal or dioxolan compounds described in Examples 1214—1216.

Also, in accordance with the procedure of Example 1229, the corresponding $E_2$-series ethylene ketal or dioxolan compound may be prepared from the $F_2$-series ethylene ketal or dioxolan compounds described in Examples 1220—1222.

### Example 1232

Preparation of 1,9 - dioxo - 11$\alpha$,16 - dihydroxy - 16 - methyl - 1 - hydroxymethylprostane from 1,9 - dioxo - 11$\alpha$,16 - dihydroxy - 16 - methyl - 1 - hydroxymethyl - 5 - *cis* - 13 - *trans*-prostadiene

A solution of 1,9 - dioxo - 11$\alpha$,16 - dihydroxy - 16 - methyl - 1 - hydroxymethyl - 5 - *cis*-13- *trans* - prostadiene in 50 ml of ethyl acetate may be completely hydrogenated in a Parr apparatus using 500 mg of 5% rhodium-on-carbon. The catalyst is removed by filtration and the filtrate is taken to dryness to furnish the title product.

### Example 1233

In accordance with the hydrogenation procedure of Example 1232 the $E_1$, $E_2$, $F_1$ or $F_2$ prostaglandin derivatives of Examples 319—1231 herein may be hydrogenated to provide the corresponding $E_0$ and $F_0$ prostaglandin derivative.

Example 1234

Preparation of 1,9 - dioxo - 11α,16 - dihydroxy - 1 - *p* - methoxybenzoyloxymethyl - 16 - methyl-13 - *trans* - prostene

To a mixture of 200 mg of 1,9 - dioxo - 11α,16 - dihydroxy - 1 - hydroxymethyl - 16 - methyl-13 - *trans* - prostene and 89.2 mg (1 eq) of *p*-anisoyl chloride is added 2 ml of dry pyridine. After agitation to complete dissolution, the solution is allowed to stand at ambient temperature for two hours. The solution is applied directly to a silica-gel dry column (31" x 1") and developed with ethyl acetate. The column is segmented and the fractions containing the product ester are eluted with ethyl acetate to afford 123 mg of the title compound.

Example 1235

Preparation of 1,9 - dioxo - 11α,16 - dihydroxy - 1 - *p* - chlorobenzoyloxymethyl - 16 - methyl-13 - *trans* prostene

To a mixture of 303 mg of 1,9 - dioxo - 11α,16 - dihydroxy - 1 - hydroxymethyl-16 - methyl - 13 - *trans* - prostene and 139 mg (1 eq) of *p* - chlorobenzoylchloride is added 2.5 ml of dry pyridine. After dissolution, the reaction mixture is allowed to stand 18 hours at ambient temperature. The reaction mixture is placed directly onto a silica-gel dry column (50" x 1") containing 225 g of silica-gel and developed with ethyl acetate. The column is segmented and the sections containing the product are eluted with ethyl acetate to provide 121 mg of the title ester as a wax.

[There is no Example 1236].

Example 1237

Preparation of 1,9-dioxo-11α,16-dihydroxy-1-acetoxymethyl-16-methyl-13-*trans*-prostene

To a solution of 218 mg of 1,9-dioxo-11α,16-dihydroxy-1-hydroxymethyl-16-methyl-13-*trans*-prostene in 1 ml of dry pyridine at 0°C is added, dropwise, a solution of 58 mg (1 eq) of acetic anhydride in 0.6 ml of dry pyridine. After stirring at 0°C for 18 hours, the reaction mixture is placed directly onto a silica-gel dry column (150 g silica-gel, 35" x 1", equilibrated with 15 ml ETOAC) and developed with ethyl acetate. The column is segmented and the portions containing the product are eluted with ethyl acetate to afford 88 mg of the 1-oxo-1-acetoxymethyl prostanoid.

Example 1238

Treatment of 1,9-dioxo-11α,16-dihydroxy-1-hydroxy-methyl-16-methyl-13-*trans*-prostene or the individual optical isomers thereof with 1.1 eq of the following acid halides by the procedure of Examples 1234—1236 or a carboxylic acid anhydride by the procedure of Example 1237 provides the corresponding 1-substituted carboxymethyl prostanoic derivative:

CARBOXYLIC HALIDES

Acetyl bromide
Acetyl chloride
*p*-Anisoyl chloride
Benzoyl bromide
Benzoyl chloride
Butyryl chloride
o-Chlorobenzoyl chloride
m-Chlorobenzoyl chloride
p-Chlorobenzoyl chloride
2,4-Dichlorobenzoyl chloride
2,6-Dichlorobenzoyl chloride
3,4-Dichlorobenzoyl chloride
Diethylcarbamyl chloride
3,5-Dimethoxybenzoyl chloride
o-Fluorobenzoyl chloride
m-Fluorobenzoyl chloride
p-Fluorobenzoyl chloride
Isobutyryl chloride
Isovaleryl chloride
Pentafluorobenzoyl chloride
o-Toluoyl chloride
m-Toluoyl chloride
p-Toluoyl chloride
3,4,5-Trimethoxybenzoyl chloride
Trimethylacetyl chloride
Valeryl chloride

CARBOXYLIC ACID ANHYDRIDES
Acetic anhydride
n-Butyric anhydride
Propionic anhydride
Benzoic anhydride

Example 1239

Treatment of 1,9 - dioxo - 11$\alpha$,16 - dihydroxy - 1 - hydroxy - methyl - 16 - methyl - 5 - *cis*,13-*trans* - prostadiene, or the individual isomers thereof, with 1.1 eq of the acid halides or anhydrides listed in Example 1238 by the procedures of Examples 1234—1237 is productive of the corresponding 1-substituted carboxymethyl prostanoic derivative.

Example 1240

Treatment of the 1-oxo-hydroxymethyl E, F and A prostanoids described herein with the acid halides and anhydrides described in Example 1238 by the procedures of Examples 1234—1237 is productive of the corresponding 1-oxo-1-substituted carboxymethyl prostanoic derivatives.

In its broad aspect, this invention encompasses prostaglandin type compounds characterized by an $\alpha$-chain having a terminal hydroxy methyl ketone group or the acyl or protected precursor derivatives thereof. The term $\alpha$-chain as employed herein is intended to connote the prostaglandin side-chain which, in natural mammalian or racemic prostaglandins carries the carboxylic acid function. Prostaglandins, as pointed out earlier in the specification, are named as derivatives of the basic prostanoic acid molecule. See, also, Journal of Medicinal Chemistry 1974, Vol. 17, No. 9, pp. 911—918 and Chemistry and Industry, April 17, 1976, pp. 334—338.

A 15-deoxy-16-hydroxy substituted prostaglandin consists of two *dl* racemates (16$\alpha$-hydroxyl and 16$\beta$-hydroxyl), and similarly a 15-hydroxy-15-substituted compound consists of two *dl*-racemates (15$\alpha$-hydroxyl and 15$\beta$-hydroxyl) unless specified otherwise, which respectively are separable into the 16$\alpha$ and 16$\beta$ epimers, and 15$\alpha$ and 15$\beta$ epimers. A species claim wherein the stereochemistry of the $C_{15}$ or $C_{16}$ carbon is not specified encompasses the *nat*-15$\alpha$ and 15$\beta$, and *nat*-16$\alpha$ and 16$\beta$ forms of the compound and the racemic mixtures thereof.

**Claims**

1. A compound of the formula:

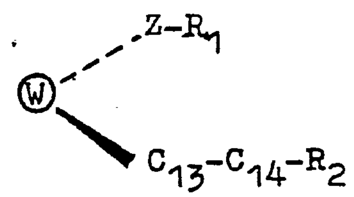

wherein Z is $-(CH_2)_f-$ or $-CH_2-\underset{cis}{CH=CH}-(CH_2)_g-$, wherein f is an integer from 5 to 7 inclusive, and g is an integer from 2 to 4, inclusive; $C_{13}$—$C_{14}$ is ethylene or *trans*-vinylene; W is selected from the group consisting of:

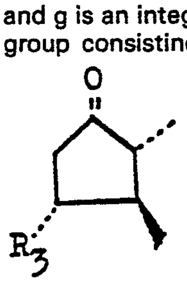 , 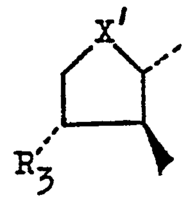 or 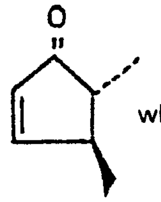 wherein X' is: 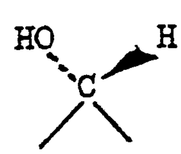 or 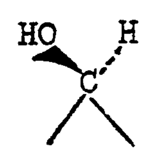

and $R_3$ is hydrogen or hydroxyl; $R_1$ is selected from the group consisting of:

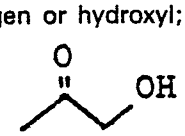

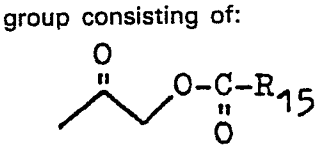

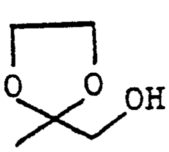

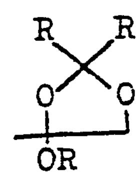

wherein R is $C_1$—$C_4$ alkyl and $R_{15}$ is selected from the group consisting of $C_1$—$C_4$ alkyl, di-($C_1$—$C_4$)-alkylamino, and phenyl or phenyl substituted with one or more substituents selected from the group consisting of $C_1$—$C_4$ alkyl, —OR, —SR, F or Cl wherein R is as previously defined, with the proviso that when W is

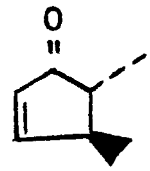

then $R_1$ cannot be

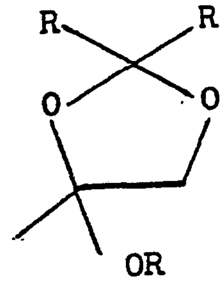

and $R_2$ is selected from the group consisting of:

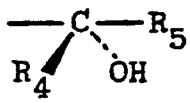

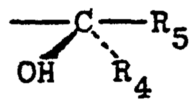

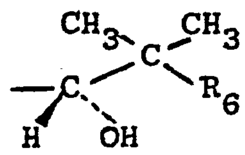

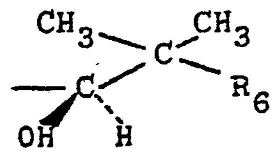

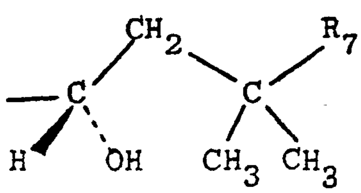

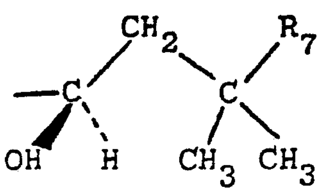

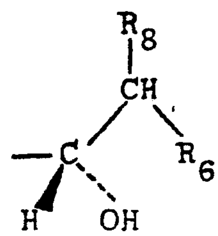

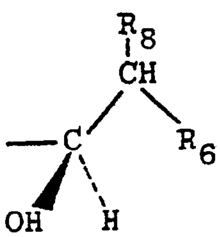

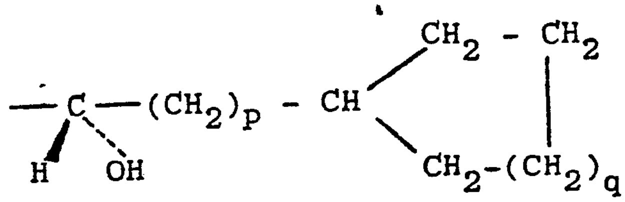

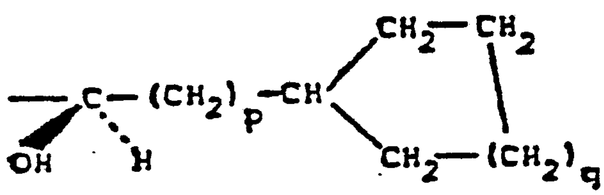 ,

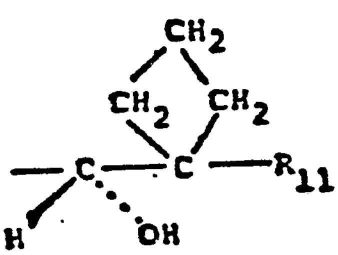 ,

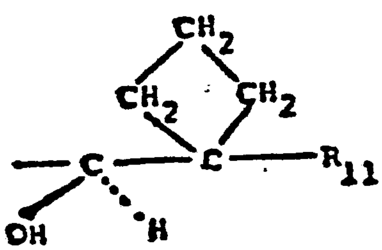 ,

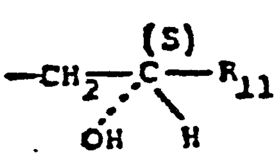 ,

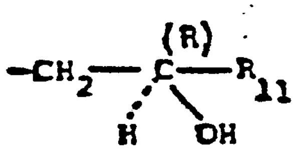 ,

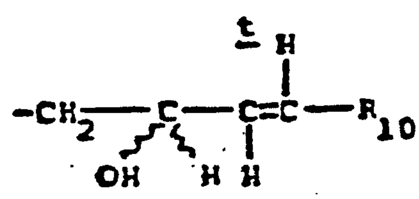

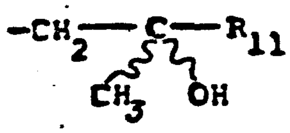,
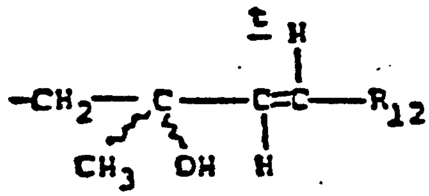,
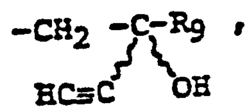,

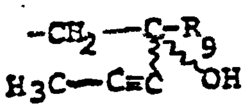

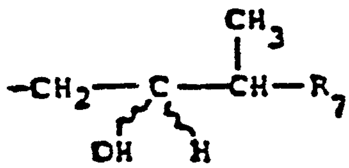,
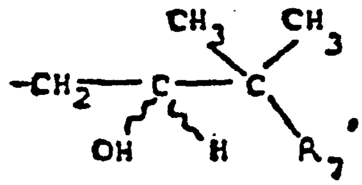,
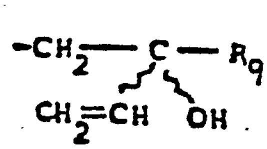;

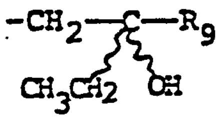,
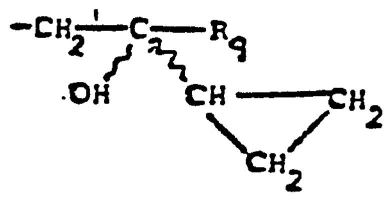;

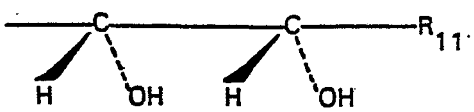,
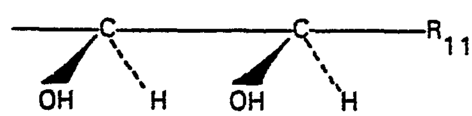,

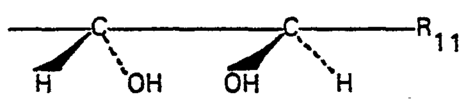,
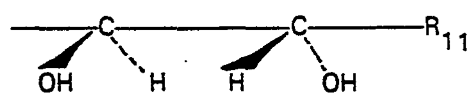,

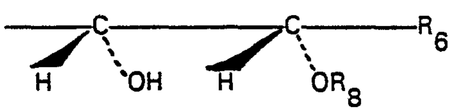,
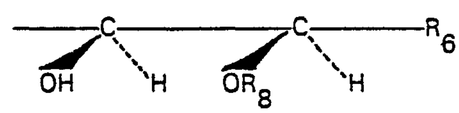

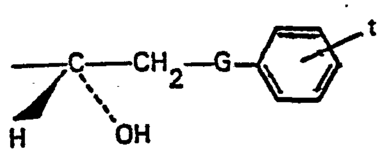

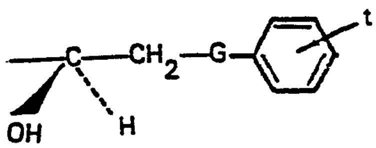

and 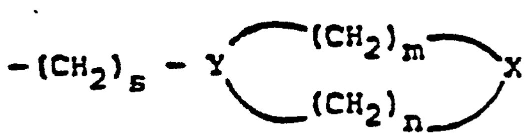

wherein $R_4$ is hydrogen or $C_1$—$C_3$ alkyl; $R_5$ is selected from the group consisting of straight chain $C_4$—$C_7$ alkyl; $R_6$ is selected from the group consisting of straight chain $C_3$—$C_6$ alkyl; $R_7$ is selected from the group consisting of straight chain $C_2$—$C_4$ alkyl; $R_8$ is selected from the group consisting of $C_1$—$C_2$ alkyl; $R_9$ is selected from the group consisting of straight chain $C_3$—$C_6$ alkyl; $R_{10}$ is selected from the group consisting of straight chain $C_1$—$C_4$ alkyl; $R_{11}$ is selected from the group consisting of straight chain $C_3$—$C_7$ alkyl; $R_{12}$ is selected from the group consisting of straight chain $C_1$—$C_4$ alkyl; p is an integer from 0 to 3; q is 1 or 2; X is a divalent radical selected from the group consisting of:

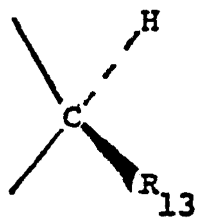

and

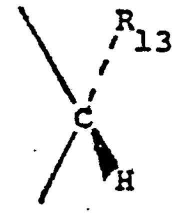

wherein $R_{13}$ is selected from the group consisting of $C_1$—$C_7$ alkyl, hydrogen, and a phenoxy group optionally substituted with a substituent selected from the group consisting of halogen, trifluoromethyl and $C_1$—$C_4$ alkyloxy; Y is a divalent radical selected from the group consisting of:

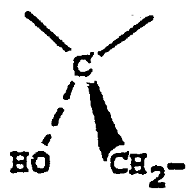

and

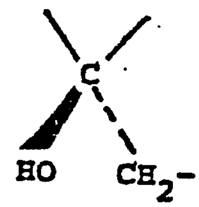

G is a divalent radical selected from the group consisting of —O— and —$CH_2$—; m is zero or an integer from 1 to 4 inclusive; n is zero or an integer from 1 to 4, inclusive; with the proviso that the sum of m and n has the value of 1 to 4; s is zero or the integer 1; and t is selected from the group consisting of hydrogen, chloro, fluoro, dichloro, trifluoro-methyl and methoxy.

2. The compound according to Claim 1: 1,9-dioxo-11$\alpha$,16-dihydroxy-1-hydroxymethyl-16-methyl-5-*cis*,13-*trans*-prostadiene.

3. The compound according to Claim 1: 1,9-dioxo-11$\alpha$,16-dihydroxy-16-methyl-1-hydroxymethyl-13-*trans*-prostene.

4. The compound according to Claim 1: *nat.*-1,9-dioxo-11$\alpha$,16(S)-dihydroxy-1-hydroxymethyl-16-methyl-5-*cis*,13-*trans*-prostadiene.

5. The compound according to Claim 1: *nat.*-1,9-dioxo-11$\alpha$,16(S)-dihydroxy-1-hydroxymethyl-16-methyl-13-*trans*-prostene.

6. The compound according to Claim 1: 1,9-dioxo-11$\alpha$,16(S)-dihydroxy-1-hydroxymethyl-16-methyl-5-*cis*,13-*trans*-prostadiene.

7. The compound according to Claim 1: *nat.*-1,9-dioxo-11$\alpha$,16(S)-dihydroxy-1-hydroxymethyl-16-methyl-13-*trans*-prostene.

8. A pharmaceutical preparation comprising a compound according to any one of Claims 1—7 and a pharmaceutically acceptable carrier or diluent therefor.

9. A method for preparing a compound according to Claim 1, comprising:

(A) reacting a racemic or optically active cyclopentenone of the formula:

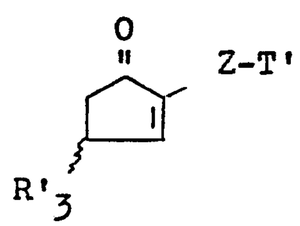

wherein Z is as previously defined, $R_3'$ is hydrogen or a protected hydroxy group, and T′ is

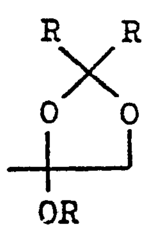

,

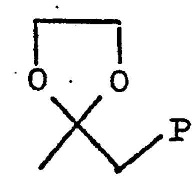

or

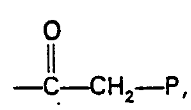

wherein P is a protected hydroxy group, and R is $C_1$—$C_4$ alkyl; with a lithiocuprate selected from the group consisting of:

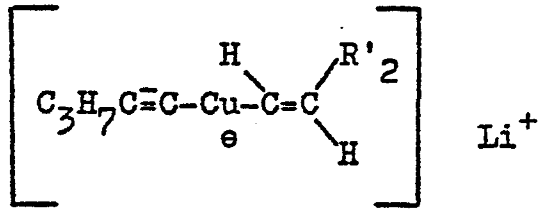 ,

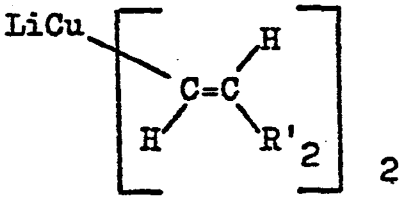 and 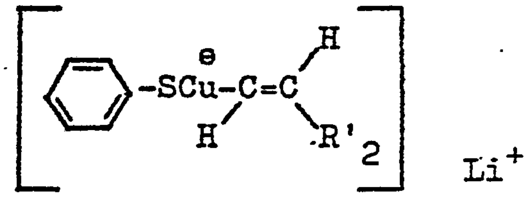

wherein $R_2'$ is a group $R_2$ wherein any hydroxyl group is blocked with a protecting group; to provide a racemic or optically active compound of the formula:

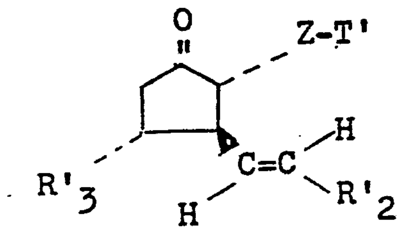

wherein Z, T', $R_3'$ and $R_2'$ are as previously defined; followed by the

(B) removal of the hydroxy protecting groups to provide compounds of the formula:

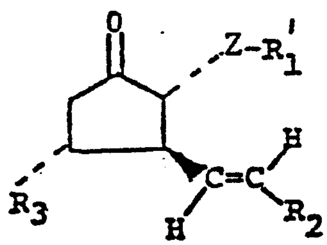

wherein Z, $R_2$, $R_3$ are as previously defined and $R_1'$ is

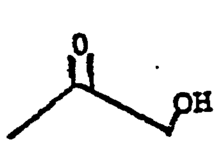 , 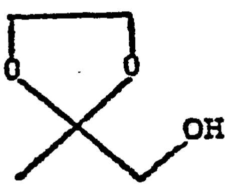 or 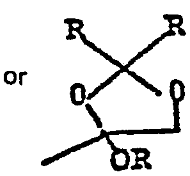

wherein R is as previously defined, and if desired, followed by

(C) the complete hydrogenation of the products of step (B) to provide a compound of the formula:

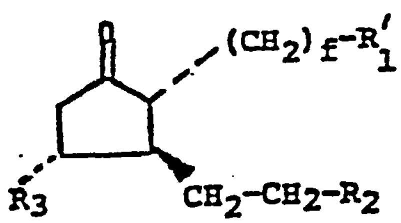

wherein f, $R_3$, $R_2$ and $R_1'$ are as previously defined; or

(D) the partial hydrogenation of the compounds of step (B) of the formula:

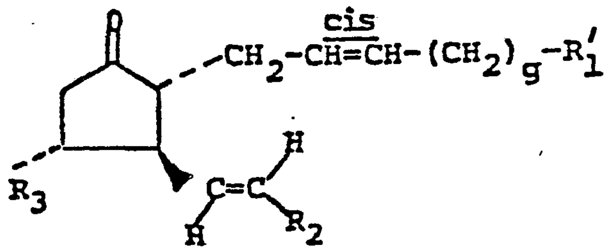

wherein g, $R_3$, $R_2$ and $R_1'$ are as previously defined, to provide the compound of the formula:

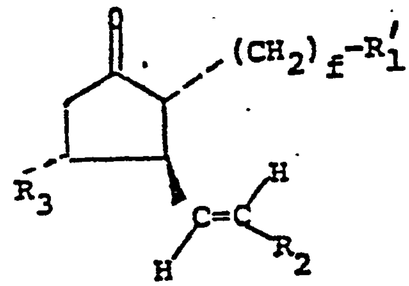

wherein $R_3$, f, $R_1'$ and $R_2$ are as previously defined; and if desired followed by

(E) the treatment of the products of step (A), with a stereo-selective carbonyl reducing agent followed by the removal of the hydroxy protecting group to provide the 9α-hydroxy derivative of the compounds of step (A), or if desired,

(F) the treatment of the products of step (A) with a carbonyl reducing agent followed by the removal of the hydroxy protecting group to provide a separable mixture of the 9α- and 9β-hydroxy derivatives of step (A), and if desired, followed by

(G) the partial hydrogenation of the products of steps (E) or (F) wherein Z is *cis*-vinylene and $C_{13}$—$C_{14}$ is *trans*-vinylene to provide from the 9α-hydroxy compounds of step (E) the compound of the formula:

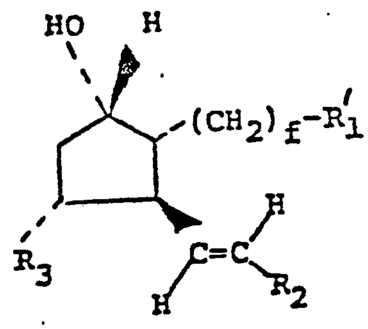

wherein f, $R_2$, $R_3$ and $R_1'$ are as previously defined; or to provide from the 9α/β compound of step (F) the separable mixture of the compounds of the formula:

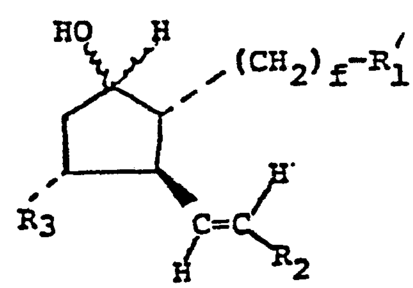

wherein f, $R_1'$, $R_2$, and $R_3$ are as previously defined, or if desired followed by

(H) the complete hydrogenation of the products of steps (E) or (F) to provide from the 9α-hydroxy product of steps (E) or (F) the compound of the formula:

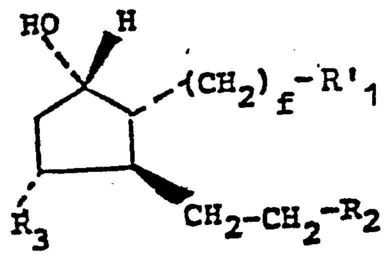

wherein f, $R_1'$, $R_2$ and $R_3$ are as previously defined; and from $9\alpha/\beta$-hydroxy products of step (F) the separable mixture of compounds of the formula:

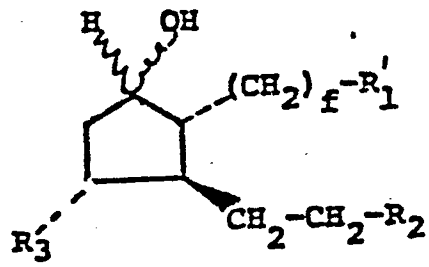

wherein f, $R_1'$, $R_3$ and $R_2$ are as previously defined, and if desired followed by

(I) the dehydration of a compound of the formula

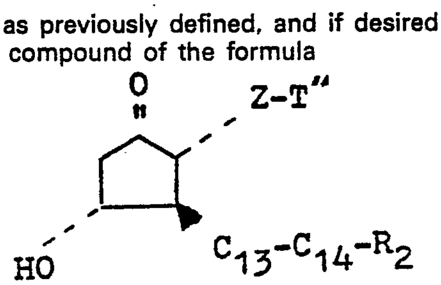

wherein Z, $R_2$ and $C_{13}$-$C_{14}$ are as previously defined and T'' is

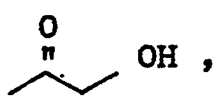 , 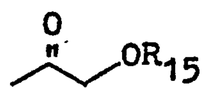 or 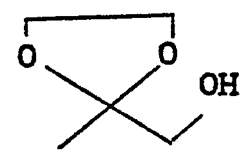

wherein $R_{15}$ is as defined in claim 1, to provide a compound of the formula

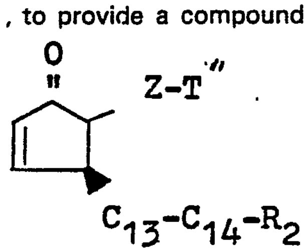

wherein $R_2$, Z, $C_{13}$—$C_{14}$ and T'' are as previously defined, and if desired

(J) the products of steps (B), (C), (D), (E), (F), (G) or (H) wherein $R_1$ is

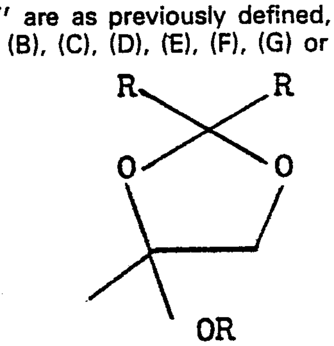

is treated with acid to provide the corresponding product of said step wherein $R_1$ is

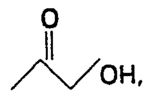 , and if desired

(K) the products of steps (B)—(J) wherein $R_1$ is

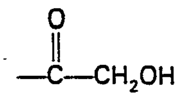

is treated with an anhydride of the formula: $(R_{15}CO)_2O$ or an acid chloride of the formula:

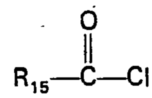

wherein $R_{15}$ is as previously defined to provide the compound of the formula:

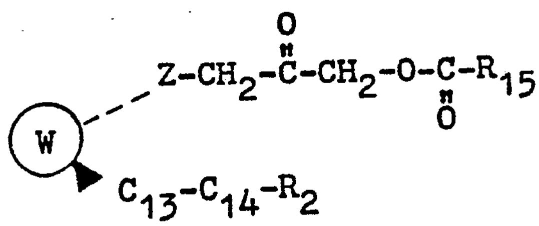

wherein $R_{15}$, Z, W, $C_{13}$—$C_{14}$ and $R_2$ are as previously defined, and if desired, followed by

(L) the resolution of the racemic products of steps (B)—(K).

10. A method for the preparation of a compound according to Claim 1 of the formula:

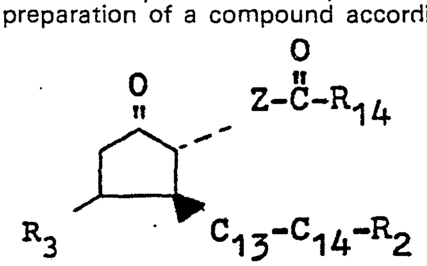

wherein $R_2$, $R_3$, Z and $C_{13}$—$C_{14}$ are as defined in Claim 1, and $R_{14}$ is —$CH_2OH$, comprising:

(A) protecting a compound of the formula:

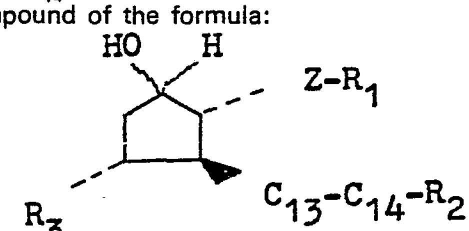

wherein $R_1$, $R_2$, $R_3$, Z and $C_{13}$—$C_{14}$ are as previously defined to give a compound of the formula:

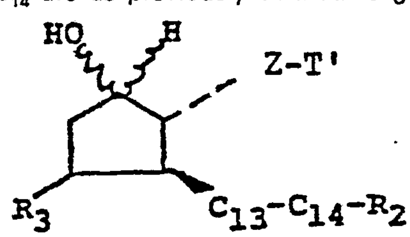

wherein T′, $R_1$, $R_2$, $R_3'$, Z and $C_{13}$—$C_{14}$ are as defined in Claim 9, followed by

(B) treatment with an oxidizing agent and removal of the protecting groups.

11. A method for preparing the compound of Claim 1 wherein $R_1$ is

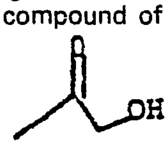 comprising

(A) converting the compound of the formula:

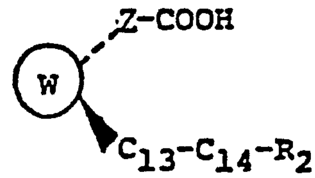

wherein W, $R_2$, Z and $C_{13}$—$C_{14}$ are as defined in Claim 1, to the compound of the formula:

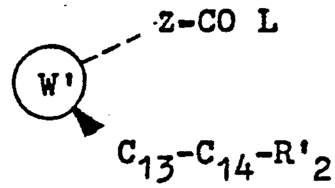

wherein W′ is selected from the group consisting of:

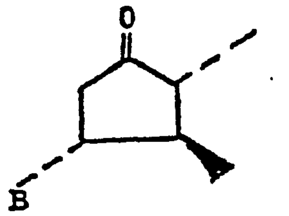

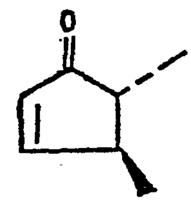

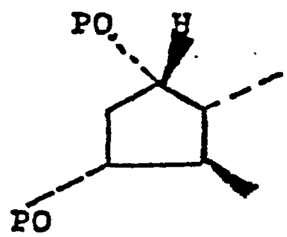

or

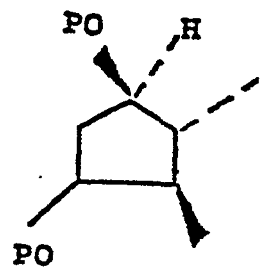

wherein P is a hydroxy protecting group and B is hydrogen or a protected hydroxy group; $R_2'$ is as defined in Claim 9; L is chlorine or bromine; and Z and $C_{13}$—$C_{14}$ are as previously defined, followed by

(B) treatment with diazomethane and then hydrolysis.

12. A method for the preparation of a compound according to Claim 1 of the formula:

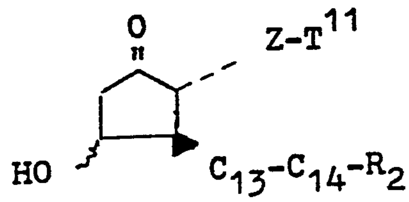

wherein Z, $C_{13}$—$C_{14}$, $R_1$ and $R_2$ are as defined in Claim 1 and T″ is as defined in Claim 9 comprising:

(A) treating the compound

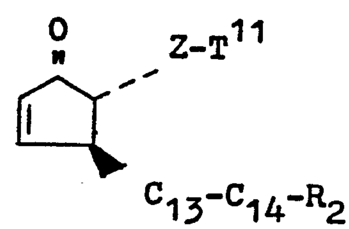

wherein T″, $R_2$, Z and $C_{13}$—$C_{14}$ are as previously defined, with alkaline hydrogen peroxide followed by

(B) treatment with chromous acetate or aluminium amalgam, to provide a separable mixture of the 11$\alpha$ and 11$\beta$ derivatives of the product compound, and if desired followed by the

(C) treatment of the product of step B wherein T'' is

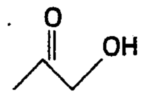

with an anhydride of the formula $(R_{15}—CO)_2$ or an acid chloride of the formula $R_{15}COCl$, where $R_{15}$ is as defined in Claim 1, to provide the product of step (B) wherein T'' is

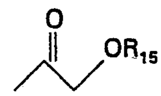

wherein $R_{15}$ is as previously defined.

**Patentansprüche**

1. Verbindung der Formel

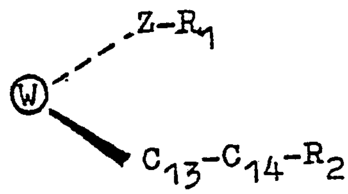

worin Z für $—(CH_2)_f—$ oder $—CH_2—\overset{cis}{CH=CH}—(CH_2)_g—$ steht, wobei f eine Zahl von 5 bis einschließlich 7 und g eine Zahl von 2 bis einschließlich 4 bedeuten; $C_{13}—C_{14}$ Äthylen oder trans-Vinylen bedeutet, W ausgewählt ist unter

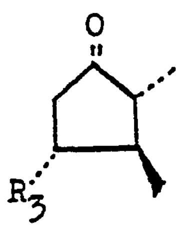 , 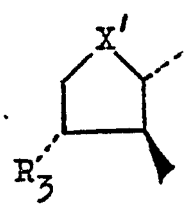 oder 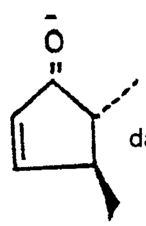 darstellt, worin X' für 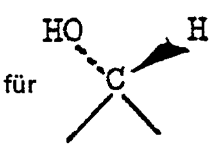 oder 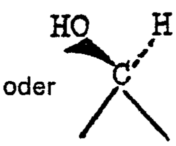

steht, und $R_3$ Wasserstoff oder Hydroxyl bedeutet; $R_1$ ausgewählt ist unter

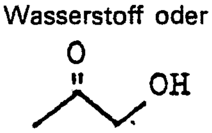 , 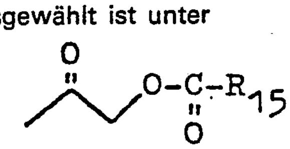

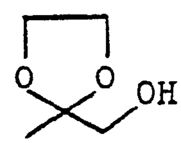 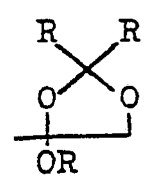

wobei R für $C_{1-4}$-Alkyl steht und $R_{15}$ ausgewählt ist unter $C_{1-4}$Alkyl, Di-$(C_{1-4})$-alkylamino und Phenyl oder Phenyl, das mit einem oder mehreren Substituenten substituiert ist, die ausgewählt sind unter $C_{1-4}$-Alkyl, —OR, —SR, F oder Cl, und wobei R die zuvor angegebene Bedeutung hat, mit der Maßgabe, daß dann, wenn W für

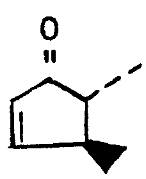 steht, $R_1$ nicht 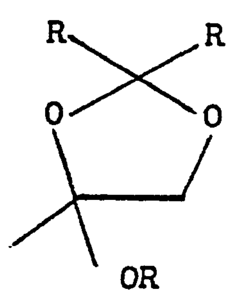 sein kann; und $R_2$ ausgewählt ist unter

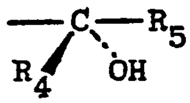 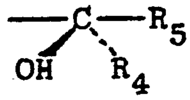 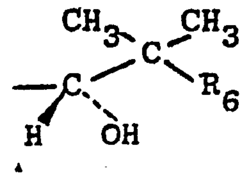 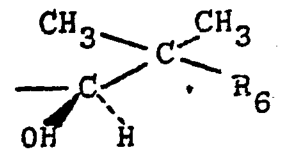

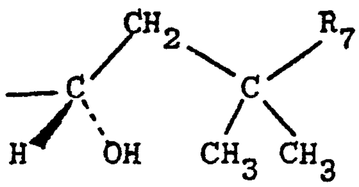 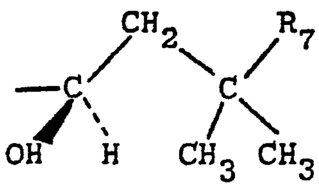 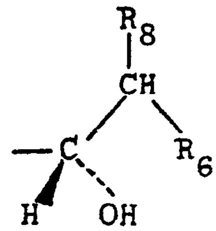

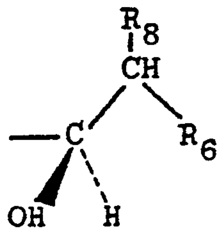 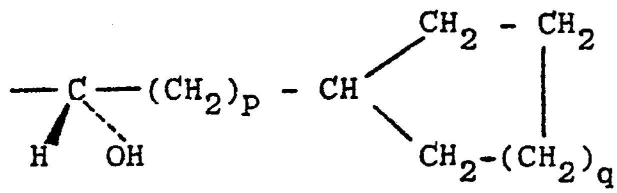

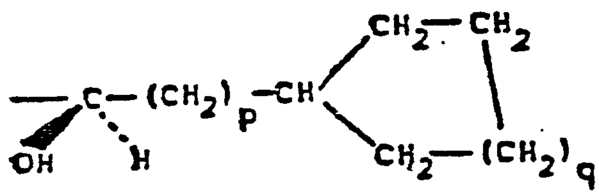

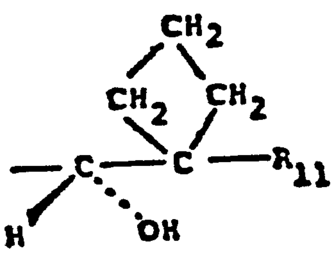 , 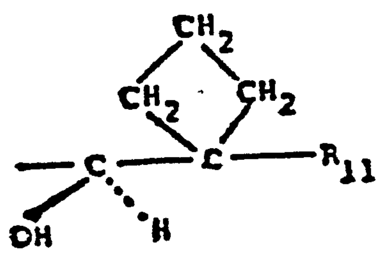 ,

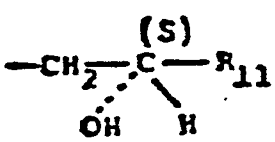 , 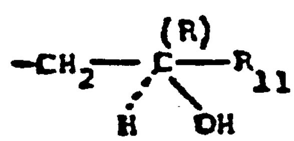 , 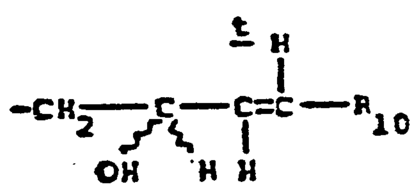

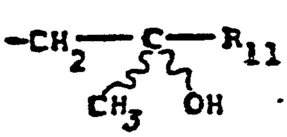 , 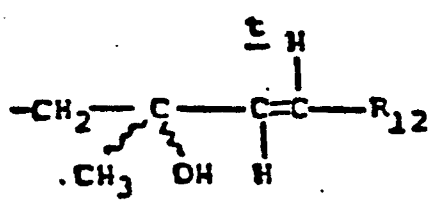 , 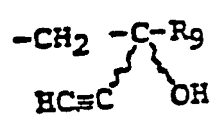 ,

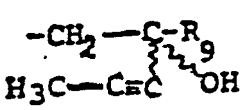

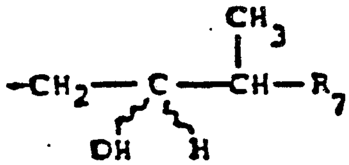 , 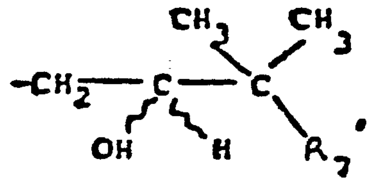 , 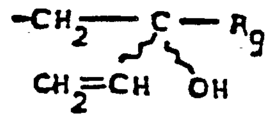 ,

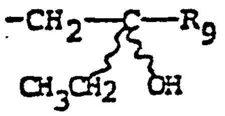 , 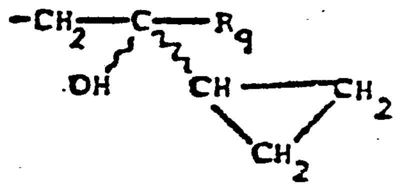 ,

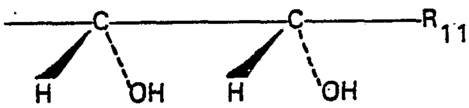 , 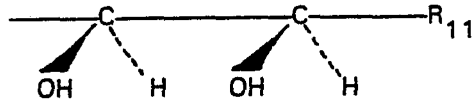 ,

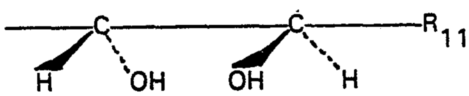 , 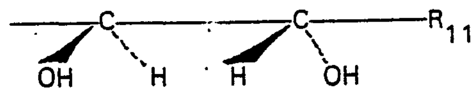 ,

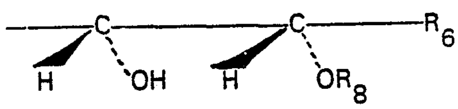 , 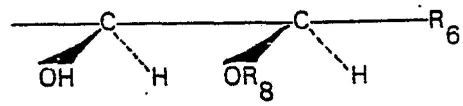

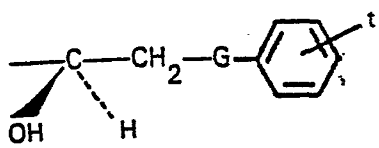

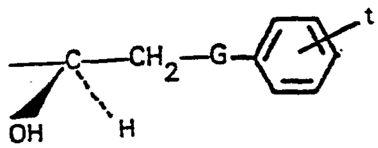

und 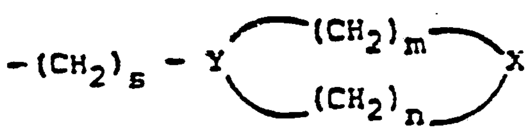

wobei $R_4$ für Wasserstoff oder $C_{1-3}$-Alkyl steht; $R_5$ aus der Gruppe der geradkettigen $C_{4-7}$-Alkylgruppen ausgewählt ist; $R_6$ aus der Gruppe der geradkettigen $C_{3-6}$-Alkylgruppen ausgewählt ist; $R_7$ aus der Gruppe der geradkettigen $C_{2-4}$-Alkylgruppen ausgewählt ist; $R_8$ aus der Gruppe der $C_{1-2}$-Alkylgruppen ausgewählt ist; $R_9$ aus der Gruppe der geradkettigen $C_{3-6}$-Alkylgruppen ausgewählt ist; $R_{10}$ aus der Gruppe der geradkettigen $C_{1-4}$-Alkylgruppen ausgewählt ist; $R_{11}$ aus der Gruppe der geradkettigen $C_{3-7}$-Alkylgruppen ausgewählt ist; $R_{12}$ aus der Gruppe der geradkettigen $C_{1-4}$-Alkylgruppen ausgewählt ist; p für eine ganze Zahl von 0 bis 3 steht; q für 1 oder 2 steht; X für einen zweiwertigen Rest steht, der ausgewählt ist unter

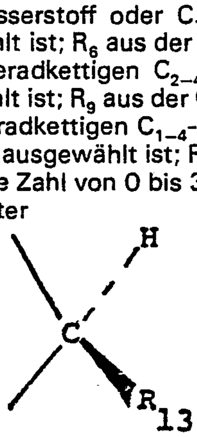 und 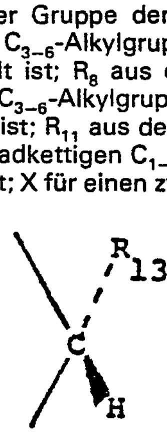

wobei $R_{13}$ ausgewählt ist unter $C_{1-7}$-Alkyl, Wasserstoff und einer Phenoxygruppe, die gegebenenfalls mit einem Substituenten substituiert ist, der ausgewählt ist aus der Gruppe, die aus Halogen, Trifluormethyl und $C_{1-4}$-Alkyloxy besteht; Y für einen zweiwertigen Rest steht, der ausgewählt ist unter

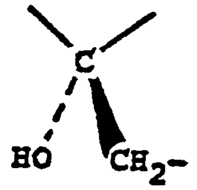

und

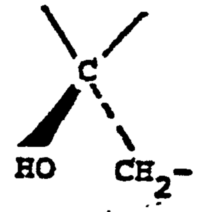

G einen zweiwertigen Rest bedeutet, der unter —O— und —$CH_2$— ausgewählt ist; m für 0 oder eine ganze Zahl von 1 bis einschließlich 4 steht; n für 0 oder eine ganze Zahl von 1 bis einschließlich 4 steht, mit der Maßgabe, daß die Summe von m + n den Wert von 1 bis 4 hat; s 0 oder die ganze Zahl 1 bedeutet; und t aus der Gruppe ausgewählt ist, die aus Wasserstoff, Chlor, Fluor, Dichlor, Trifluormethyl und Methoxy besteht.

2. Verbindung nach Anspruch 1, nämlich 1,9-Dioxo-11α,16-dihydroxy-1-hydroxymethyl-16-methyl-5-cis,13-trans-prostadien.

3. Verbindung nach Anspruch 1, nämlich 1,9-Dioxo-11α,16-dihydroxy-16-methyl-1-hydroxymethyl-13-trans-prosten.

4. Verbindung nach Anspruch 1, nämlich nat.-1,9-Dioxo-11α,16(S)-dihydroxy-1-hydroxymethyl-16-methyl-5-cis,13-trans-prostadien.

5. Verbindung nach Anspruch 1, nämlich nat.-1,9-Dioxo-11α,16(S)-dihydroxy-1-hydroxymethyl-16-methyl-13-trans-prosten.

6. Verbindung nach Anspruch 1, nämlich 1,9-Dioxo-11α,16(S)-dihydroxy-1-hydroxymethyl-16-methyl-5-cis,13-trans-prostadien.

7. Verbindung nach Anspruch 1, nämlich nat.-1,9-Dioxo-11α,16(S)-dihydroxy-1-hydroxymethyl-16-methyl-13-trans-prosten.

8. Pharmazeutische Präparation, gekennzeichnet durch eine Verbindung nach einem der Ansprüche 1 bis 7 und ein pharmazeutisch akzeptables Trägermaterial oder Verdünnungsmittel für die Verbindung.

9. Verfahren zur Herstellung einer Verbindung nach Anspruch 1, dadurch gekennzeichnet, daß man

(A) ein racemisches oder optisch aktives Cyclopentenon der Formel

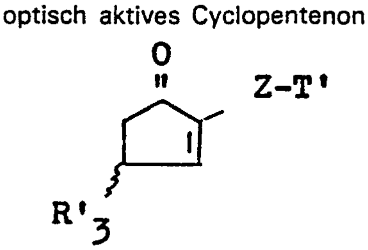

wobei Z die oben angegebene Bedeutung hat; $R'_3$ Wasserstoff oder eine geschützte Hydroxylgruppe bedeutet und T' für

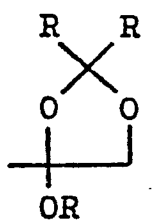

,

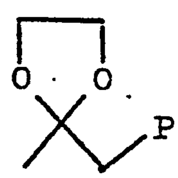

oder

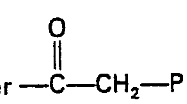

steht, wobei P eine geschützte Hydroxylgruppe bedeutet und R für $C_{1-4}$-Alkyl steht; mit einem Lithiocuprat umsetzt, das aus der Gruppe ausgewählt ist, die aus

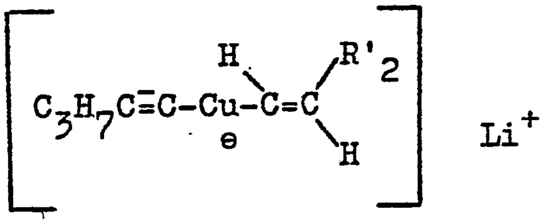 ,

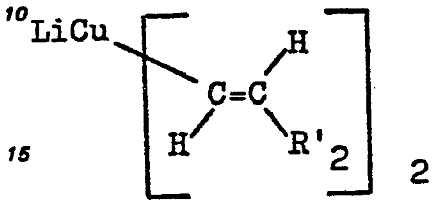 und 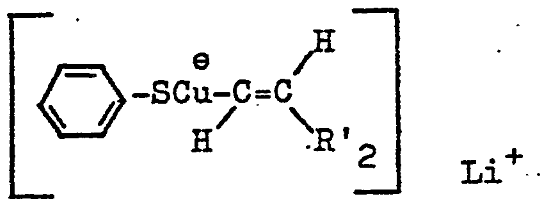

besteht, wobei $R'_2$ eine Gruppe $R_2$ bedeutet, bei der jede Hydroxylgruppe mit einer Schutzgruppe blockiert ist, unter Bildung einer racemischen oder optisch aktiven Verbindung der allgemeinen Formel

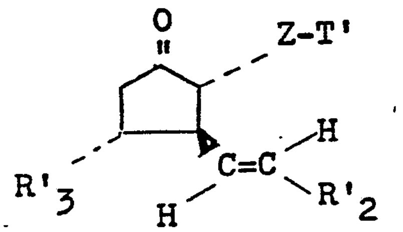

wobei Z, T', $R'_3$ und $R'_2$ die oben angegebenen Bedeutungen haben; daß man anschließend

(B) die Hydroxyl-Schutzgruppen unter Bildung von Verbindungen der Formel

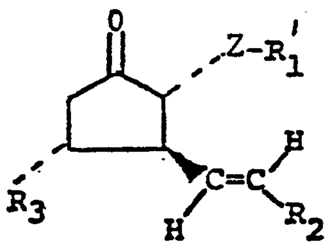

wobei Z, $R_2$ und $R_3$ die oben angegebenen Bedeutungen haben und $R'_1$ für

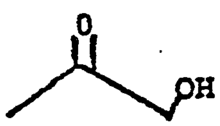 , 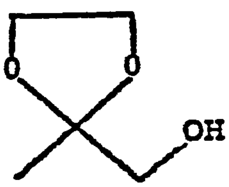 oder 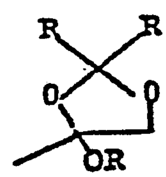

steht, wobei R die oben angegebene Bedeutung hat, entfernt und gewünschtenfalls anschließend

(C) die Produkte der Stufe (B) unter Bildung einer Verbindung der Formel

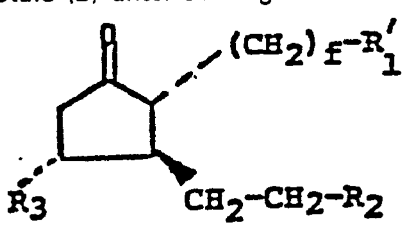

wobei f, $R_3$, $R_2$ und $R'_1$ die oben angegebenen Bedeutungen haben, vollständig hydriert; oder

(D) die Verbindungen der Stufe (B) der Formel

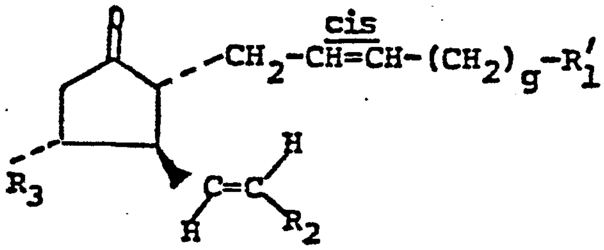

wobei g, $R_3$, $R_2$ und $R'_1$ die oben angegebenen Bedeutungen haben, unter Bildung der Verbindung der Formel

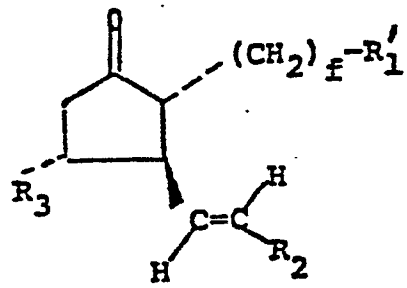

wobei $R_3$, f, $R'_1$ und $R_2$ die oben angegebenen Bedeutungen haben, teilweise hydriert; und gewünschtenfalls anschließend

(E) die Produkte der Stufe (A) mit einem stereo-selektiven Carbonyl-Reduktionsmittel behandelt und nachfolgend die Hydroxyl-Schutzgruppe unter Bildung des 9$\alpha$-Hydroxy-Derivats der Verbindungen der Stufe (A) entfernt, oder gewünschtenfalls

(F) die Produkte der Stufe (A) mit einem Carbonyl-Reduktionsmittel behandelt und nachfolgend die Hydroxyl-Schutzgruppe unter Bildung einer trennbaren Mischung aus den 9$\alpha$- und 9$\beta$-Hydroxy-Derivaten der Stufe (A) entfernt, und gewünschtenfalls anschließend

(G) die Produkte der Stufen (E) oder (F), bei denen Z für cis-Vinylen und $C_{13}$—$C_{14}$ für trans-Vinylen steht, teilweise hydriert, um aus den 9$\alpha$-Hydroxyverbindungen der Stufe (E) die Verbindung der Formel

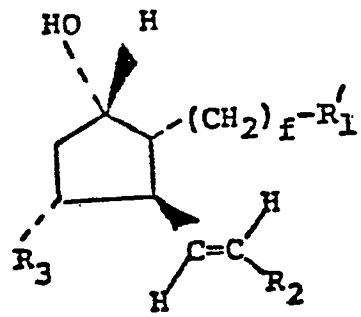

zu bilden, wobei f, $R_2$, $R_3$ und $R'_1$ die oben angegebenen Bedeutungen haben; oder um aus der 9$\alpha$/$\beta$-Verbindung der Stufe (F) die trennbare Mischung der Verbindungen der Formel

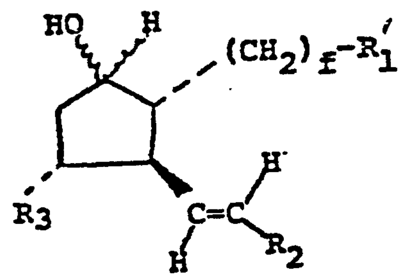

zu bilden, wobei f, $R_1$, $R_2$ und $R_3$ die oben angegebenen Bedeutungen haben, oder gewünschtenfalls anschließend

(H) die Produkte der Stufen (E) oder (F) vollständig hydriert, um aus dem 9$\alpha$-Hydroxyprodukt der Stufen (E) oder (F) die Verbindung der Formel

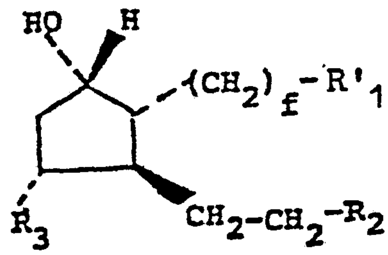

zu bilden, wobei f, $R'_1$, $R_2$ und $R_3$ die oben angegebenen Bedeutungen haben; und aus den 9α/β-Hydroxyprodukten der Stufe (F) die trennbare Mischung von Verbindungen der Formel

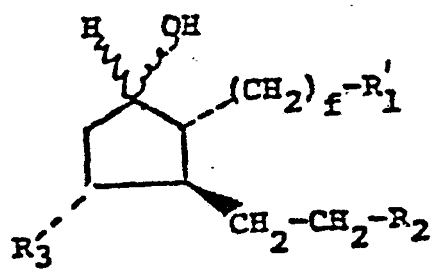

zu bilden, wobei f, $R'_1$, $R_3$ und $R_2$ die oben angegebenen Bedeutungen haben; und gewünschtenfalls anschließende

(I) Dehydratation einer Verbindung der Formel

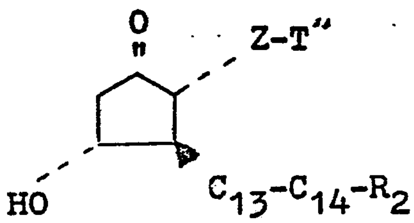

wobei Z, $R_2$ und $C_{13}$—$C_{14}$ die oben angegebenen Bedeutungen haben und T'' für

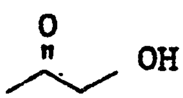 , 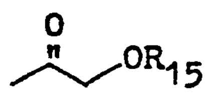 oder 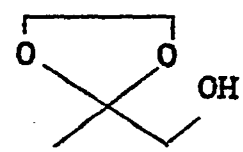

steht, wobei $R_{15}$ die in Anspruch 1 angegebene Bedeutung hat, unter Bildung einer Verbindung der Formel

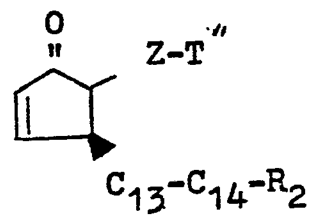

wobei Z, $C_{13}$—$C_{14}$, T'' und $R_2$ die oben angegebenen Bedeutungen haben, und man gewünschtenfalls

(J) die Produkte der Stufen (B), (C), (D), (E), (F), (G) oder (H), bei denen $R_1$ für

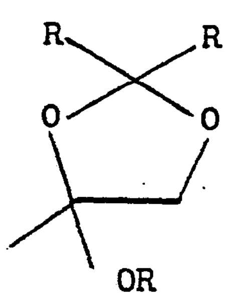

steht, mit einer Säure unter Bildung des entsprechenden Produktes dieser Stufe behandelt, bei dem $R_1$ für

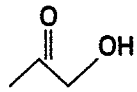

steht, und gewünschtenfalls

(K) die Produkte der Stufen (B) bis (J), bei denen $R_1$ für

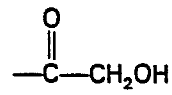

steht, mit einem Anhydrid der Formel: $(R_{15}CO)_2O$ oder einem Säurechlorid der Formel:

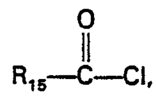

wobei $R_{15}$ die oben angegebene Bedeutung hat, unter Bildung der Verbindung der Formel

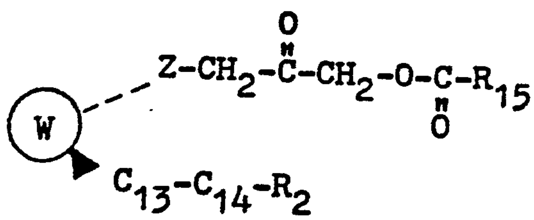

wobei $R_{15}$, Z, W, $C_{13}$—$C_{14}$ und $R_2$ die oben angegebenen Bedeutungen haben, behandelt, und gewünschtenfalls anschließend

(L) die racemischen Produkte der Stufen (B) bis (K) spaltet.

10. Verfahren zur Herstellung einer Verbindung nach Anspruch 1 der Formel

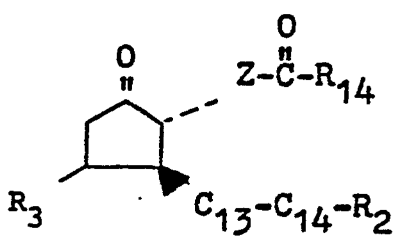

wobei $R_2$, $R_3$, Z und $C_{13}$—$C_{14}$ die in Anspruch 1 angegebenen Bedeutungen haben und $R_{14}$ für —$CH_2OH$ steht, dadurch gekennzeichnet, daß man

(A) eine Verbindung der Formel

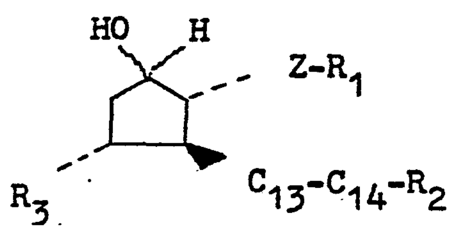

bei der $R_1$, $R_2$, $R_3$, Z und $C_{13}$—$C_{14}$ die oben angegebenen Bedeutungen haben, unter Bildung einer Verbindung der Formel

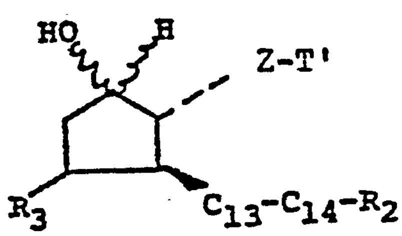

wobei T', $R_1$, $R_2$, $R_3$, Z und $C_{13}$—$C_{14}$ die in Anspruch 9 angegebenen Bedeutungen haben, schützt, anschließend

(B) mit einem Oxidationsmittel behandelt und die Schutzgruppen entfernt.

11. Verfahren zur Herstellung der Verbindung nach Anspruch 1, bei der $R_1$

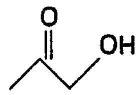

bedeutet, dadurch gekennzeichnet, daß man

(A) die Verbindung der Formel

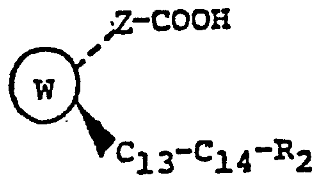

wobei W, $R_2$, Z und $C_{13}$—$C_{14}$ die in Anspruch 1 angegebenen Bedeutungen haben, in die Verbindung der Formel

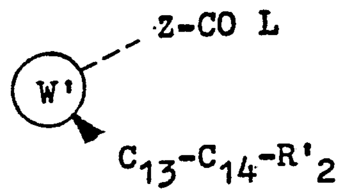

wobei W' ausgewählt ist unter

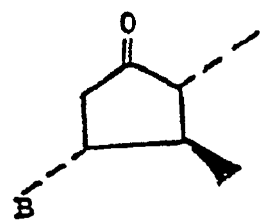

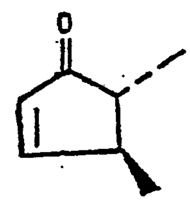

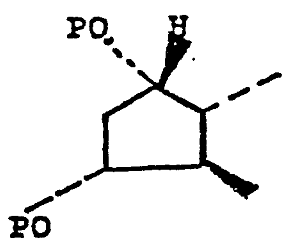

oder

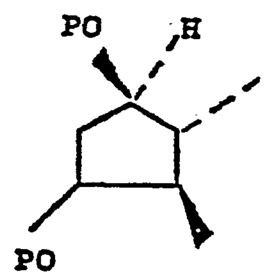

wobei P eine Hydroxyl-Schutzgruppe bedeutet und B für Wasserstoff oder eine geschützte Hydroxylgruppe steht; $R'_2$ die in Anspruch 9 angegebene Bedeutung hat; L für Chlor oder Brom steht und Z und $C_{13}$—$C_{14}$ die oben angegebenen Bedeutungen haben, umwandelt, anschließend

(B) mit Diazomethan behandelt und daraufhin hydrolysiert.

12. Verfahren zur Herstellung einer Verbindung nach Anspruch 1 der Formel

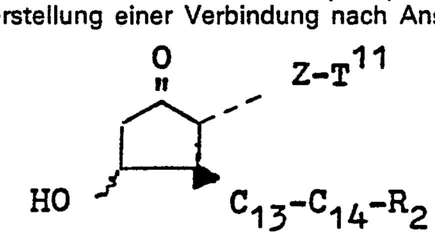

wobei Z, $C_{13}$—$C_{14}$, $R_1$ und $R_2$ die in Anspruch 1 angegebenen Bedeutungen haben und $T^{11}$ die in Anspruch 9 angegebene Bedeutung hat, dadurch gekennzeichnet, daß man

(A) die Verbindung

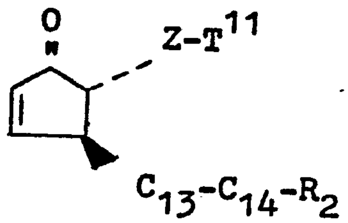

wobei $T^{11}$, $R_2$, Z und $C_{13}—C_{14}$ die zuvor angegebenen Bedeutungen haben, mit alkalischem Hydrogenperoxid behandelt, anschließend

(B) zur Bildung einer trennbaren Mischung der 11α- und 11β-Derivate der Produktverbindung eine Behandlung mit Chromacetat oder Aluminiumamalgam durchführt und gewünschtenfalls anschließend

(C) das Produkt der Stufe (B), bei dem $T^{11}$ für

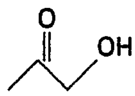

steht, mit einem Anhydrid der Formel $(R_{15}—CO)_2$ oder einem Säurechlorid der Formel $R_{15}—COCl$, wobei $R_{15}$ die in Anspruch 1 angegebene Bedeutung hat, behandelt unter Bildung des Produkts der Stufe (B), bei dem $T^{11}$ für

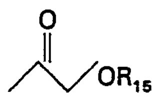

steht, wobei $R_{15}$ die zuvor angegebene Bedeutung hat.

**Revendications**

1. Composé de la formule

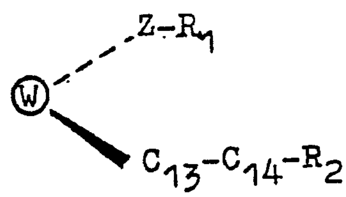

dans laquelle Z représente un groupe $—(CH_2)_f—$ ou $—CH_2—\underset{cis}{CH=CH}—(CH_2)_g—$, où f est un nombre entier dont la valeur varie de 5 à 7 inclusivement et g est un nombre entier dont la valeur varie de 2 à 4 inclusivement; $C_{13}—C_{14}$ représente un groupe éthylène ou *trans*-vinylène; W est choisi parmi les radicaux des formules suivantes:

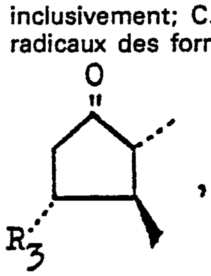 , 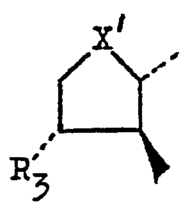 et 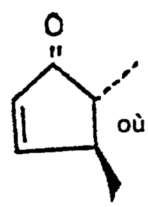 où X' représente un groupe 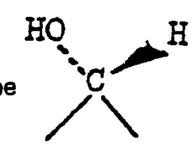 ou 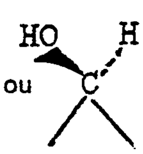

et $R_3$ représente un atome d'hydrogène ou le radical hydroxyle; $R_1$ est choisi parmi les radicaux suivants:

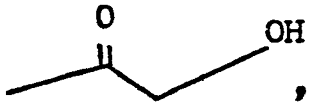 , 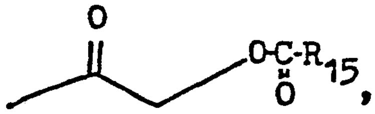 , 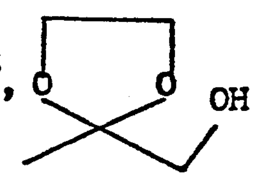 ou 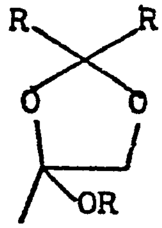 ,

où R représente un groupe alkyle en $C_1$ à $C_4$ et $R_{15}$ est choisi dans le groupe formé par les radicaux alkyle en $C_1$ à $C_4$, di-(alkyl en $C_1$ à $C_4$)-amino et phényle ou phényle substitué par un ou plusieurs substituants choisis dans le groupe formé par les radicaux alkyle en $C_1$ à $C_4$, —OR, —SR, F ou Cl, où R possède les significations précédemment définies, sauf que $R_1$ ne peut désigner un groupe

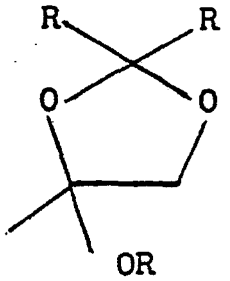

lorsque W représente un groupe

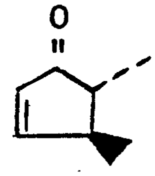

et $R_2$ est choisi dans le groupe formé par les radicaux suivants:

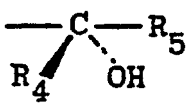

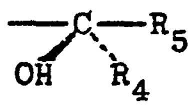

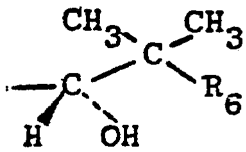

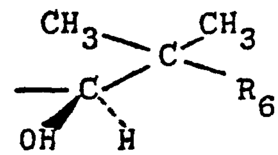

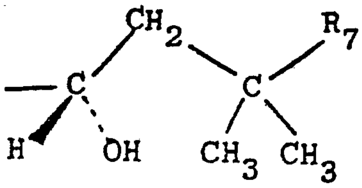

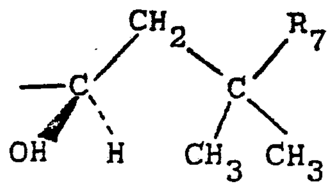

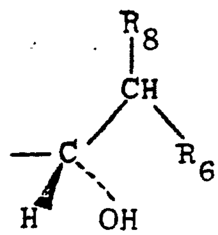

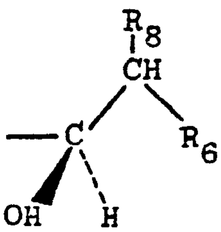

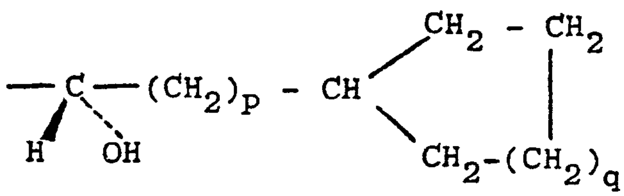

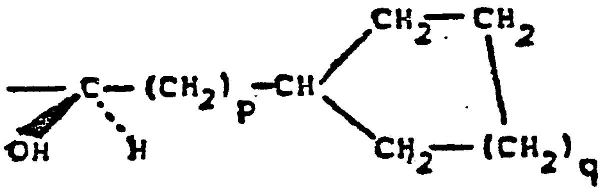

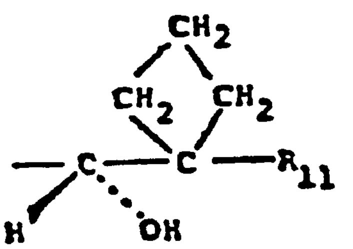

,

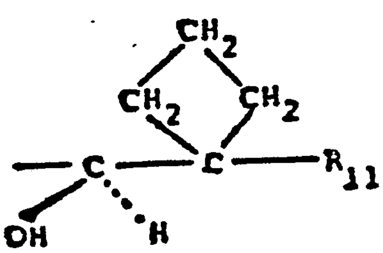

,

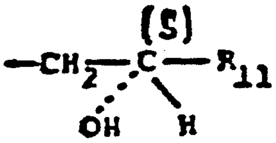 ,

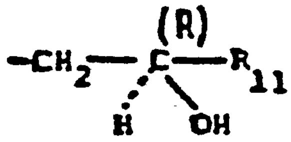 ,

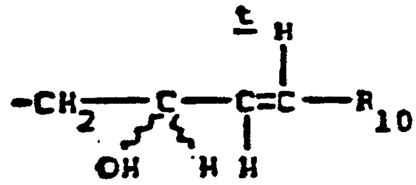

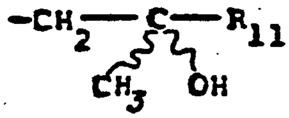 ,

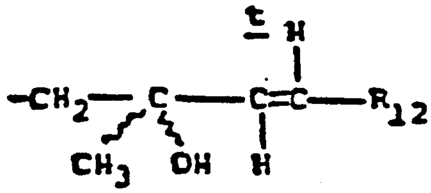 ,

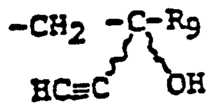 ,

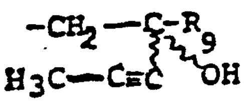

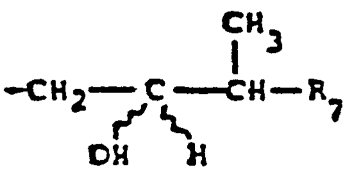 ,

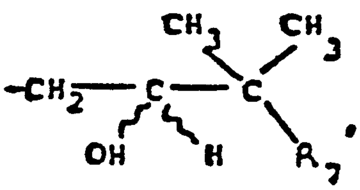 ,

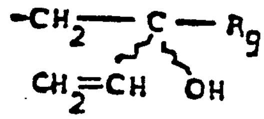 ,

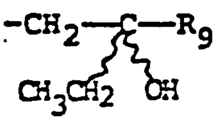 ,

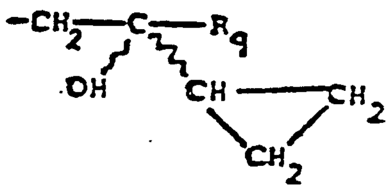 ,

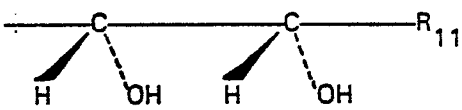 ,

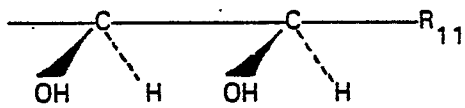 ,

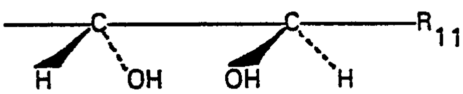 ,

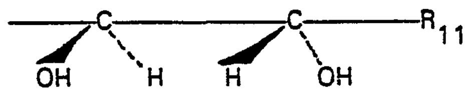 ,

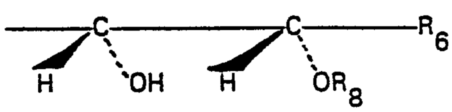 ,

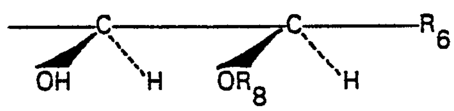

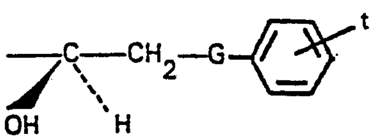

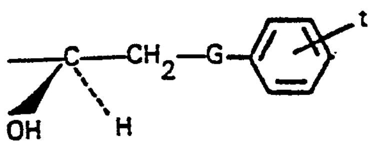

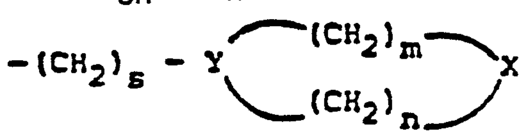

où $R_4$ est choisi parmi l'hydrogène et les radicaux alkyle en $C_1$ à $C_3$; $R_5$ est choisi dans le groupe formé par les radicaux alkyle à chaîne droite en $C_4$ à $C_7$; $R_6$ est choisi dans le groupe formé par les radicaux alkyle à chaîne droite en $C_3$ à $C_6$; $R_7$ est choisi dans le groupe formé par les radicaux alkyle à chaîne droite en $C_2$ à $C_4$; $R_8$ est choisi dans le groupe formé par les radicaux alkyle en $C_1$ ou $C_2$; $R_9$ est choisi dans le groupe formé par les radicaux alkyle à chaîne droite en $C_3$ à $C_6$; $R_{10}$ est choisi dans le groupe formé par les radicaux alkyle à chaîne droite en $C_1$ à $C_4$; $R_{11}$ est choisi dans le groupe formé par les radicaux alkyle à chaîne droite en $C_3$ à $C_7$; $R_{12}$ est choisi dans le groupe formé par les radicaux alkyle à chaîne droite en $C_1$ à $C_4$; p est un nombre entier dont la valeur varie de 0 à 3; q est égal à 1 ou à 2; X représente un radical divalent choisi dans le groupe formé par les radicaux

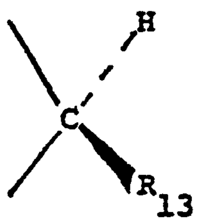

et

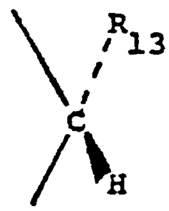

où $R_{13}$ est choisi par le groupe formé par l'hydrogène, les radicaux alkyle en $C_1$ à $C_7$ et phénoxy éventuellement substitué par un substituant choisi dans le groupe formé par les halogènes, les radicaux trifluorométhyle et alkyloxy en $C_1$ à $C_4$; Y représente un radical bivalent choisi dans le groupe formé par les radicaux suivants:

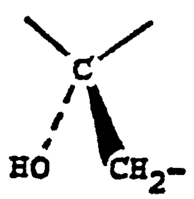

et

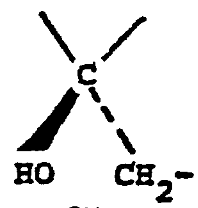

G est un radical bivalent choisi dans le groupe formé par —O— et —$CH_2$—; m est égal à 0 ou est un nombre entier dont la valeur varie de 1 à 4 inclusivement; n est égal à 0 ou est un nombre entier dont la valeur varie de 1 à 4 inclusivement, à condition que la somme de m et de n ait une valeur qui varie de 1 à 4; s est égal à 0 ou à 1 et t est choisi dans le groupe formé par l'hydrogène, le chlore, le fluor, les radicaux dichloro, trifluorométhyle et méthoxy.

2. Composé suivant la revendication 1: le 1,9-dioxo-11$\alpha$,16-dihydroxy-1-hydroxyméthyl-16-méthyl-5-*cis*,13-*trans*-prostadiène.

3. Composé suivant la revendication 1: le 1,9-dioxo-11$\alpha$,16-dihydroxy-16-méthyl-1-hydroxyméthyl-13-*trans*-prostène.

4. Composé suivant la revendication 1: le *nat.*-1,9-dioxo-11$\alpha$,16(S)-dihydroxy-1-hydroxyméthyl-16-méthyl-5-*cis*,13-*trans*-prostadiène.

5. Composé suivant la revendication 1: le *nat.*-1,9-dioxo-11$\alpha$,16(S)-dihydroxy-1-hydroxyméthyl-16-méthyl-13-*trans*-prostène.

6. Composé suivant la revendication 1: le 1,9-dioxo-11$\alpha$,16(S)-dihydroxy-1-hydroxyméthyl-16-méthyl-5-*cis*,13-*trans*-prostadiène.

7. Composé suivant la revendication 1: le *nat.*-1,9-dioxo-11$\alpha$,16(S)-dihydroxy-1-hydroxyméthyl-16-méthyl-13-*trans*-prostène.

8. Composition pharmaceutique contenant un composé suivant l'une quelconque des revendications 1 à 7 dans un véhicule ou diluant physiologiquement acceptable.

9. Procédé de préparation d'un composé suivant la revendication 1, caractérisé en ce que:

(A) on fait réagir une cyclopenténone optiquement active ou son racémique, répondant à la formule suivante:

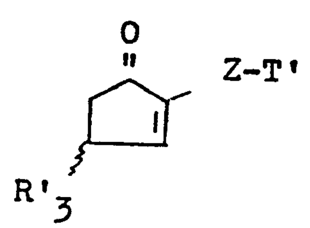

dans laquelle Z possède les significations précédemment définies, $R'_3$ représente un atome d'hydrogène ou un groupe hydroxyle protégé et T' est choisi dans le groupe formé par les radicaux suivants:

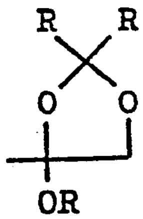
,
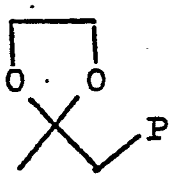
ou
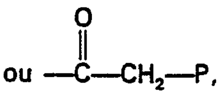

où P représente un groupe hydroxyle protégé et R représente un groupe alkyle en $C_1$ à $C_4$, avec un lithiocuprate choisi dans le groupe des composés des formules ci-après:

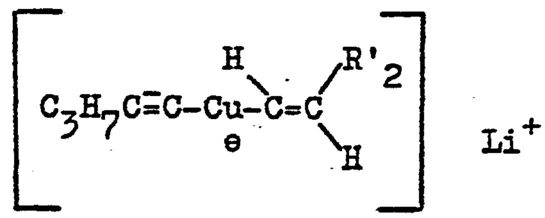
,

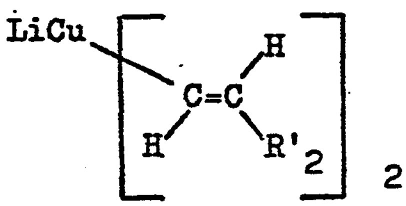
et
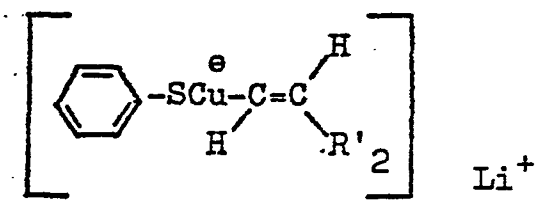

dans lesquelles $R'_2$ est un groupe $R_2$, dont tous les groupes hydroxyle sont bloqués par un groupe protecteur, de manière à obtenir un composé optiquement actif ou son racémique de la formule

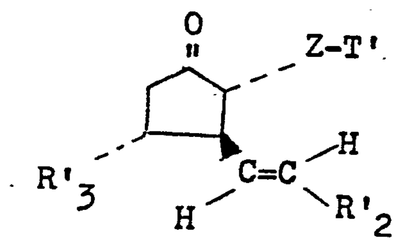

dans laquelle Z, T', $R'_3$ et $R'_2$ possèdent les significations précédemment définies, cette réaction étant suivie:

(B) de l'élimination des groupes protecteurs des groupes hydroxyle de façon à obtenir un composé de la formule

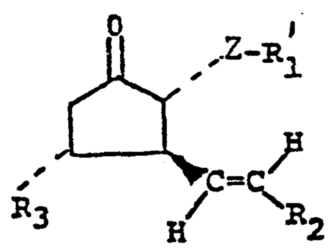

dans laquelle Z, $R_2$ et $R_3$ possèdent les significations précédemment définies et $R'_1$ représente un groupe

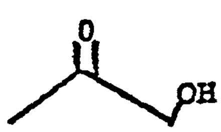
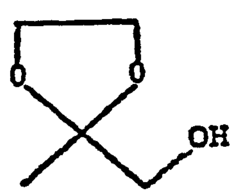
ou
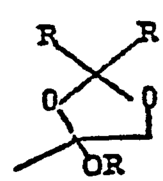

où R possède les significations précédemment définies et, si on le souhaite, suivie de

(C) l'hydrogénation complète des produits de l'étape (B), de façon à obtenir un composé de la formule

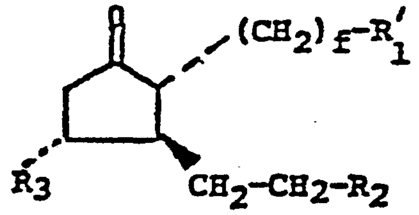

dans laquelle f, $R_3$, $R_2$ et $R'_1$ possèdent les significations précédemment définies, ou de

(D) l'hydrogénation partielle des composés de l'étape (B) de la formule

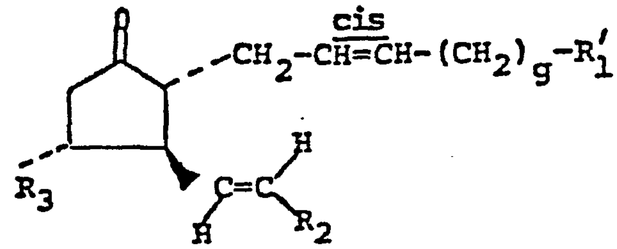

dans laquelle g, $R_3$, $R_2$ et $R'_1$ possèdent les significations définies, de façon à obtenir le composé de la formule

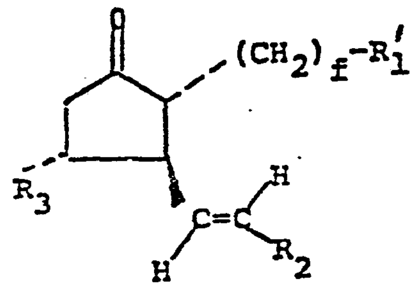

dans laquelle $R_3$, f, $R'_1$ et $R_2$ possèdent les significations précédemment définies et, si on le souhaite, suivie:

(E) d'un traitement des produits de l'étape (A) avec un agent réducteur de la fonction carbonyle stéréo-sélectif, suivi de l'enlèvement du groupe protégeant le groupe hydroxyle, de façon à obtenir le dérivé 9$\alpha$-hydroxy des composés de l'étape (A), ou bien, si on le souhaite:

(F) d'un traitement des produits de l'étape (A) avec un agent réducteur de la fonction carbonyle, suivi de l'enlèvement du groupe protégeant le groupe hydroxyle, de façon à obtenir un mélange séparable des dérivés 9$\alpha$- et 9$\beta$-hydroxy de l'étape (A) et, si on le souhaite, suivi de:

(G) l'hydrogénation partielle des produits des étapes (E) ou (F), où Z représente un groupe *cis*-vinylène et $C_{13}$—$C_{14}$ représente un groupe *trans*-vinylène, de manière à obtenir, à partir des composés 9$\alpha$-hydroxy du stade (E), le composé de la formule

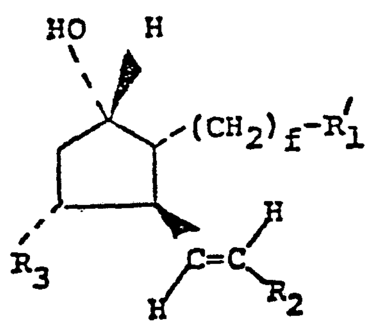

dans laquelle f, $R_2$, $R_3$ et $R'_1$ possèdent les significations définies, ou à obtenir, à partir du composé 9$\alpha$/$\beta$ du stade (F), le mélange séparable des composés de la formule

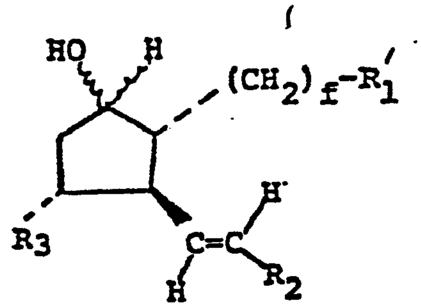

dans laquelle f', $R_1$, $R_2$ et $R_3$ sont tels que précédemment définis ou bien, si on le souhaite, on fait suivre ces opérations de

(H) l'hydrogénation complète des produits des étapes (E) ou (F) de façon à obtenir, à partir du produit 9$\alpha$-hydroxy des stades (E) ou (F), le composé de la formule

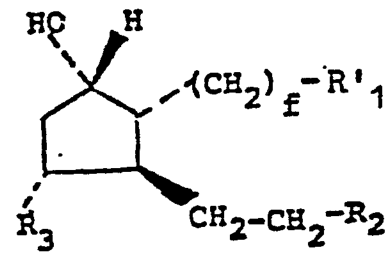

dans laquelle f, $R'_1$, $R_2$ et $R_3$ possèdent les significations définies, et, à partir des produits 9$\alpha$/$\beta$-hydroxy de l'étape (F), le mélange séparable de composés de la formule

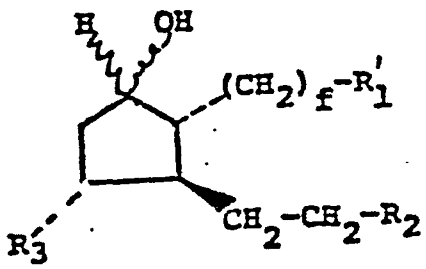

dans laquelle f, $R_3$, $R_2$ et $R'_1$ possèdent les significations précédemment définies et, si on le souhaite, suivie de:

(I) la déshydratation d'un composé de la formule

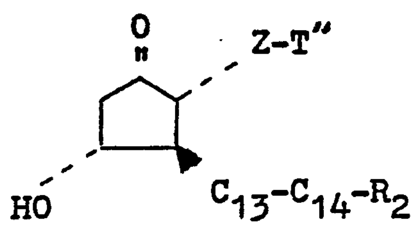

dans laquelle Z, $C_{13}$—$C_{14}$ et $R_2$ possèdent les significations précédemment définies et T'' est un groupe

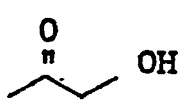 , 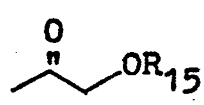 ou 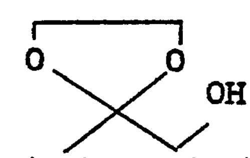

où $R_{15}$ possède les significations définies dans la revendication 1, pour obtenir un composé de la formule

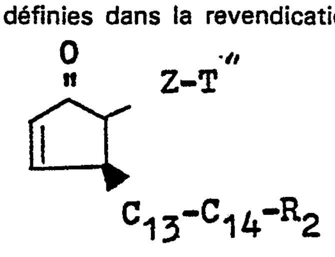

dans laquelle Z, $C_{13}$—$C_{14}$, $R_2$ et T'' possèdent les significations définies et, si on le souhaite,

(J) on peut traiter les produits des étapes (B), (C), (D), (E), (F), (G) ou (H), où $R_1$ représente un groupe

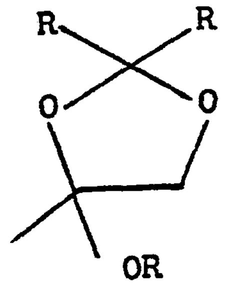

par un acide pour obtenir le produit correspondant de l'étape considérée, où $R_1$ représente un groupe

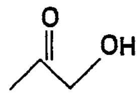

et, si on le souhaite,

(K) on peut traiter les produits des étapes (B) à (J), où $R_1$ représente un groupe

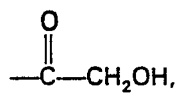

par un anhydride de la formule $(R_{15}CO)_2O$ ou un chlorure d'acide de la formule

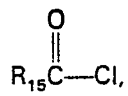

où $R_{15}$ possède les significations précédemment définies, de façon à obtenir le composé de la formule

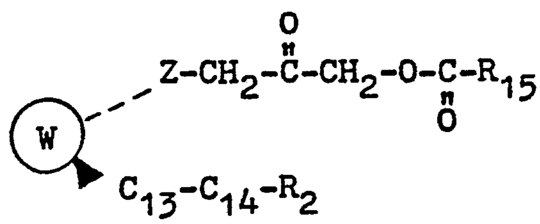

dans laquelle $R_{15}$, Z, W, $C_{13}$—$C_{14}$ et $R_2$ possèdent les significations précédemment définies et, si on le souhaite, on fait suivre les opérations de

(L) la résolution des racémiques des étapes (B) à (K).

10. Procédé de préparation d'un composé suivant la revendication 1 de la formule

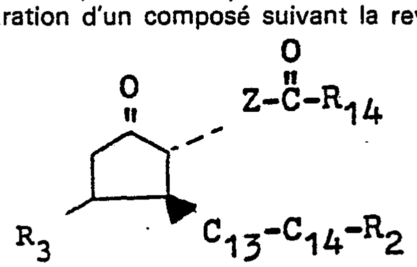

dans laquelle $R_2$, $R_3$, Z et $C_{13}$—$C_{14}$ possèdent les significations définies dans la revendication 1 et $R_{14}$ représente un groupe —$CH_2OH$, caractérisé en ce que:

(A) on protège le composé de la formule suivante:

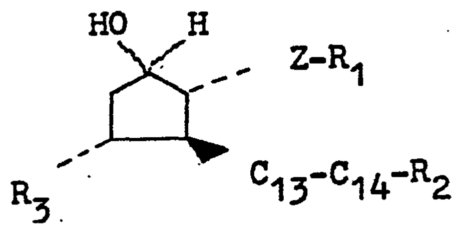

dans laquelle $R_1$, $R_2$, $R_3$, Z et $C_{13}$—$C_{14}$ possèdent les significations définies, de manière à obtenir un composé de la formule ci-dessus, dans lequel $R_1 = T'$ avec T' possédant l'une des significations définies dans la revendication 9, suivi de

(B) le traitement par un agent oxydant et l'élimination des groupes protecteurs.

11. Procédé de préparation d'un composé suivant la revendication 1, où $R_1$ représente un groupe

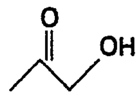

caractérisé en ce que

(A) on convertit le composé de la formule

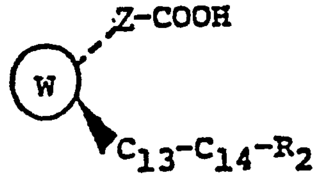

dans laquelle W, $R_2$, Z et $C_{13}$—$C_{14}$ possèdent les significations définies dans la revendication 1, en le composé de la formule

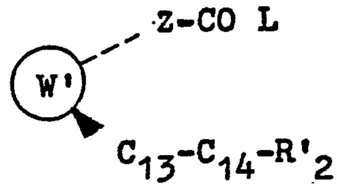

dans laquelle W' représente un groupe

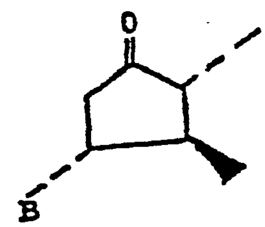

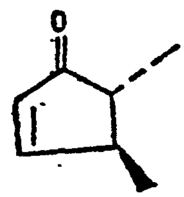

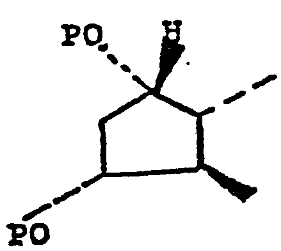

ou

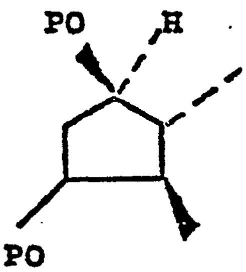

où P représente un groupe protecteur pour les groupes hydroxyle et B représente un atome d'hydrogène ou un groupe hydroxyle protégé; $R'_2$ possède les significations définies dans la revendication 9, L représente un atome de chlore ou de brome et Z et $C_{13}$—$C_{14}$ possèdent les significations définies précédemment, suivi de

(B) un traitement avec le diazométhane, puis d'une hydrolyse.

12. Procédé de préparation d'un composé suivant la revendication 1 de la formule

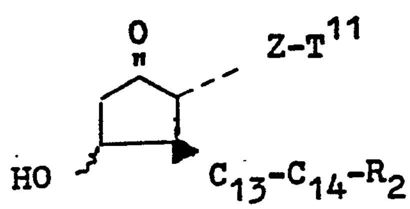

dans laquelle Z, $C_{13}$—$C_{14}$, $R_1$ et $R_2$ possèdent les significations définies dans la revendication 1 et T'' celles définies dans la revendication 9, caractérisé en ce que:

(A) on traite le composé de la formule

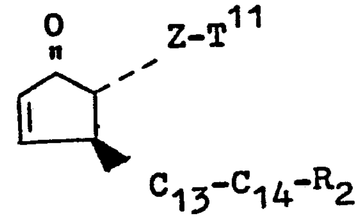

dans laquelle T'', Z, $R_2$ et $C_{13}$—$C_{14}$ possèdent les significations définies, par du peroxyde d'hydrogène en milieu alcalin, suivi de

(B) un traitement par de l'acétate chromeux ou un amalgame d'aluminium, de façon à obtenir un mélange séparable des dérivés 11$\alpha$ et 11$\beta$ du composé produit et, si on le souhaite, on fait suivre de.

(C) un traitement du produit de l'étape (B), où T'' représente un groupe

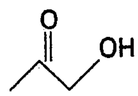

avec un anhydride de la formule $(R_{15}—CO)_2O$ ou un chlorure d'acide de la formule $R_{15}—COCl$, où $R_{15}$ possède les significations définies dans la revendication 1, de façon à obtenir le produit de l'étape (B), où T'' représente un groupe

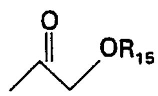

où $R_{15}$ possède l'une des significations définies.